# EUROPÄISCHE PATENTSCHRIFT

(11) **EP 4 541 156 B1**
(45) Veröffentlichungstag und Bekanntmachung des Hinweises auf die Patenterteilung: **18.02.2026**
(21) Anmeldenummer: 23733303.4
(22) Anmeldetag: 16.06.2023
(51) Int. Cl.: H10K 85/40, H10K 85/60, H10K 30/20, H10K 30/00

(54) **ORGANISCHE HETEROCYCLEN FÜR PHOTOELEKTRISCHE VORRICHTUNGEN**
ORGANIC HETEROCYCLES FOR PHOTOELECTRIC DEVICES
HÉTÉROCYCLES ORGANIQUES POUR DISPOSITIFS PHOTOÉLECTRIQUES

(30) Priorität: 20.06.2022 EP 22179953; 19.12.2022 EP 22214766
(43) Veröffentlichungstag der Anmeldung: 23.04.2025
(73) Patentinhaber: Merck Patent GmbH, 64293 Darmstadt (DE)
(72) Erfinder: STOESSEL, Philipp, 64293 Darmstadt (DE)
(74) Vertreter: Merck Patent Association
(86) Internationale Anmeldenummer: PCT/EP2023/066198
(87) Internationale Veröffentlichungsnummer: WO 2023/247338

(56) Entgegenhaltungen:
- US-A1- 2020 194 679

## Beschreibung

Die vorliegende Erfindung betrifft organische Heterocyclen für die Verwendung in elektronischen Vorrichtungen, insbesondere in organischen, photoelektrischen Vorrichtungen, sowie elektronische Vorrichtungen, insbesondere organische, photoelektrische Vorrichtungen, enthaltend diese heterocyclischen Verbindungen.

In organischen optischen Detektoren werden als Photosensitizer häufig heterocyclische Verbindungen eingesetzt. Aus US 2020/19467 A1, CN 110964007 A EP 3026722 A1, EP 3243822 A1, EP 3473622 A1, EP 3757108 A1, EP 3770163 A1, US 2019/131541 A1 und EP 3848374 A1 sind heterocyclische Verbindungen bekannt, die in optischen Detektoren eingesetzt werden können. Verbindungen gemäß der vorliegenden Erfindung sind nicht offenbart.

Generell besteht bei diesen heterocyclischen Verbindungen, beispielsweise für die Verwendung als Photosensitizer, noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einem organischen optischen Detektor, eignen und welche bei Verwendung in dieser Vorrichtung zu guten Deviceeigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen.

Weiterhin sollten die Verbindungen eine ausgezeichnete Verarbeitbarkeit aufweisen, wobei die Verbindungen insbesondere eine gute Löslichkeit zeigen sollten.

Eine weitere Aufgabe der vorliegenden Erfindung kann darin gesehen werden, Verbindungen bereitzustellen, welche sich für den Einsatz in einem organischen optischen Detektor eignen, insbesondere als Photosensitizer. Insbesondere ist es eine Aufgabe der vorliegenden Erfindung, Photosensitizer bereitzustellen, welche sich für infrarote, rote, grüne oder blaue optische Detektoren, vorzugweise für grüne optische Detektoren eignen.

Eine weitere Aufgabe kann darin gesehen werden, elektronische Vorrichtungen mit einer ausgezeichneten Leistungsfähigkeit möglichst kostengünstig und in konstanter Qualität bereitzustellen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich sehr gut für die Verwendung in elektronischen Vorrichtungen eignen und zu organischen optischen Detektoren führen, die insbesondere in Bezug auf die Lebensdauer, der Effizienz und der Betriebsspannung sehr gute Eigenschaften vorweisen. Diese Verbindungen sowie elektronische Vorrichtungen, insbesondere organische optische Detektoren, welche derartige Verbindungen enthalten, sind daher der Gegenstand der vorliegenden Erfindung.

Gegenstand der vorliegenden Erfindung ist eine Verbindung umfassend mindestens eine Struktur der Formel (I), vorzugsweise eine Verbindung gemäß der Formel (I), 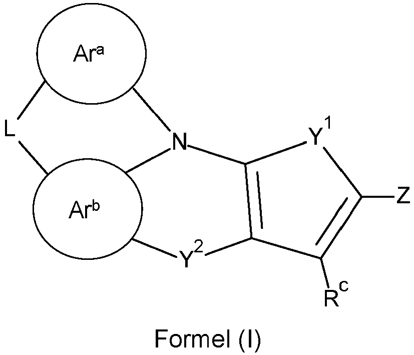 wobei für die verwendeten Symbole gilt:
- Ar^{a}: ist bei jedem Auftreten gleich oder verschieden ein aromatisches oder heteroaromatisches Ringsystem mit 5 bis 60 aromatischen Ringatomen, das mit einem oder mehreren Resten Ar oder R^{a} substituiert sein kann;
- Ar^{b}: ist bei jedem Auftreten gleich oder verschieden ein aromatisches oder heteroaromatisches Ringsystem mit 5 bis 60 aromatischen Ringatomen, das mit einem oder mehreren Resten Ar oder R^{b} substituiert sein kann;
- Z: ist bei jedem Auftreten gleich oder verschieden eine Alkenylgruppe mit 2 bis 40 C-Atomen, wobei die Alkenylgruppe mit einem oder mehreren Resten R^{d} substituiert sein kann, wobei eine oder mehrere nicht benachbarte CH₂-Gruppen durch R^{d}C=CR^{d}, C=C, Si(R^{d})₂, C=O, C=S, C=Se, C=NR, C(=O)O, C(=O)NR^{d}, NR, P(=O)(R^{d}), O, S, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann; dabei kann die Gruppe mit einer weiteren Gruppe, beispielsweise einer Gruppe R oder R^{c} ein Ringsystem bilden; mit der Maßgabe, dass die Gruppe Z mindestens zwei CN-Gruppen aufweist;
- Y¹: ist bei jedem Auftreten gleich oder verschieden O, S, Se, Te, NAr, NR, C(R)₂,Ge(R)₂, Si(R)₂, vorzugsweise O, S, Se, Te, NAr, besonders bevorzugt S oder Se;
- Y²: ist bei jedem Auftreten gleich oder verschieden eine Bindung, O, S, Se, Te, NAr, NR^{e}, BAr, BR^{e}, C(R^{e})₂, Ge(R^{e})₂, Si(R^{e})₂, CR^{e}=CR^{e}, C=NR^{e}, S=O, SO₂, vorzugsweise eine Bindung, C(R^{e})₂, O, S, NAr, besonders bevorzugt C(R^{e})₂;
- L: ist bei jedem Auftreten gleich oder verschieden eine Verbindungsgruppe, vorzugsweise eine Bindung, C(R)₂, O, S, NR, NAr, C(=O), BAr, B(R), Si(R)₂, C=NR, C=C(R)₂, S=O, SO₂, P(R), P(=O)R, CR=CR oder ein aromatisches oder heteroaromatisches Ringsystem mit 5 bis 40, bevorzugt 5 bis 30 aromatischen Ringatomen, welches durch einen oder mehrere Reste R substituiert sein kann, besonders bevorzugt eine Bindung, C(R)₂, O, S, NAr, NR, CR=CR oder eine ortho-verknüpfte Arylen- oder Heteroarylengruppe mit 5 bis 14 aromatischen Ringatomen, ganz besonders bevorzugt eine Bindung, C(R)₂, O, S, NAr oder NR;
- Ar: ist bei jedem Auftreten gleich oder verschieden ein aromatisches oder heteroaromatisches Ringsystem mit 5 bis 60 aromatischen Ringatomen, das mit einem oder mehreren Resten R substituiert sein kann; hierbei kann die Gruppe Ar mit mindestens einer Gruppe Ar, R, R^{a}, R^{b}, R^{c}, R^{d}, R^{e} oder einer weiteren Gruppe ein Ringsystem bilden;
- R, R^{a}, R^{b}, R^{c}, R^{d}, R^{e}: ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R', S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, C(=O)O, C(=O)NR¹, NR¹, P(=O)(R¹), Se, Te, NAr', BR¹, Ge(R¹)₂, O, S, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann, oder eine Arylthio- oder Heteroarylthiogruppe mit 5 bis 60 aromatischen Ringatomen, die durch einen oder mehrere Reste R¹ substituiert sein kann, oder eine Diarylamino-, Arylheteroarylamino-, Diheteroarylaminogruppe mit 5 bis 60 aromatischen Ringatomen, die durch einen oder mehrere Reste R¹ substituiert sein kann, oder eine Aralkyl- oder Heteroarylalkylgruppe mit 5 bis 60 aromatischen Ringatomen und 1 bis 10 C-Atomen im Alkylrest, die durch einen oder mehrere Reste R¹ substituiert sein kann; dabei können zwei Reste R, R^{a}, R^{b}, R^{c}, R^{d}, R^{e} auch miteinander oder einer weiteren Gruppe ein Ringsystem bilden;
- Ar': ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann; dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R', miteinander verbrückt sein;
- R¹: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², C(=O)O, C(=O)NR², NR², P(=O)(R²), O, S, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden; dabei können einer oder mehrere Reste R¹ mit einem weiteren Teil der Verbindung ein Ringsystem bilden;
- Ar": ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann; dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden.

Die Gruppe Z kann bei jedem Auftreten gleich oder verschieden eine Alkenylgruppe mit 2 bis 40 C-Atomen darstellen. Diese Gruppe kann geradkettig, cyclisch oder verzweigt sein, wobei die verzweigten Gruppen mindestens 3, die cyclischen Gruppen mindestens 4 Kohlenstoffatome aufweisen. Dabei können die cyclischen Gruppen auch ein oder mehrere Heteroatome aufweisen.

Erfindungsgemäß weist die Gruppe Z mindestens zwei CN-Gruppen auf. Hierbei sind Strukturen bzw. Verbindungen der Formel (I) bevorzugt, bei denen die Gruppe Z zwei CN-Gruppen aufweist, die an dasselbe Kohlenstoffatom gebunden sind.

Ferner sind Strukturen bzw. Verbindungen der Formel (I) besonders bevorzugt, bei denen die mindestens zwei CN-Gruppen der Gruppe Z mit dem 5-Ring, der die Gruppe Y¹ umfasst, durchgängig konjugiert sind.

Der Begriff "Konjugation" beziehungsweise "konjugiert" ist in der Fachwelt weithin bekannt, so dass hierauf verwiesen wird. Eine durchgängige Konjugation der mindestens zwei CN-Gruppen der Gruppe Z wird ausgebildet, sobald alternierende Doppel- und Einfachbindungen zwischen den mindestens zwei CN-Gruppen der Gruppe Z und dem 5-Ring, der die Gruppe Y¹ umfasst, vorliegen. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht.

Bevorzugte Ausführungsformen der Gruppen Ar^{a}, Ar^{b}, R^{c}, R^{d}, Y¹, Y² und L ergeben sich insbesondere aus Strukturen oder Verbindungen, die im Stand der Technik beschrieben sind.

Strukturen beziehungsweise Verbindungen, die den vorliegenden mit Bezug auf die Gruppen Ar^{a}, Ar^{b}, R^{c}, R^{d}, Y¹, Y² und L ähnlich sind, sind unter anderem aus der europäischen Patentanmeldung EP 3848374 A1, hinterlegt beim Europäischen Patentamt am 13.01.2021 mit der Anmeldenummer EP 21151295.9, wobei diese Druckschrift zu Offenbarungszwecken durch Referenz hierauf eingefügt wird.

Die Struktur der Formel (I) der in EP 3848374 A1 dargelegten Verbindungen unterscheidet sich unter anderem von den vorliegenden Verbindungen insbesondere durch die Gruppe Z, die in EP 3848374 A1 nicht offenbart ist, so dass sich bevorzugte Strukturen beziehungsweise Verbindungen, die den vorliegenden mit Bezug auf die Gruppen Ar^{a}, Ar^{b}, R^{c}, R^{d}, Y¹, Y² und L ähnlich sind, dadurch ergeben, dass das in Formel (I) der Druckschrift EP 3848374 A1 dargelegte Teilelement der Formel 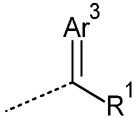 durch die Gruppe Z zu ersetzen ist.

Unter den zuvor dargelegten Voraussetzungen, nämlich den Ersatz der in den nachfolgend dargelegten und in EP 3848374 A1 näher dargelegten Teilstruktur, umfassend die Reste Ar³ und R¹ durch die Gruppe Z, werden insbesondere die in EP 3848374 A1 näher ausgeführten Strukturen gemäß Formel (1) (vgl. EP 3848374 A1, Seite 3, Absatz [0009], Formel (2A) (vgl. EP 3848374 A1, Seite 4, Absatz [0011], Formel (2A-1) (vgl. EP 3848374 A1, Seite 5, Absatz [0013], Formel (2A-2) (vgl. EP 3848374 A1, Seite 5, Absatz [0014], Formel (2B) (vgl. EP 3848374 A1, Seite 6, Absatz [0016], Formel (2B-1) (vgl. EP 3848374 A1, Seite 6, Absatz [0019], Formel (2B-2) (vgl. EP 3848374 A1, Seite 7, Absatz [0020], Formel (2C) (vgl. EP 3848374 A1, Seite 7, Absatz [0022], Formel (2C-1) (vgl. EP 3848374 A1, Seite 8, Absatz [0026], Formel (2C-2) (vgl. EP 3848374 A1, Seite 9, Absatz [0027], Formel (2D) (vgl. EP 3848374 A1, Seite 9, Absatz [0029], Formel (2D-1) (vgl. EP 3848374 A1, Seite 10, Absatz [0032], Formel (2D-2) (vgl. EP 3848374 A1, Seite 10, Absatz [0034], Formel (2E) (vgl. EP 3848374 A1, Seite 11, Absatz [0035], Formel (2E-1) (vgl. EP 3848374 A1, Seite 12, Absatz [0038], Formel (2E-2) (vgl. EP 3848374 A1, Seite 12, Absatz [0039], durch Referenz hierauf in die vorliegende Anmeldung zu Offenbarungszwecken eingefügt.

Besonders bevorzugte Strukturen werden später explizit näher ausgeführt. Diese sind gegenüber den zuvor dargelegten bevorzugt.

In einer bevorzugten Ausführungsform der Erfindung ist Z eine Alkenylgruppe mit 2 bis 10 C-Atomen, bevorzugt mit 2 bis 6 C-Atomen, besonders bevorzugt mit 2 bis 4 C-Atomen, welche mit einem oder mehreren Resten R^{d} substituiert sein kann, wobei mindestens zwei CN-Gruppen an die Alkenylgruppe gebunden sind; dabei kann die Gruppe mit einer Gruppe R oder R^{c} ein Ringsystem bilden. Besonders bevorzugt ist Z eine Alkenylgruppe mit 2 C-Atomen, welche mit einem Rest R^{d} und zwei CN-Gruppen substituiert ist, wobei die beiden CN-Gruppen bevorzugt an dasselbe Kohlenstoffatom gebunden sind.

Ferner kann vorgesehen sein, dass die Gruppe Z eine Teilstruktur der Formel (Z-1) oder (Z-2) darstellt oder umfasst, vorzugsweise darstellt, 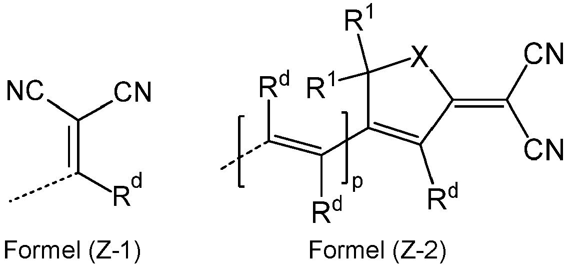 wobei R^{d} und R¹ die zuvor, insbesondere für Formel (I) genannte Bedeutung aufweisen, die gestrichelte Bindung die Anbindungsstelle darstellt und weiterhin gilt:
- X: ist O, S oder Se, bevorzugt O oder S und besonders bevorzugt O;
- p: ist 0, 1 oder 2, bevorzugt 0 oder 1 und besonders bevorzugt 1.

Vorzugsweise ist die Teilstruktur der Formel (Z-1) bzw. (Z-2) mit dem 5-Ring, der die Gruppe Y¹ umfasst, durchgängig konjugiert. Dabei steht R^{d} in Formel (Z-1) bevorzugt für H oder D.

Für Formel (Z-2) gilt bevorzugt:
R^{d}, welches an die nicht-cyclische Alkenylgruppe in Formel (Z-2) gebunden ist, ist bevorzugt gleich oder verschieden bei jedem Auftreten H, D oder eine Alkylgruppe mit 1 bis 5 C-Atomen, besonders bevorzugt H, D oder Methyl und ganz besonders bevorzugt H oder D.
R^{d}, welches an den Fünfring in Formel (Z-2) gebunden ist, ist bevorzugt H, D, CN, F, eine Alkylgruppe mit 1 bis 5 C-Atomen oder eine Phenylgruppe, welche auch durch einen oder mehrere bevorzugt nicht-aromatische Reste R¹ substituiert sein kann. Bevorzugt ist dieses R^{d} H, D, Methyl oder CN.

Die Gruppen R¹, welche an den Fünfring in Formel (Z-2) gebunden sind, sind gleich oder verschieden bei jedem Auftreten H, D, eine Alkylgruppe mit 1 bis 5 C-Atomen oder eine Phenylgruppe, welche auch durch einen oder mehrere bevorzugt nicht-aromatische Reste R¹ substituiert sein kann. Dabei können die beiden Gruppen R¹ auch miteinander ein Ringsystem bilden. Bevorzugt sind diese Gruppen R¹ gleich oder verschieden bei jedem Auftreten eine Alkylgruppe mit 1 bis 4 C-Atomen, insbesondere Methylgruppen.

Bevorzugte Ausführungsformen der Formel (Z-2) sind die Strukturen der folgenden Formeln (Z-2') und (Z-2"), wobei diese Gruppen auch teilweise oder vollständig deuteriert sein können, 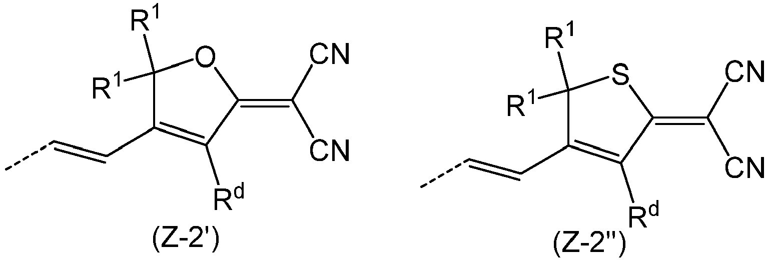 wobei die gestrichelte Bindung die Anbindungsstelle darstellt, R^{d} für H, D, Methyl oder CN steht und R¹ gleich oder verschieden bei jedem Auftreten für H, D oder Methyl, insbesondere für Methyl steht.

Bevorzugte Ausführungsformen der Formel (Z-2) sind die Strukturen der folgenden Formeln (Z-2a) bis (Z-2d), wobei diese Gruppen auch teilweise oder vollständig deuteriert sein können, 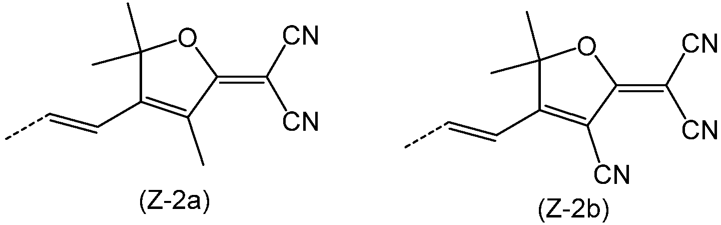 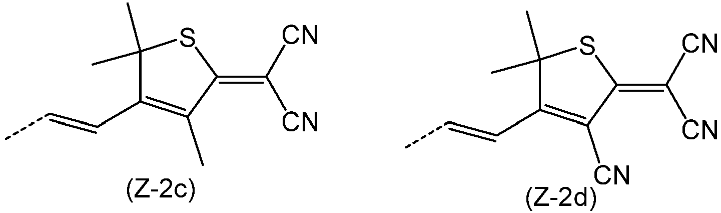 wobei die gestrichelte Bindung die Anbindungsstelle darstellt.

Die Strukturen bzw. Verbindungen der Formel (I) können ein, zwei, drei, vier oder mehr Teilstrukturen der Formel (Z-1) umfassen.

In einer bevorzugten Ausgestaltung kann vorgesehen sein, dass die erfindungsgemäßen Verbindungen eine Struktur der Formel (II) oder (IIa) umfassen, wobei besonders bevorzugt die erfindungsgemäßen Verbindungen ausgewählt sein können aus den Verbindungen der Formeln (II) oder (IIIa), 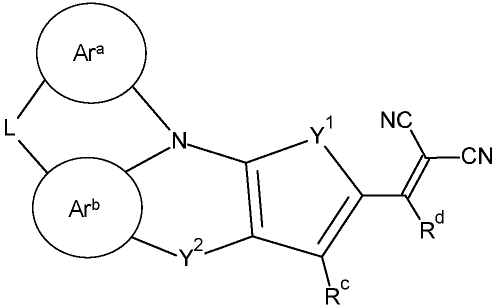 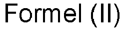 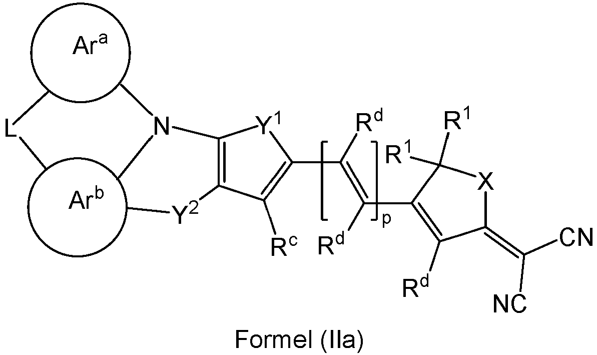 wobei die Symbole und Indizes Ar^{a}, Ar^{b}, R^{c}, R^{d}, Y¹, Y², L, R¹, X und p die zuvor, insbesondere für Formel (I) genannten Bedeutungen aufweisen und R^{d} in Formel (II) und in Formel (IIa) an der nicht-cyclischen Alkenylgruppe bevorzugt für H oder D steht und in Formel (IIa) R^{d} in dem Fünfring bevorzugt für Methyl oder CN steht. Weiterhin steht X in Formel (IIa) bevorzugt für O oder S, besonders bevorzugt für O, die explizit eingezeichneten R¹ in Formel (IIa) stehen bevorzugt für optional deuteriertes Methyl und der Index p in Formel (IIa) steht bevorzugt für 1.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem, vorzugsweise 6 bis 40 C-Atome im Ringsystem. Ein heteroaromatisches Ringsystem im Sinne dieser Erfindung enthält 2 bis 60 C-Atome, vorzugsweise 3 bis 40 C-Atome, und mindestens ein Heteroatom im Ringsystem, mit der Maßgabe, dass die Summe aus C-Atomen und Heteroatomen mindestens 5 ergibt. Die Heteroatome sind bevorzugt ausgewählt aus **N,** O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 bis 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinazolin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

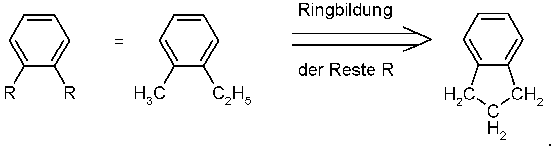

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

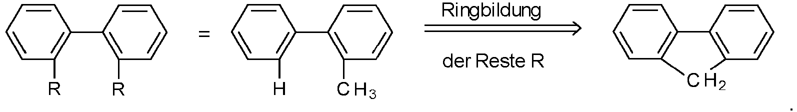

In einer bevorzugten Ausführungsform der Erfindung stehen Ar^{a} und Ar^{b} gleich oder verschieden bei jedem Auftreten für ein aromatisches oder heteroaromatisches Ringsystem mit 6 bis 14 aromatischen Ringatomen steht, das mit einem oder mehreren Resten Ar oder R^{a} substituiert sein kann, bevorzugt für eine Aryl- oder Heteroarylgruppe. In einer ganz besonders bevorzugten Ausführungsform der Erfindung stehen Ar^{a} und Ar^{b} gleich oder verschieden bei jedem Auftreten für Benzol, Naphthalin, Phenanthren, Dibenzofuran, Dibenzothiophen oder Carbazol, welches jeweils mit einem oder mehreren Resten Ar oder R^{a} substituiert sein kann. Dabei steht bevorzugt mindestens eine der Gruppen Ar^{a} und/oder Ar^{b} für Benzol.

In einer bevorzugten Ausgestaltung kann vorgesehen sein, dass die erfindungsgemäßen Verbindungen eine Struktur der Formel (III) oder (IIIa) umfassen, wobei besonders bevorzugt die erfindungsgemäßen Verbindungen ausgewählt sein können aus den Verbindungen der Formeln (III) oder (IIIa), 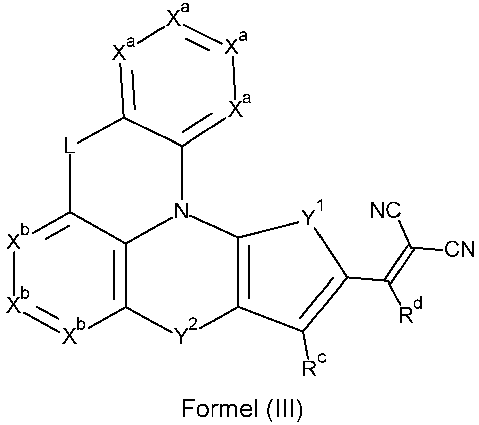 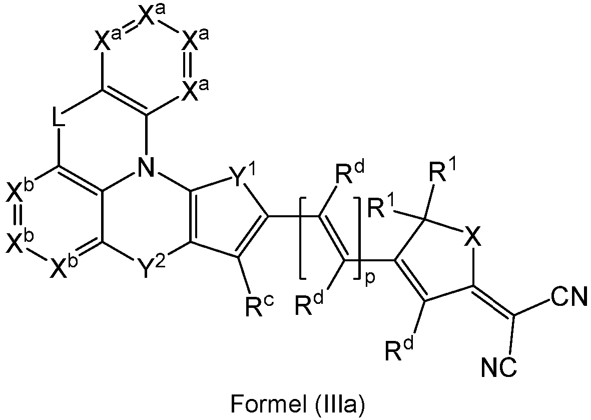 wobei die Symbole R^{c}, R^{d}, Y¹, Y², L, X, R¹ und p die zuvor, insbesondere für Formel (I) genannten Bedeutungen aufweisen und die weiteren Symbole die folgende Bedeutung aufweisen:
- X^{a}: steht bei jedem Auftreten gleich oder verschieden für N oder CR^{a}, vorzugsweise für CR^{a}, mit der Maßgabe, dass nicht mehr als zwei der Gruppen X^{a} in einem Cyclus für N stehen, wobei R^{a} die zuvor, insbesondere für Formel (I) dargelegte Bedeutung aufweist; oder zwei benachbarte X^{a} stehen zusammen für eine Gruppe der folgenden Formel, 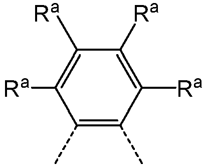 wobei die gestrichelten Bindungen die Verknüpfung der Gruppe innerhalb der Struktur darstellen;
- X^{b}: steht bei jedem Auftreten gleich oder verschieden für N oder CR^{b}, vorzugsweise für CR^{b}, mit der Maßgabe, dass nicht mehr als zwei der Gruppen X^{b} in einem Cyclus für N stehen, wobei R^{b} die zuvor, insbesondere für Formel (I) dargelegte Bedeutung aufweist; oder zwei benachbarte X^{b} stehen zusammen für eine Gruppe der folgenden Formel, 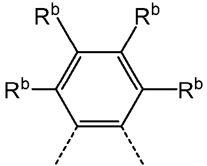 wobei die gestrichelten Bindungen die Verknüpfung der Gruppe innerhalb der Struktur darstellen.

Weiterhin steht R^{d} in Formel (III) und in Formel (IIIa) an der nicht-cyclischen Alkenylgruppe bevorzugt für H oder D, und R^{d} in Formel (IIIa) in dem Fünfring steht bevorzugt für Methyl oder CN. Weiterhin steht X in Formel (IIIa) bevorzugt für O oder S, besonders bevorzugt für O, die explizit eingezeichneten R¹ in Formel (IIIa) stehen bevorzugt für optional deuteriertes Methyl und der Index p in Formel (IIIa) steht bevorzugt für 1.

Vorzugsweise kann vorgesehen sein, dass in Formeln (III) (bzw. (IIIa) insgesamt nicht mehr als drei, vorzugsweise nicht mehr als zwei Gruppen X^{a} und X^{b} für N stehen. Besonders bevorzugt stehen alle Gruppen X^{a} und X^{b} für CR^{a} bzw. CR^{b} oder für die oben ausgeführten ankondensierten Benzogruppen.

In einer besonders bevorzugten Ausgestaltung kann vorgesehen sein, dass die erfindungsgemäßen Verbindungen eine Struktur der Formel (IV) bzw. (IVa) bis (IVf) umfassen, wobei besonders bevorzugt die erfindungsgemäßen Verbindungen ausgewählt sein können aus den Verbindungen der Formel (IV) bzw. (IVa) bis (IVf), 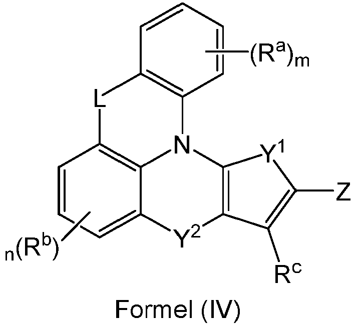 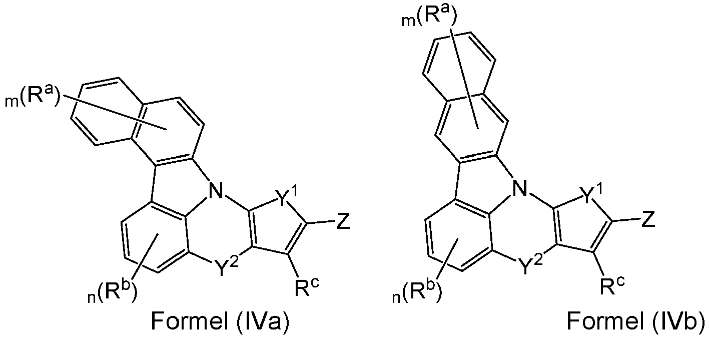 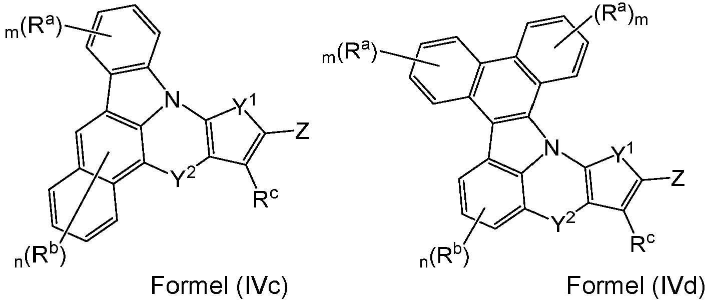 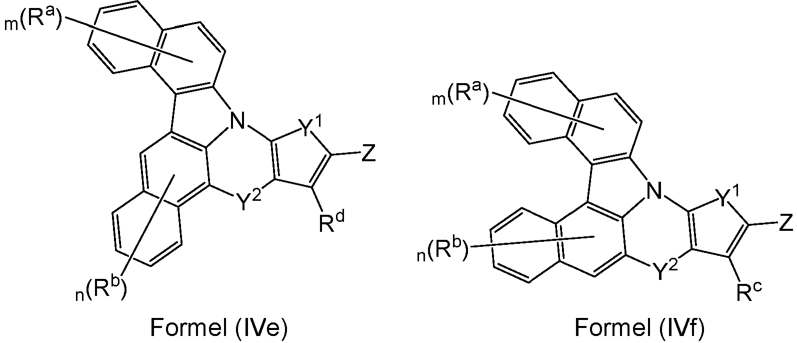 wobei die Verbindungen auch teilweise oder vollständig deuteriert sein können, Z für eine Gruppe der Formel (Z-1) oder (Z-2) steht, die Symbole R^{a}, R^{b}, R^{c}, R^{d}, Y¹, Y² und L die zuvor, insbesondere für Formel (I) genannten Bedeutungen aufweisen und die weiteren Symbole die folgende Bedeutung aufweisen:
- m: ist 0, 1, 2, 3 oder 4, vorzugsweise 0, 1 oder 2, besonders bevorzugt 0 oder 1 ;
- n: ist 0, 1, 2 oder 3, vorzugsweise 0, 1 oder 2, besonders bevorzugt 0 oder 1.

Geeignet sind daher die folgenden Ausführungsformen:

| Formel | Gruppe Z |
|---|---|
| (IV) | (Z-1) |
| (IV) | (Z-2) |
| (IVa) | (Z-1) |
| (IVa) | (Z-2) |
| (IVb) | (Z-1) |
| (IVb) | (Z-2) |
| (IVc) | (Z-1) |
| (IVc) | (Z-2) |
| (IVd) | (Z-1) |
| (IVd) | (Z-2) |
| (IVe) | (Z-1) |
| (IVe) | (Z-2) |
| (IVf) | (Z-1) |
| (IVf) | (Z-2) |

Bevorzugt sind die Gruppen der Z ausgewählt aus den Gruppen der Formeln (Z-1), (Z-2') und (Z-2") und insbesondere aus den Gruppen der Formeln (Z-1) und (Z-2a) bis (Z-2d).

Wenn Z für eine Gruppe der Formel (Z-2) steht, sind somit die folgenden Ausführungsformen bevorzugt:

| Formel | Gruppe Z |
|---|---|
| (IV) | (Z-2') |
| (IV) | (Z-2") |
| (IVa) | (Z-2') |
| (IVa) | (Z-2") |
| (IVb) | (Z-2') |
| (IVb) | (Z-2") |
| (IVc) | (Z-2') |
| (IVc) | (Z-2") |
| (IVd) | (Z-2') |
| (IVd) | (Z-2") |
| (IVe) | (Z-2') |
| (IVe) | (Z-2") |
| (IVf) | (Z-2') |
| (IVf) | (Z-2") |

Ferner kann bevorzugt, unter anderem in den Formeln (I), (II), (IIa), (III), (IIIa), (IV) und (IVa) bis (IVf) vorgesehen sein, dass die Gruppe L für eine Bindung, C(R)₂, O, S, NAr oder NR steht, und gleichzeitig die Gruppe Y² für C(R^{e})₂ steht und gleichzeitig die Gruppe Y¹ für O, S, Se, Te, NR, NAr, vorzugsweise S oder Se steht. In einer besonders bevorzugten Ausführungsform der Erfindung steht L für eine Bindung oder C(R)₂, insbesondere für eine Bindung, und Y² steht für C(R^{e})₂ und Y¹ steht für S oder Se. In den Verbindungen der Formeln (IVa) bis (IVf) steht bevorzugt die Gruppe Y² für C(R^{e})₂ und die Gruppe Y¹ steht für O, S, Se, Te, NR, NAr, vorzugsweise S oder Se.

Weiterhin kann bevorzugt, unter anderem in Formel (I), (II), (IIa), (III), (IIIa) und/oder (IV) bzw. (IVa) bis (IVf) vorgesehen sein, dass die Gruppe Y² für NR^{e}, BR^{e}, C(R^{e})₂, Ge(R^{e})₂, Si(R^{e})₂, -CR^{e}=CR^{e}-, C=NR^{e}, vorzugsweise für C(R^{e})₂ steht und der Rest R^{e} der Gruppe Y² für eine geradkettige Alkylgruppe mit 1 bis 10 C Atomen oder für eine verzweigte oder cyclische Alkylgruppe mit 3 bis 12 C-Atomen, wobei die Alkylgruppe jeweils teilweise oder vollständig deuteriert sein kann und mit einem oder mehreren Resten R¹ substituiert sein kann, oder für eine Aryl- oder Heteroarylgruppe mit 5 bis 12 aromatischen Ringatomen, vorzugsweise eine Phenylgruppe, steht, die teilweise oder vollständig deuteriert sein kann und durch einen oder mehrere Reste R¹ substituiert sein kann, wobei die zwei Reste R^{e} der Gruppe Y² zusammen einen Ring mit 5 bis 10 Ringatomen bilden können, der durch einen oder mehrere Reste R¹ substituiert sein kann. In einer speziell bevorzugten Ausführungsform steht die Gruppe Y² für C(R^{e})₂ und die zwei Reste R^{e} der Gruppe Y² bilden zusammen einen Ring mit 5 bis 10 Ringatomen, der durch einen oder mehrere Reste R¹ substituiert sein kann. Hierdurch entsteht vorzugsweise ein Spiro-System, wobei der durch die zwei Reste R^{e} gebildete Ring vorzugsweise einen 5-Ring, einen 6-Ring, einen 7-Ring oder einen 8-Ring darstellt, der verbrückt sein kann. Besonders bevorzugt kann vorgesehen sein, dass der durch die zwei Reste R^{e} gebildete Ring ein monocyclischer Cyclopentyl-, Cyclohexyl- oder Cycloheptylring, vorzugsweise ein monocyclischer Cyclopentyl- oder Cyclohexyl ist oder einen tricyclischen Adamantylring darstellt. Wenn Y² für C(R^{e})₂ steht, steht R^{e} bevorzugt für F, Methyl, Ethyl, neo-Pentyl oder Phenyl, wobei diese Gruppen jeweils auch teilweise oder vollständig deuteriert sein können und wobei die beiden Gruppen R^{e} auch miteinander einen Ring bilden können, oder die beiden Gruppen R^{e} bilden zusammen mit dem C-Atom, an das sie binden, eine Cyclopentyl-, Cyclohexyl- oder Adamantanylgruppe, die auch teilweise oder vollständig deuteriert sein kann. Besonders bevorzugt steht R^{e} für Methyl, welches auch teilweise oder vollständig deuteriert sein kann.

Darüber hinaus kann vorgesehen sein, dass der Rest R^{c} für H, D, für eine geradkettige Alkylgruppe mit 1 bis 10 C Atomen, für eine verzweigte oder cyclische Alkylgruppe mit 3 bis 12 C-Atomen, wobei die Alkylgruppe jeweils mit einem oder mehreren Resten R¹ substituiert sein kann, oder für eine Aryl- oder Heteroarylgruppe mit 5 bis 12 aromatischen Ringatomen, vorzugsweise eine Phenylgruppe, steht, die durch einen oder mehrere Reste R¹ substituiert sein kann, wobei der Rest R^{c} mit dem Rest R^{d} zusammen einen Ring mit 5 bis 10 Ringatomen bilden kann, der durch einen oder mehrere Reste R¹ substituiert sein kann. In einer besonders bevorzugten Ausführungsform der Erfindung steht der Rest R^{c} für H, D, Methyl oder Phenyl, ganz besonders bevorzugt H oder D.

Darüber hinaus kann vorgesehen sein, dass der Rest R^{d} für H, D, für eine geradkettige Alkylgruppe mit 1 bis 10 C Atomen, für eine verzweigte oder cyclische Alkylgruppe mit 3 bis 12 C-Atomen, wobei die Alkylgruppe jeweils mit einem oder mehreren Resten R¹ substituiert sein kann, oder für eine Aryl- oder Heteroarylgruppe mit 5 bis 12 aromatischen Ringatomen, vorzugsweise eine Phenylgruppe, steht, die durch einen oder mehrere Reste R¹ substituiert sein kann, wobei der Rest R^{d} mit dem Rest R^{c} zusammen einen Ring mit 5 bis 10 Ringatomen bilden kann, der durch einen oder mehrere Reste R¹ substituiert sein kann. In einer besonders bevorzugten Ausführungsform der Erfindung steht der Rest R^{d} für H oder D.

Weiterhin kann vorgesehen sein, dass die Reste R^{c} und R^{d} gleich sind. Ferner kann vorgesehen sein, dass die Reste R^{c} und R^{d} verschieden sind.

In einer besonders bevorzugten Ausgestaltung kann vorgesehen sein, dass R^{c} und R^{d} gleich sind und jeweils für H oder D stehen. In einer weiteren besonders bevorzugten Ausgestaltung kann vorgesehen sein, dass der Rest R^{c} mit dem Rest R^{d} zusammen einen Ring mit 5 bis 10 Ringatomen bilden. Darüber hinaus kann vorgesehen sein, dass R^{c} für H oder D steht und R^{d} für eine geradkettige Alkylgruppe mit 1 bis 10 C Atomen, für eine verzweigte oder cyclische Alkylgruppe mit 3 bis 12 C-Atomen, wobei die Alkylgruppe jeweils mit einem oder mehreren Resten R¹ substituiert sein kann, oder für eine Aryl- oder Heteroarylgruppe mit 5 bis 12 aromatischen Ringatomen, vorzugsweise eine Phenylgruppe, steht, die durch einen oder mehrere Reste R¹ substituiert sein kann.

In einer bevorzugten Ausgestaltung können die erfindungsgemäßen Verbindungen eine Struktur der Formeln (V-1) bis (V-80) umfassen, besonders bevorzugt können die erfindungsgemäßen Verbindungen ausgewählt sein aus den Verbindungen der Formeln (V-1) bis (V-80), 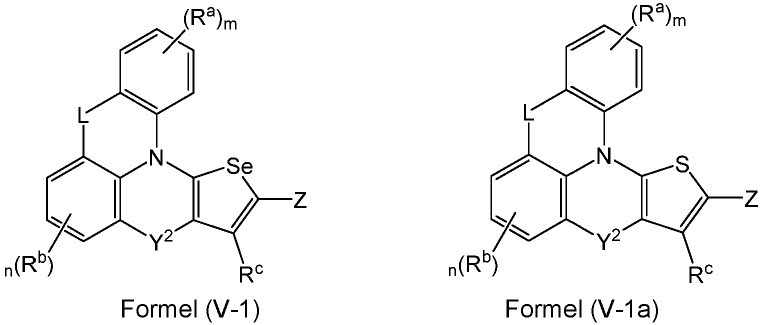 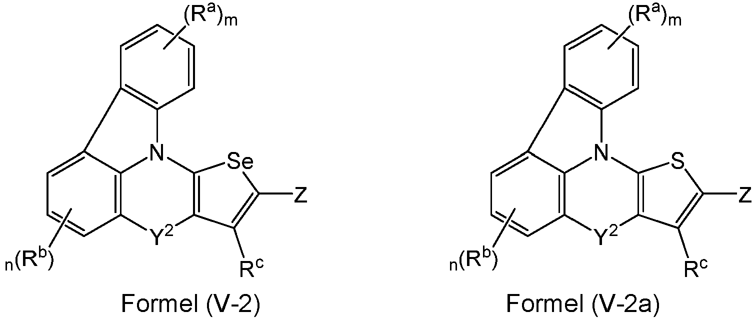 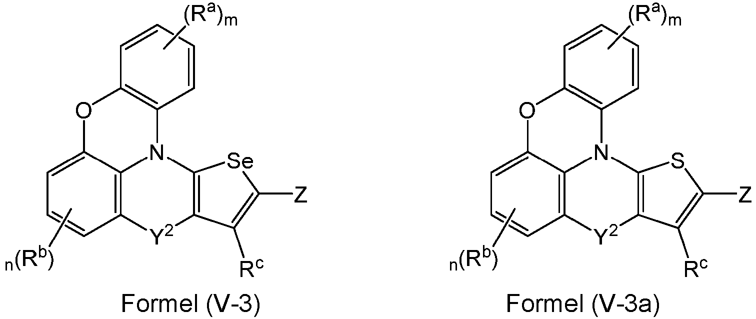 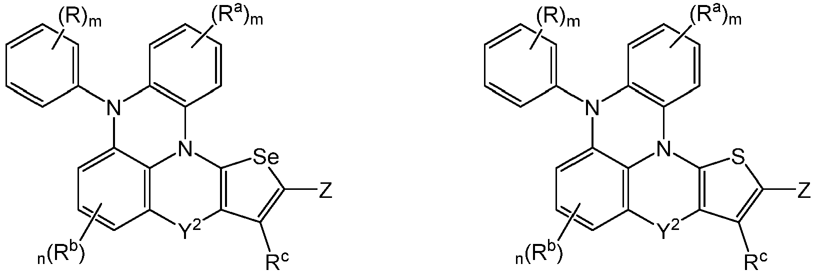 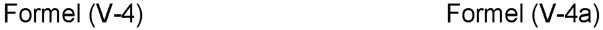 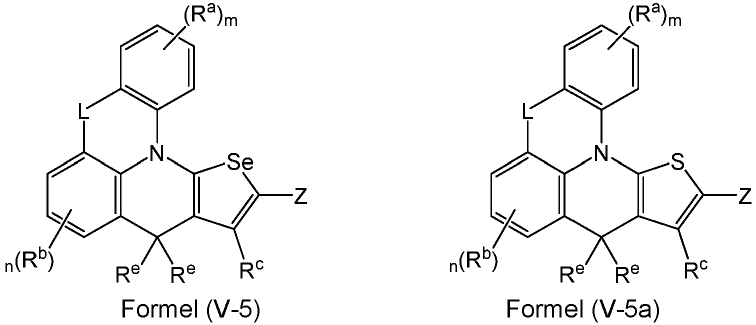 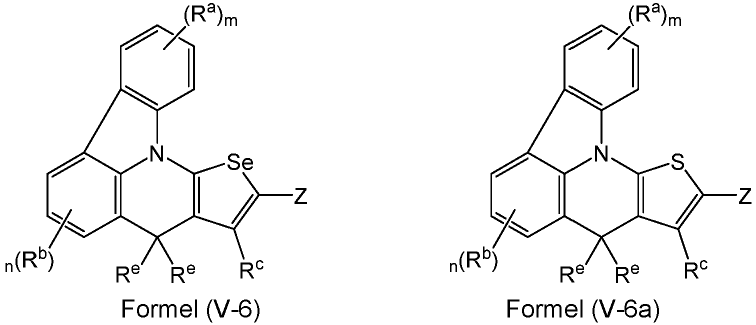 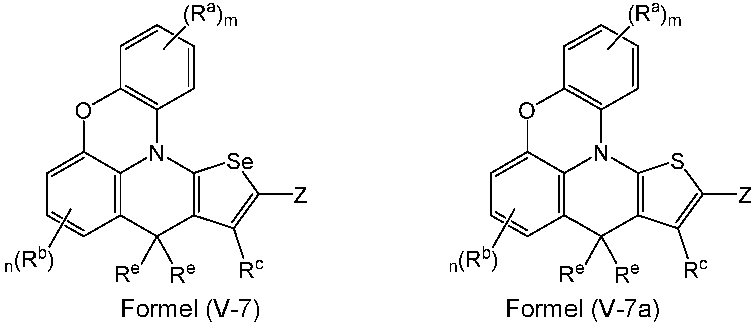 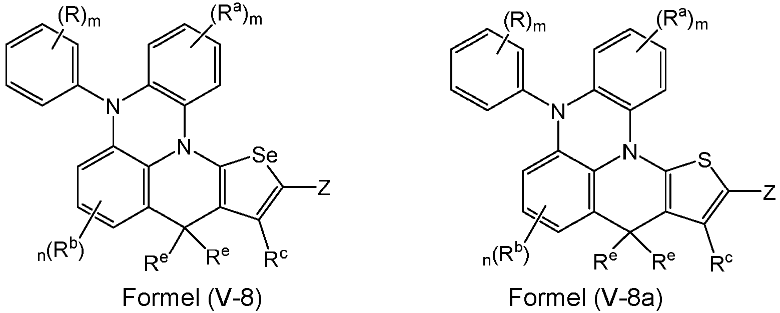 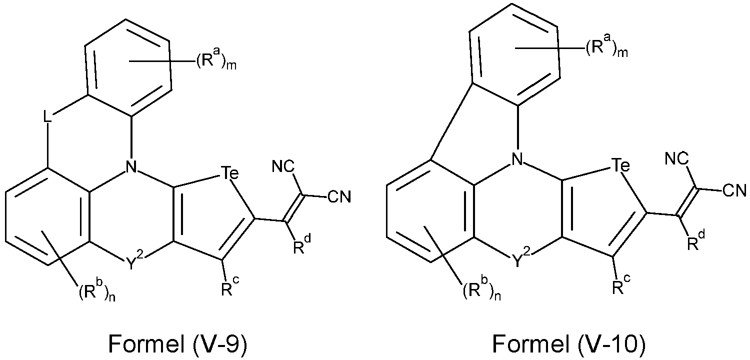 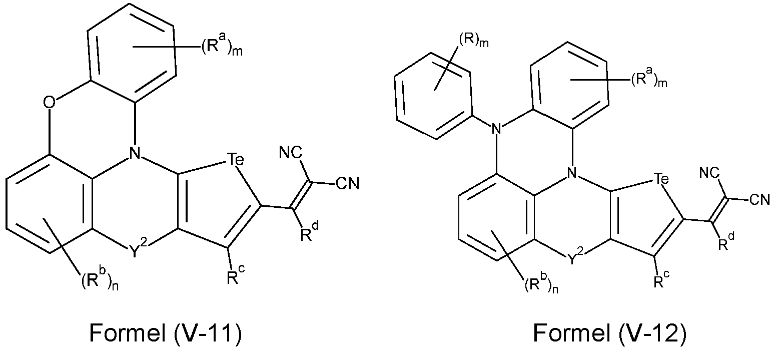 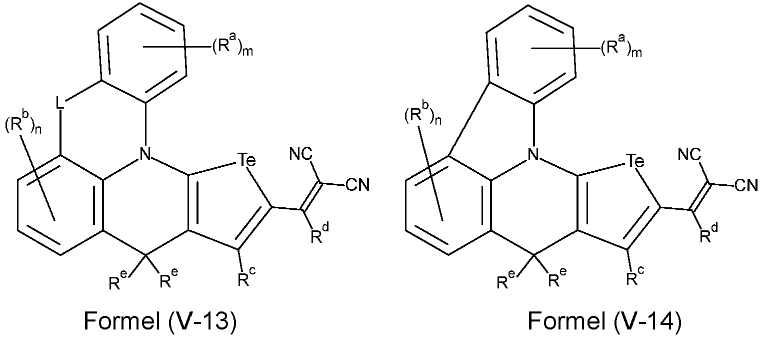 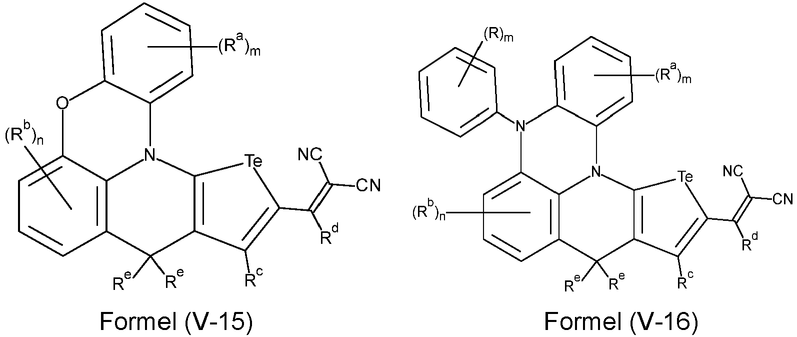 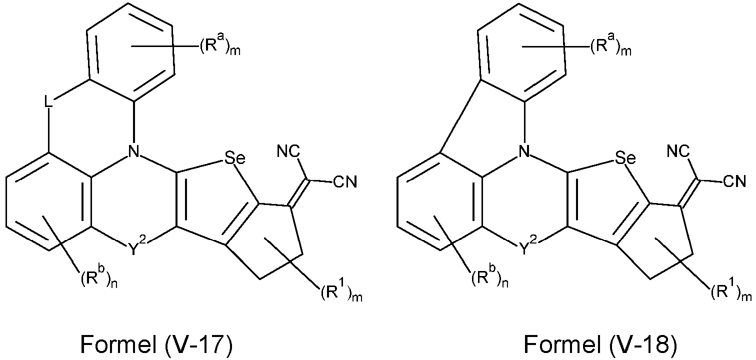 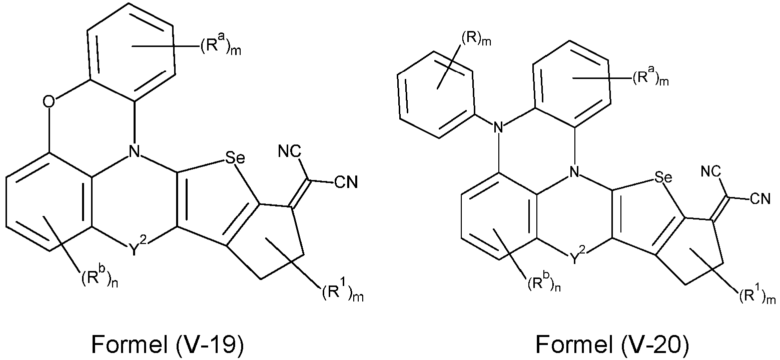 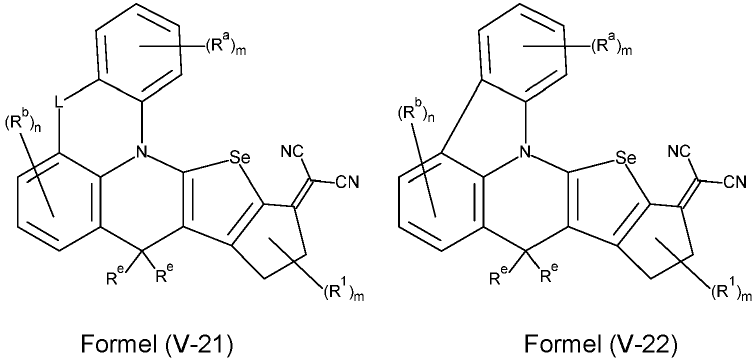 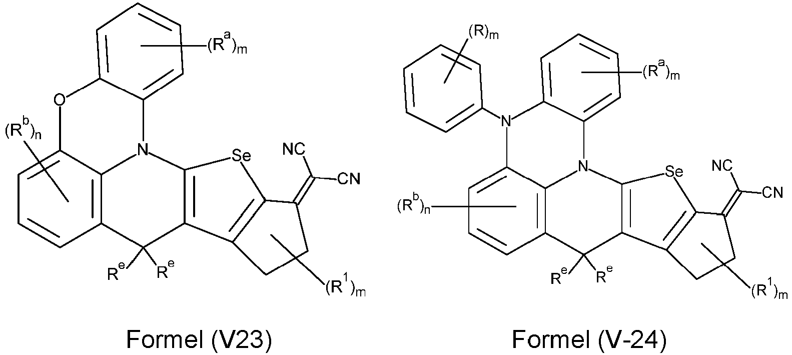 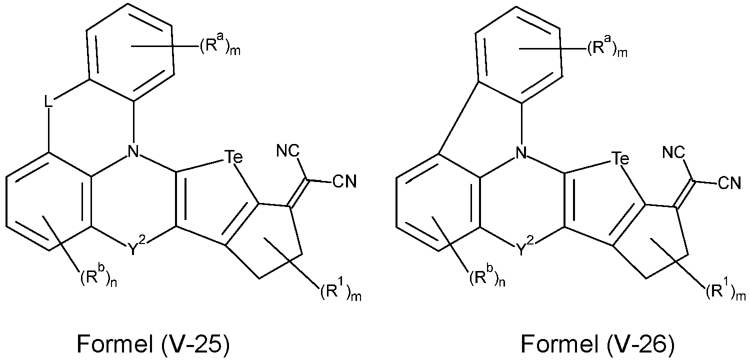 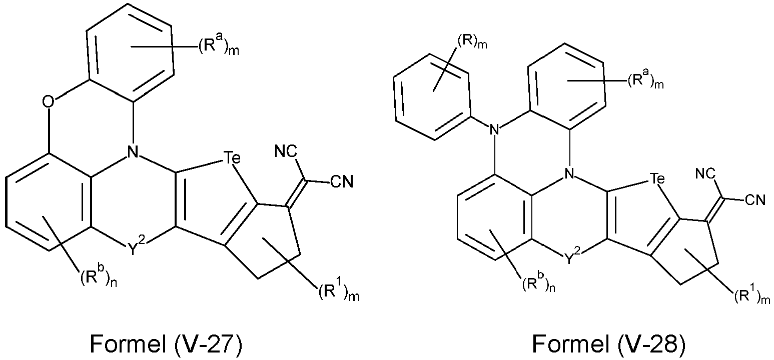 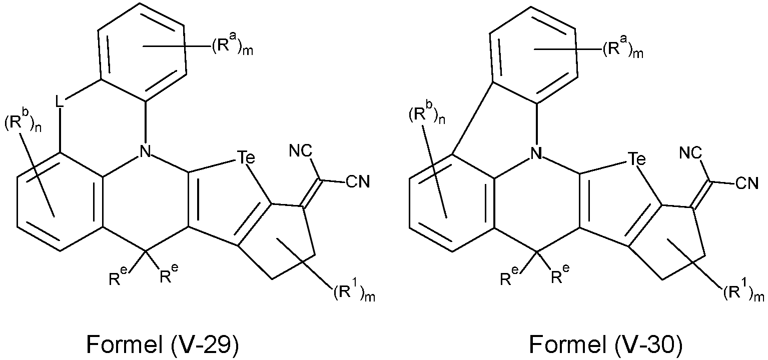 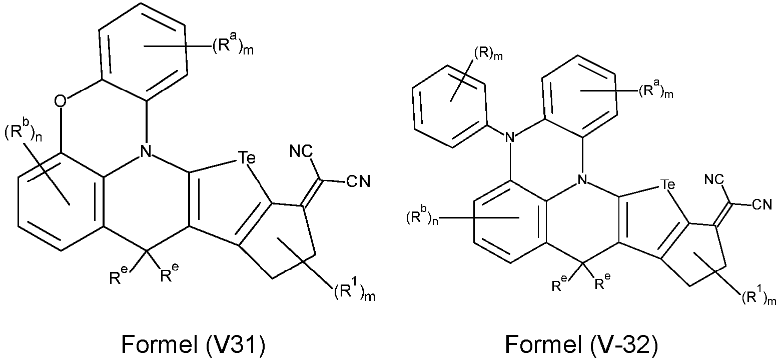 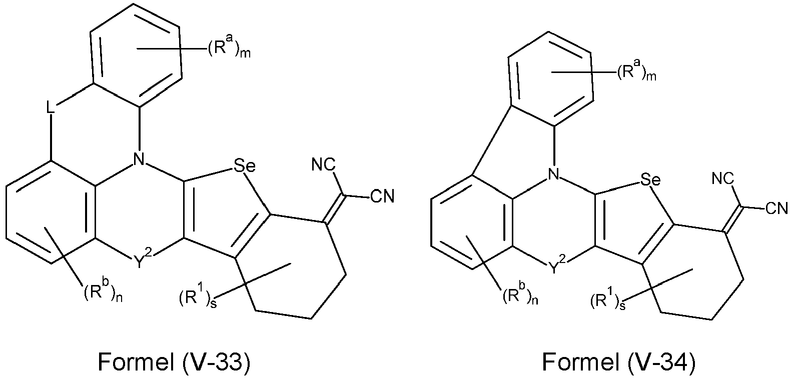 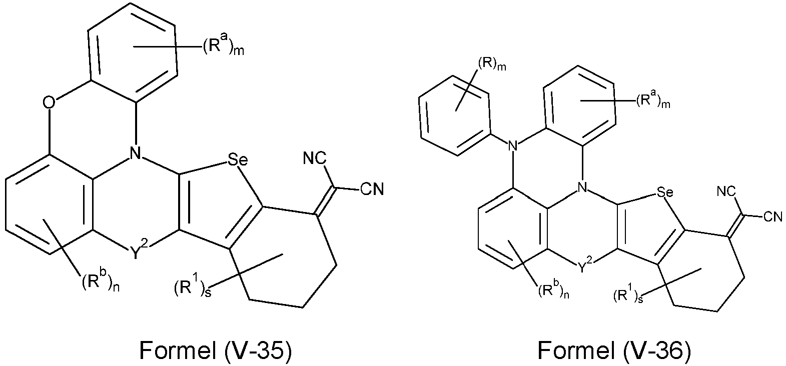 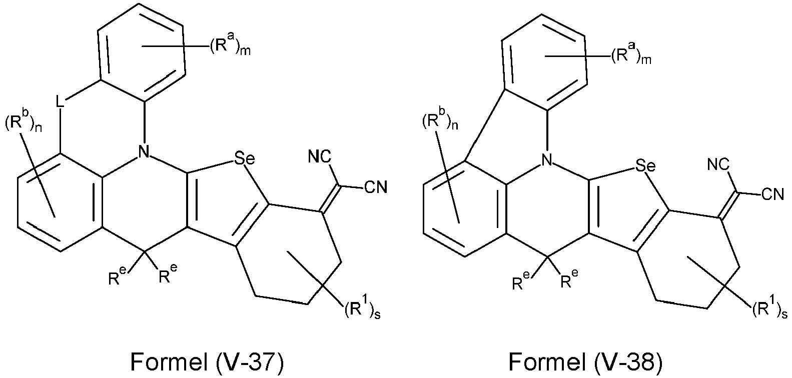 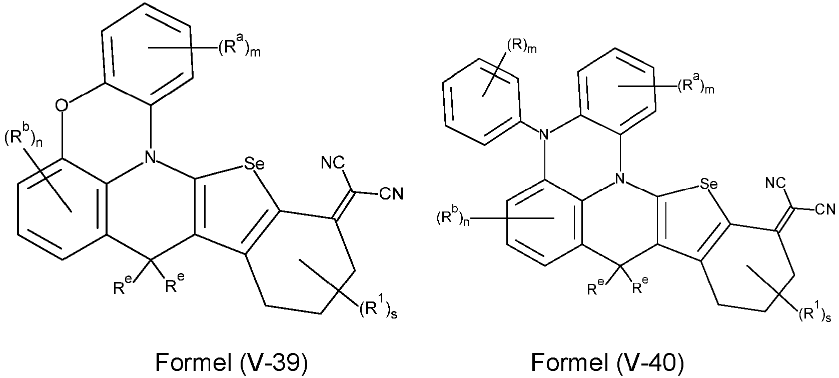 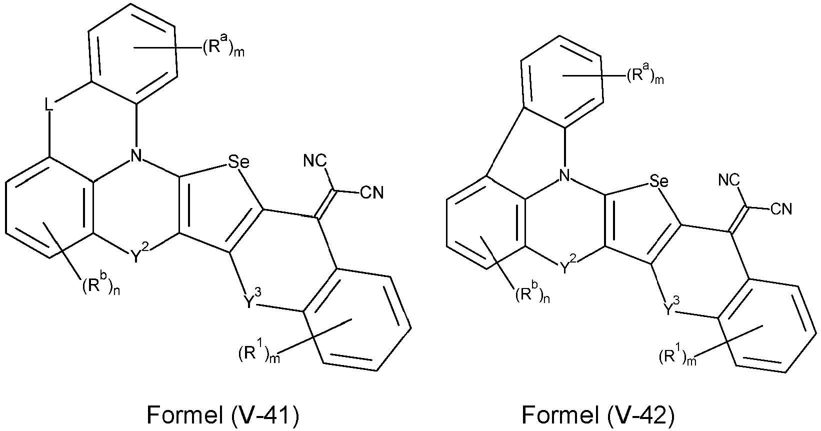 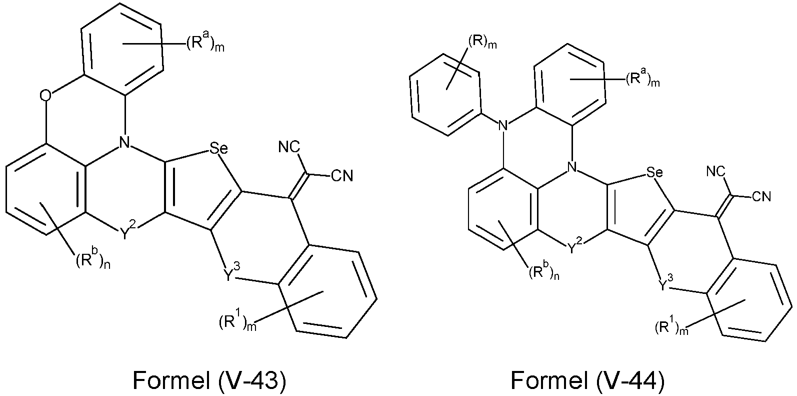 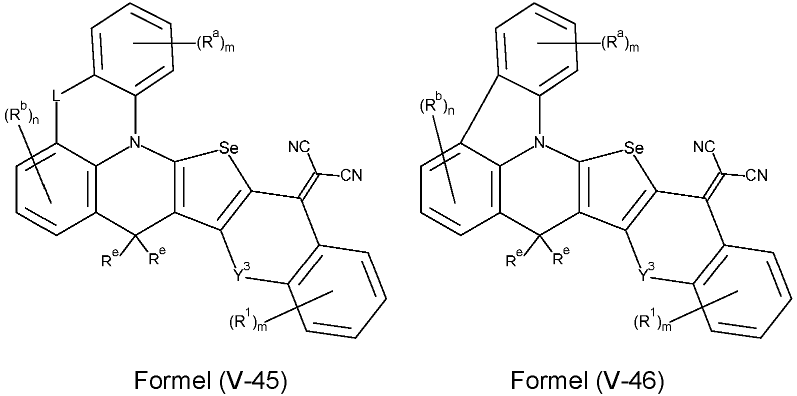 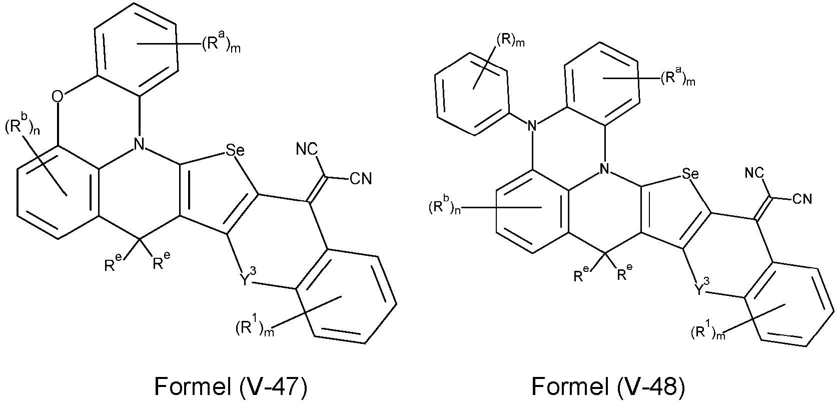 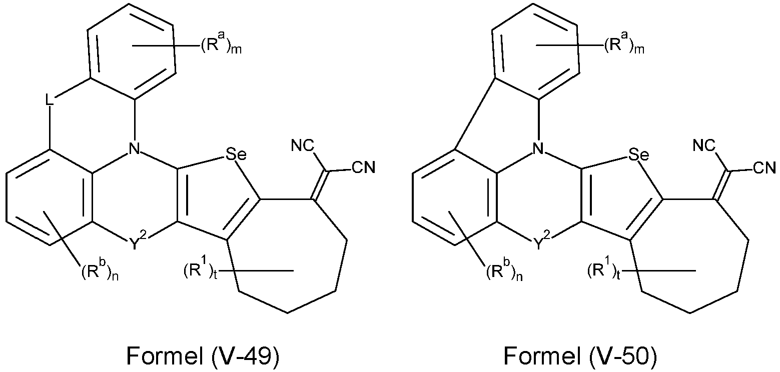 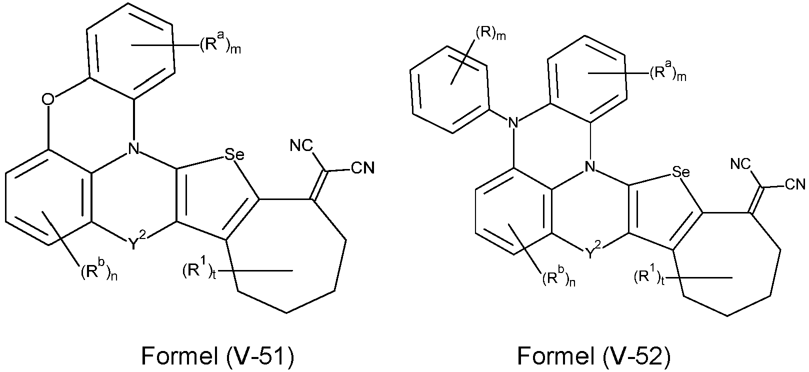 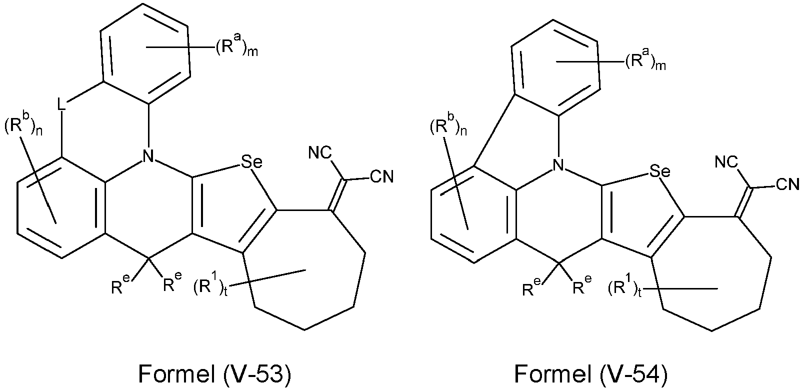 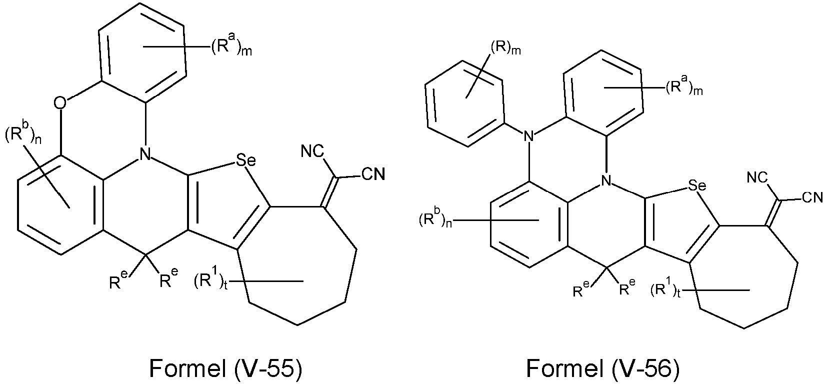 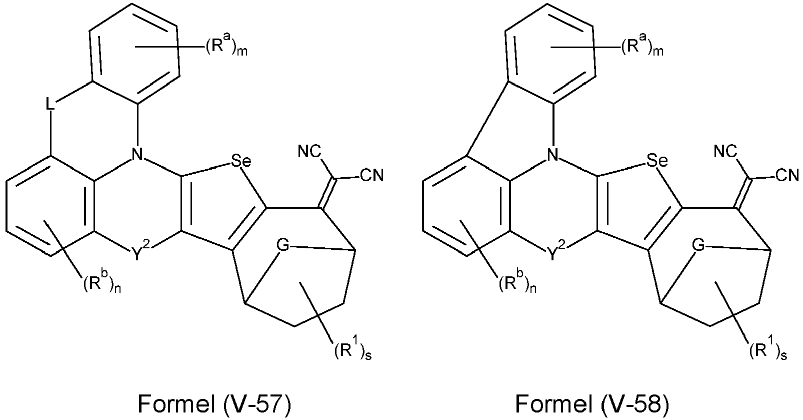 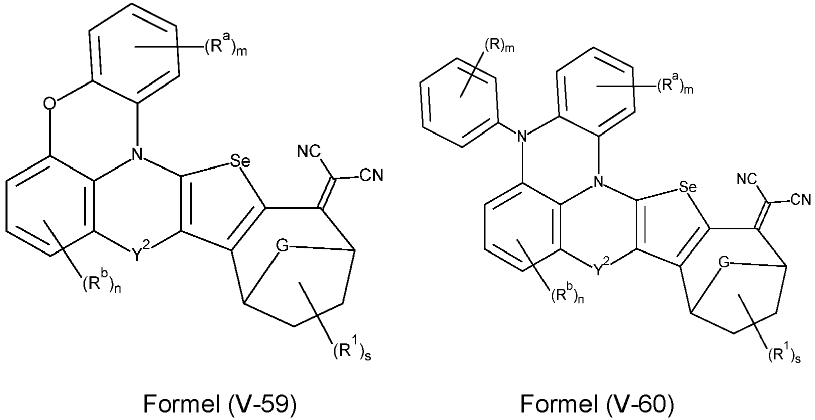 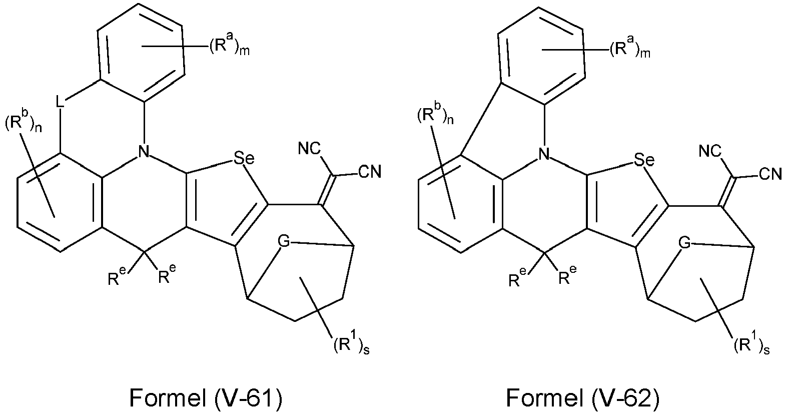 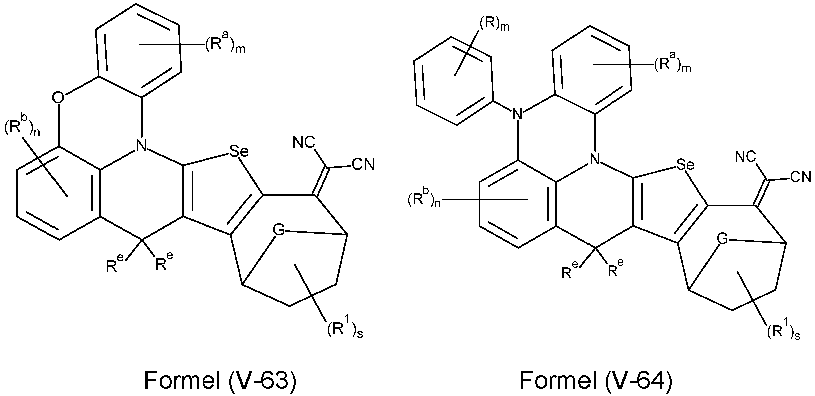 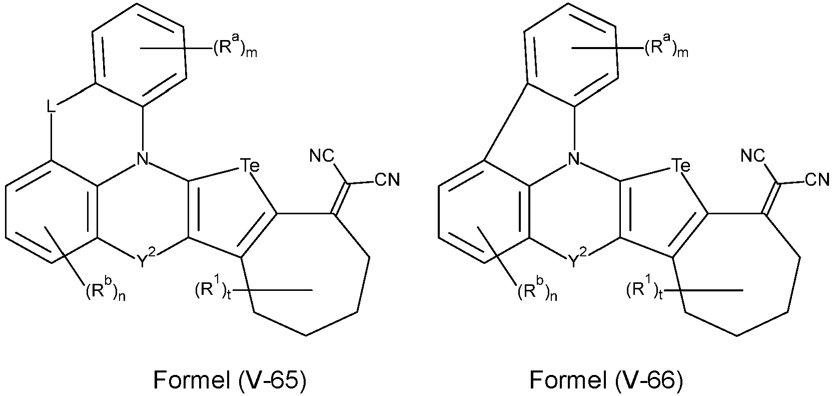 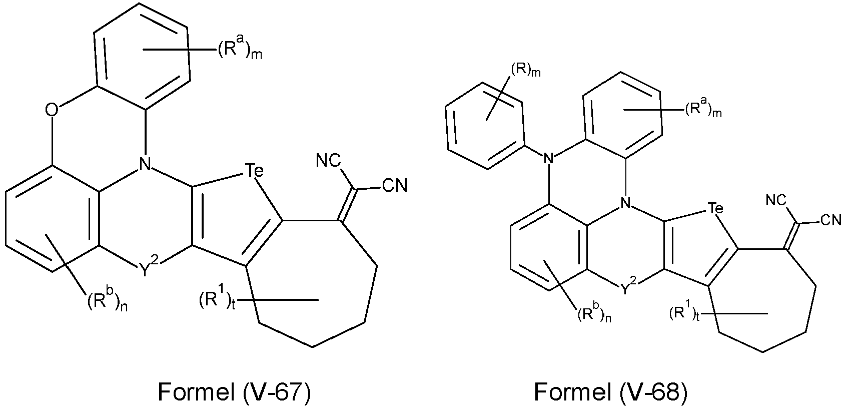 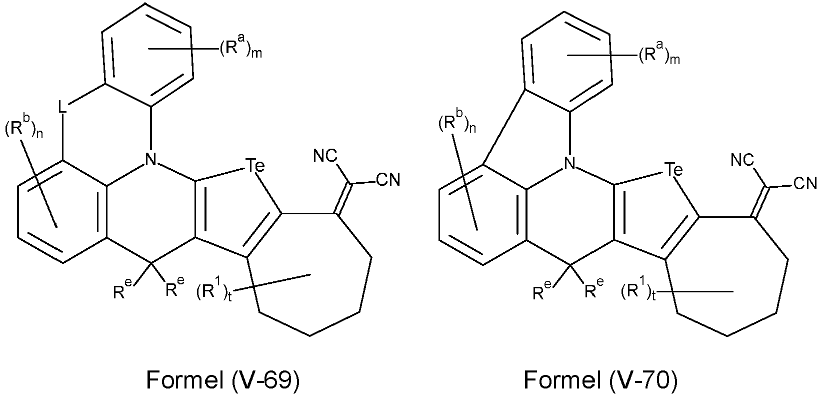 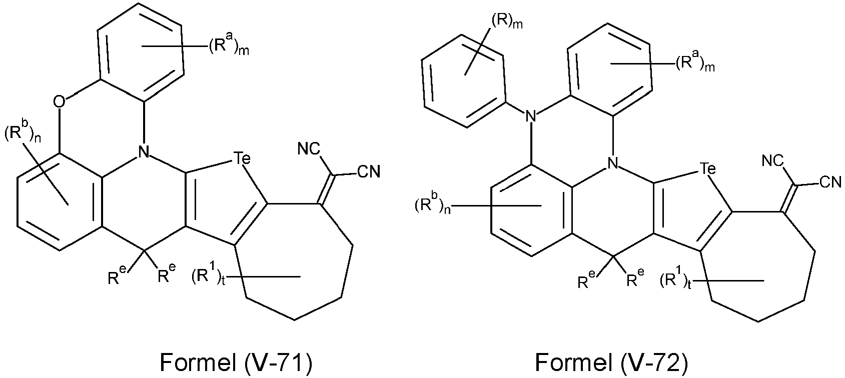 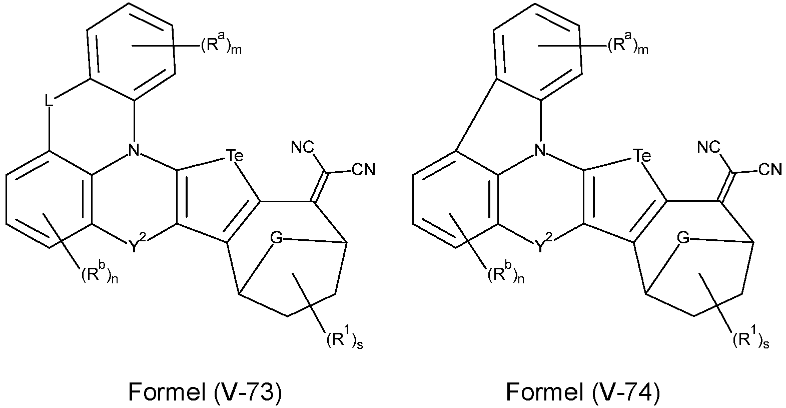 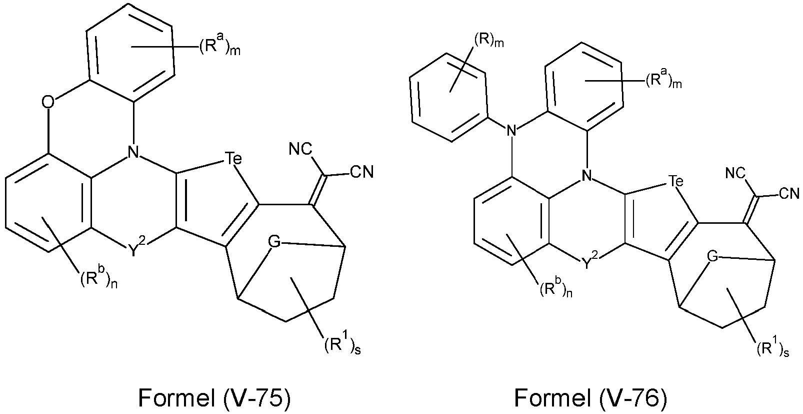 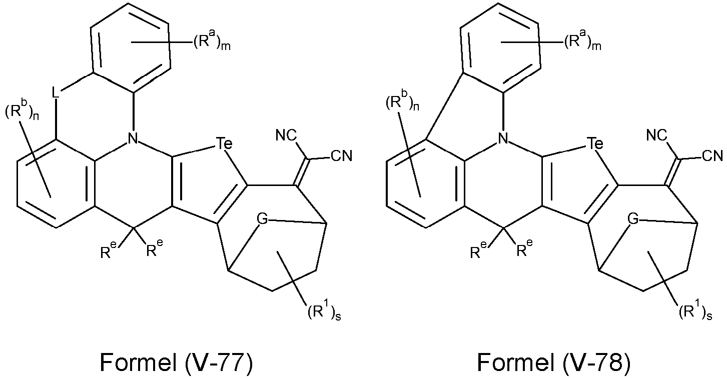 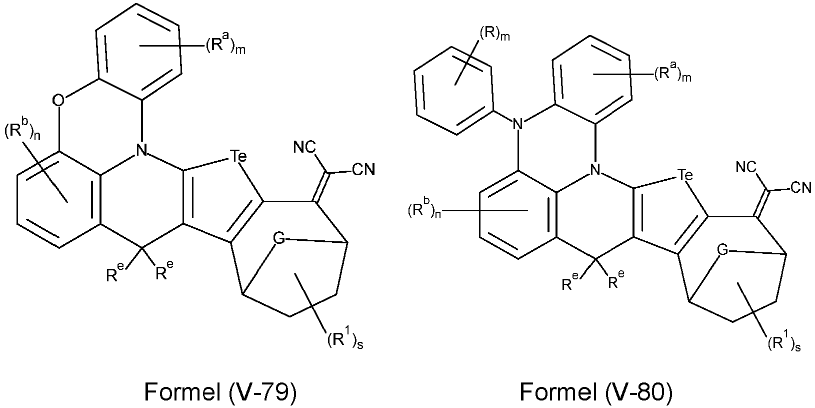 wobei die Symbole R^{a}, R^{b}, R^{c}, R^{d}, R^{e}, Y² und L die zuvor, insbesondere für Formel (I) genannten Bedeutungen aufweisen, Z für eine Gruppe der Formel (Z-1) oder (Z-2) steht und die weiteren Symbole die folgende Bedeutung aufweisen:
- G: ist bei jedem Auftreten gleich oder verschieden O, S, Se, Te, NAr', NR¹, BR¹, C(R¹)₂, Ge(R¹)₂, Si(R¹)₂, -C(R¹)₂-(CR¹)₂-, -CR¹=CR¹-, C=NR¹, S=O, SO₂ oder eine ortho-verknüpfte Arylen- oder Heteroarylengruppe mit 5 bis 14 aromatischen Ringatomen, welche durch einen oder mehrere Reste R¹ substituiert sein kann, wobei der Rest R¹ die zuvor, insbesondere für Formel (I) genannte Bedeutung aufweist, vorzugsweise C(R¹)₂, -C(R¹)₂-(CR¹)₂-, -CR¹=CR¹-, O, S;
- Y³: ist bei jedem Auftreten gleich oder verschieden eine Bindung, O, S, Se, Te, NAr', NR¹, BR¹, C(R¹)₂, Ge(R¹)₂, Si(R¹)₂, -CR¹=CR¹-, C=NR¹, S=O, SO₂ oder eine ortho-verknüpfte Arylen- oder Heteroarylengruppe mit 5 bis 14 aromatischen Ringatomen, welche durch einen oder mehrere Reste R¹ substituiert sein kann, wobei der Rest R¹ die zuvor, insbesondere für Formel (I) genannte Bedeutung aufweist, vorzugsweise eine Bindung, C(R¹)₂, O, S, NAr;
- t: ist 0, 1, 2, 3, 4, 5, 6, 7 oder 8, vorzugsweise 0, 1, 2, 3, oder 4, besonders bevorzugt 0, 1 oder 2;
- s: ist 0, 1, 2, 3, 4, 5 oder 6, vorzugsweise 0, 1, 2, 3, oder 4, besonders bevorzugt 0, 1 oder 2;
- m: ist 0, 1, 2, 3 oder 4, vorzugsweise 0, 1 oder 2, besonders bevorzugt 0 oder 1;
- n: ist 0, 1, 2 oder 3, vorzugsweise 0, 1 oder 2, besonders bevorzugt 0 oder 1.

Weitere bevorzugte Ausführungsformen sind die Strukturen der Formeln (V-17) bis (V-80), die S statt Se enthalten.

Geeignet sind für die Formeln (V-1) bis (V-8a) daher die folgenden Ausführungsformen:

| Formel | Gruppe Z |
|---|---|
| (V-1) | (Z-1) |
| (V-1) | (Z-2) |
| (V-1a) | (Z-1) |
| (V-1a) | (Z-2) |
| (V-2) | (Z-1) |
| (V-2) | (Z-2) |
| (V-2a) | (Z-1) |
| (V-2a) | (Z-2) |
| (V-3) | (Z-1) |
| (V-3) | (Z-2) |
| (V-3a) | (Z-1) |
| (V-3a) | (Z-2) |
| (V-4) | (Z-1) |
| (V-4) | (Z-2) |
| (V-4a) | (Z-1) |
| (V-4a) | (Z-2) |
| (V-5) | (Z-1) |
| (V-5) | (Z-2) |
| (V-5a) | (Z-1) |
| (V-5a) | (Z-2) |
| (V-6) | (Z-1) |
| (V-6) | (Z-2) |
| (V-6a) | (Z-1) |
| (V-6a) | (Z-2) |
| (V-7) | (Z-1) |
| (V-7) | (Z-2) |
| (V-7a) | (Z-1) |
| (V-7a) | (Z-2) |
| (V-8) | (Z-1) |
| (V-8) | (Z-2) |
| (V-8a) | (Z-1) |
| (V-8a) | (Z-2) |

Bevorzugt sind die Gruppen der Z ausgewählt aus den Gruppen der Formeln (Z-1), (Z-2') und (Z-2") und insbesondere aus den Gruppen der Formeln (Z-1) und (Z-2a) bis (Z-2d).

Hierbei sind Strukturen bzw. Verbindungen der Formeln (V-1), (V-1a), (V-2), (V-2a), (V-3), (V-3a), (V-5), (V-5a), (V-6), (V-6a), (V-7), (V-7a), (V-17), (V-18), (V-21), (V-22), (V-33), (V-34), (V-37), (V-38), (V-41), (V-42), (V-45), (V-46), (V-61) und/oder (V-62) bevorzugt, Strukturen bzw. Verbindungen der Formeln (V-2), (V-2a), (V-6), (V-6a), (V-18), (V-22), (V-34), (V-38), (V-42), (V-46) und/oder (V-62) besonders bevorzugt sind und Strukturen bzw. Verbindungen der Formeln (V-6), (V-6a), (V-22), (V-38), (V-46) und/oder (V-62) ganz besonders bevorzugt.

Die zuvor für den Rest R^{e} der Gruppe Y² im Hinblick auf die Formeln (I), (II), (IIa), (III), (IIIa), (IV) und/oder (IVa) bis (IVf) zuvor dargelegten Bevorzugungen gelten auch für die Formeln (V-1) bis (V-80) entsprechend.

Ferner kann unter anderem für die Formel (IV), (IVa) bis (IVf) und/oder Formeln (V-1) bis (V-80) vorgesehen sein, dass die Summe der Indices m und n höchstens 10, vorzugsweise höchstens 8, insbesondere bevorzugt höchstens 6 und besonders bevorzugt höchstens 4 und ganz besonders bevorzugt höchtens 2 beträgt.

In einer bevorzugten Weiterbildung der vorliegenden Erfindung kann vorgesehen sein, dass mindestens zwei Reste R, R^{a}, R^{b} mit den weiteren Gruppen, an die die zwei Reste R, R^{a}, R^{b} binden, einen kondensierten Ring bilden, wobei die zwei Reste R, R^{a}, R^{b} mindestens eine Struktur der Formeln (RA-1) bis (RA-12) formen 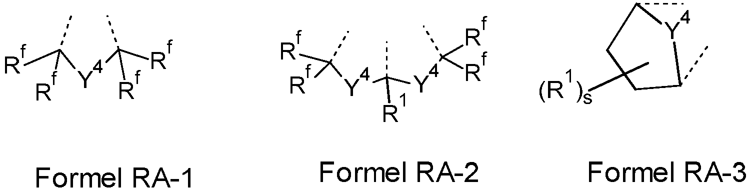 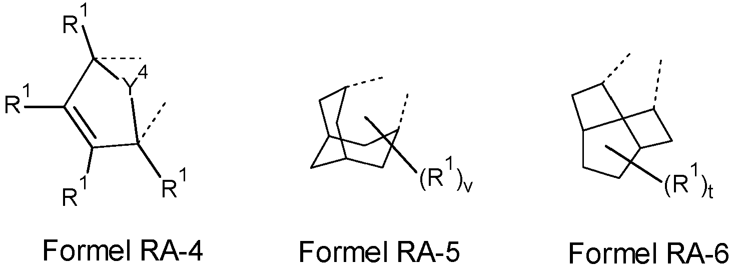 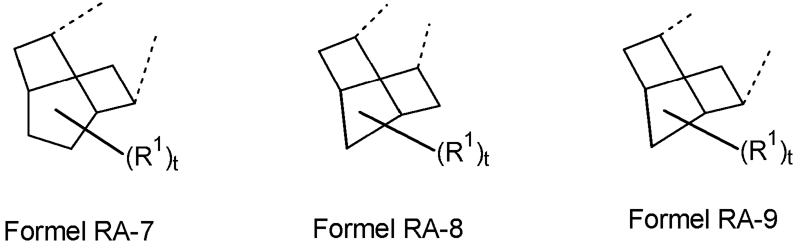 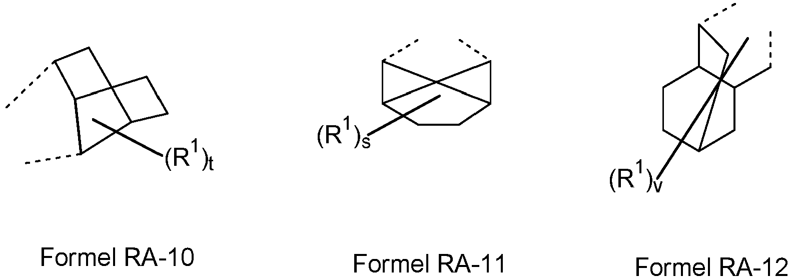 wobei R¹ die zuvor dargelegte Bedeutung hat, die gestrichelten Bindungen die Anbindungsstellen an die Atome der Gruppen, an die die zwei Reste R, R^{a}, R^{b} binden, darstellen und die weiteren Symbole die folgende Bedeutung aufweisen:
- Y⁴: ist bei jedem Auftreten gleich oder verschieden C(R¹)₂, (R¹)₂C-C(R¹)₂, (R¹)C=C(R¹), NR¹, NAr', O oder S, vorzugsweise C(R¹)₂, (R¹)₂C-C(R¹)₂, (R¹)C=C(R¹), O oder S;
- R^{f}: ist bei jedem Auftreten gleich oder verschieden H, D, F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², C(=O)O, C(=O)NR², NR², P(=O)(R¹), O, S, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{f} auch miteinander oder ein Rest R^{f} mit einem Rest R¹ oder mit einer weiteren Gruppe ein Ringsystem bilden, wobei der Rest R², die zuvor, insbesondere für Formel (I) genannte Bedeutung aufweist;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2.

Hierbei sind Strukturen der Formeln RA-1, RA-3, RA-4 und RA-5 bevorzugt und Strukturen der Formeln RA-4 und RA-5 besonders bevorzugt.

In einer bevorzugten Weiterbildung der vorliegenden Erfindung kann vorgesehen sein, dass mindestens zwei Reste R, R^{a}, R^{b} mit den weiteren Gruppen, an die die zwei Reste R, R^{a}, R^{b} binden, einen kondensierten Ring bilden, wobei die zwei Reste R, R^{a}, R^{b} Strukturen der Formeln (RA-1a) bis (RA-4f) formen 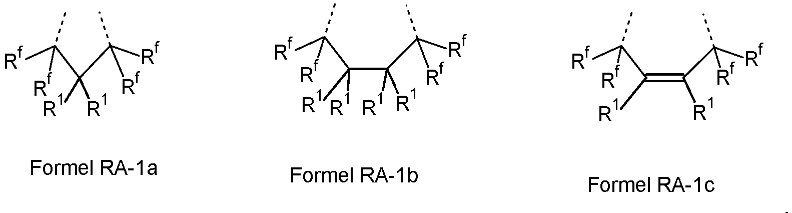 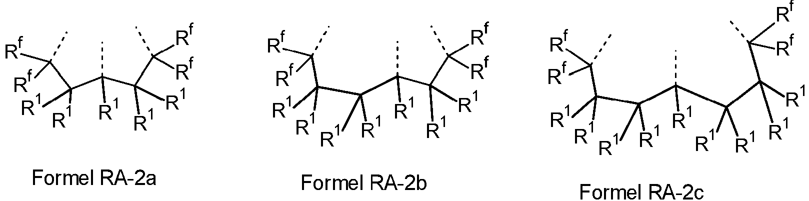 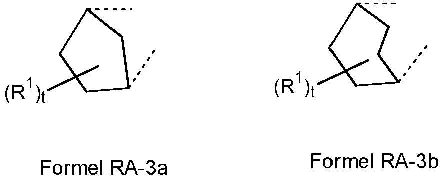 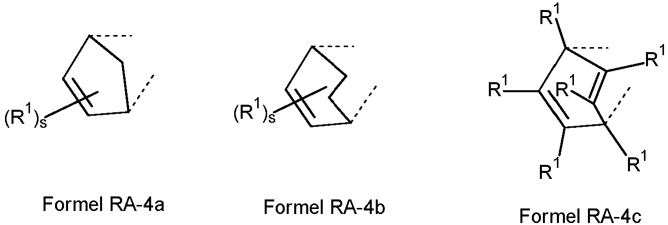 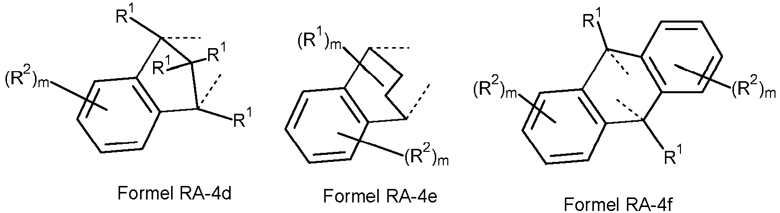 wobei die gestrichelten Bindungen die Anbindungsstellen an die Atome der Gruppen, an die die zwei Reste R, R^{a}, R^{b} binden, darstellen, der Index m 0, 1, 2, 3 oder 4, vorzugsweise 0, 1 oder 2 ist und die Symbole R¹, R², R^{f} und die Indices s, und t die zuvor, insbesondere für Formel (I) und/oder Formeln (RA-1) bis (RA-12) dargelegte Bedeutung haben.

Hierbei sind Strukturen der Formeln RA-4f bevorzugt.

Ferner kann vorgesehen sein, dass die mindestens zwei Reste R, R^{a}, R^{b} die Strukturen der Formeln (RA-1) bis (RA-12) formen und einen kondensierten Ring bilden, Reste R, R^{a}, R^{b} aus benachbarten Gruppen X^{a}, X^{b} darstellen oder Reste R, R^{a}, R^{b} darstellen, die jeweils an benachbarte C-Atome binden, wobei diese C-Atome vorzugsweise über eine Bindung verbunden sind.

In einer weiterhin bevorzugten Ausgestaltung kann vorgesehen sein, dass mindestens zwei Reste R, R^{a}, R^{b} mit den weiteren Gruppen, an die die zwei Reste R, R^{a}, R^{b} binden, einen kondensierten Ring bilden, wobei die zwei Reste R, R^{a}, R^{b} der Strukturen der Formel (RB) formen, 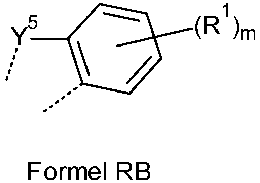 wobei R¹ die zuvor, insbesondere für Formel (I) genannte Bedeutung aufweist, die gestrichelten Bindungen die Anbindungsstellen an die Atome der Gruppen, an die die zwei Reste R, R^{a}, R^{b} binden, darstellen, der Index m 0, 1, 2, 3 oder 4, vorzugsweise 0, 1 oder 2 ist, und Y⁵ C(R¹)₂, NR¹, NAr', BR¹, BAr', O oder S ist, vorzugsweise C(R¹)₂, NAr' oder O, besonders bevorzugt C(R¹)₂ oder O, wobei Ar' die zuvor, insbesondere für Formel (I) genannte Bedeutung aufweist.

Hierbei kann vorgesehen sein, dass die mindestens zwei Reste R, R^{a}, R^{b}, die die Strukturen der Formel (RB) formen und einen kondensierten Ring bilden, Reste R, R^{a}, R^{b} aus benachbarten Gruppen X^{a}, X^{b}, darstellen oder Reste R, R^{a}, R^{b} darstellen, die jeweils an benachbarte C-Atome binden, wobei diese C-Atome vorzugsweise über eine Bindung miteinander verbunden sind.

Insbesondere kann vorgesehen sein, dass in bevorzugten Strukturen bzw. Verbindungen die Summe der Indices r, s, t, v, m und n vorzugsweise 0, 1, 2 oder 3, besonders bevorzugt 1 oder 2 ist.

Besonders bevorzugt umfassen die Verbindungen mindestens eine Struktur der Struktur der Formeln (VI-1) bis (VI-5), besonders bevorzugt sind die Verbindungen ausgewählt aus Verbindungen der Formeln (VI-1) bis (VI-5), wobei die Verbindungen mindestens einen kondensierten Ring aufweisen, 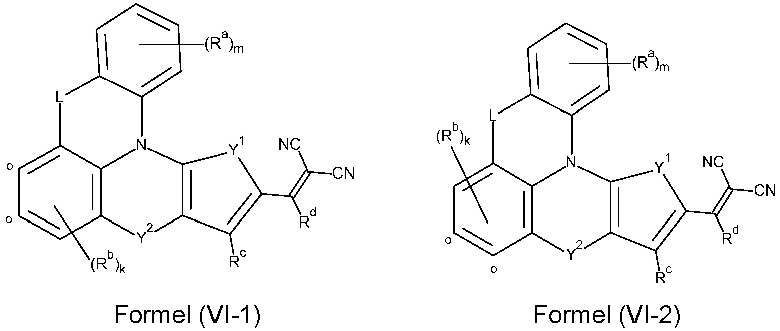 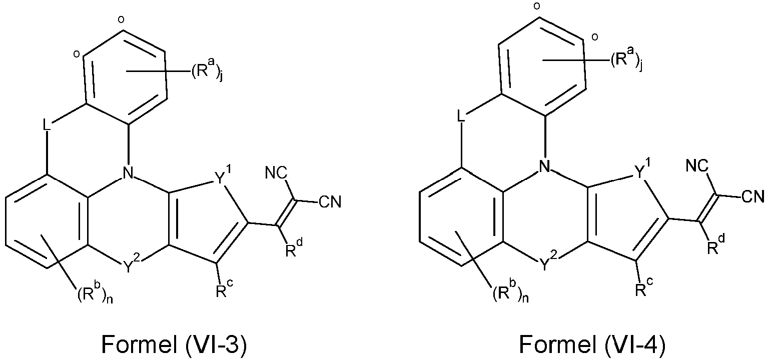 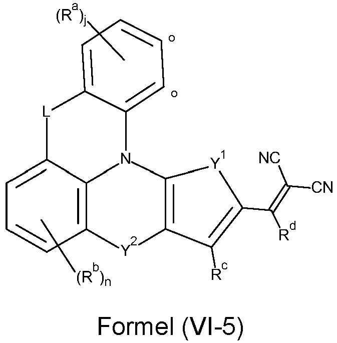 wobei die Symbole R^{a}, R^{b}, R^{c}, R^{d}, Y¹, Y² und L die zuvor, insbesondere für Formel (I) genannten Bedeutungen aufweisen, das Symbol o für die Kondensationsstellen des mindestens einen kondensierten Rings steht und die weiteren Indices die folgende Bedeutung haben:
- m: ist 0, 1, 2, 3 oder 4, vorzugsweise 0, 1 oder 2;
- n: ist 0, 1, 2 oder 3, vorzugsweise 0, 1 oder 2;
- j: ist 0, 1 oder 2, vorzugsweise 0 oder 1;
- k: ist 0 oder 1.

Analog geeignet sind die Strukturen der Formeln (VI-1) bis (VI-5), die als Gruppe Z statt der Gruppe der Formel (Z-1) eine Gruppe der Formel (Z-2) enthält.

Ferner kann vorgesehen sein, dass der kondensierte Ring durch mindestens zwei Reste R^{a}, R^{b} und den weiteren Gruppen, an die die zwei Reste R^{a}, R^{b} binden, gebildet wird, wobei die mindestens zwei Reste R^{a}, R^{b} Strukturen der Formeln (RA-1) bis (RA-12) und/oder der Formel (RB) formen, vorzugsweise Strukturen der Formeln (RA-1) bis (RA-12).

Ferner kann insbesondere in Formeln (VI-1) bis (VI-5) vorgesehen sein, dass die Summe der Indices k, j, n und m 0, 1, 2 oder 3, vorzugsweise 0, 1 oder 2 ist.

Bevorzugt kann vorgesehen sein, dass die Verbindungen mindestens zwei kondensierte Ringe aufweisen, wobei mindestens ein kondensierter Ring durch Strukturen der Formeln (RA-1) bis (RA-12) und/oder (RA-1a) bis (RA-4f) gebildet ist und ein weiterer Ring durch Strukturen der Formeln (RA-1) bis (RA-12), (RA-1a) bis (RA-4f) oder (RB) gebildet ist.

Weiterhin kann vorgesehen sein, dass die Substituenten R, R^{a}, R^{b}, R^{c}, R^{d}, R^{e}, R^{f} und R¹ gemäß obigen Formeln mit den Ringatomen des Ringsystems, an das die Substituenten R, R^{a}, R^{b}, R^{c}, R^{d}, R^{e}, R^{f} und R¹ binden, kein kondensiertes aromatisches oder heteroaromatisches Ringsystem bilden. Dies schließt die Bildung eines kondensierten aromatischen oder heteroaromatischen Ringsystems mit möglichen Substituenten R¹ und R² ein, die an die Substituenten R, R^{a}, R^{b}, R^{c}, R^{d}, R^{e}, R^{f} und R¹ gebunden sein können.

Wenn zwei Reste, die insbesondere ausgewählt sein können aus R, R^{a}, R^{b}, R^{c}, R^{d}, R^{e}, R^{f}, R¹ und/oder R², miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R^{a}, R^{b}, R^{c}, R^{d}, R^{e}, R^{f}, R¹ und/oder R² versehenen Ringsysteme auch über eine Bindung miteinander verbunden sein, so dass hierdurch ein Ringschluss bewirkt werden kann. In diesem Fall ist jede der entsprechenden Bindungsstellen vorzugsweise mit einem Substituenten R, R^{a}, R^{b}, R^{c}, R^{d}, R^{e}, R^{f}, R¹ und/oder R² versehen.

Gemäß einer bevorzugten Ausgestaltung ist eine erfindungsgemäße Verbindung durch mindestens eine der Strukturen gemäß Formeln (I), (II), (IIa), (III), (IIIa), (IV), (IVa) bis (IVf) und/oder (V-1) bis (V-80) darstellbar. Vorzugsweise weisen erfindungsgemäße Verbindungen, bevorzugt umfassend Strukturen oder dargestellt durch Strukturen gemäß Formeln (I), (II), (IIa), (III), (IIIa), (IV), (IVa) bis (IVf) und/oder (V-1) bis (V-80) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Bevorzugte aromatische bzw. heteroaromatische Ringsysteme Ar, R, R^{a}, R^{b}, R^{c}, R^{d}, R^{e}, R^{f} und/oder Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R, R¹ bzw. R² substituiert sein können.

Vorzugsweise kann vorgesehen sein, dass mindestens ein Rest R, R^{a}, R^{b}, R^{c}, R^{d}, R^{e} gleich oder verschieden bei jedem Auftreten ausgewählt ist aus der Gruppe bestehend aus H, D, einer geradkettigen Alkylgruppe mit 1 bis 4 C-Atomen, einer verzweigten oder cyclischen Alkylgruppe mit 3 bis 10 C-Atomen oder einem aromatischen oder heteroaromatischen Ringsystem ausgewählt aus den Gruppen der folgenden Formeln Ar-1 bis Ar-78, vorzugsweise die Reste R, R^{a}, R^{b}, R^{c}, R^{d}, R^{e} entweder einen kondensierten Ring, vorzugsweise gemäß den Strukturen der Formeln (RA-1) bis (RA-12) oder (RB) bilden oder der Rest R, R^{a}, R^{b}, R^{c}, R^{d}, R^{e} gleich oder verschieden bei jedem Auftreten ausgewählt ist aus der Gruppe bestehend aus H, D, Methyl oder einem aromatischen oder heteroaromatischen Ringsystem ausgewählt aus den Gruppen der folgenden Formeln Ar-1 bis Ar-78, und/oder die Gruppe Ar' gleich oder verschieden bei jedem Auftreten ausgewählt ist aus den Gruppen der folgenden Formeln Ar-1 bis Ar-78, 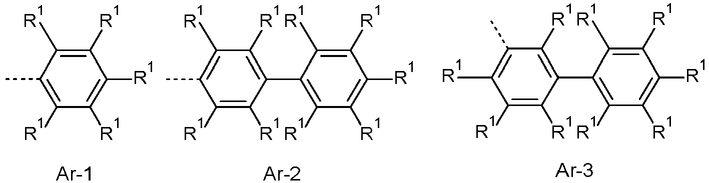 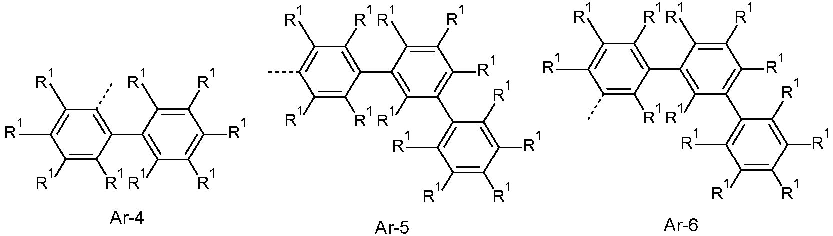 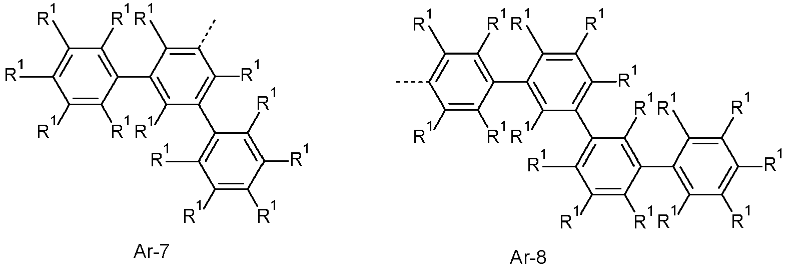 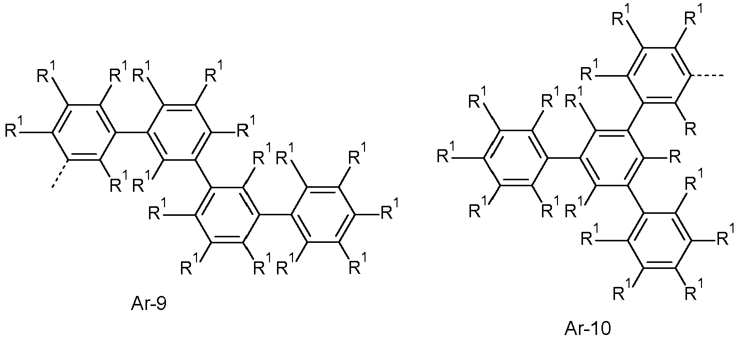 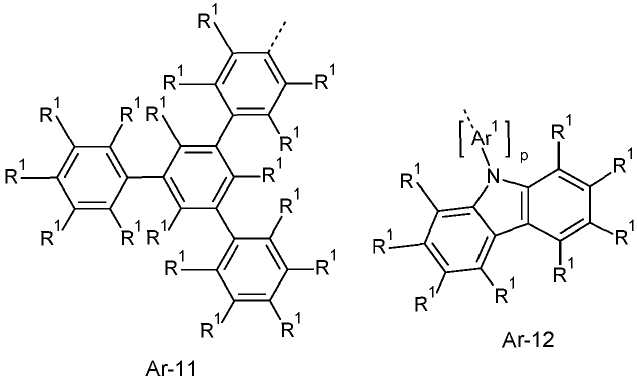 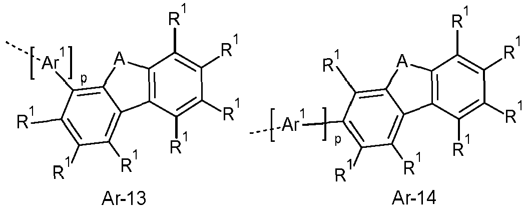 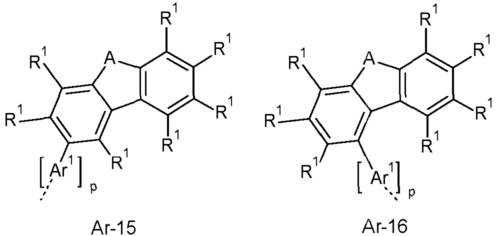 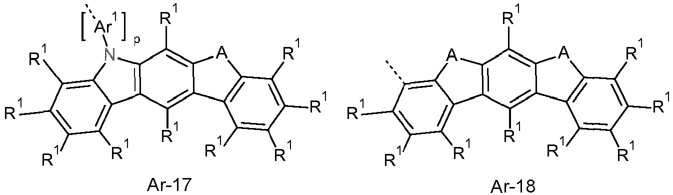 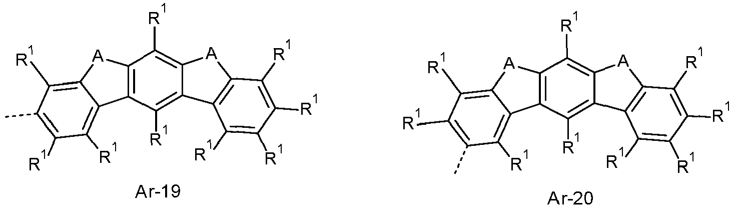 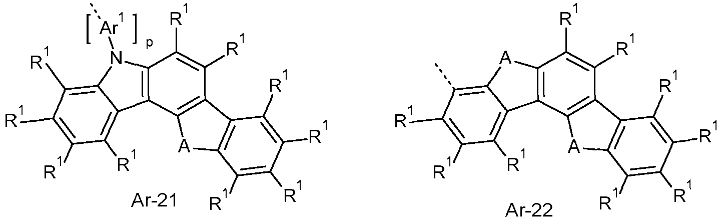 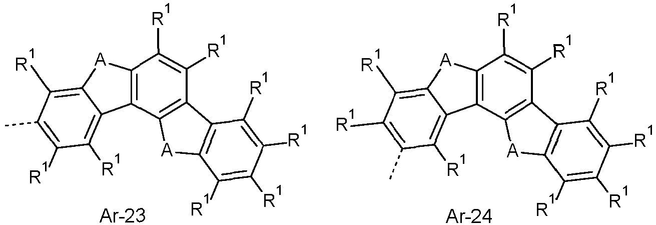 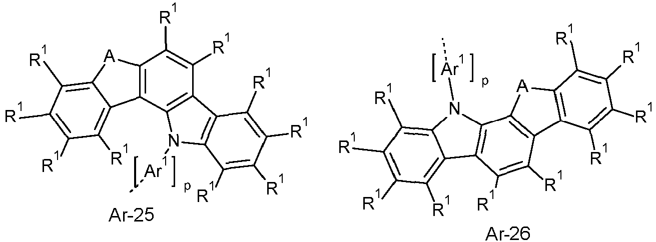 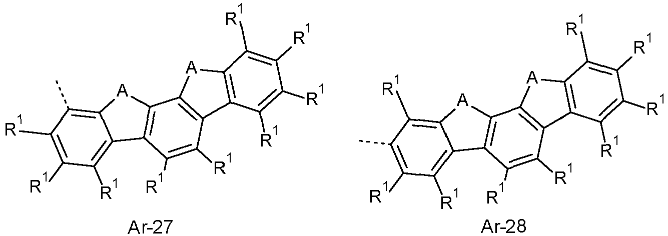 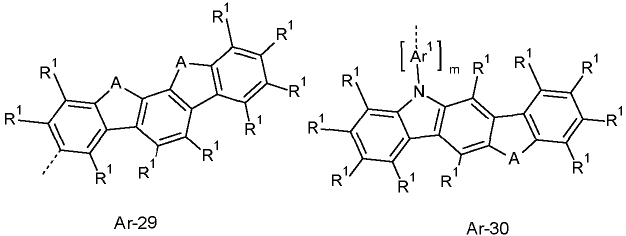 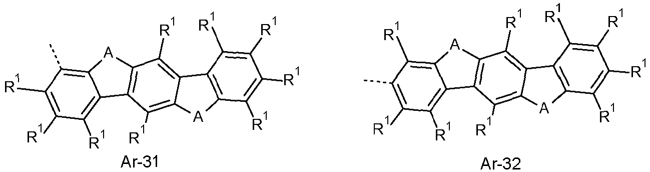 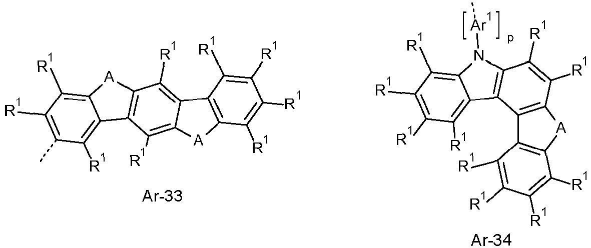 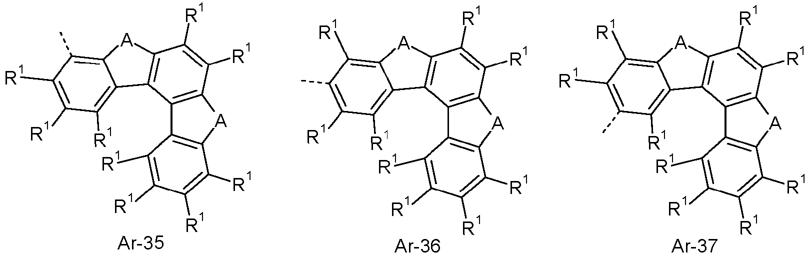 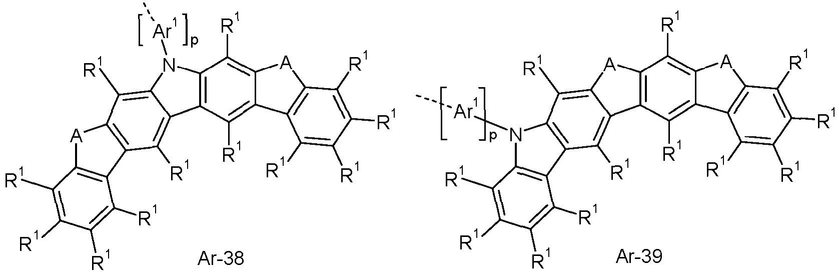 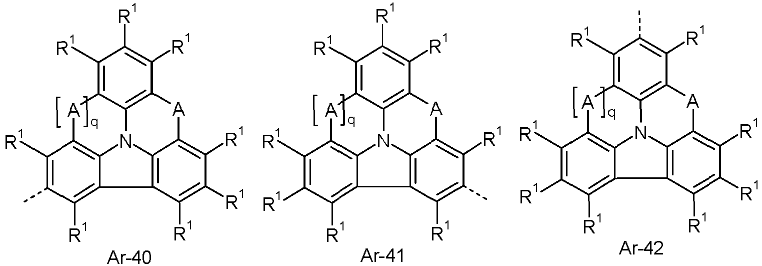 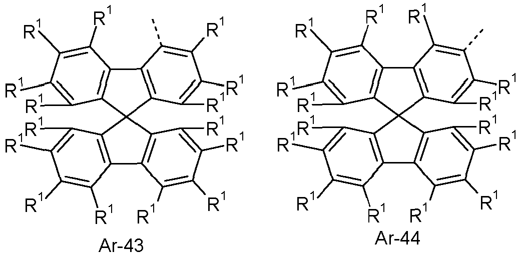 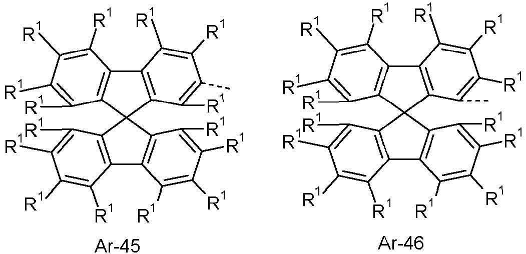 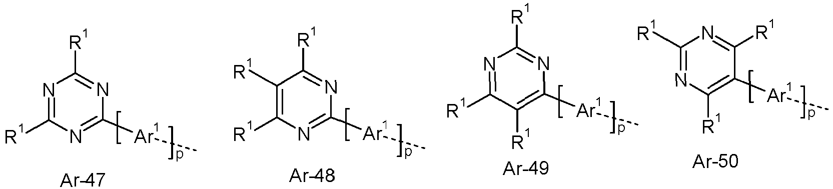 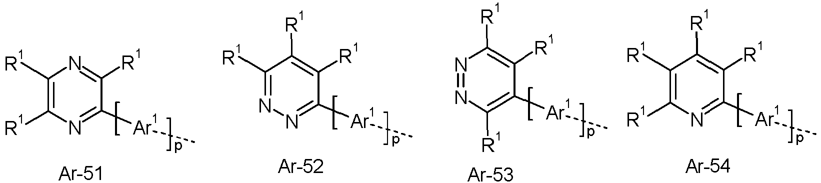 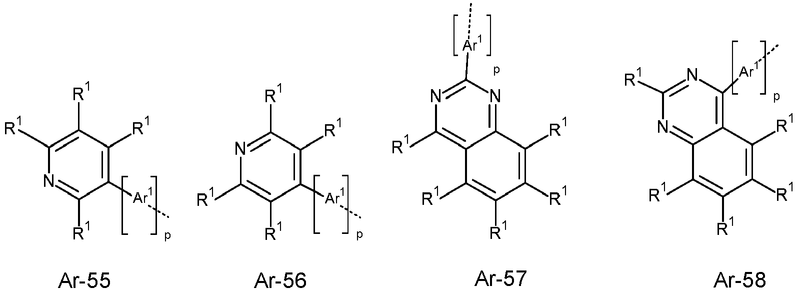 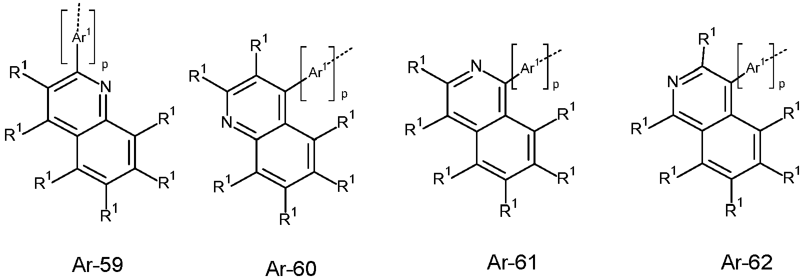 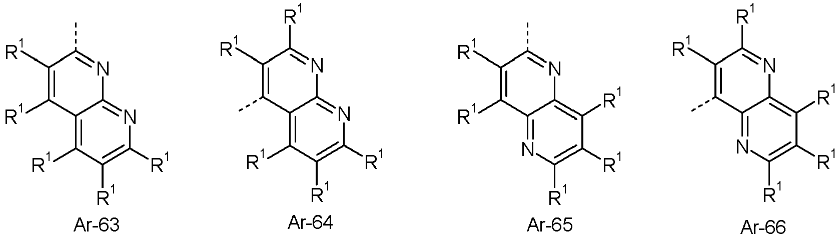 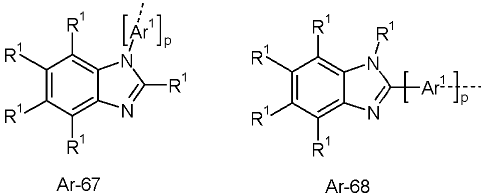 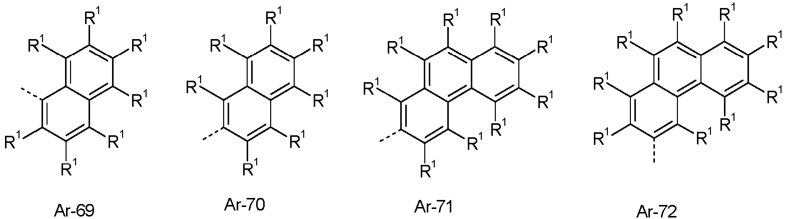 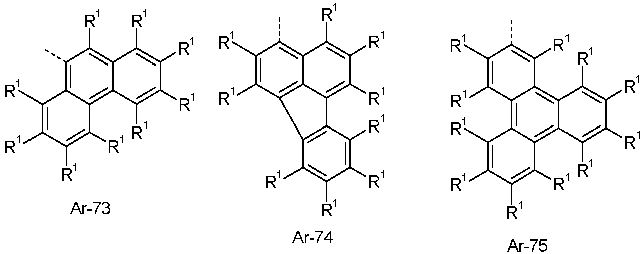 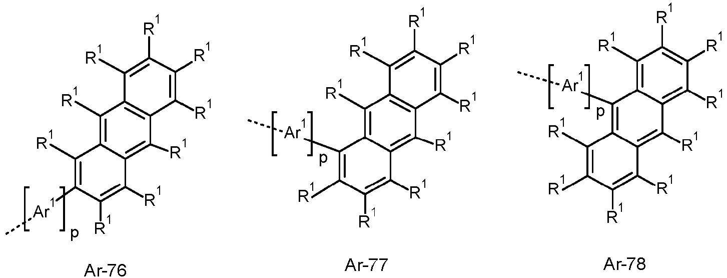 wobei R¹ die oben genannten Bedeutungen aufweist, die gestrichelte Bindung die Anbindungstelle an die entsprechende Gruppe darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Hierbei sind die Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-44), (Ar-45), (Ar-46), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Im Folgenden werden bevorzugte Substituenten R, R^{a}, R^{b}, R^{c}, R^{d}, R^{e} und R^{f} beschrieben.

In einer bevorzugten Ausführungsform der Erfindung ist R, R^{a}, R^{b}, R^{c}, R^{d}, R^{e} gleich oder verschieden bei jedem Auftreten ausgewählt aus der Gruppe bestehend aus H, D, F, CN, Si(R¹)₃, B(OR¹)₂, einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R¹ substituiert sein kann, oder einem aromatischen oder heteroaromatischen Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin besonders bevorzugten Ausführungsform der Erfindung ist Substituent R, R^{a}, R^{b}, R^{c}, R^{d}, R^{e} gleich oder verschieden bei jedem Auftreten ausgewählt aus der Gruppe bestehend aus H, D, F, einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R¹ substituiert sein kann, oder einem aromatischen oder heteroaromatischen Ringsystem mit 6 bis 30 aromatischen Ringatomen, bevorzugt mit 6 bis 24 aromatischen Ringatomen, besonders bevorzugt mit 6 bis 18 aromatischen Ringatomen, ganz besonders bevorzugt mit 6 bis 13 aromatischen Ringatomen, das jeweils durch einen oder mehrere Reste R¹ substituiert sein kann.

Ferner kann vorgesehen sein, dass mindestens ein Substituent R, R^{a}, R^{b}, R^{c}, R^{d}, R^{e} gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, bevorzugt mit 6 bis 24 aromatischen Ringatomen, besonders bevorzugt mit 6 bis 18 aromatischen Ringatomen, ganz besonders bevorzugt mit 6 bis 13 aromatischen Ringatomen, welches jeweils mit einem oder mehreren Resten R¹ substituiert sein kann, oder einer Gruppe N(Ar')₂. Bevorzugte Ausführungsformen des aromatischen bzw. heteroaromatischen Ringsystems sind die oben abgebildeten Strukturen (Ar-1) bis (Ar-78).

In einer weiterhin bevorzugten Ausführungsform der Erfindung bilden die Substituenten R, R^{a}, R^{b}, R^{c}, R^{d}, R^{e} entweder einen Ring gemäß den Strukturen der Formeln (RA-1) bis (RA-12), (RA-1a) bis (RA-4f) oder (RB) oder der Substituent R, R^{a}, R^{b}, R^{c}, R^{d}, R^{e} ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Besonders bevorzugt ist Substituent R, R^{a}, R^{b} gleich oder verschieden bei jedem Auftreten ausgewählt aus der Gruppe bestehend aus H, D oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

Bevorzugt kann vorgesehen sein, dass mindestens ein Substituent R, R^{a}, R^{b}, R^{c}, R^{d}, R^{e} ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R¹ substituiert sein können. Hierbei bedeutet der Ausdruck Substituent insbesondere, dass R, R^{a}, R^{b}, R^{c}, R^{d}, R^{e} ungleich H sind. Ferner können die Substituenten R, R^{a}, R^{b}, R^{c}, R^{d}, R^{e} gleich oder verschieden sein, falls zwei oder mehr Substituenten vorhanden sind, die aus den genannten aromatischen oder heteroaromatischen Gruppe ausgewählt sind.

In einer weiteren Ausführungsform kann vorgesehen sein, dass mindestens ein Substituent R, R^{a}, R^{b}, R^{c}, R^{d}, R^{e} ausgewählt ist aus o-Biphenyl, o,o'-Terphenyl, o,o',p-Quaterphenyl, 4,6-Diphenyl-pyrimidin-2-yl, 4,6-Diphenyl-triazin-2-yl, Naphthalin, Phenanthren, Chrysen, Spirobifluoren, Triphenylen, Anthracen, Benzanthracen, Fluoren und/oder Pyren, welche jeweils mit einem oder mehreren Resten R¹ substituiert sein können. Hierbei sind Spirobifluoren, o-Biphenyl, o,o'-Terphenyl, o,o',p-Quaterphenyl, 4,6-Diphenyl-pyrimidin-2-yl, 4,6-Diphenyl-triazin-2-yl-Reste bevorzugt. Die Substituenten R, R^{a}, R^{b}, R^{c}, R^{d}, R^{e} können gleich oder verschieden sein, falls zwei oder mehr Substituenten vorhanden sind, die aus den genannten aromatischen Gruppe ausgewählt sind.

In einer bevorzugten Ausführungsform der Erfindung ist R^{f} gleich oder verschieden bei jedem Auftreten ausgewählt aus der Gruppe bestehend aus einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R¹ substituiert sein kann, oder einem aromatischen oder heteroaromatischen Ringsystem mit 5 bis 60 aromatischen Ringatomen, bevorzugt mit 5 bis 40 aromatischen Ringatomen, das jeweils durch einen oder mehrere Reste R² substituiert sein kann.

In einer weiterhin bevorzugten Ausführungsform der Erfindung ist R^{f} gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 30 aromatischen Ringatomen, welches mit einem oder mehreren Resten R² substituiert sein kann.

In einer bevorzugten Ausführungsform der Erfindung ist R^{f} bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann; dabei können zwei Reste R^{f} auch miteinander ein Ringsystem bilden. Besonders bevorzugt ist R^{f} bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R² substituiert sein kann, bevorzugt aber unsubstituiert ist, oder einem aromatischen oder heteroaromatischen Ringsystem mit 6 bis 18 aromatischen Ringatomen, bevorzugt mit 6 bis 13 aromatischen Ringatomen, insbesondere mit 6 aromatischen Ringatomen, das jeweils durch einen oder mehrere, bevorzugt nicht-aromatische Reste R² substituiert sein kann, bevorzugt aber unsubstituiert ist; dabei können zwei Reste R^{f} miteinander ein Ringsystem bilden. Ganz besonders bevorzugt ist R^{f} bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen. Ganz besonders bevorzugt steht R^{f} für eine Methylgruppe oder für eine Phenylgruppe, wobei zwei Phenylgruppen zusammen ein Ringsystem bilden können, wobei eine Methylgruppe gegenüber einer Phenylgruppe bevorzugt ist.

Bevorzugte aromatische bzw. heteroaromatische Ringsysteme, für die Substituent R, R^{a}, R^{b}, R^{c}, R^{d}, R^{e}, R^{f} beziehungsweise Ar oder Ar' stehen, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R, R¹ beziehungsweise R² substituiert sein können. Besonders bevorzugt sind die oben aufgeführten Strukturen Ar-1 bis Ar-78, wobei Strukturen der Formeln (Ar-1), (Ar-2), (Ar-3), (Ar-12), (Ar-13), (Ar-14), (Ar-15), (Ar-16), (Ar-69), (Ar-70), (Ar-75) bevorzugt und Strukturen der Formeln (Ar-1), (Ar-2), (Ar-3), (Ar-12), (Ar-13), (Ar-14), (Ar-15), (Ar-16) besonders bevorzugt sind. Hinsichtlich der Strukturen Ar-1 bis Ar-78 ist festzuhalten, dass diese mit einem Substituenten R¹ dargestellt sind. Im Falle der Ringsysteme Ar sind diese Substituenten R¹ durch R und im Falle R^{f} sind diese Substituenten R¹ durch R² zu ersetzen.

Weitere geeignete Gruppen R, R^{a}, R^{b}, R^{c}, R^{d} sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R² substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, D, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Ferner kann vorgesehen sein, dass die Verbindung genau zwei oder genau drei Strukturen gemäß Formel (I), (II), (IIa), (III), (IIIa), (IV), (IVa) bis (IVf), (V-1) bis (V-80) und/oder (VI-1) bis (VI-5) umfasst.

In einer bevorzugten Ausgestaltung sind die Verbindungen ausgewählt aus Verbindungen gemäß der Formel (D-1), 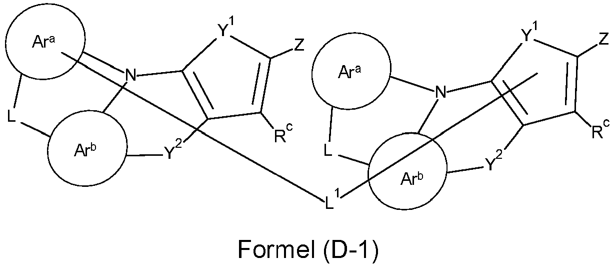 wobei die Gruppe L' eine Verbindungsgruppe, vorzugsweise eine Bindung oder ein aromatisches oder heteroaromatisches Ringsystem mit 5 bis 40, bevorzugt 5 bis 30 aromatischen Ringatomen darstellt, welches durch einen oder mehrere Reste R¹ substituiert sein kann, und die weiteren verwendeten Symbole die zuvor, insbesondere für Formel (I) genannten Bedeutungen aufweisen. Dabei kann L¹ an Ar^{a}, Ar^{b}, L, Y¹ oder Y² binden oder kann an die Position von R^{c} binden, wobei R^{c} in diesem Fall nicht vorhanden ist.

In einer weiteren bevorzugten Ausführungsform der Erfindung steht L¹ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen oder ein heteroaromatisches Ringsystem mit 6 bis 13 aromatischen Ringatomen, welches jeweils durch einen oder mehrere Reste R¹ substituiert sein kann, bevorzugt aber unsubstituiert ist, wobei R¹ die zuvor, insbesondere für Formel (I) genannte Bedeutung aufweisen kann. Besonders bevorzugt steht L¹ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R¹ substituiert sein kann, bevorzugt aber unsubstituiert ist, wobei R¹ die zuvor, insbesondere für Formel (I) genannte Bedeutung aufweisen kann.

Weiterhin bevorzugt steht das unter anderem in Formel (D1) dargelegte Symbol L' gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (D1) dargelegte Gruppe L¹ ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L¹ sind ausgewählt aus der Gruppe bestehend aus ortho-, meta- oder para-Phenylen, ortho-, meta- oder para-Biphenyl, Terphenyl, insbesondere verzweigtes Terphenyl, Quaterphenyl, insbesondere verzweigtes Quaterphenyl, Fluorenylen, Spirobifluorenylen, Dibenzofuranylen, Dibenzothienylen und Carbazolylen, die jeweils durch einen oder mehrere Reste R¹ substituiert sein können, bevorzugt aber unsubstituiert sind.

Die oben genannten bevorzugten Ausführungsformen können beliebig innerhalb der in Anspruch 1 definierten Einschränkungen miteinander kombiniert werden. **In** einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen:

| | |
|---|---|
| 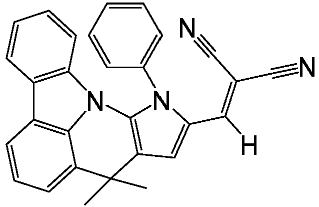 | 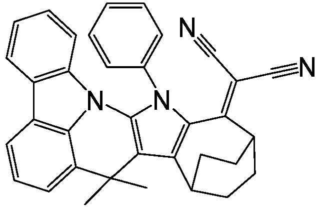 |
| 1 | 2 |
| 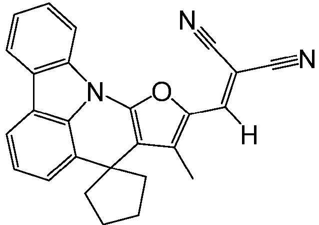 | 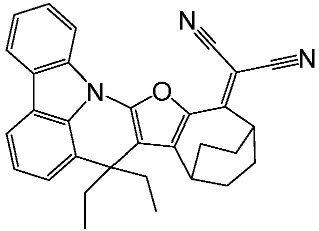 |
| 3 | 4 |
| 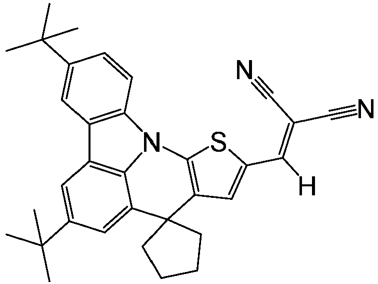 | 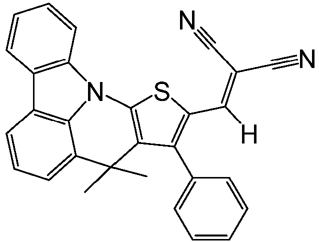 |
| 5 | 6 |
| 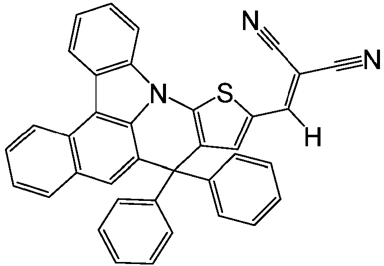 | 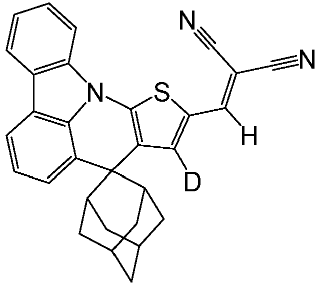 |
| 7 | 8 |
| 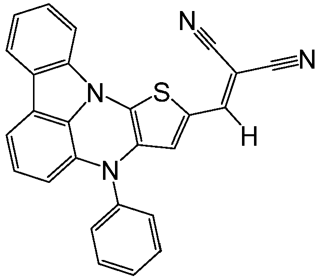 | 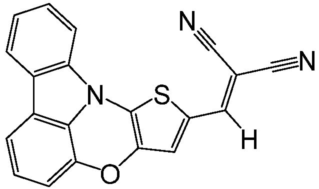 |
| 9 | 10 |
| 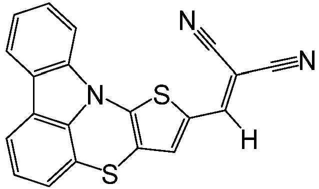 | 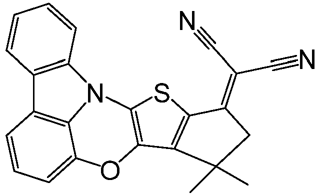 |
| 11 | 12 |
| 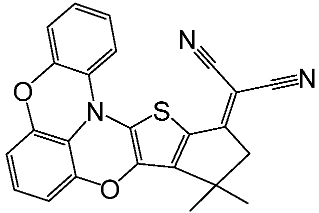 | 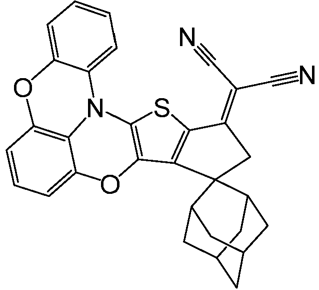 |
| 13 | 14 |
| 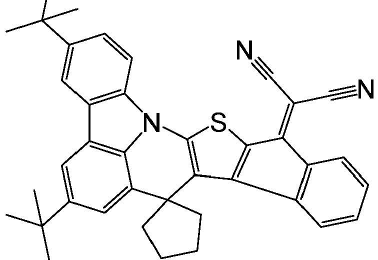 | 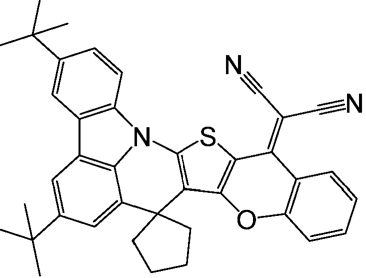 |
| 15 | 16 |
| 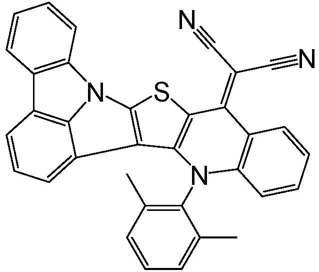 | 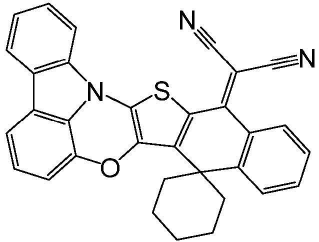 |
| 17 | 18 |
| 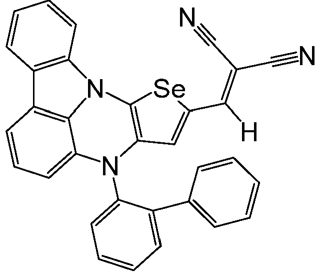 | 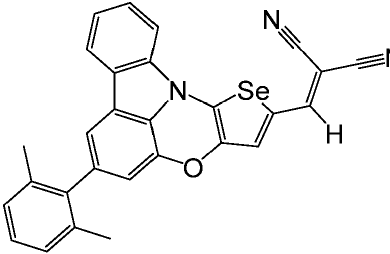 |
| 19 | 20 |
| 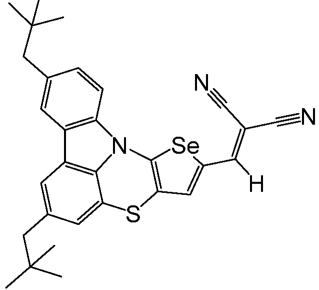 | 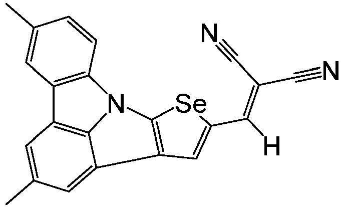 |
| 21 | 22 |
| 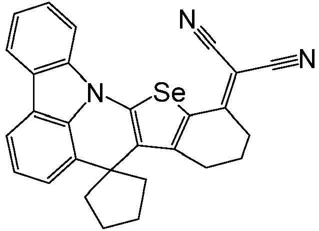 | 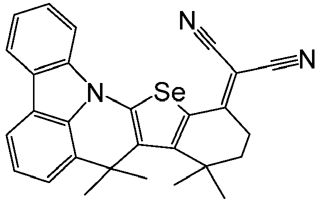 |
| 23 | 24 |
| 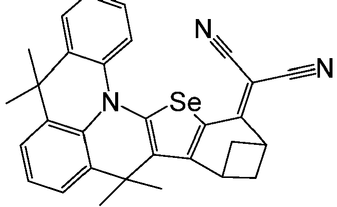 | 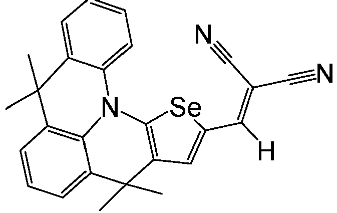 |
| 25 | 26 |
| 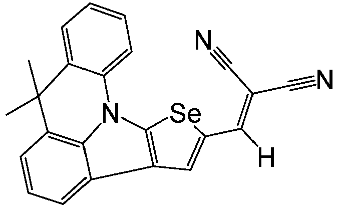 | 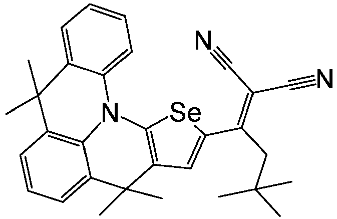 |
| 27 | 28 |
| 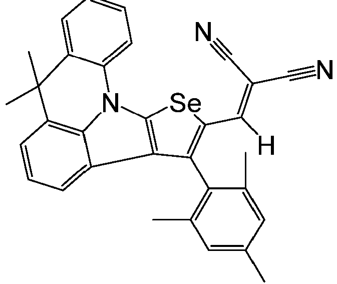 | 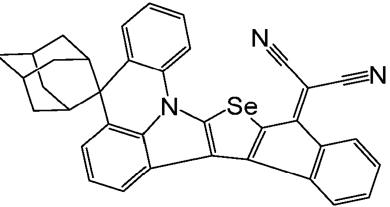 |
| 29 | 30 |
| 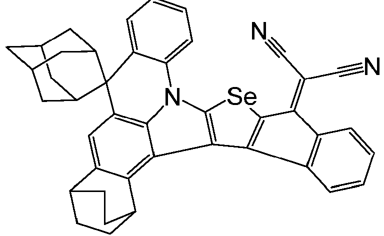 | 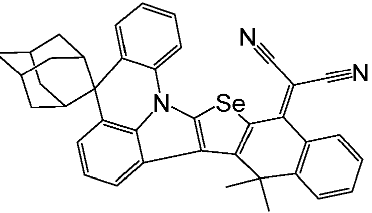 |
| 31 | 32 |
| 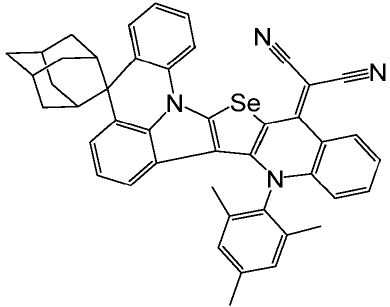 | 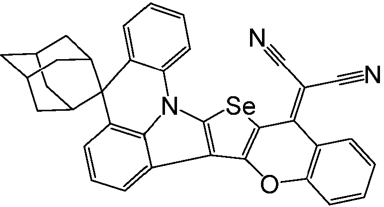 |
| 33 | 34 |
| 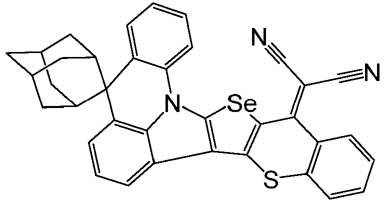 | 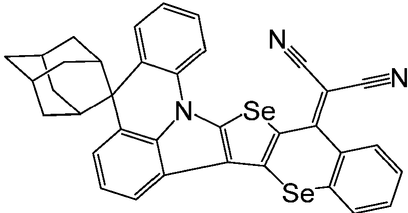 |
| 35 | 36 |
| 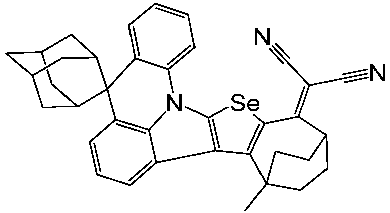 | 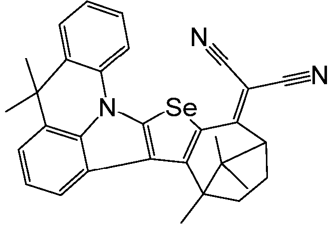 |
| 37 | 38 |
| 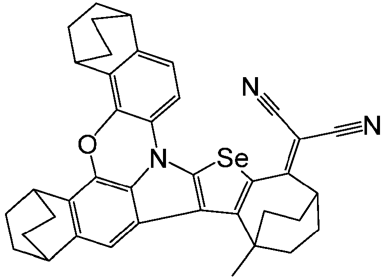 | 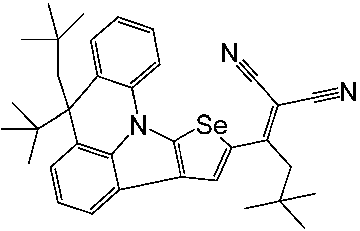 |
| 39 | 40 |
| 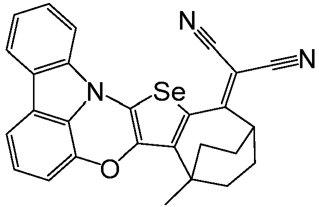 | 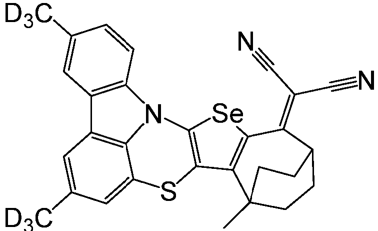 |
| 41 | 42 |
| 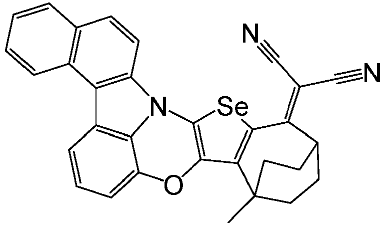 | 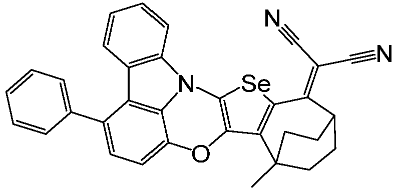 |
| 43 | 44 |
| 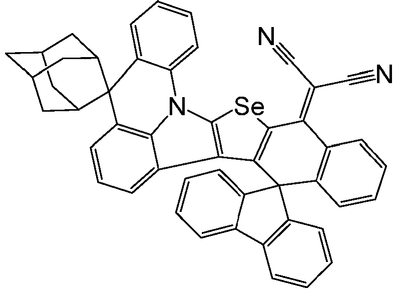 | 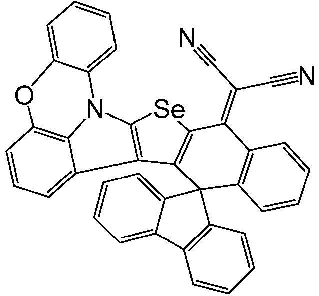 |
| 45 | 46 |
| 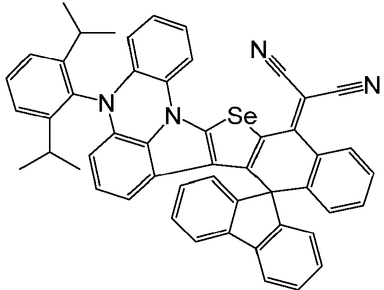 | 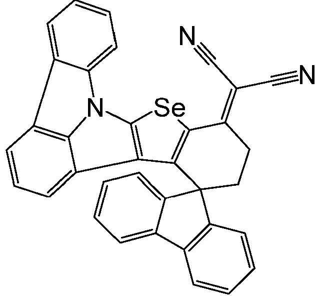 |
| 47 | 48 |
| 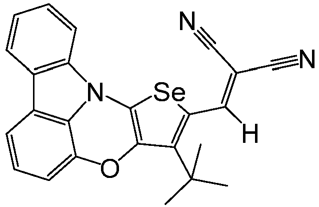 | 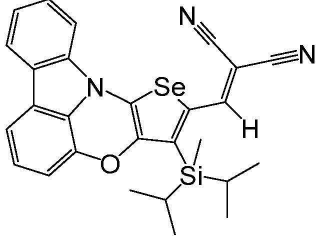 |
| 49 | 50 |
| 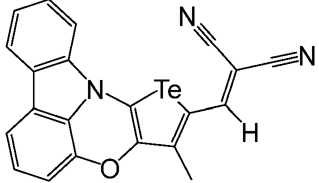 | 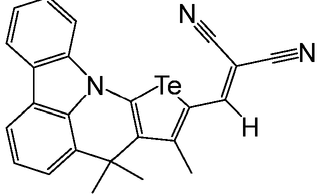 |
| 51 | 52 |
| 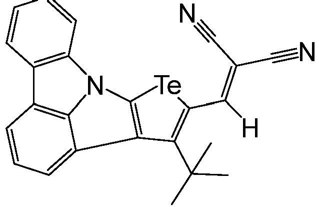 | 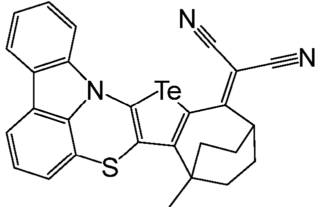 |
| 53 | 54 |
| 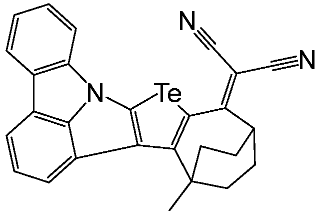 | 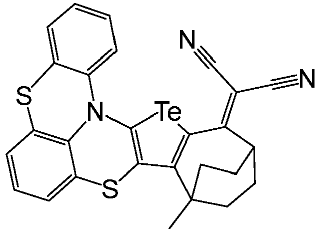 |
| 55 | 56 |
| 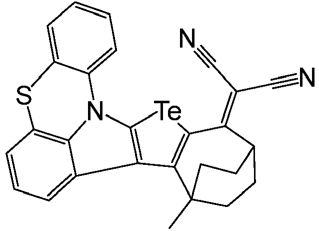 | 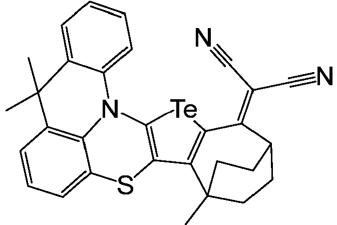 |
| 57 | 58 |
| 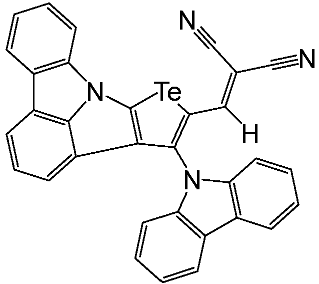 | 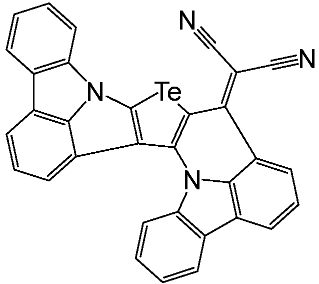 |
| 59 | 60 |
| 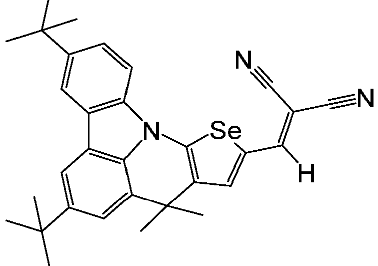 | 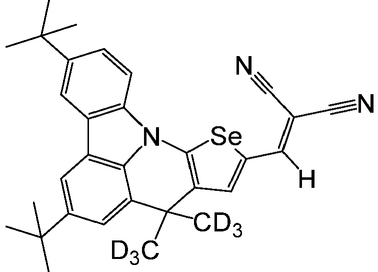 |
| 61 | 62 |
| 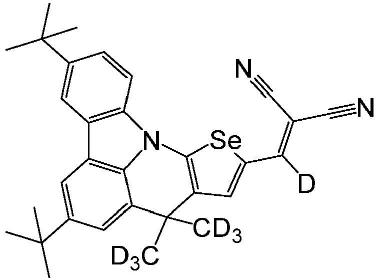 | 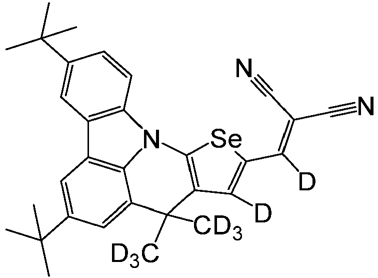 |
| 63 | 64 |
| 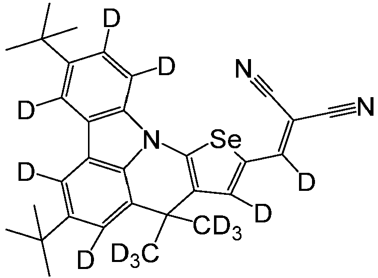 | 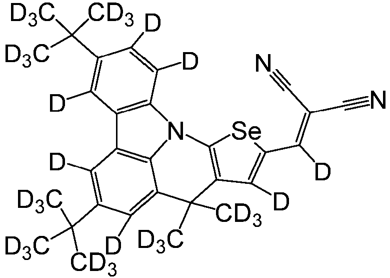 |
| 65 | 66 |
| 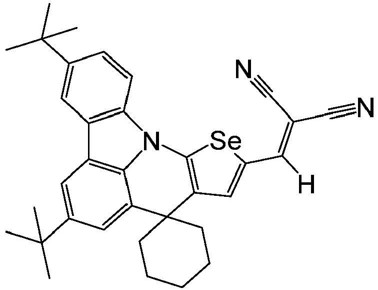 | 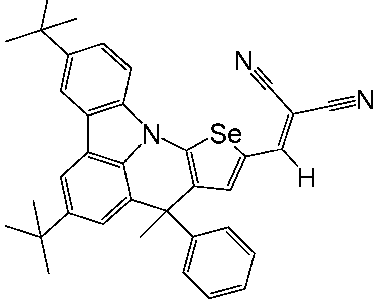 |
| 67 | 68 |
| 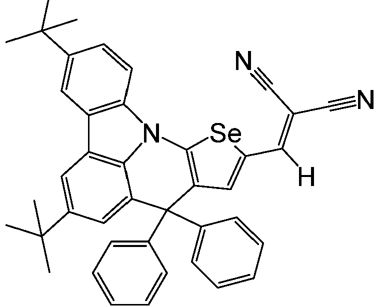 | 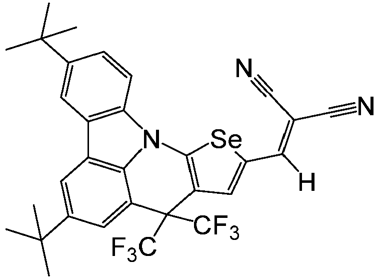 |
| 69 | 70 |
| 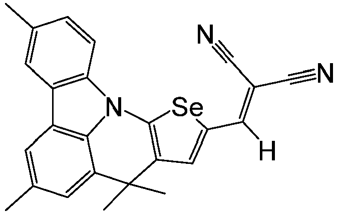 | 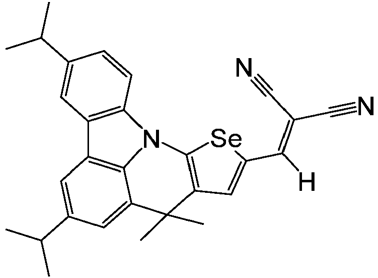 |
| 71 | 72 |
| 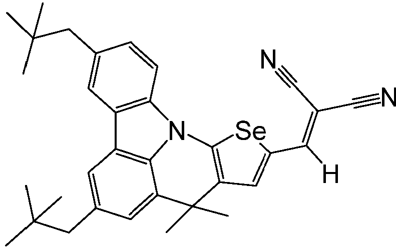 | 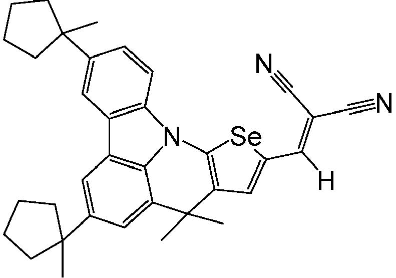 |
| 73 | 74 |
| 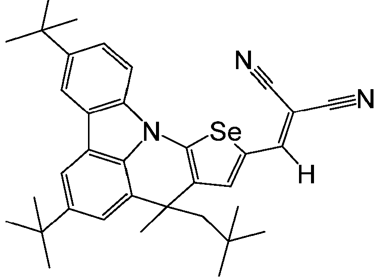 | 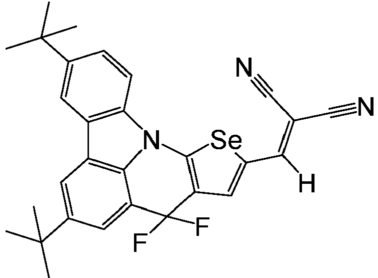 |
| 75 | 76 |
| 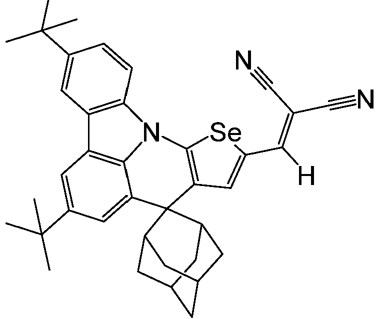 | 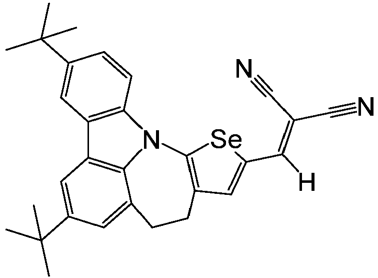 |
| 77 | 78 |
| 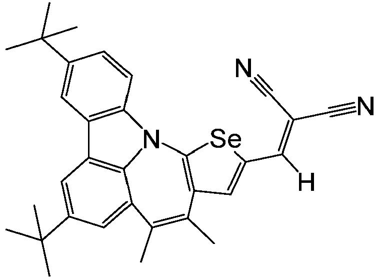 | 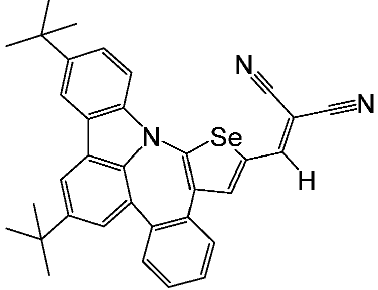 |
| 79 | 80 |
| 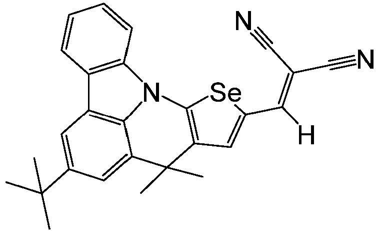 | 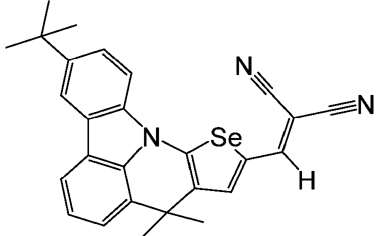 |
| 81 | 82 |
| 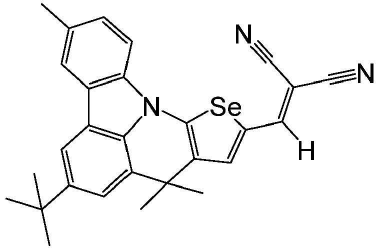 | 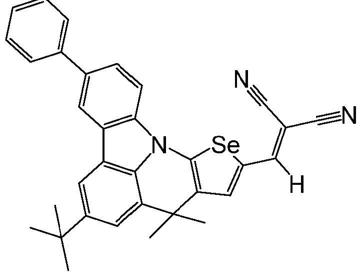 |
| 83 | 84 |
| 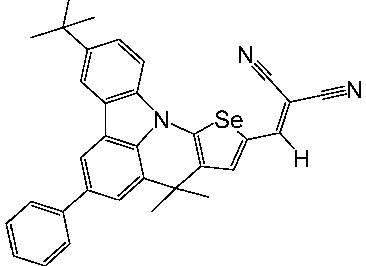 | 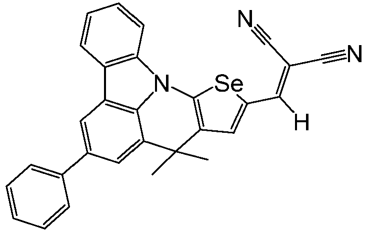 |
| 85 | 86 |
| 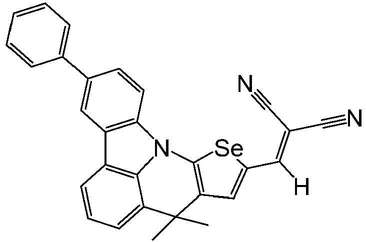 | 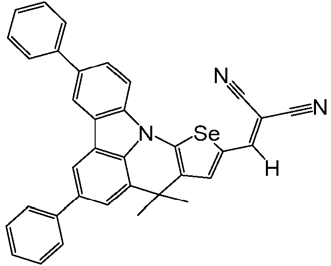 |
| 87 | 88 |
| 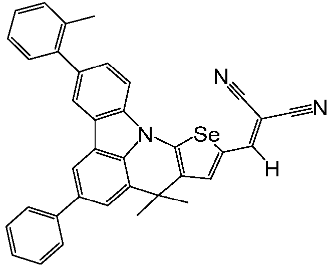 | 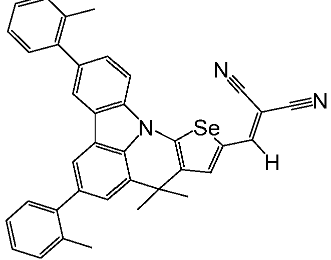 |
| 89 | 90 |
| 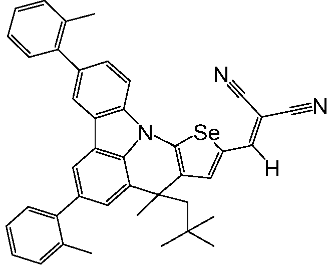 | 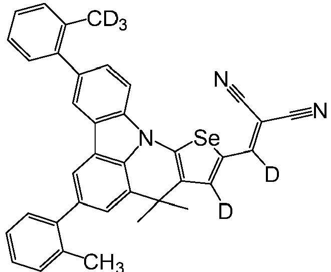 |
| 91 | 92 |
| 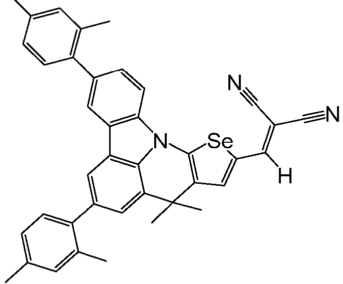 | 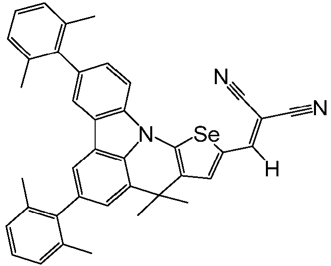 |
| 93 | 94 |
| 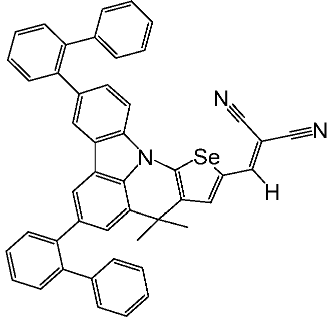 | 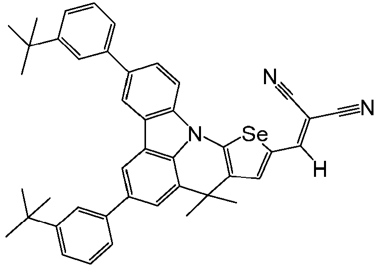 |
| 95 | 96 |
| 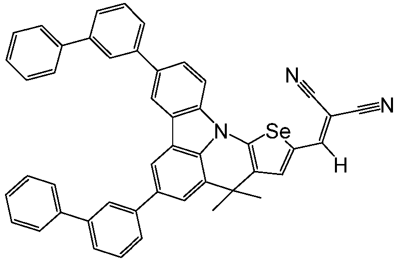 | 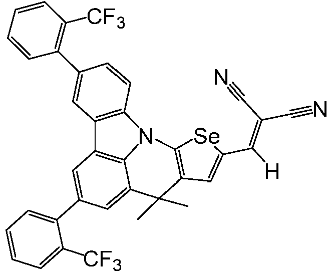 |
| 97 | 98 |
| 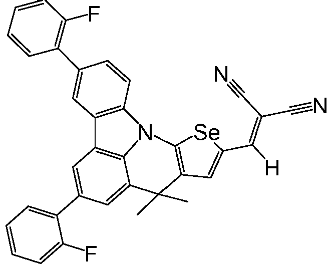 | 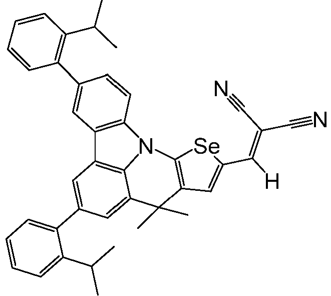 |
| 99 | 100 |
| 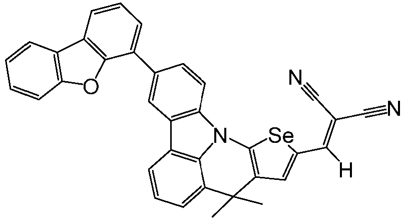 | 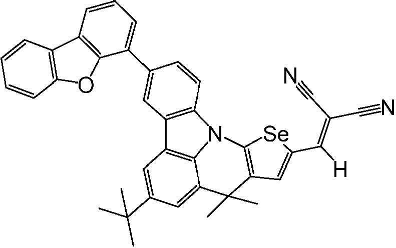 |
| 101 | 102 |
| 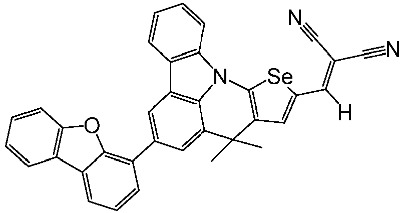 | 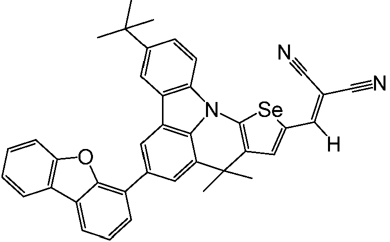 |
| 103 | 104 |
| 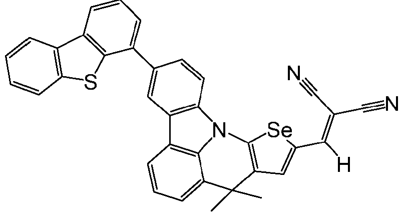 | 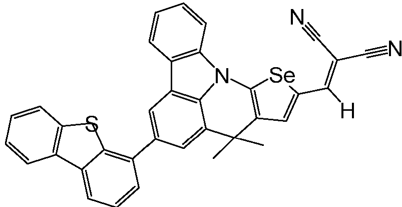 |
| 105 | 106 |
| 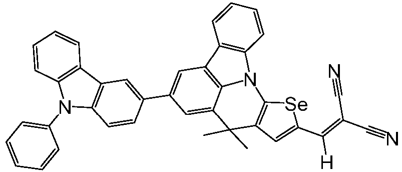 | 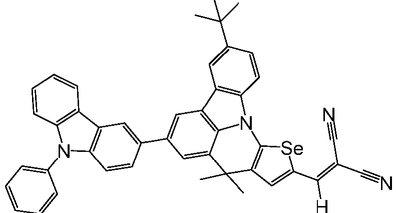 |
| 107 | 108 |
| 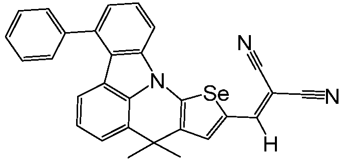 | 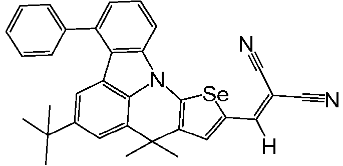 |
| 109 | 110 |
| 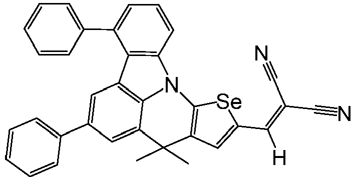 | 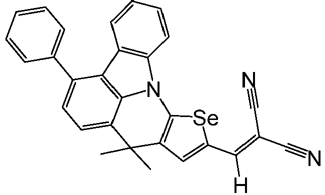 |
| 111 | 112 |
| 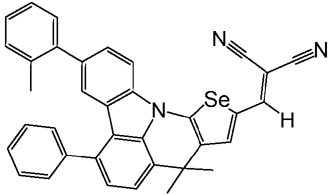 | 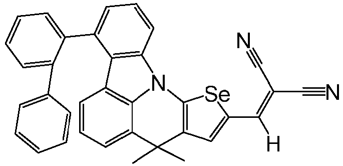 |
| 113 | 114 |
| 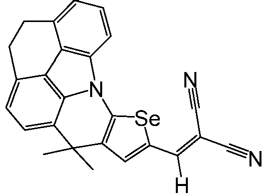 | 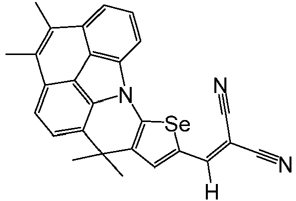 |
| 115 | 116 |
| 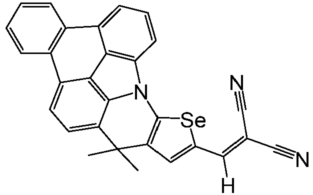 | 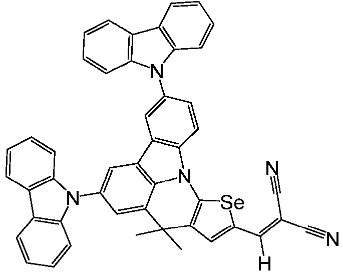 |
| 117 | 118 |
| 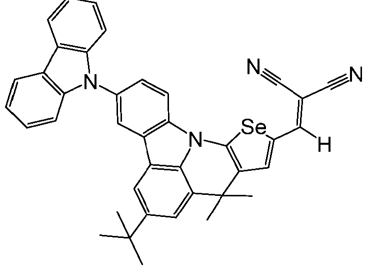 | 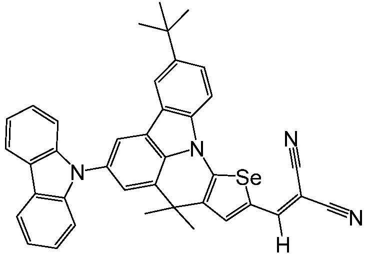 |
| 119 | 120 |
| 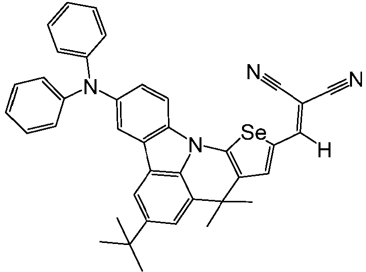 | 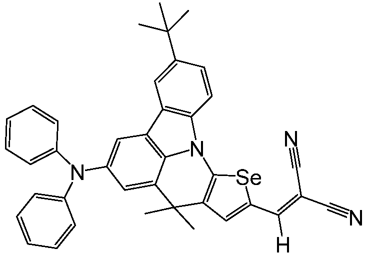 |
| 121 | 122 |
| 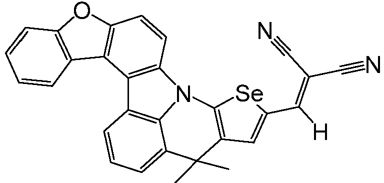 | 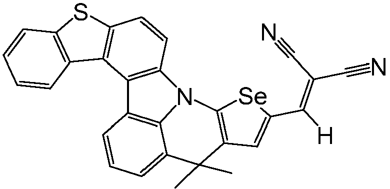 |
| 123 | 124 |
| 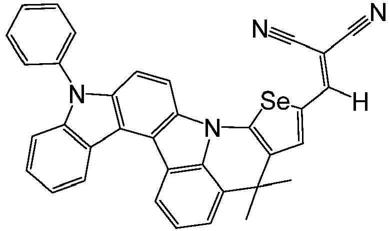 | 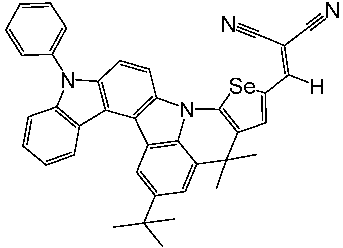 |
| 125 | 126 |
| 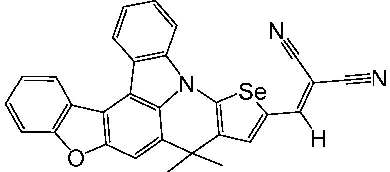 | 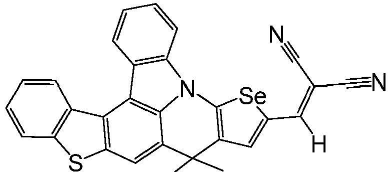 |
| 127 | 128 |
| 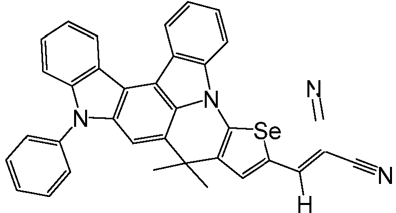 | 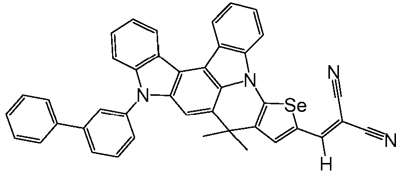 |
| 129 | 130 |
| 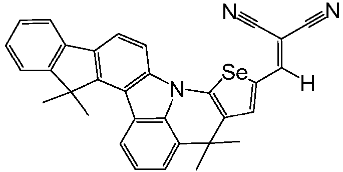 | 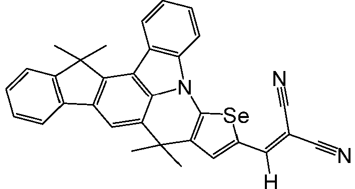 |
| 131 | 132 |
| 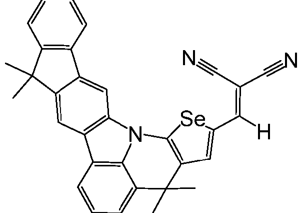 | 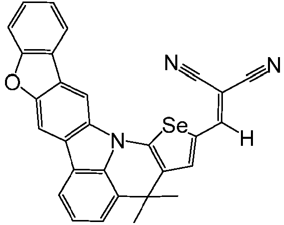 |
| 133 | 134 |
| 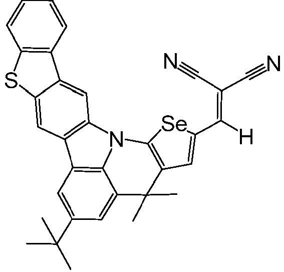 | 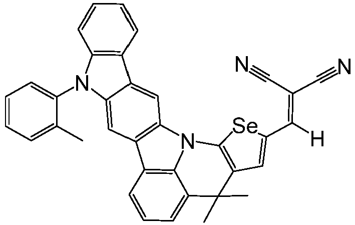 |
| 135 | 136 |
| 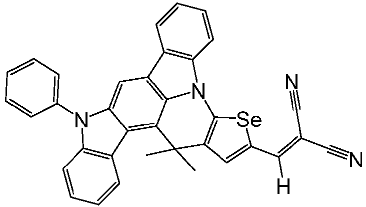 | 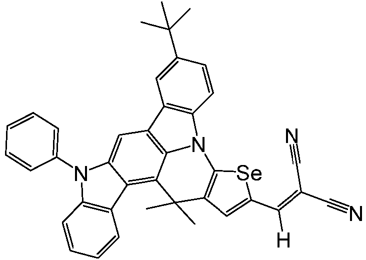 |
| 137 | 138 |
| 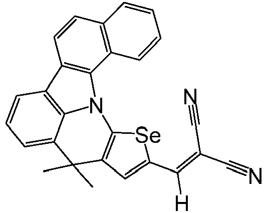 | 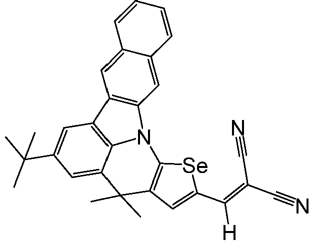 |
| 139 | 140 |
| 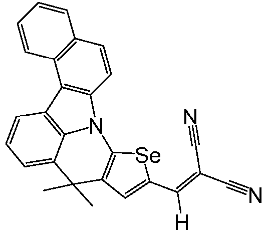 | 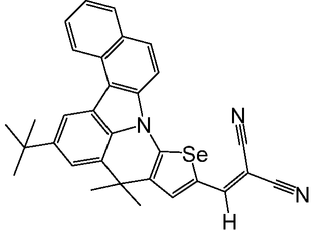 |
| 141 | 142 |
| 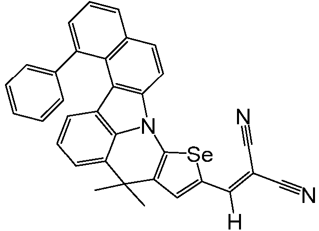 | 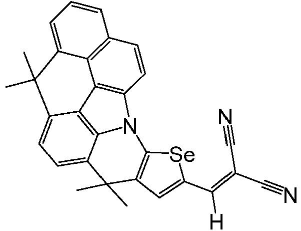 |
| 143 | 144 |
| 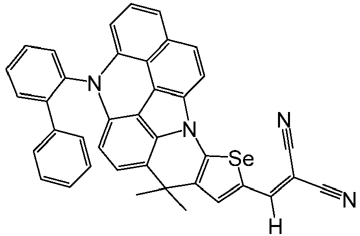 | 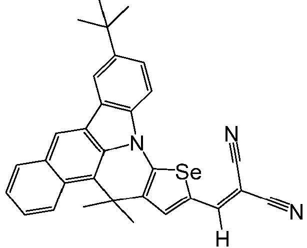 |
| 145 | 146 |
| 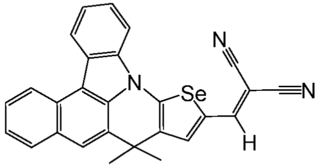 | 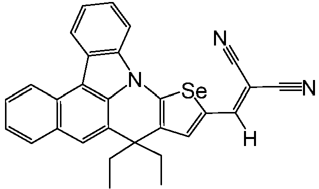 |
| 147 | 148 |
| 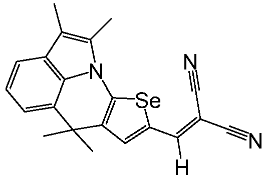 | 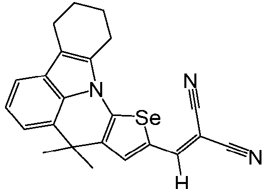 |
| 149 | 150 |
| 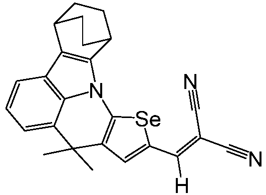 | 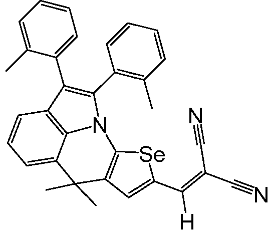 |
| 151 | 152 |
| 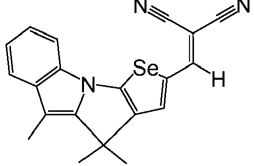 | 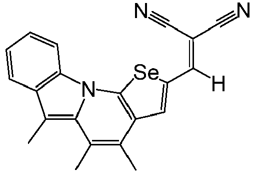 |
| 153 | 154 |
| 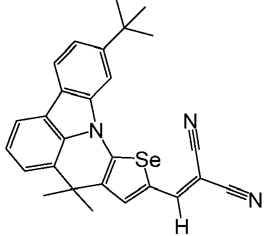 | 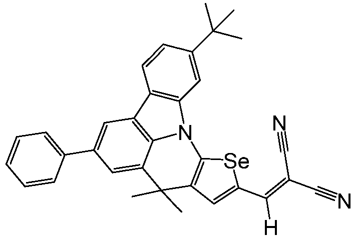 |
| 155 | 156 |
| 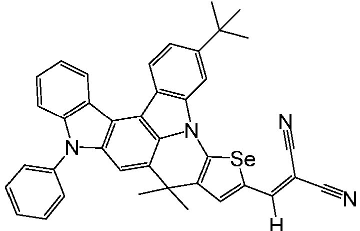 | 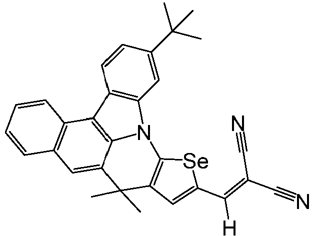 |
| 157 | 158 |
| 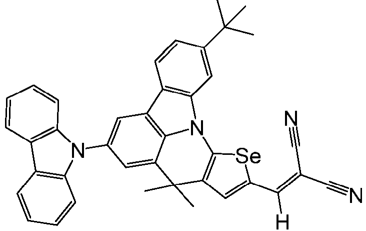 | 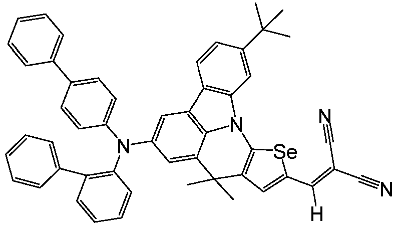 |
| 159 | 160 |
| 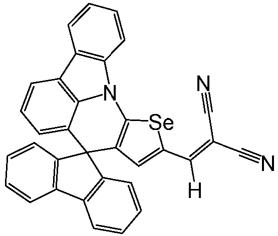 | 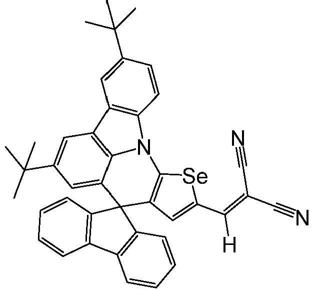 |
| 161 | 162 |
| 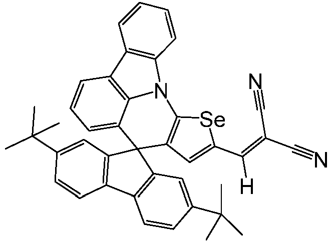 | 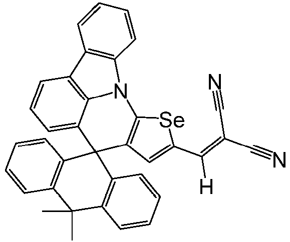 |
| 163 | 164 |
| 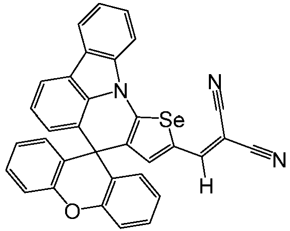 | 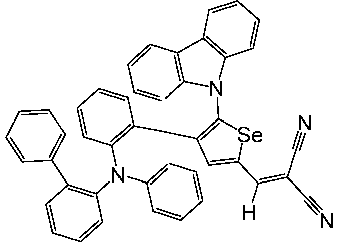 |
| 165 | 166 |
| 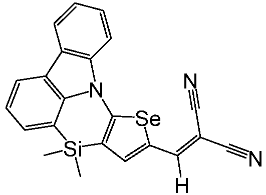 | 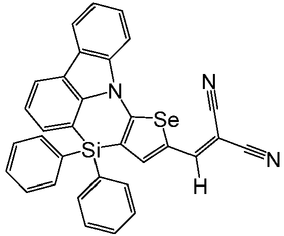 |
| 167 | 168 |
| 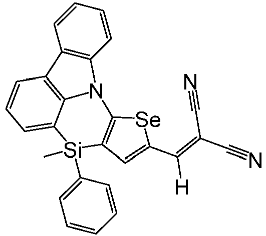 | 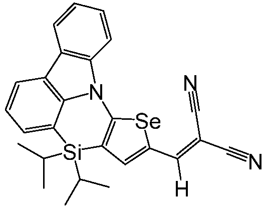 |
| 169 | 170 |
| 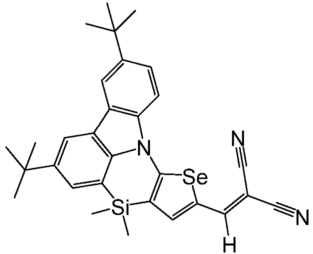 | 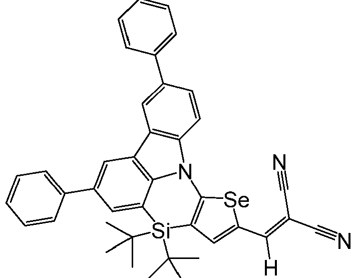 |
| 171 | 172 |
| 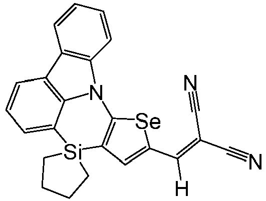 | 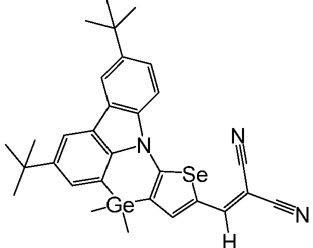 - |
| 173 | 174 |
| 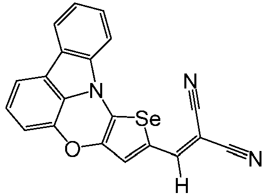 | 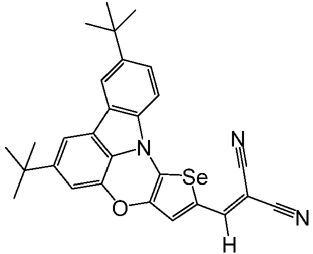 |
| 175 | 176 |
| 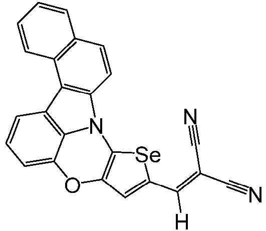 | 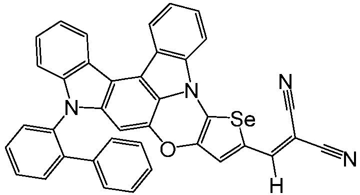 |
| 177 | 178 |
| 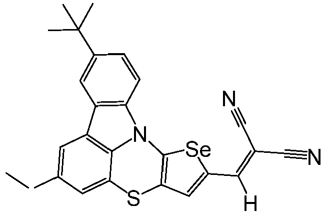 | 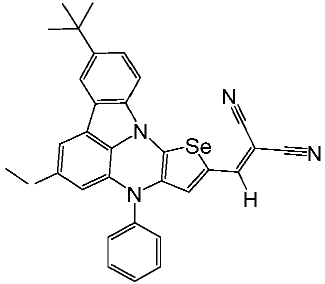 |
| 179 | 180 |
| 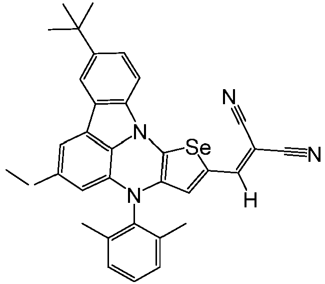 | 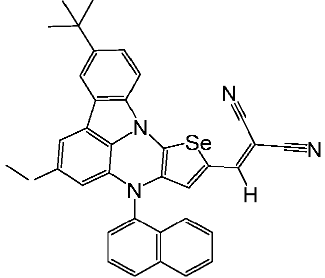 |
| 181 | 182 |
| 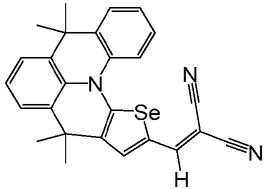 | 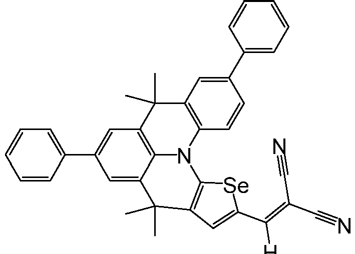 |
| 183 | 184 |
| 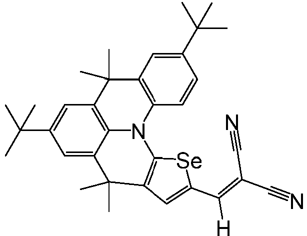 | 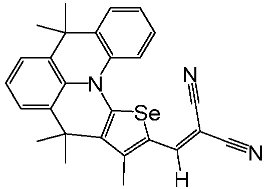 |
| 185 | 186 |
| 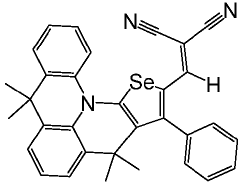 | 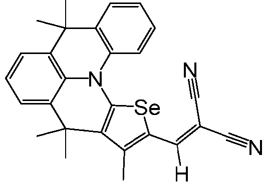 |
| 187 | 188 |
| 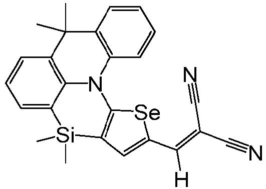 | 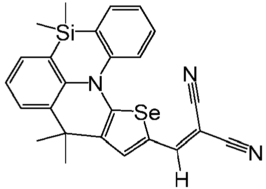 |
| 189 | |
| 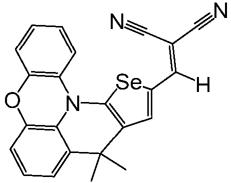 | 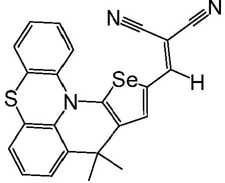 |
| 191 | 192 |
| 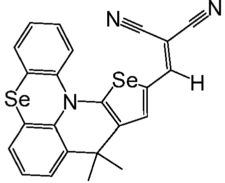 | 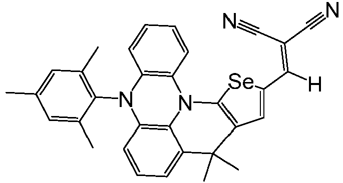 |
| 193 | 194 |
| 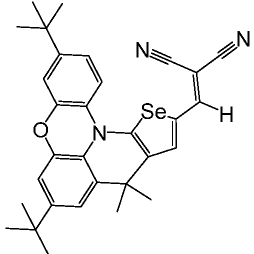 | 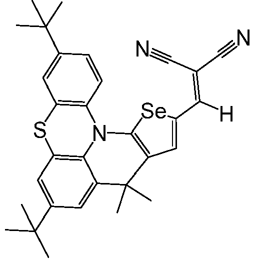 |
| 195 | 196 |
| 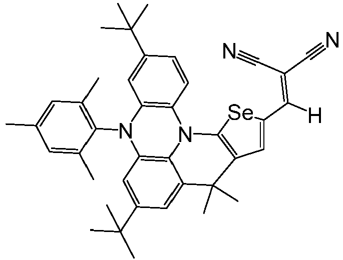 | 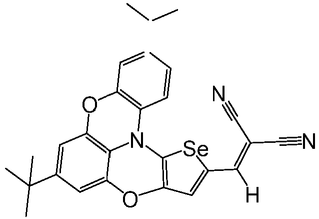 |
| 197 | 198 |
| 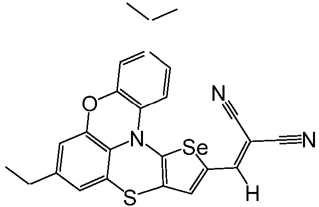 | 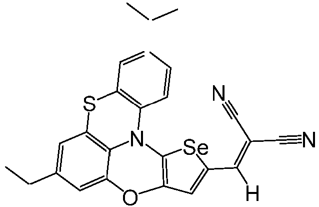 |
| 199 | 200 |
| 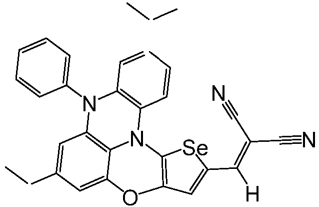 | 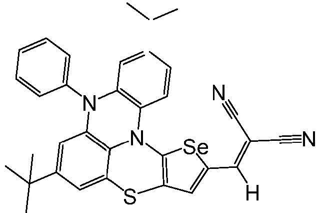 |
| 201 | 202 |
| 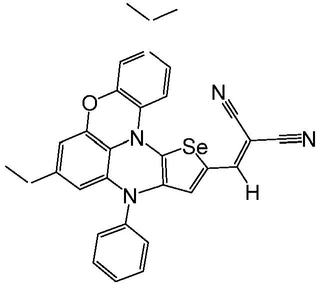 | 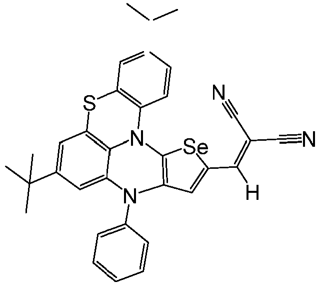 |
| 203 | 204 |
| 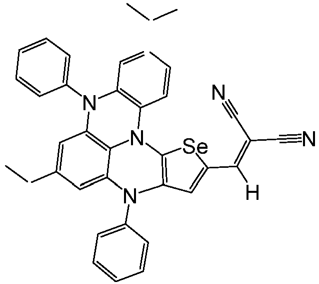 | 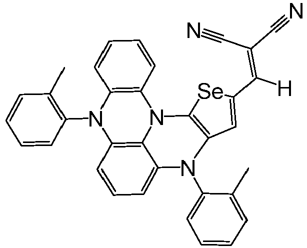 |
| 205 | 206 |
| 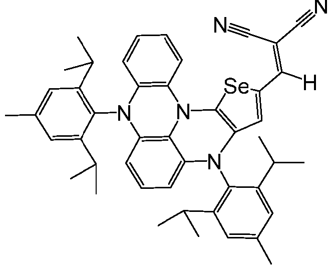 | 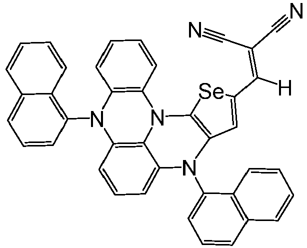 |
| 207 | 208 |
| 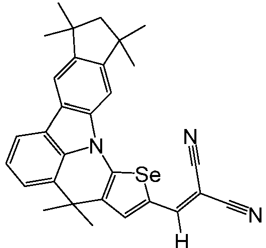 | 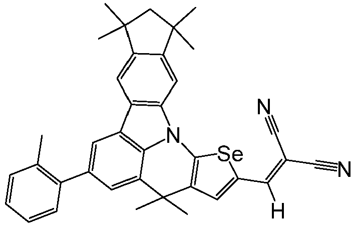 |
| 209 | 210 |
| 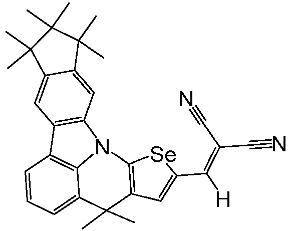 | 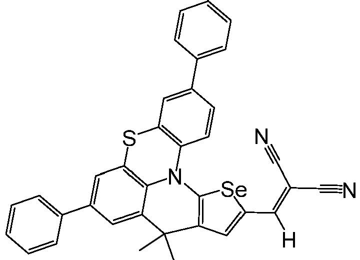 |
| 211 | 212 |
| 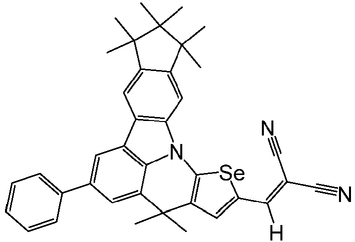 | 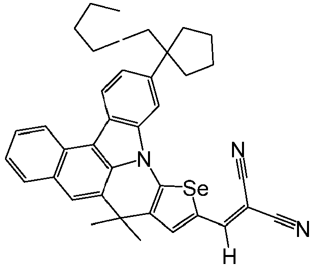 |
| 213 | 214 |
| 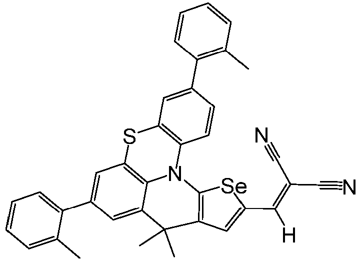 | 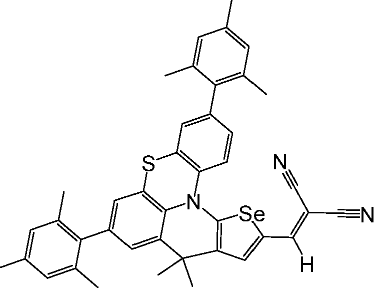 |
| 215 | 216 |
| 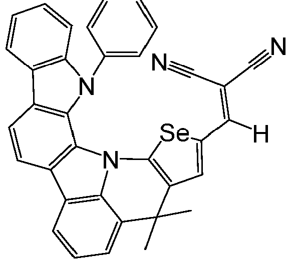 | 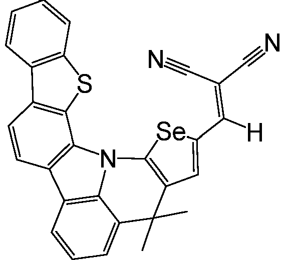 |
| 217 | 218 |
| 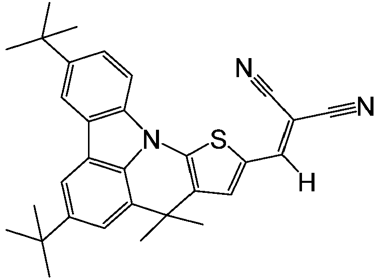 | 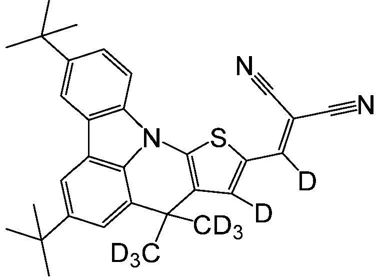 |
| 219 | 220 |
| 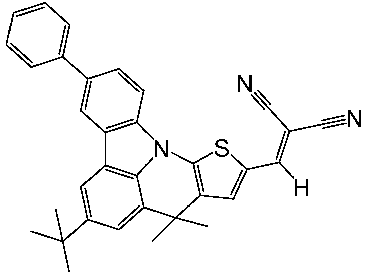 | 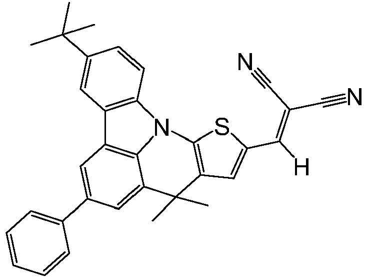 |
| 221 | 222 |
| 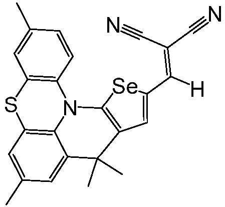 | 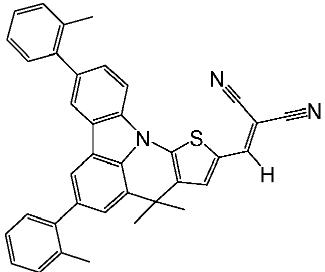 |
| 223 | 224 |
| 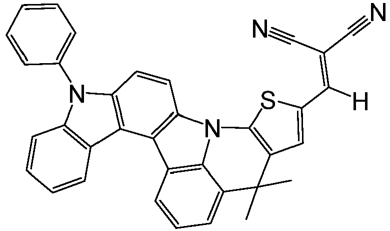 | 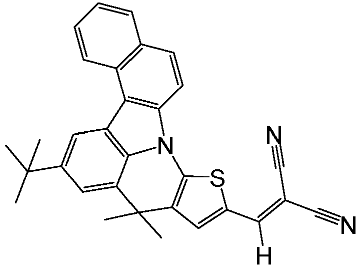 |
| 225 | 226 |
| 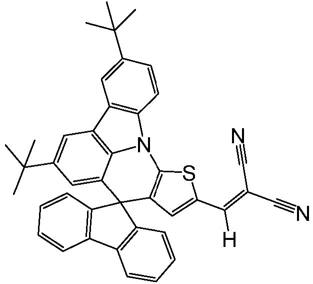 | 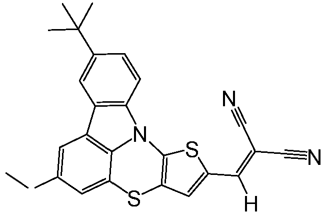 |
| 227 | 228 |
| 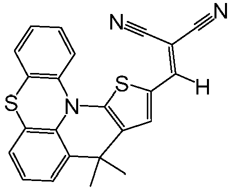 | 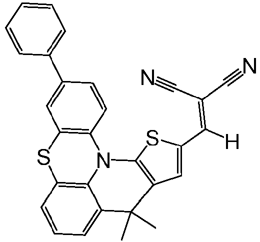 |
| 229 | 230 |
| 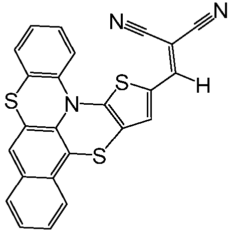 | 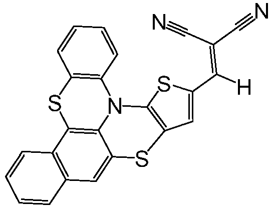 |
| 231 | 232 |
| 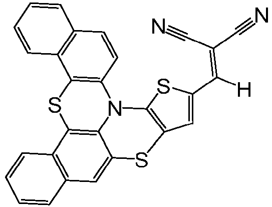 | 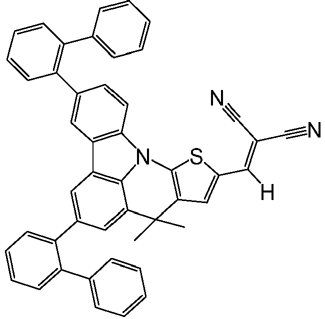 |
| 233 | 234 |
| 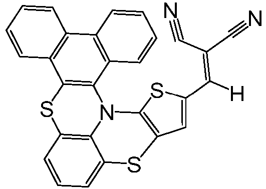 | 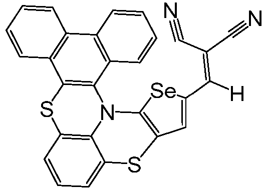 |
| 235 | 236 |
| 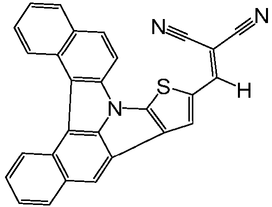 | 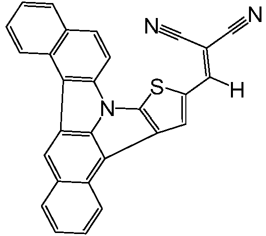 |
| 237 | 238 |
| 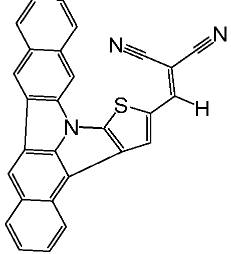 | 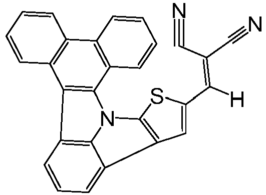 |
| 239 | 240 |
| 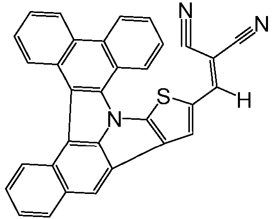 | 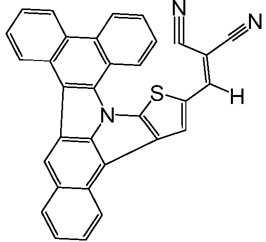 |
| 241 | 242 |
| 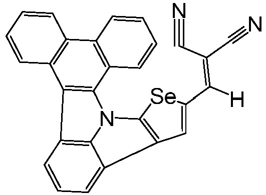 | 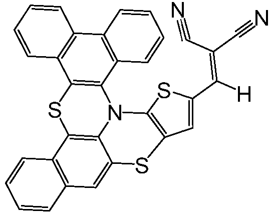 |
| 243 | 244 |
| 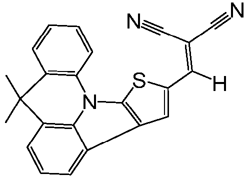 | 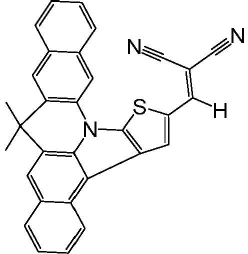 |
| 245 | 246 |
| 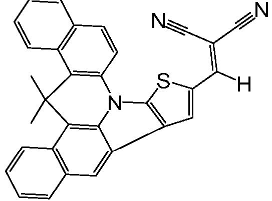 | 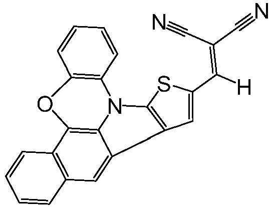 |
| 247 | 248 |
| 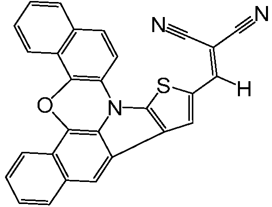 | 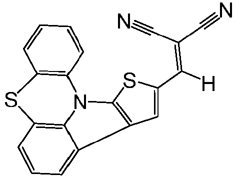 |
| 249 | 250 |
| 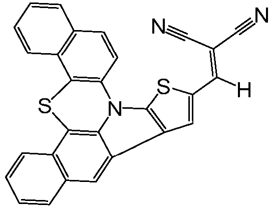 | 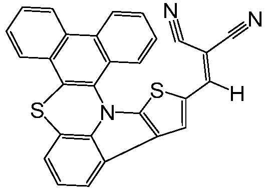 |
| 251 | 252 |
| 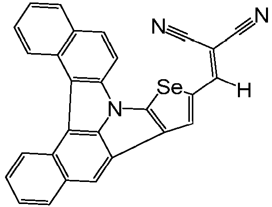 | 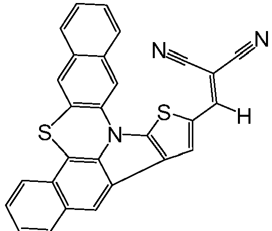 |
| 254 | 255 |
| 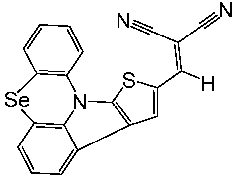 | 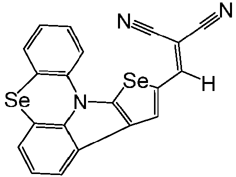 |
| 256 | 257 |
| 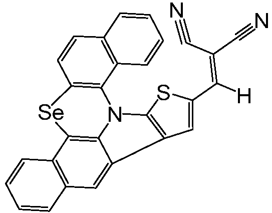 | 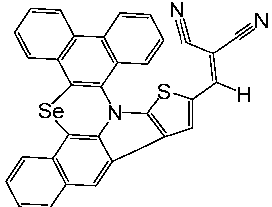 |
| 258 | 259 |
| 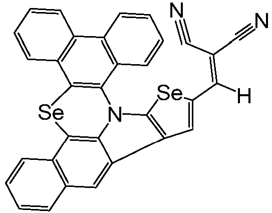 | 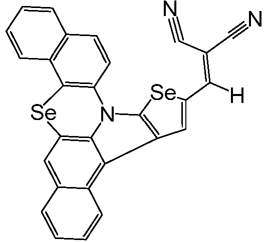 |
| 260 | 261 |
| 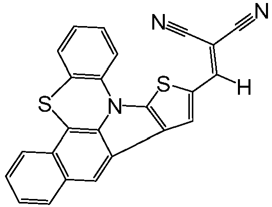 | 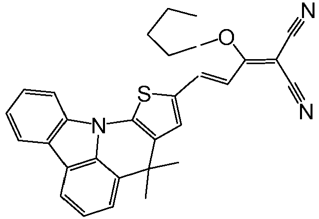 |
| 262 | 263 |
| 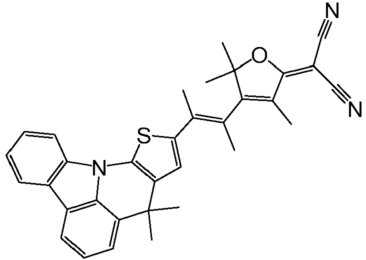 | 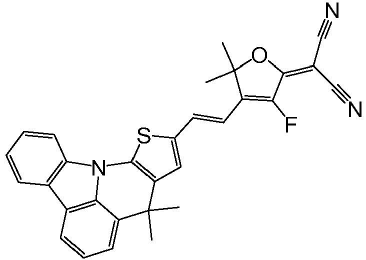 |
| 264 | 265 |
| 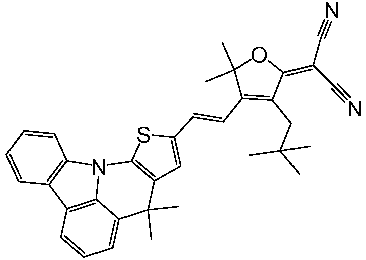 | 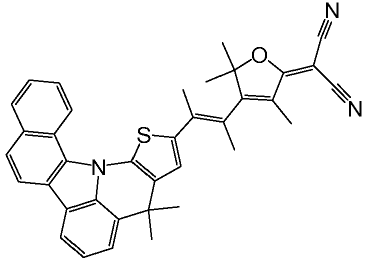 |
| 266 | 267 |
| 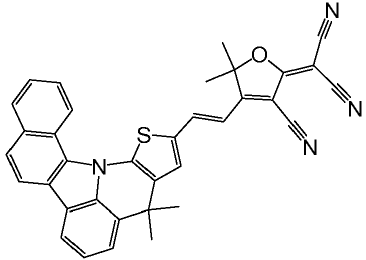 | 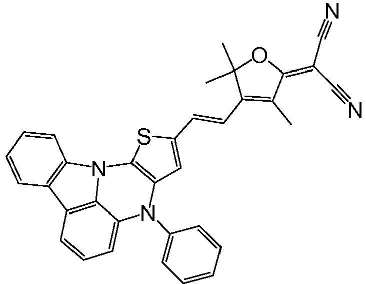 |
| 268 | 269 |
| 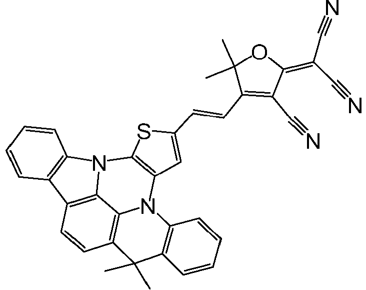 | 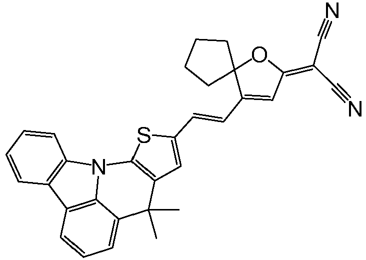 |
| 270 | 271 |
| 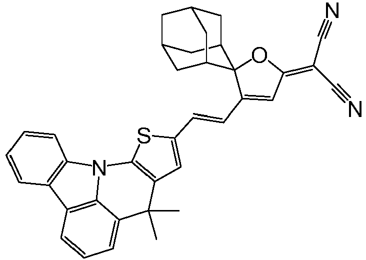 | 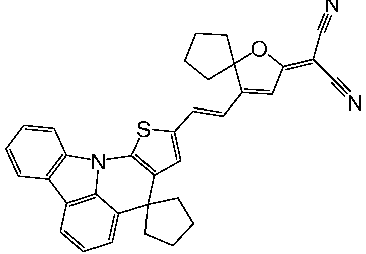 |
| 272 | 273 |
| 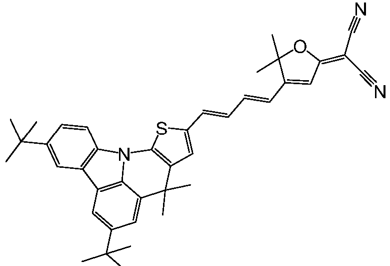 | 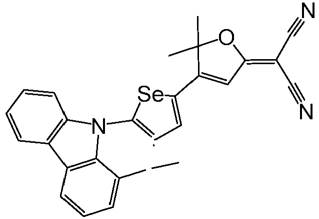 |
| 274 | 275 |
| 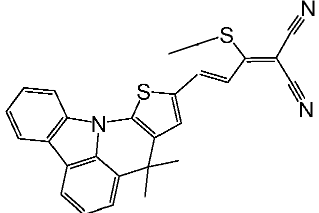 | 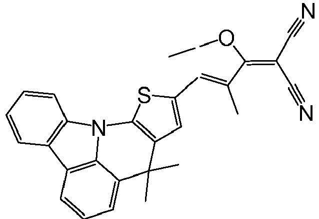 |
| 276 | 277 |
| 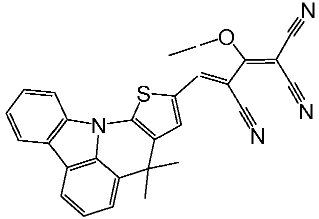 | 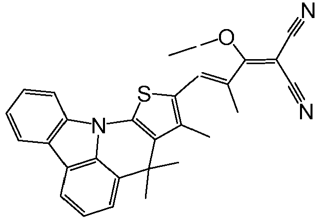 |
| 278 | 279 |
| 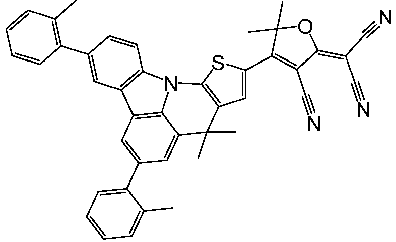 | 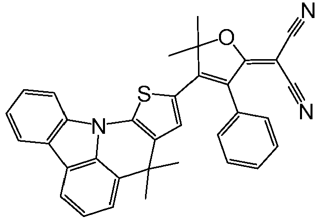 |
| 280 | 281 |
| 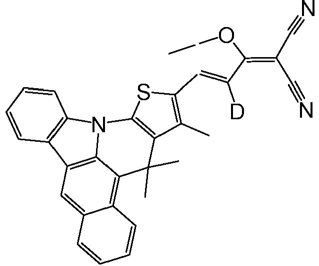 | |
| 282 | |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem ein Grundgerüst mit einer aromatischen Aminogruppe synthetisiert wird und mindestens ein heterocyclischer Rest eingeführt wird, vorzugsweise mittels einer nukleophilen aromatischen Substitutionsreaktion oder einer Kupplungsreaktion.

Geeignete Verbindungen, umfassend ein Grundgerüst mit einer aromatischen Aminogruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, ULLMANN, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Synthese der erfindungsgemäßen Verbindungen kann unter anderem gemäß dem nachfolgenden Schema 1 erfolgen.

In einem ersten Schritt kann eine Verbindung mit einer aromatischen Aminogruppe (1) mit einem Heterocyclus (2) umgesetzt werden. Bevorzugte Heterocyclen sind beispielsweise Pyrrol-, Furan-, Thiophen-, Selenophen- oder Tellurophen-Derivate. Die erhaltene Verbindung (3) kann in einer Hydroxyalkylierungsreaktion mit einer Carbonylverbindung (4) zu einer stickstoffhaltigen Hydroxyverbindung (5) umgesetzt werden, die im vorliegenden Schema als Schritt 2 dargestellt ist. In Schritt 3 wird diese Hydroxyverbindung (5) cyclisiert und nachfolgend durch eine Formylierung bzw. Acylierung in eine Carbonylverbindung (8) derivatisiert. In Schritt 5 kann die Carbonylverbindung (8) in einer Knoevenagel-Kondensation mit Malonsäuredinitril (9) zu einer erfindungsgemäßen Verbindung (10) umgesetzt werden.

Die Knoevenagel-Kondensation wird unter anderem in S. Haig et al., Chem. Mat., 2011, 23(20), 4435 dargelegt.

### Schema 1:

### Schritt 1: Ullmann-Kupplung

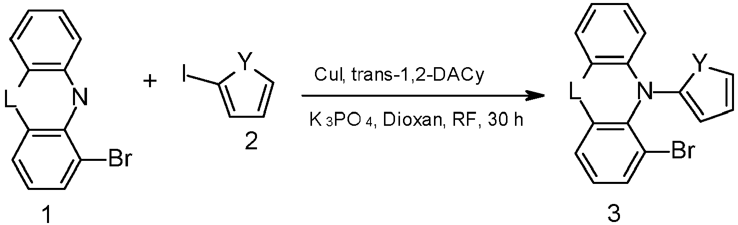

### Schritt 2: Hydroxyalkylierung

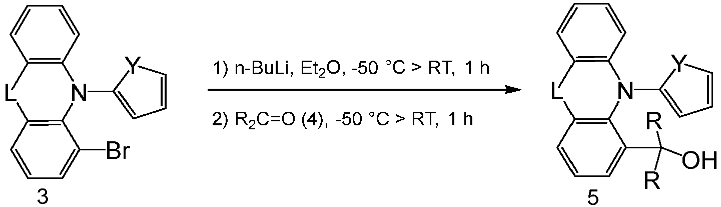

### Schritt 3: Cyclisierung

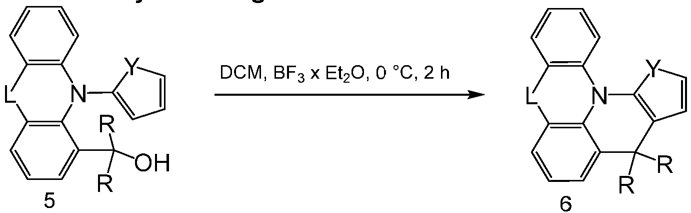

### Schritt 4: Formylierung bzw. Acylierung

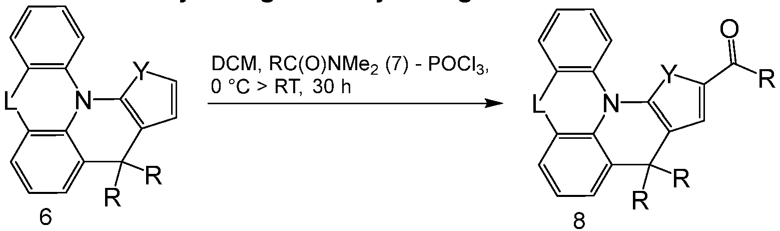

### Schritt 5: Knoevenagel-Kondensation

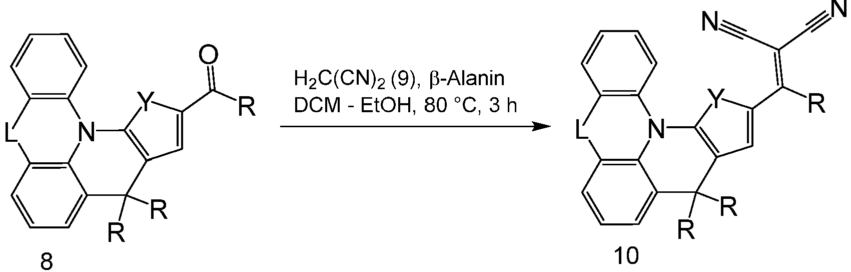

Die Synthese der erfindungsgemäßen Verbindungen kann weiterin gemäß dem nachfolgenden Schema 2 erfolgen, durch welches insbesondere Verbindungen mit weiteren Gruppen Y² erhalten werden können, wie beispielsweise O, S, Se, Te, NAr, NR^{e}, BAr, BR^{e}, Ge(R^{e})₂, Si(R^{e})₂, -CR^{e}=CR^{e}-, C=NR^{e}, S=O, SO₂.

In einem ersten Schritt kann eine Verbindung mit einer aromatischen Aminogruppe (11) mit einem Heterocyclus (12) durch doppelte Ullmann-Kupplung umgesetzt werden. Bevorzugte Heterocyclen sind beispielsweise Pyrrol-, Furan-, Thiophen-, Selenophen- oder Tellurophen-Derivate. Die erhaltene Verbindung (13) kann durch eine Formylierung bzw. Acylierung in eine Carbonylverbindung (15) derivatisiert werden. In Schritt 3 kann die Carbonylverbindung (15) in einer Knoevenagel-Kondensation mit Malonsäuredinitril (16) zu einer erfindungsgemäßen Verbindung (17) umgesetzt werden.

Vorzugsweise können als aromatischen Aminogruppe (11) sekundäre Amine und als Heterocyclen (12) literaturbekannte 1,2-Bis-Halogenfunktionalisierte 5-Ring-Heterocyclen eingesetzt werden.

### Schema 2:

### Schritt 1: Ullmann-Kupplung

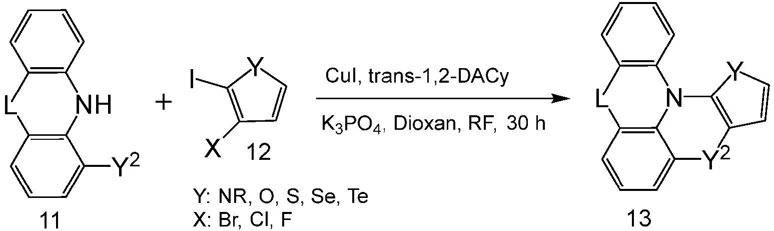

Y²: NHR, OH, SH, SeH

### Schritt 2: Formylierung bzw. Acylierung

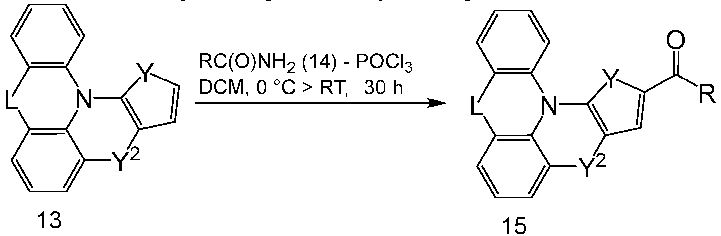

### Schritt 3: Knoevenagel-Kondensation

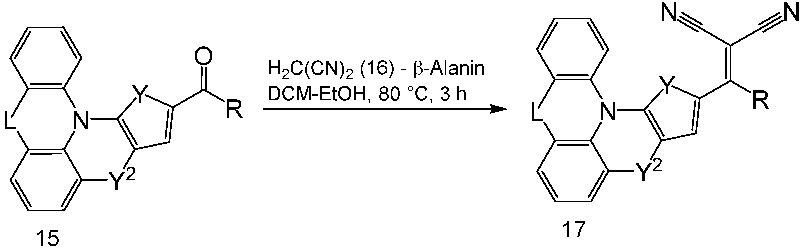

Abwandlungen des zuvor dargelegten Schemas zur Herstellung von Verbindungen/Strukturen, bei denen die Gruppe Y² beispielsweise für Te, BR^{e}, Ge(R^{e})₂, Si(R^{e})₂, -CR^{e}=CR^{e}-, C=NR^{e}, S=O, SO₂ steht, sind dem Fachmann bekannt.

Die Synthese der erfindungsgemäßen Verbindungen kann darüber hinaus gemäß dem nachfolgenden Schema 3 erfolgen, durch welches insbesondere Verbindungen mit weiteren Gruppen Y² erhalten werden können, wie beispielsweise eine Bindung.

Ausgehend von (18) und (19) können nach D. Bader et al., J. Org. Chem., 2020, 85(5), 3865 die Intermediate (20) erhalten werden. Die Intermediate (20) können dann mittels Schritt 2 und 3 zu den entsprechenden erfindungsgemäßen Verbindungen (22) umgesetzt werden.

### Schema 3:

### Schritt 1: Ullmann-Kupplung

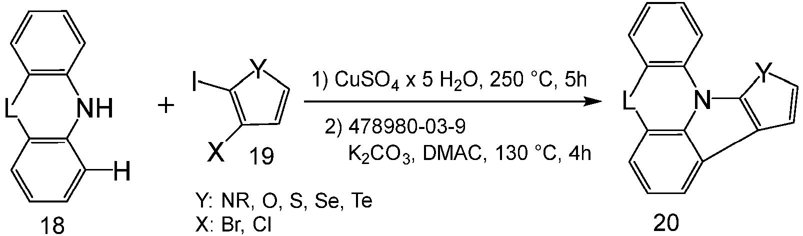

Y²: NHR, OH, SH, SeH

### Analog Schritt 2: Formylierung bzw. Acylierung

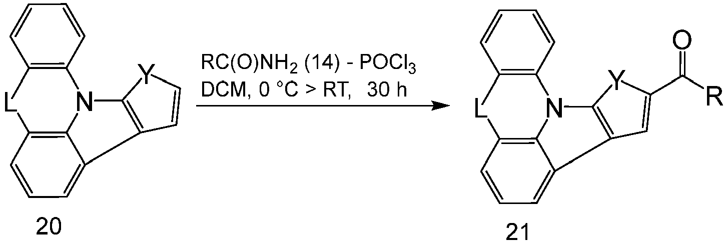

### Analog Schritt 3: Knoevenagel-Kondensation

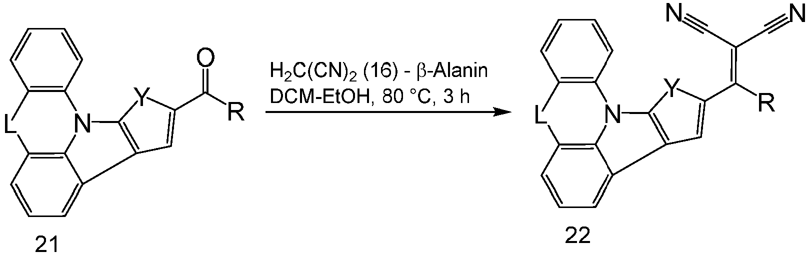

Zur Einführung der Gruppen der Formel (Z-2) mit p = 1 kann die Knoevenagel-Kondensation anstelle von Maleodinitril (H₂C(CN)₂) mit 2-(4,5,5-Trimethyl-2(5H)-furanyliden)-propandinitril bzw. 2-(4,5,5-Trimethyl-2(5H)-thiophenyliden)-propandinitril und deren Derivaten durchgeführt werden.

Die Bedeutung der in Schemata 1, 2 und 3 verwendeten Symbole entspricht im Wesentlichen denen, die für Formel (I) beziehungsweise bevorzugte Ausführungsformen dieser Strukturen definiert wurde, wobei aus Gründen der Übersichtlichkeit auf eine Nummerierung sowie auf eine vollständige Darstellung aller Symbole verzichtet wurde. Darüber hinaus wurde aus Gründen der Übersichtlichkeit vielfach auf die Verwendung von Symbolen zur Darstellung möglicher Stickstoffatome in den heteroaromatischen Ringen verzichtet, wie diese insbesondere in Formel (III) durch die Symbole X^{a} und X^{b} dargelegt sind. Diese Angaben sind daher beispielhaft zu verstehen, wobei der Fachmann in der Lage ist, die zuvor und nachfolgend, insbesondere in den Beispielen dargelegten Synthesen auf Verbindungen zu übertragen, bei denen ein oder mehrere der Symbole Symbole X^{a} und X^{b} für Stickstoff stehen oder die statt den Benzolgruppen im Grundgerüst andere aromatische Gruppe, wie beispielsweise Naphthalin oder Phenanthren enthalten.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels ¹H-NMR und/oder HPLC) erhalten.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, lod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) und bevorzugten Ausführungsformen dieser Formel oder erfindungsgemäße Verbindungen, wobei ein oder mehrere Bindungen der erfindungsgemäßen Verbindungen oder der Strukturen der Formel (I) und bevorzugten Ausführungsformen dieser Formel zum Polymer, Oligomer oder Dendrimer vorhanden sind. Je nach Verknüpfung der Strukturen der Formel (I) und bevorzugten Ausführungsformen dieser Formel bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß der Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise ein Comaterial, wobei sich diese Verbindungen von den erfindungsgemäßen Verbindungen unterscheiden. Geeignete Comaterialien sind hinten im Zusammenhang mit der organischen elektronischen Vorrichtung aufgeführt. Die weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus Photosensitizern, Elektronentransportmaterialien, Elektroneninjektionsmaterialien, Lochleitermaterialien, Lochinjektionsmaterialien, Elektronenblockiermaterialien und Lochblockiermaterialien, vorzugsweise Photosensitizern, Elektronentransportmaterialien, Elektroneninjektionsmaterialien, Lochblockiermaterialien.

Ein weiterer Gegenstand der vorliegenden Erfindung ist die Verwendung einer erfindungsgemäßen Verbindung in einer elektronischen Vorrichtung, vorzugsweise einer organischen, photoelektrischen Vorrichtung, insbesondere in einem organischen optischen Detektor, vorzugsweise als Photosensitizer, besonders bevorzugt als grüner, roter, infraroter oder blauer Photosensitizer, speziell bevorzugt als grüner Photosensitizer.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus Besonders bevorzugt ist elektronische Vorrichtung ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen optischen Detektoren, organischen Photorezeptoren, organischen elektrischen Sensoren, organischen Elektrolumineszenzvorrichtungen (OLEDs, sOLED, PLEDs, LECs, etc.), besonders bevorzugt organischen optischen Detektoren.

Der organische optische Detektor enthält Kathode, Anode und mindestens eine lichtabsorbierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei lichtabsorbierenden Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann der organische optische Detektor eine lichtabsorbierende Schicht enthalten, oder er kann mehrere lichtabsorbierende Schichten enthalten.

Die erfindungsgemäße Verbindung kann dabei in unterschiedlichen Schichten eingesetzt werden, je nach genauer Struktur. Bevorzugt ist ein organischer optischer Detektor, enthaltend eine Verbindung gemäß Formel (I) bzw. die oben ausgeführten bevorzugten Ausführungsformen in einer lichtabsorbierenden Schicht als Photosensitizer, vorzugsweise infraroter, roter, grüner oder blauer Photosensitizer, besonders bevorzugt als grüner Photosensitizer, wobei die Farbe jeweils die Farbe des Lichtes angibt, die von dem Photosensitizer absorbiert wird.

Wenn die erfindungsgemäße Verbindung als Photosensitizer in einer lichtabsorbierenden Schicht eingesetzt wird, wird bevorzugt ein geeignetes Co-Material eingesetzt, welches als solches bekannt ist. Dabei wird das Co-Material entweder als Mischung mit dem Photosensitizer eingesetzt oder in einer Schicht, die der Schicht enthaltend den Photosensitizer benachbart ist.

Geeignete Co-Materialien, welche in Kombination, also als Mischung mit den erfindungsgemäßen Verbindungen oder in einer Schicht benachbart zu der Schicht enthaltend die erfindungsgemäßen Verbindungen, eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Comaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

In einer besonders bevorzugten Ausführungsform kann vorgesehen sein, dass eine oder mehrere erfindungsgemäße Verbindungen gemäß Formel (I) oder den bevorzugten Ausführungsformen in Kombination mit Elektronentransportmaterialien, Elektroneninjektionsmaterialien, Lochblockiermaterialien eingesetzt werden. Besonders bevorzugt werden unter anderem Subphthalocyanine, Subphthalocyanin-Derivate, Fullerene oder Fulleren-Derivate verwendet. Derartige Verbindungen sind dem Fachmann für die Verwendung in organischen optischen Detektoren bekannt.

Diese Ausgestaltung ist insbesondere bevorzugt für den Fall, dass die erfindungsgemäße Verbindung als Lochleitermaterialien, Lochinjektionsmaterialien und/oder Elektronenblockiermaterialien eingesetzt werden können.

Bevorzugte einsetzbare Subphthalocyanine, Subphthalocyanin-Derivate, Fullerene oder Fulleren-Derivate werden unter anderem in der europäischen Patentanmeldung EP 3848374 A1, hinterlegt beim Europäischen Patentamt am 13.01.2021 mit der Anmeldenummer EP 21151295.9 beschrieben, wobei diese Druckschrift zu Offenbarungszwecken durch Referenz hierauf eingefügt wird. Diese Materialien werden insbesondere auf Seiten 84 bis 86 dargelegt (vgl. Absätze [322] bis [332]).

Weiterhin kann als Comaterial eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Comaterial Verbindungen, welche eine große Bandlücke aufweisen und selber nicht oder zumindest nicht in wesentlichem Maße am Ladungstransport der lichtabsorbierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

In einer weiteren Ausführungsform der Erfindung enthält der erfindungsgemäße organische optische Detektor keine separate Lochinjektionsschicht und/oder Lochtransportschicht und/oder Lochblockierschicht und/oder Elektronentransportschicht, d. h. die lichtabsorbierende Schicht grenzt direkt an die Lochinjektionschicht oder die Anode an, und/oder die lichtabsorbierende Schicht grenzt direkt an die Elektronentransportschicht oder die Elektroneninjektionsschicht oder die Kathode an.

In den weiteren Schichten des erfindungsgemäßen organischen optischen Detektors können alle Materialien verwendet werden, wie sie üblicherweise gemäß dem Stand der Technik eingesetzt werden. Der Fachmann kann daher ohne erfinderisches Zutun alle für organische optische Detektoren bekannten Materialien in Kombination mit den erfindungsgemäßen Verbindungen gemäß Formel (I) bzw. den oben ausgeführten bevorzugten Ausführungsformen einsetzen.

Weiterhin bevorzugt ist ein organischer optischer Detektor, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls ein organischer optischer Detektor, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist ein organischer optischer Detektor, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung gemäß Formel (I) oder deren zuvor dargelegten bevorzugten Ausführungsformen sind neu. Ein weiterer Gegenstand der vorliegenden Erfindung ist daher eine Formulierung, enthaltend mindestens ein Lösungsmittel und eine Verbindung gemäß Formel (I) oder deren zuvor dargelegten bevorzugten Ausführungsformen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische optische Detektoren enthaltend die erfindungsgemäßen Verbindungen angewandt werden.

Weitere Einzelheiten bevorzugter elektronischer Vorrichtungen, insbesondere organischer optischer Detektoren, sowie deren Herstellung sind aus dem Stand der Technik bekannt. Diese werden unter anderem in der europäischen Patentanmeldung EP 3848374 A1, hinterlegt beim Europäischen Patentamt am 13.01.2021 mit der Anmeldenummer EP 21151295.9 beschrieben, wobei diese Druckschrift zu Offenbarungszwecken durch Referenz hierauf eingefügt wird. Hierzu wird insbesondere auf die in EP 3848374 A1 beschriebenen Figuren 1 bis 10 verwiesen, die unter anderem auf Seiten 83 bis 90 der Druckschrift EP 3848374 A1 dargelegt sind.

Die erfindungsgemäßen elektronischen Vorrichtungen, insbesondere organische optische Detektoren, zeichnen sich durch einen oder mehrere der folgenden überraschenden Vorteile gegenüber dem Stand der Technik aus:
1. Elektronische Vorrichtungen, insbesondere organische optische Detektoren enthaltend Verbindungen gemäß Formel (I) bzw. die zuvor und nachfolgend ausgeführten bevorzugten Ausführungsformen, insbesondere als Photosensitizer, weisen eine sehr gute Lebensdauer auf.
2. Elektronische Vorrichtungen, insbesondere organische optische Detektoren enthaltend Verbindungen gemäß Formel (I) bzw. die zuvor und nachfolgend ausgeführten bevorzugten Ausführungsformen als Photosensitizer weisen eine hervorragende Effizienz auf. Hierbei bewirken erfindungsgemäße Verbindungen gemäß Formel (I) bzw. die zuvor und nachfolgend ausgeführten bevorzugten Ausführungsformen eine geringe Betriebsspannung bei Verwendung in elektronischen Vorrichtungen.
3. Die erfindungsgemäßen Verbindungen gemäß Formel (I) bzw. die zuvor und nachfolgend ausgeführten bevorzugten Ausführungsformen zeigen eine sehr hohe Stabilität, insbesondere thermische Stabilität und geringere Aufdampftemperaturen.
4. Mit Verbindungen gemäß Formel (I) bzw. den zuvor und nachfolgend ausgeführten bevorzugten Ausführungsformen kann in elektronischen Vorrichtungen, insbesondere organischen optischen Detektoren, die Bildung von optischen Verlustkanäle vermieden werden. Hierdurch zeichnen sich diese Vorrichtungen durch eine hohe Photostrom-Effizienz von Photosensitizer bzw. eine ausgezeichnete Energieübertragung aus.
5. Verbindungen gemäß Formel (I) bzw. die zuvor und nachfolgend ausgeführten bevorzugten Ausführungsformen weisen eine ausgezeichnete Glasfilmbildung auf.
6. Verbindungen gemäß Formel (I) bzw. die zuvor und nachfolgend ausgeführten bevorzugten Ausführungsformen bilden aus Lösungen sehr gute Filme und zeigen eine ausgezeichnete Löslichkeit.

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn, dass bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbindungen, die mehrere enantiomere, diastereomere oder tautomere Formen aufweisen können, wird eine Form stellvertretend gezeigt.

### Literaturbekannte Synthone LS:

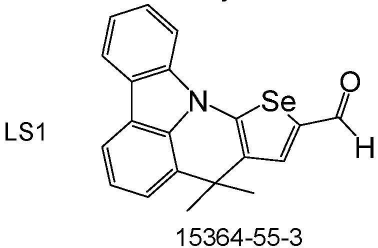

### Synthese von Synthonen:

### Beispiel S1:

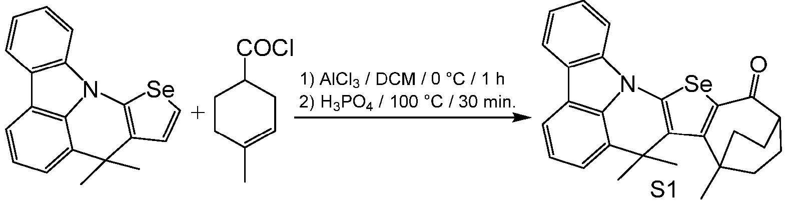

Durchführung analog K. Mazzio et al, Appl. Mat. & Interfaces, 2011, 3, 2, 271. Eine auf 0 °C gekühlte Lösung von 3.36 g (10 mmol) 8,8-Dimethyl-8H-seleno-[3',2':5,6]pyrido[3,2,1-jk]carbazol [2661611-42-1] und 1.59 g (10 mmol) 4-Methyl-3-cyclohexen-1-carbonylchlorid [16695-95-7] in 150 ml DCM wird tropfenweise während 10 min. mit einer Lösung von 1.73 g (13 mmol) Aluminiumchlorid, wasserfrei, versetzt und 1 h nachgerührt. Man quencht durch vorsichtige Zugabe von 50 g Eis, trennt die org. Phase ab, wäscht diese zweimal mit je 50 ml Wasser, einmal mit 50 ml ges.

Kochsalzlösung und trocknet über Magnesiumsulfat: Natriumcarbonat (1:1). Man filtriert vom Trockenmittel ab, engt das Filtrat im Vakuum ein, versetzt das orange Öl mit 30 g Polyphosphorsäure (85 %ig), homogenisiert das Gemisch bei 50 °C und steigert dann die Temperatur auf 100 °C. Nach 30 min. lässt man auf 50 °C erkalten und versetzt dann tropfenweise unter Kühlung (Vorsicht exotherm, Induktionsperiode, maximale Temperatur 80 °C) mit 200 ml Wasser. Man saugt vom ausgefallenen Feststoff ab, nimmt diesen in 150 ml DCM auf, wäscht einmal mit 50 ml ges. Natriumhydrogencarbonat-Lösung, zweimal mit je 50 ml Wasser, einmal mit 50 ml ges. Kochsalzlösung und trocknet über Magnesiumsulfat. Man saugt vom Trockenmittel ab und engt das Filtrat im Vakuum zur Trockene ein. Die weitere Reinigung des Rohprodukts erfolgt durch Chromatographie (Torrent Säulenautomat der Fa. A. Semrau). Ausbeute: 1.03 g (2.3 mmol) 23 %; Reinheit: ca. 97 %ig n. ¹H-NMR.

Analog können folgende Verbindungen dargestellt werden:

| Bsp. | Edukt | Produkt | Ausbeute |
|---|---|---|---|
| S2 | B12 - Schritt 3 | 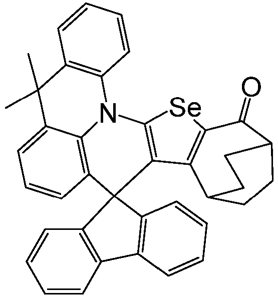 | 25 % |
| | 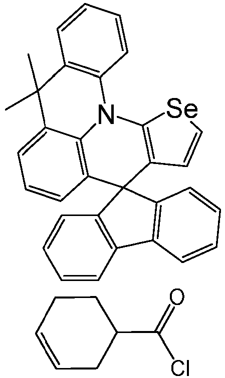 | | |
| | 932-67-2 | | |
| S3 | B14 - Schritt 3 | 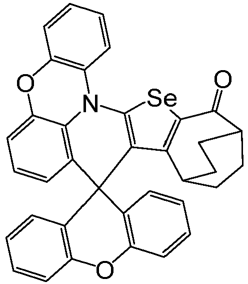 | 27 % |
| | 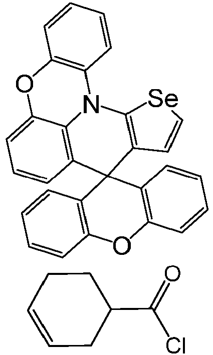 | | |
| | 932-67-2 | | |
| S4 | 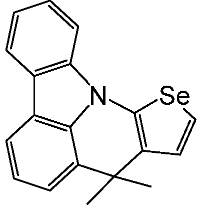 | 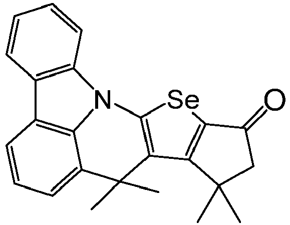 | 25% |
| | 2661611-42-1 | | |
| | 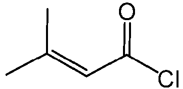 | | |
| | 3350-78-5 | | |
| S5 | 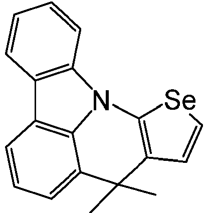 | 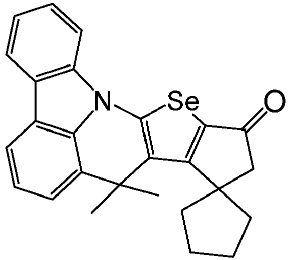 | 21 % |
| | 2661611-42-1 | | |
| | 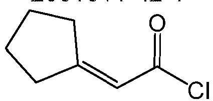 | | |
| | 80385-46-2 | | |
| S6 | 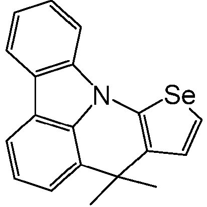 | 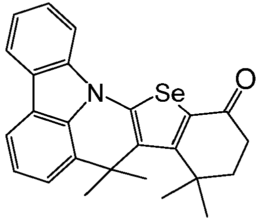 | 23 % |
| | 2661611-42-1 | | |
| | 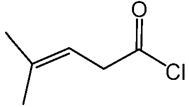 | | |
| | 41199-96-6 | | |
| S100 | 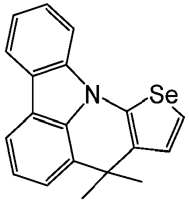 | 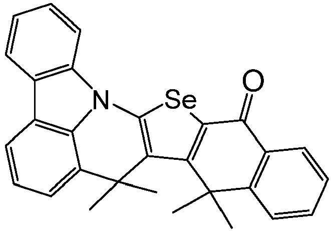 | 47 % |
| | 2661611-42-1 | | |
| | 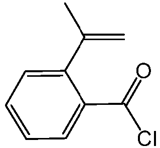 | | |
| | 65680-40-2 | | |

### Synthese der erfindungsgemäßen Verbindungen:

Die Aldehyde (8) können analog zu US 2021/0234103 Seite 70, durch Einsatz der entsprechend unten aufgeführten Edukte, einem o-Brom-Amin (1), einem 5-Ring Heterocyclus (2) (Pyrrol, Furan, Thiophen, Selenophen, Tellurophen), einem Keton (4), und anschließende Formylierung der Verbindungen (6) mit einem Formamid (7) bzw. Acylierung dargestellt werden, s. Schema 1, Schritte 1 bis 4. Im letzten Schritt 5, werden die Carbonylverbindungen (8) in einer Knoevenagel-Kondensation (z.B. S. Haig et al., Chem. Mat., 2011, 23(20), 4435) mit Malonsäuredinitril (9) zu den erfindungsgemäßen Verbindungen (10) umgesetzt.

### Schema 1:

### Schritt 1: Ullmann-Kupplung

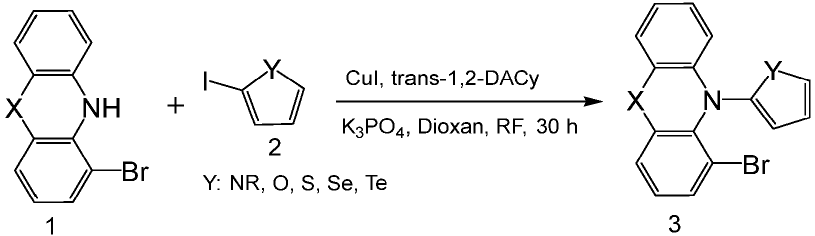

X: Einfachbindung,
CR₂, NR, O, S, Se, Te

### Schritt 2: Hydroxyalkylierung

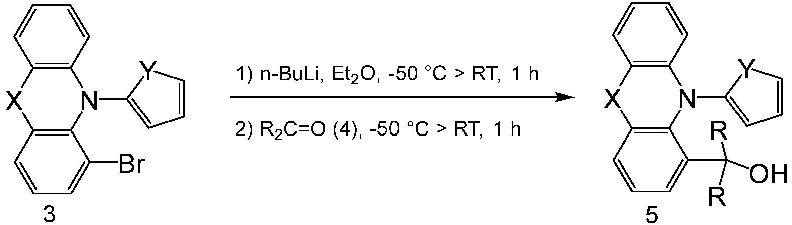

### Schritt 3: Cyclisierung

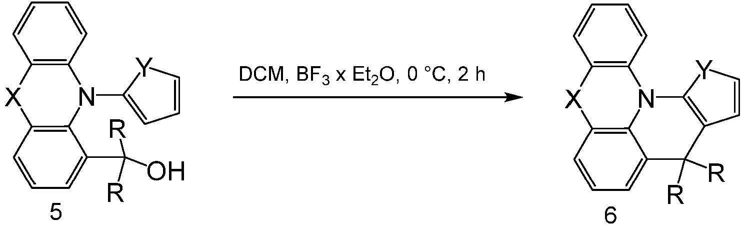

### Schritt 4: Formylierung bzw. Acylierung

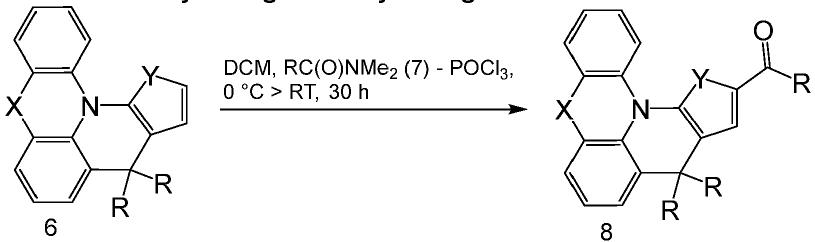

### Schritt 5: Knoevenagel-Kondensation

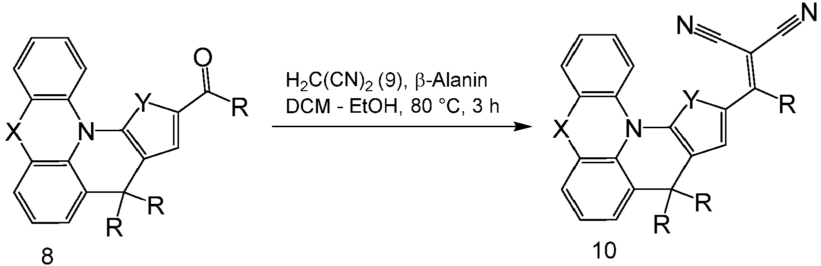

### Alternatives Verfahren zu Schritt 2: Suzuki-Kupplung

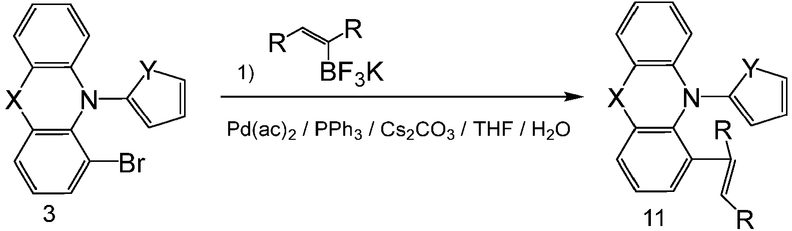

Ein gut gerührtes Gemisch aus 100 mmol (3), 120 mmol des Alkenyl-BF₃K Salzes, 250 mmol Cäsiumcarbonat, 3 mmol Palladium(II)acetat, 9 mmol Triphenylphosphin, 1000 ml THF und 200 ml Wasser wird 16 h unter Rückfluss erhitzt. Nach Erkalten fügt man 500 ml Ethylacetat (EE) zu, trennt die wässrige Phase ab, wäscht die org. Phase dreimal mit je 300 ml Wasser und einmal mit 200 ml ges. Kochsalzlösung und trocknet über Magnesiumsulfat. Man filtriert vom Trockenmittel über ein mit EE vorgeschlämmtes Kieselgelbett ab und entfernt das Lösungsmittel im Vakuum. Der Rückstand wird ohne weitere Reinungung weiter umgesetzt.

### Alternatives Verfahren zu Schritt 3: Cyclisierung

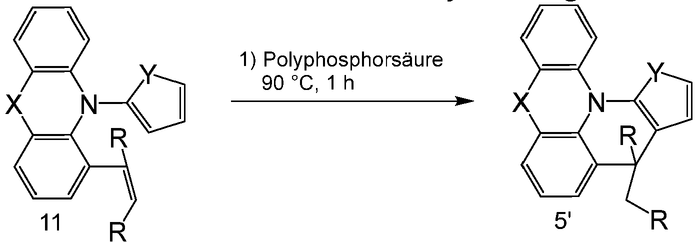

Ein gut gerührtes Gemisch aus 100 mmol (11) und 400 g Polyphosphorsäure wird 1 h auf 90 °C erwärmt. Man lässt auf 60 °C erkalten, gießt die Reaktionsmischung unter gutem Rünren auf 5 l Eiswasser, rührt 30 min. nach, saugt vom ausgefallenen Feststoff ab oder arbeitet extraktiv, mit EE oder Dichlormethan (DCM), auf. Das Rohprodukt wird aus DCM/Acetonitril umkristallisiert oder chromatographiert.

### Alternatives Verfahren zur Einführung von BR-, SiR₂-, GeR₂-, NR-, S-Brücken:

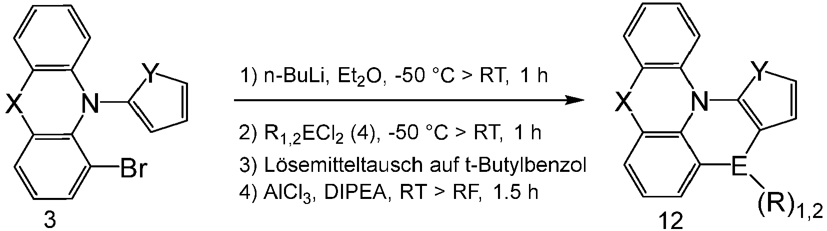

### Beispiel B1:

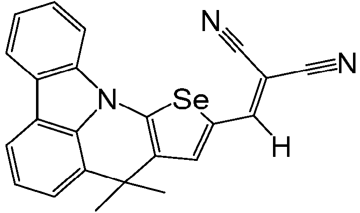

Durchführung analog S. Haig et al., Chem. Mat., 2011, 23(20), 4435. Ein Gemisch aus 36.4 g (100 mmol) LS1, 14.6 g (220 mmol) Malonsäuredinitril [109-77-3], 446 mg (5 mmol) β-Alanin [107-95-9], 300 ml Ethanol und 200 ml Dichlormethan (DCM) wird in einem Rührautoklaven 3 h bei 80 °C gerührt. Man lässt unter Rühren erkalten, saugt vom ausgefallenen Produkt ab, wäscht dreimal mit je 30 ml kaltem Ethanol nach und trocknet im Vakuum. Die weitere Reinigung erfolgt durch Chromatographie (Torrent Säulenautomat der Fa. A. Semrau) oder Heißextraktionskristallisation (übliche org. Lösungsmittel, bevorzugt Acetonitril oder Acetonitril - DCM Gemische (4:1 - 1:2 vv) und durch fraktionierte Sublimation im Hochvakuum (p. ca. 10⁻⁵ mbar). Ausbeute: 31.9 g (77 mmol) 77 %; Reinheit: ca. 99.9 %ig n. HPLC.

Analog können folgende Verbindungen, in den angegebenen Ausbeuten über die 5 Stufen, dargestellt werden:

| Bsp. | Edukte | Produkt | Ausbeute |
|---|---|---|---|
| B2 | 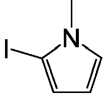 | 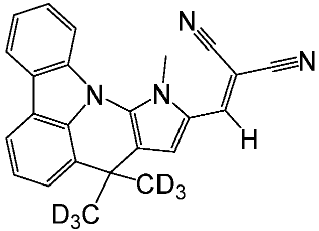 | 16 % |
| | 34671-29-9 | | |
| | 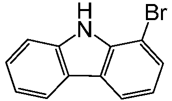 | | |
| | 16807-11-7 | | |
| | 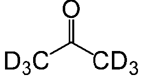 666-52-4 | | |
| B3 | 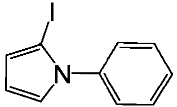 | 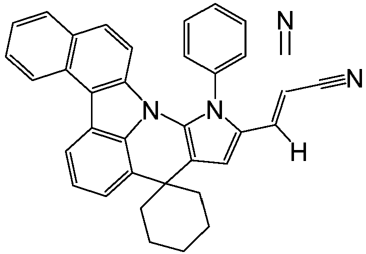 | 19 % |
| | 1008533-34-3 | | |
| | 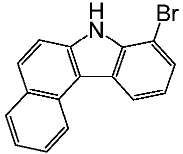 | | |
| | 1686099-80-8 | | |
| | 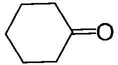 108-94-1 | | |
| B4 | 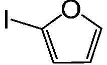 | 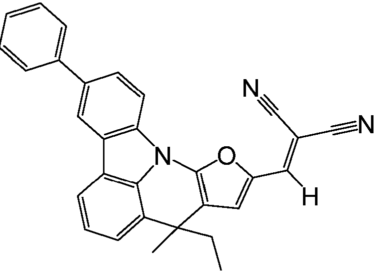 | 22 % |
| | 54829-48-0 | | |
| | 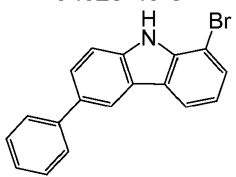 | | |
| | 1686099-79-5 | | |
| | 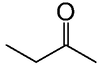 78-93-3 | | |
| B5 | 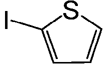 | 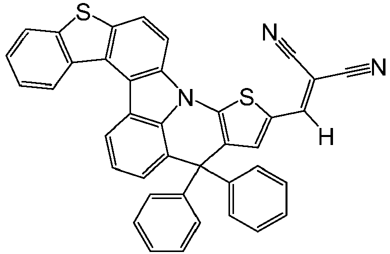 | 20 % |
| | 3437-95-4 | | |
| | 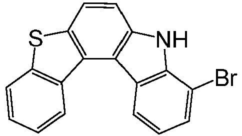 | | |
| | 1628067-03-7 | | |
| | 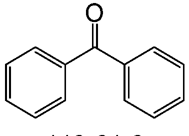 119-61-9 | | |
| B6 | 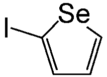 | 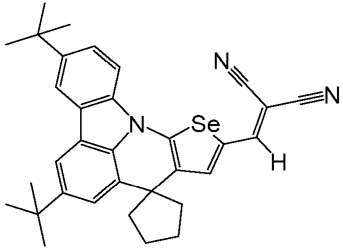 | 21 % |
| | 37686-36-5 | | |
| | 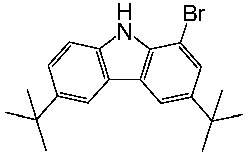 | | |
| | 1357359-52-4 | | |
| | 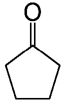 120-92-3 | | |
| B7 | 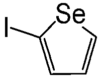 | 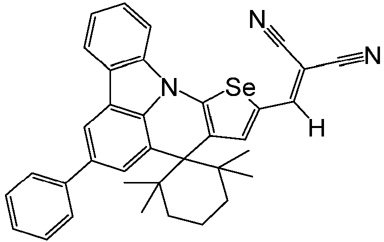 | 7% |
| | 37686-36-5 | | |
| | 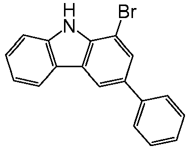 | | |
| | 2304744-36-1 | | |
| | 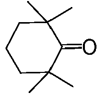 1195-93-3 | | |
| B8 | 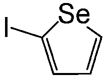 | 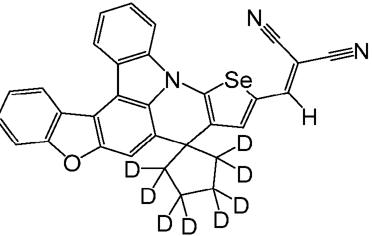 | 19 % |
| | 37686-36-5 | | |
| | 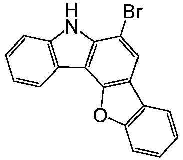 | | |
| | 1438427-35-0 | | |
| | 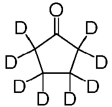 4477-17-2 | | |
| B9 | 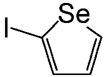 | 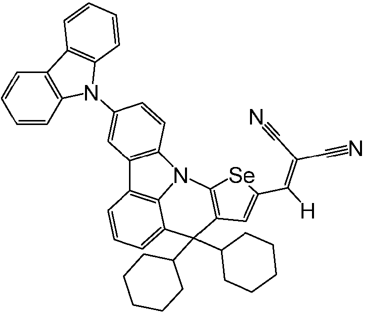 | 15 % |
| | 37686-36-5 | | |
| | 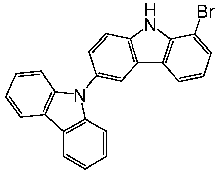 | | |
| | 2311845-06-2 | | |
| | 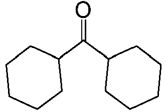 119-60-8 | | |
| B10 | 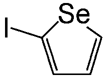 | 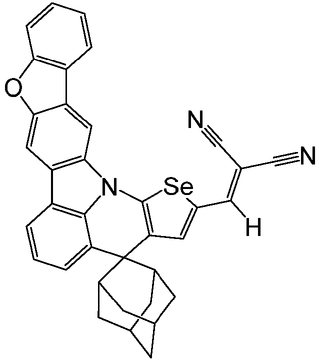 | 23 % |
| | 37686-36-5 | | |
| | 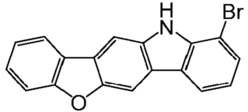 | | |
| | 2444315-80-2 | | |
| | 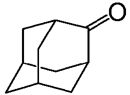 700-58-3 | | |
| B11 | 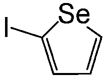 | 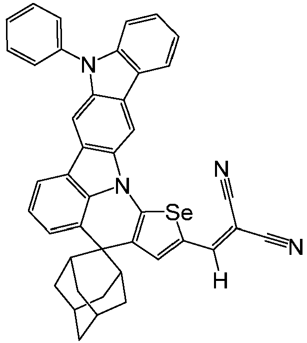 | 25 % |
| | 37686-36-5 | | |
| | 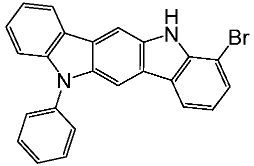 | | |
| | 2250435-63-1 | | |
| | 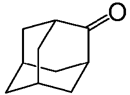 700-58-3 | | |
| B12 | 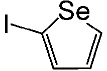 | 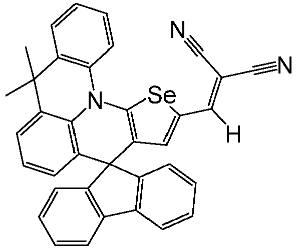 | 23 % |
| | 37686-36-5 | | |
| | 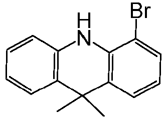 | | |
| | 2222130-32-5 | | |
| | 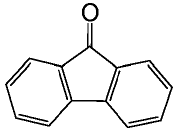 486-25-9 | | |
| B13 | | 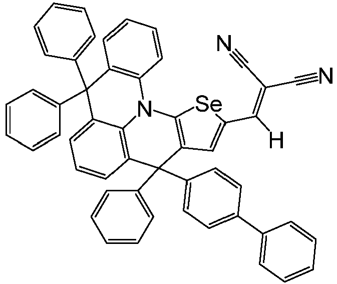 | 24 % |
| | 37686-36-5 | | |
| | 2244888-30-8 | | |
| | 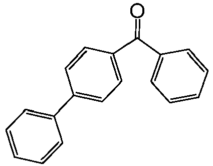 2128-93-0 | | |
| B14 | 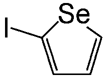 | 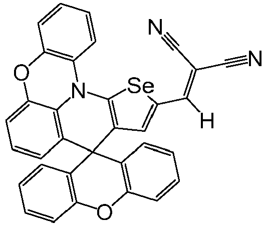 | 18 % |
| | 37686-36-5 | | |
| | 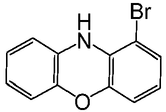 | | |
| | 1838714-71-8 | | |
| | 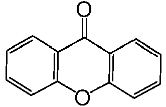 90-47-1 | | |
| B15 | 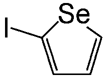 | 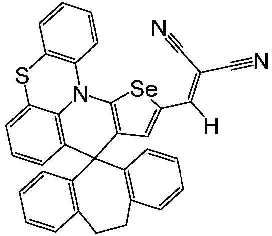 | 16 % |
| | 37686-36-5 | | |
| | 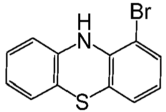 | | |
| | 31609-96-8 | | |
| | 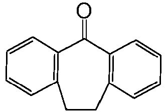 1210-35-1 | | |
| B16 | 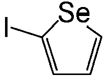 | 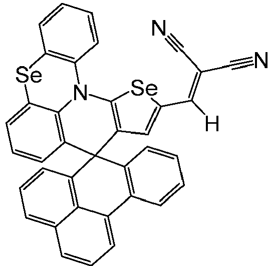 | 11 % |
| | 37686-36-5 | | |
| | 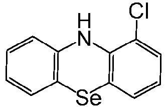 118044-75-0 | | |
| | 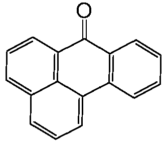 82-05-3 | | |
| B17 | 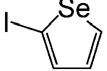 | 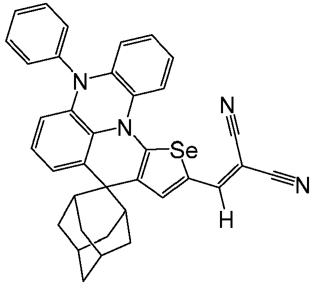 | 21 % |
| | 37686-36-5 | | |
| | 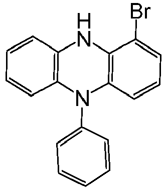 | | |
| | 2493276-29-0 | | |
| | 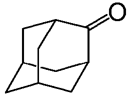 700-58-3 | | |
| B18 | 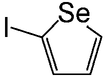 | 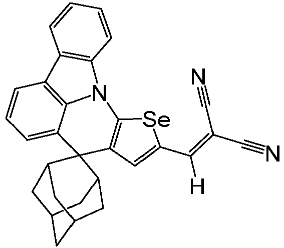 | 24 % |
| | 37686-36-5 | | |
| | 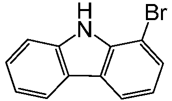 | | |
| | 16807-11-7 | | |
| | 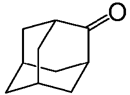 700-58-3 | | |
| B19 | 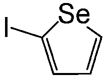 | 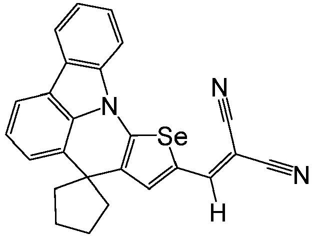 | 23 % |
| | 37686-36-5 | | |
| | 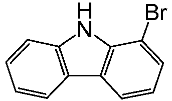 16807-11-7 | | |
| | 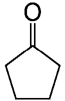 120-92-3 | | |
| B20 | 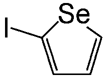 | 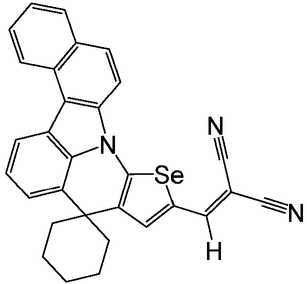 | 26 % |
| | 37686-36-5 | | |
| | 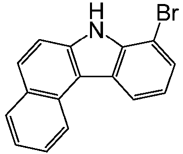 | | |
| | 1686099-80-8 | | |
| | 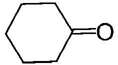 108-94-1 | | |
| B21 | 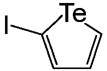 | 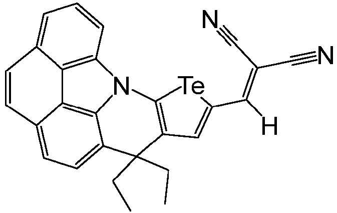 | 19 % |
| | 59163-68-7 | | |
| | 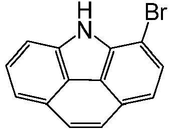 | | |
| | 2118984-35-1 | | |
| | 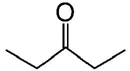 96-22-0 | | |
| B22 | 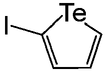 | 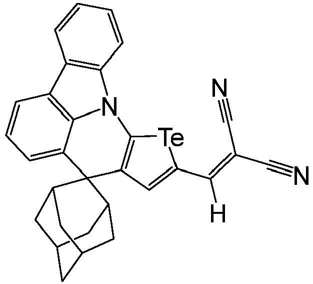 | 13 % |
| | 59163-68-7 | | |
| | 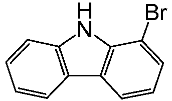 | | |
| | 16807-11-7 | | |
| | 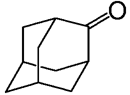 700-58-3 | | |
| B23 | 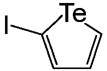 | 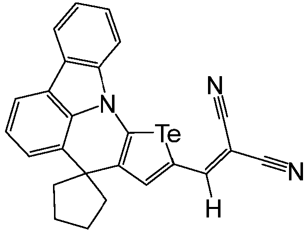 | 11 % |
| | 59163-68-7 | | |
| | 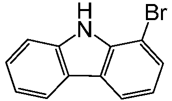 | | |
| | 16807-11-7 | | |
| | 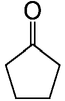 120-92-3 | | |
| B24 | 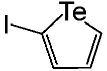 | 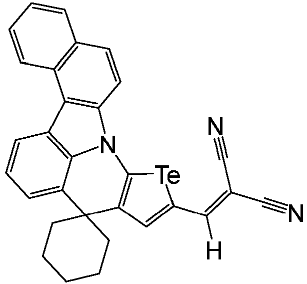 | 12 % |
| | 59163-68-7 | | |
| | 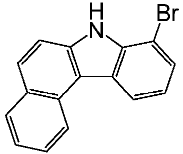 | | |
| | 1686099-80-8 | | |
| | 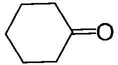 108-94-1 | | |
| B25 | 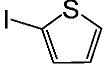 | 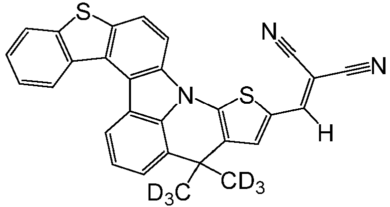 | 27 % |
| | 3437-95-4 | | |
| | 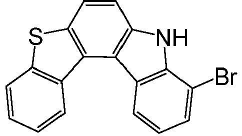 | | |
| | 1628067-03-7 | | |
| | 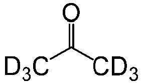 666-52-4 | | |
| B26 | 3437-95-4 | 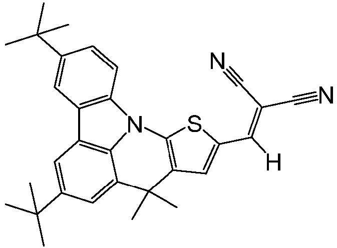 | 27 % |
| | 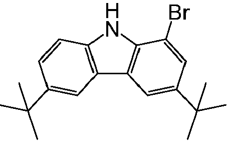 | | |
| | 1357359-52-4 | | |
| | 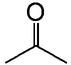 67-64-1 | | |
| B27 | 3437-95-4 | 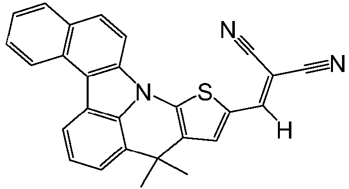 | 30 % |
| | 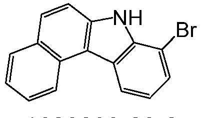 | | |
| | 1686099-80-8 | | |
| | 67-64-1 | | |
| B28 | 3437-95-4 | 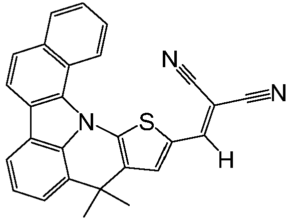 | 32 % |
| | 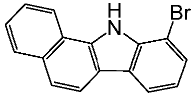 | | |
| | 1686100-42-4 | | |
| | 67-64-1 | | |
| B29 | 3437-95-4 | 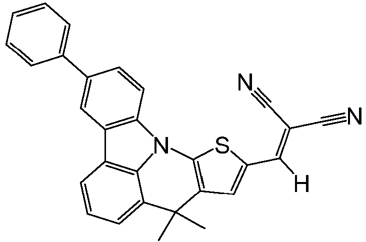 | 32 % |
| | 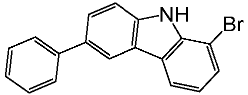 | | |
| | 1686099-79-5 | | |
| | 67-64-1 | | |
| B30 | 3437-95-4 | 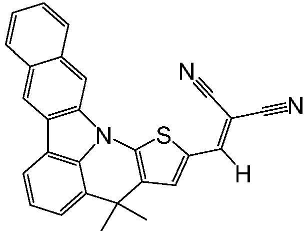 | 26 % |
| | 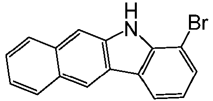 | | |
| | 1686100-43-5 | | |
| | 67-64-1 | | |
| B31 | 3437-95-4 | 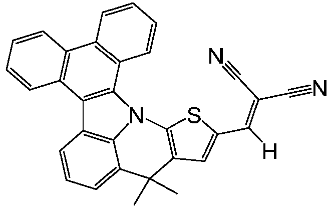 | 31 % |
| | 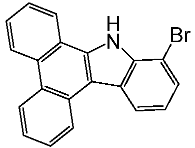 | | |
| | 1686100-44-6 | | |
| | 67-64-1 | | |
| B32 | 3437-95-4 | 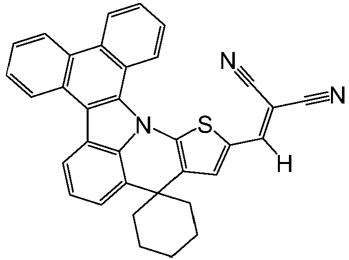 | 27 % |
| | 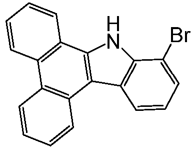 | | |
| | 1686100-44-6 | | |
| | 108-94-1 | | |
| B33 | 3437-95-4 | 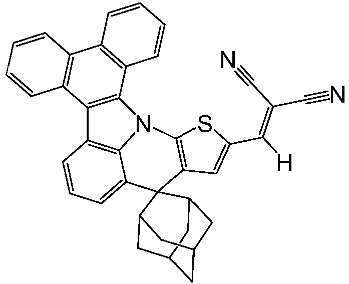 | 30 % |
| | 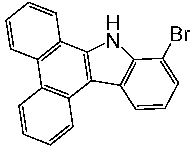 | | |
| | 1686100-44-6 | | |
| | 700-58-3 | | |
| B34 | 3437-95-4 | 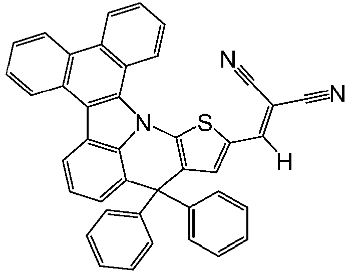 | 30 % |
| | 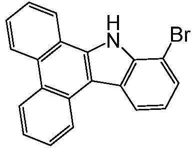 | | |
| | 1686100-44-6 | | |
| | 119-61-9 | | |
| B35 | 3437-95-4 | 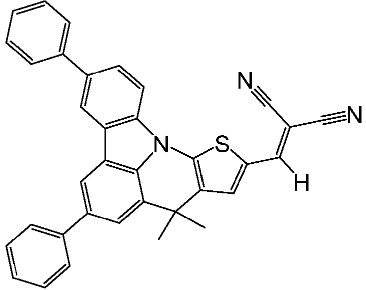 | 32 % |
| | 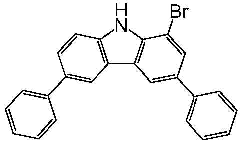 | | |
| | 2351179-71-8 | | |
| | 67-64-1 | | |
| B36 | 3437-95-4 | 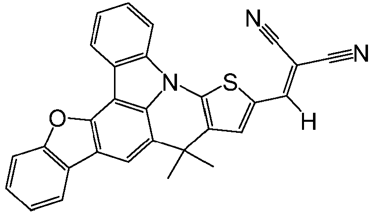 | 33 % |
| | 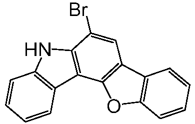 | | |
| | 1438427-35-0 | | |
| | 67-64-1 | | |
| B37 | 3437-95-4 | 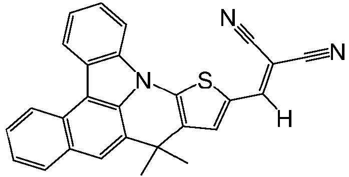 | 25 % |
| | 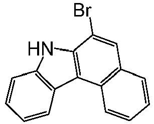 | | |
| | 1639452-47-3 | | |
| | 67-64-1 | | |
| B38 | 3437-95-4 | 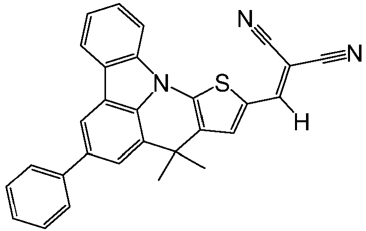 | 30 % |
| | 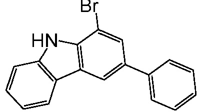 | | |
| | 2304744-36-1 | | |
| | 67-64-1 | | |
| B39 | 3437-95-4 | 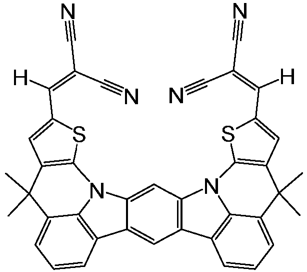 | 14 % |
| | 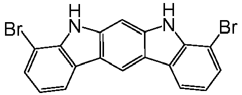 | | |
| | 845619-92-3 | | |
| | 67-64-1 | | |
| B40 | 3437-95-4 | 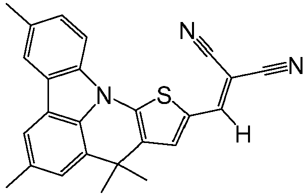 | 29 % |
| | 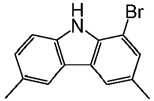 | | |
| | 2393016-93-6 | | |
| | 67-64-1 | | |
| B41 | 3437-95-4 | 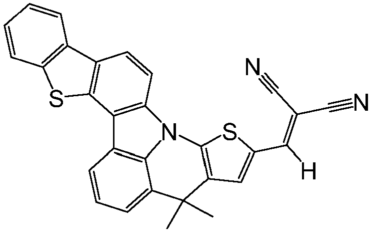 | 31 % |
| | 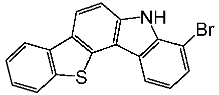 | | |
| | 1628066-90-9 | | |
| | 67-64-1 | | |
| B42 | 3437-95-4 | 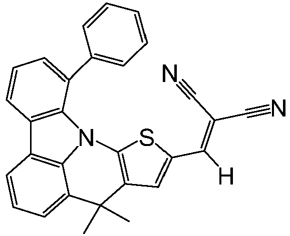 | 34 % |
| | 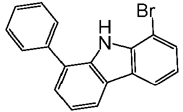 | | |
| | 2619631-74-0 | | |
| | 67-64-1 | | |
| B43 | 3437-95-4 | 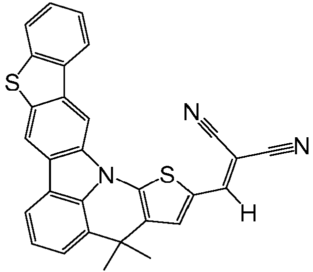 | 28 % |
| | 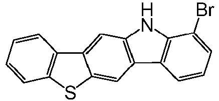 | | |
| | 1628066-98-7 | | |
| | 67-64-1 | | |
| B44 | 3437-95-4 | 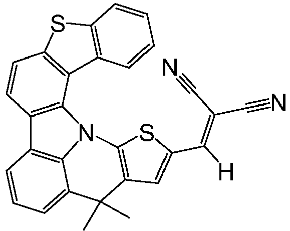 | 29 % |
| | 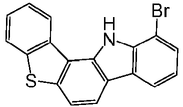 | | |
| | 1628067-07-1 | | |
| | 67-64-1 | | |
| B45 | 3437-95-4 | 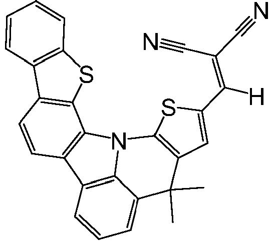 | 29 % |
| | 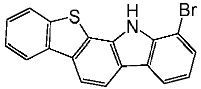 | | |
| | 1628066-87-4 | | |
| | 67-64-1 | | |
| B46 | 3437-95-4 | 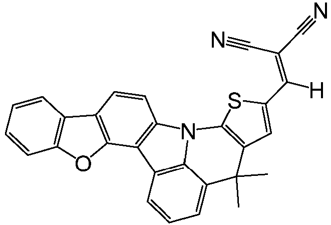 | 30 % |
| | 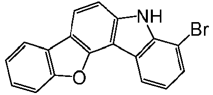 | | |
| | 2088187-43-1 | | |
| | 67-64-1 | | |
| B47 | 3437-95-4 | 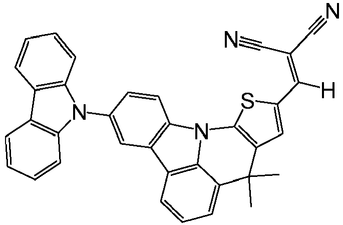 | 26 % |
| | 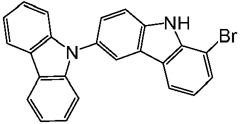 | | |
| | 2311845-06-2 | | |
| | 67-64-1 | | |
| B48 | 3437-95-4 | 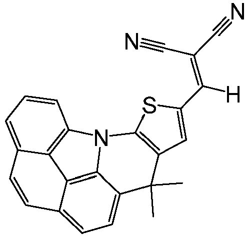 | 21 % |
| | 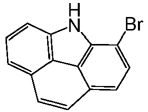 | | |
| | 2118984-35-1 | | |
| | 67-64-1 | | |
| B49 | 3437-95-4 | 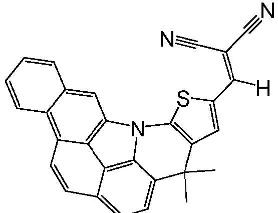 | 27 % |
| | 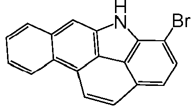 | | |
| | 2209068-29-9 | | |
| | 67-64-1 | | |
| B50 | 3437-95-4 | 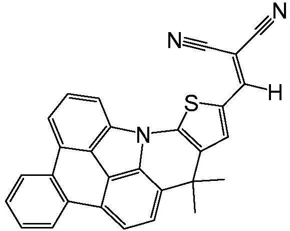 | 30 % |
| | 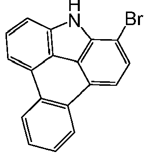 | | |
| | 2209068-40-4 | | |
| | 67-64-1 | | |
| B51 | 3437-95-4 | 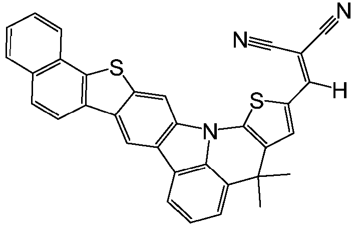 | 28 % |
| | 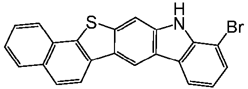 | | |
| | 1628066-94-3 | | |
| | 67-64-1 | | |
| B52 | 3437-95-4 | 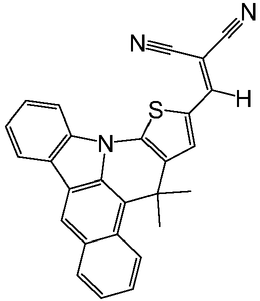 | 31 % |
| | 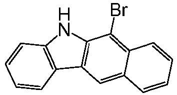 | | |
| | 2097681-89-3 | | |
| | 67-64-1 | | |
| B53 | 3437-95-4 | 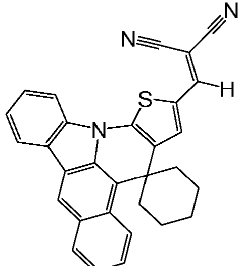 | 22 % |
| | 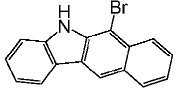 | | |
| | 2097681-89-3 | | |
| | 108-94-1 | | |
| B54 | 3437-95-4 | 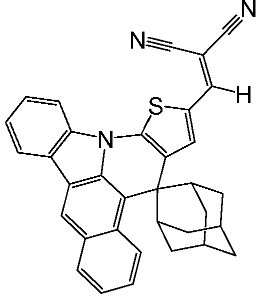 | 24 % |
| | 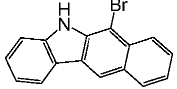 | | |
| | 2097681-89-3 | | |
| | 700-58-3 | | |
| B55 | 3437-95-4 | 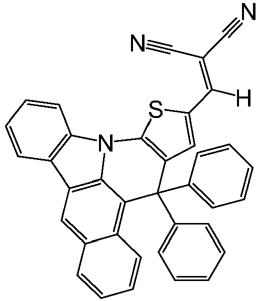 | 27 % |
| | 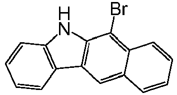 | | |
| | 2097681-89-3 | | |
| | 119-61-9 | | |
| B56 | 3437-95-4 | 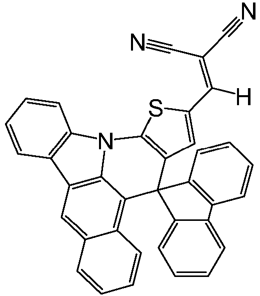 | 25 % |
| | 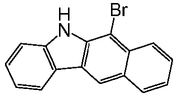 | | |
| | 2097681-89-3 | | |
| | 486-25-9 | | |
| B57 | 3437-95-4 | 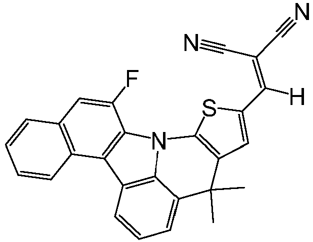 | 27 % |
| | 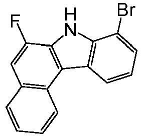 | | |
| | 2364364-94-1 | | |
| | 67-64-1 | | |
| B59 | 3437-95-4 | 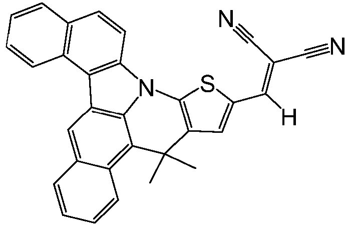 | 33 % |
| | 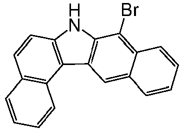 | | |
| | 1807910-69-5 | | |
| | 67-64-1 | | |
| B60 | 3437-95-4 | 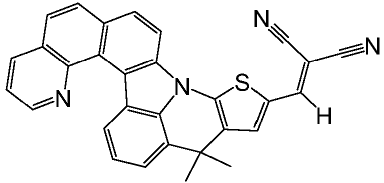 | 17 % |
| | 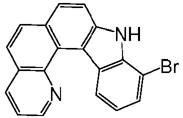 | | |
| | 2796247-53-3 | | |
| | 67-64-1 | | |
| B61 | 3437-95-4 | 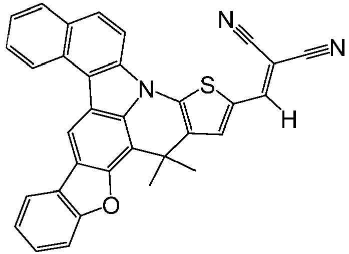 | 26 % |
| | 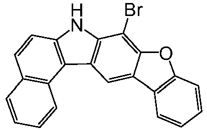 | | |
| | 2624319-17-9 | | |
| | 67-64-1 | | |
| B62 | 3437-95-4 | 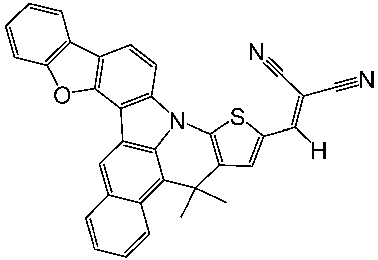 | 22 % |
| | 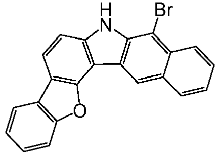 | | |
| | 2444315-86-8 | | |
| | 67-64-1 | | |
| B63 | 3437-95-4 | 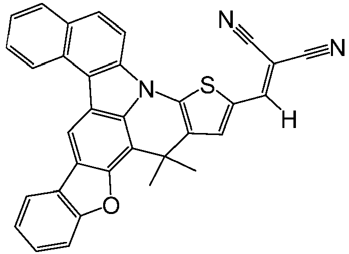 | 20 % |
| | 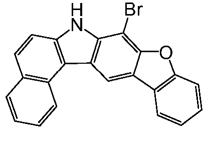 | | |
| | 2444315-84-6 | | |
| | 67-64-1 | | |
| B64 | 3437-95-4 | 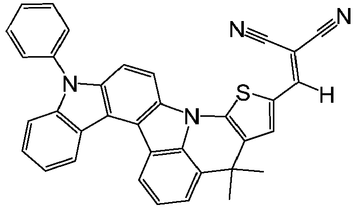 | 25 % |
| | 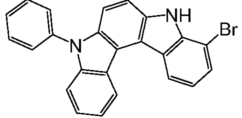 | | |
| | 1370555-67-1 | | |
| | 67-64-1 | | |
| B65 | 3437-95-4 | 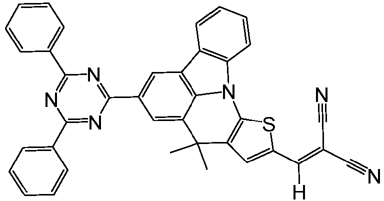 | 27 % |
| | 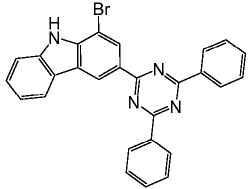 | | |
| | 2660185-57-7 | | |
| | 67-64-1 | | |
| B66 | 3437-95-4 | 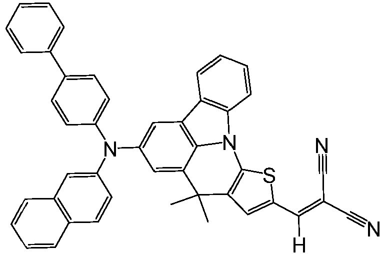 | 21 % |
| | 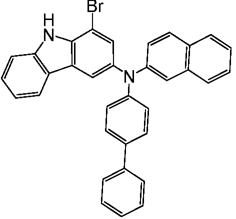 | | |
| | 2450439-85-5 | | |
| | 67-64-1 | | |
| B67 | 3437-95-4 | 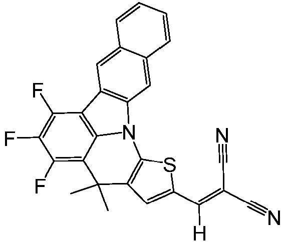 | 20 % |
| | 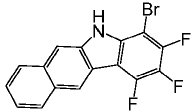 | | |
| | 2636078-73-2 | | |
| | 67-64-1 | | |
| B68 | 3437-95-4 | 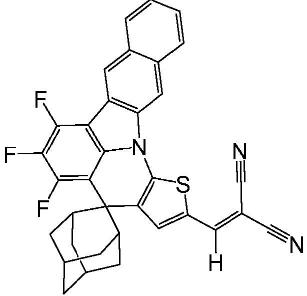 | 22 % |
| | 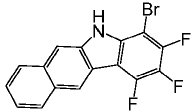 | | |
| | 2636078-73-2 | | |
| | 700-58-3 | | |
| B69 | 3437-95-4 | 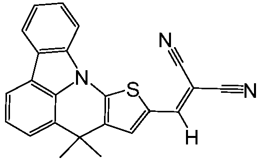 | 35 % |
| | 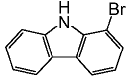 | | |
| | 16807-11-7 | | |
| | 67-64-1 | | |
| B70 | 37686-36-5 | 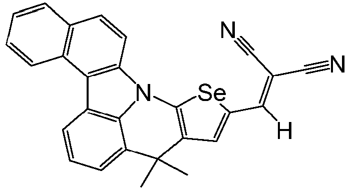 | 30 % |
| | 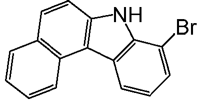 | | |
| | 1686099-80-8 | | |
| | 67-64-1 | | |
| B71 | 37686-36-5 | 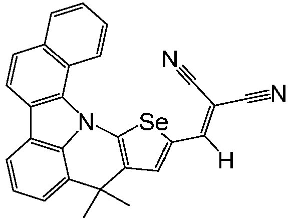 | 32 % |
| | 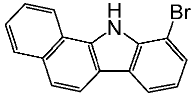 | | |
| | 1686100-42-4 | | |
| | 67-64-1 | | |
| B72 | 37686-36-5 | 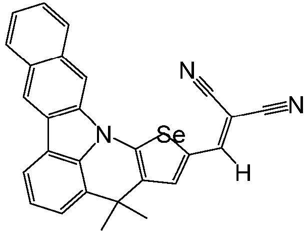 | 26 % |
| | 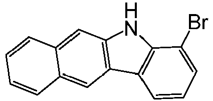 | | |
| | 1686100-43-5 | | |
| | 67-64-1 | | |
| B73 | 37686-36-5 | 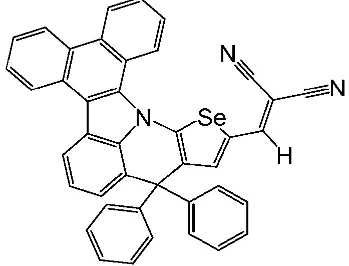 | 30 % |
| | 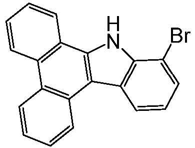 | | |
| | 1686100-44-6 | | |
| | 119-61-9 | | |
| B74 | 37686-36-5 | 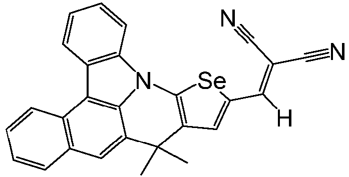 | 35 % |
| | 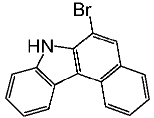 | | |
| | 1639452-47-3 | | |
| | 67-64-1 | | |
| B75 | 37686-36-5 | 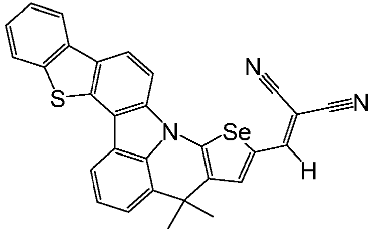 | 28 % |
| | 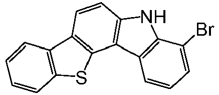 | | |
| | 1628066-90-9 | | |
| | 67-64-1 | | |
| B76 | 37686-36-5 | 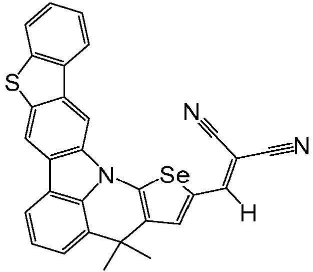 | 29 % |
| | 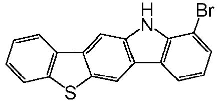 | | |
| | 1628066-98-7 | | |
| | 67-64-1 | | |
| B77 | 37686-36-5 | 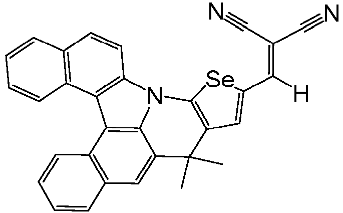 | 27 % |
| | 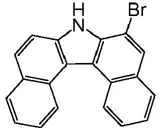 | | |
| | 2685783-06-4 | | |
| | 67-64-1 | | |
| B78 | 37686-36-5 | 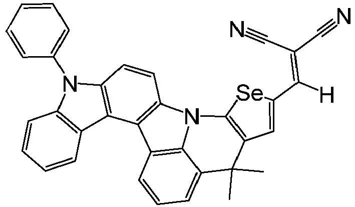 | 27 % |
| | 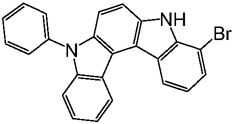 | | |
| | 1370555-67-1 | | |
| | 67-64-1 | | |
| B79 | 3437-95-4 | 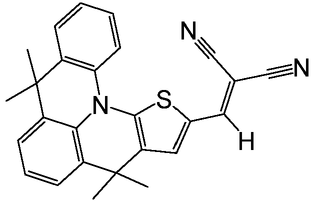 | 25 % |
| | 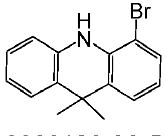 | | |
| | 2222130-32-5 | | |
| | 67-64-1 | | |
| B80 | 3437-95-4 | 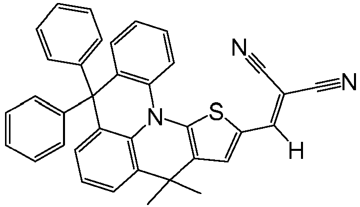 | 23 % |
| | 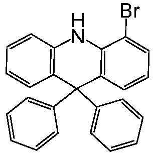 | | |
| | 2244888-30-8 | | |
| | 67-64-1 | | |
| B81 | 3437-95-4 | 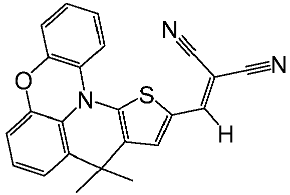 | 19 % |
| | 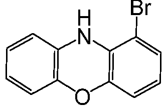 | | |
| | 1838714-71-8 | | |
| | 67-64-1 | | |
| B82 | 3437-95-4 | 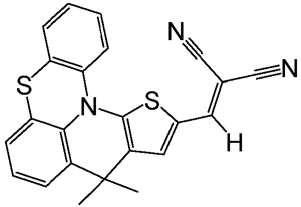 | 22 % |
| | 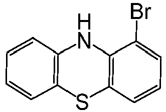 | | |
| | 31609-96-8 | | |
| | 67-64-1 | | |
| B83 | 3437-95-4 | 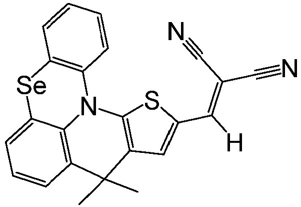 | 15 % |
| | 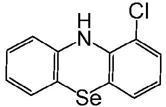 | | |
| | 118044-75-0 | | |
| | 67-64-1 | | |
| B84 | 3437-95-4 | 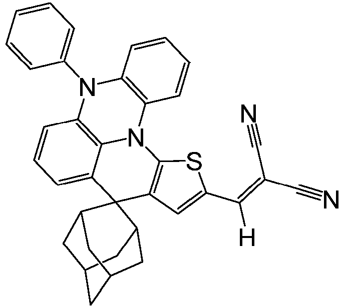 | 24 $ |
| | 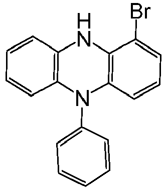 | | |
| | 2493276-29-0 | | |
| | 700-58-3 | | |
| B85 | 37686-36-5 | 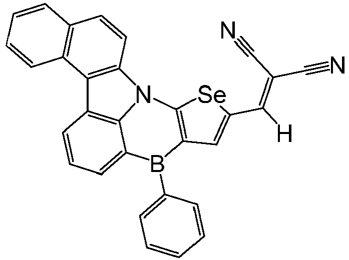 | 28 % |
| | 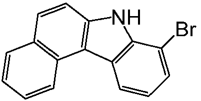 | | |
| | 1686099-80-8 | | |
| | 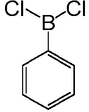 | | |
| | 873-51-8 | | |
| B86 | 3437-95-4 | 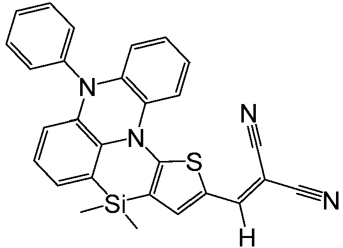 | 18 % |
| | 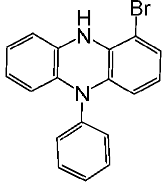 | | |
| | 2493276-29-0 | | |
| | 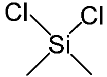 | | |
| | 75-78-5 | | |
| B87 | 3437-95-4 | 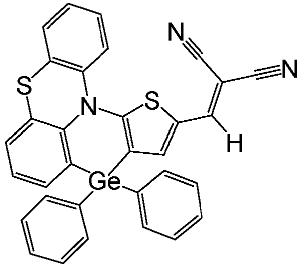 | 17 % |
| | 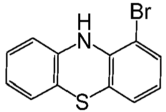 | | |
| | 31609-96-8 | | |
| | 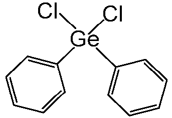 | | |
| | 1613-66-7 | | |
| B88 | 37686-36-5 | 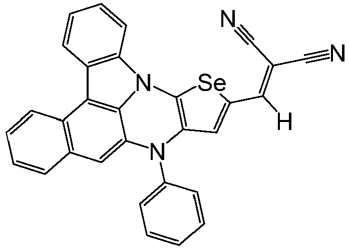 | 11 % |
| | 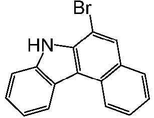 | | |
| | 1639452-47-3 | | |
| | 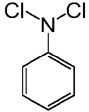 | | |
| | 70278-00-1 | | |
| B89 | 3437-95-4 | 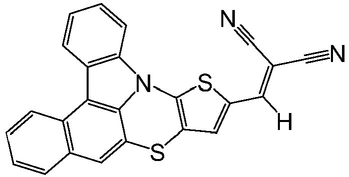 | 14 % |
| | 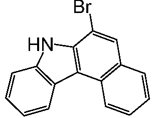 | | |
| | 1639452-47-3 | | |
| | 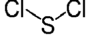 | | |
| | 10545-99-0 | | |
| B90 | 3437-95-4 | 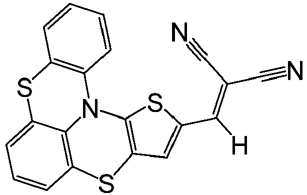 | 16 % |
| | 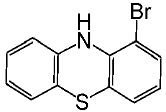 | | |
| | 31609-96-8 | | |
| | 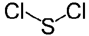 | | |
| | 10545-99-0 | | |
| B100 | 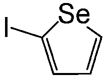 | 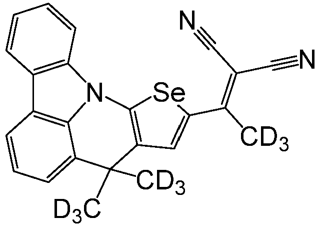 | 17 % |
| | 37686-36-5 | | |
| | 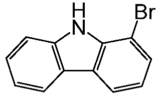 | | |
| | 16807-11-7 | | |
| | 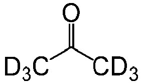 | | |
| | 666-52-4 | | |
| | 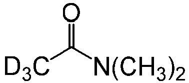 | | |
| | 20255-66-7 | | |
| B101 | 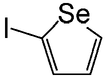 | 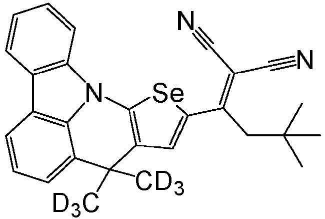 | 15 % |
| | 37686-36-5 | | |
| | 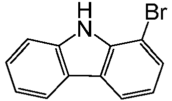 | | |
| | 16807-11-7 | | |
| | 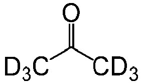 | | |
| | 666-52-4 | | |
| | 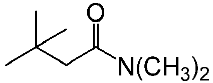 | | |
| | 26153-90-2 | | |
| B102 | 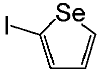 | 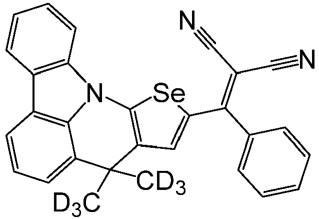 | 19 % |
| | 37686-36-5 | | |
| | 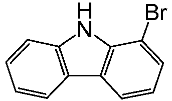 | | |
| | 16807-11-7 | | |
| | 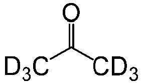 | | |
| | 666-52-4 | | |
| | 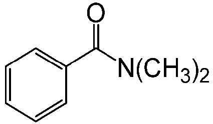 611-74-5 | | |
| B200 | S1 | 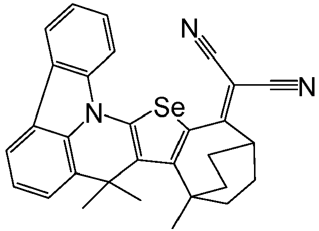 | 45 % |
| | nur Schritt 5 | | |
| | 100 °C / 5 h | | |
| B201 | S2 | 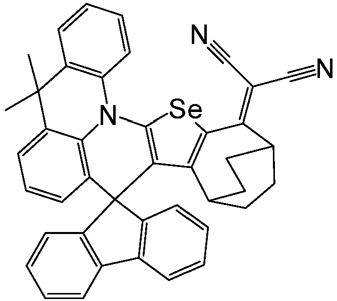 | 57 % |
| | nur Schritt 5 | | |
| | 100 °C / 5 h | | |
| B202 | S3 | 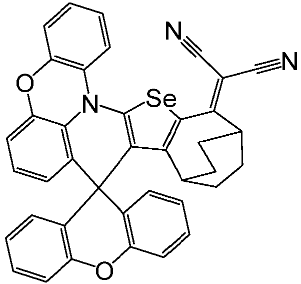 | 53 % |
| | nur Schritt 5 | | |
| | 100 °C / 5 h | | |
| B203 | S4 | 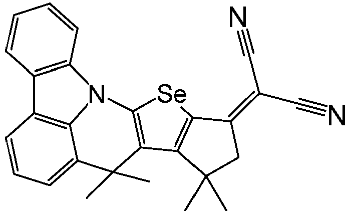 | 44 % |
| | nur Schritt 5 | | |
| | 100 °C / 5 h | | |
| B204 | S5 | 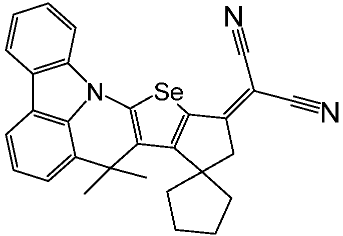 | 41 % |
| | nur Schritt 5 100 °C / 5 h | | |
| B205 | S6 | 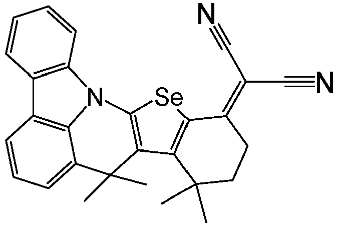 | 53 % |
| | nur Schritt 5 100 °C / 5 h | | |
| B300 | S100 | 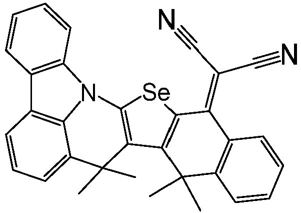 | 49 % |
| | nur Schritt 5 100 °C / 5 h | | |
| B400 | 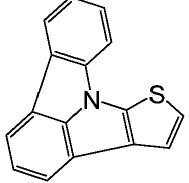 2201847-06-3 | 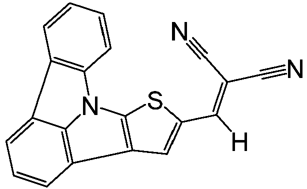 | 75 % |
| | nur Schritt 5 | | |
| B401 | 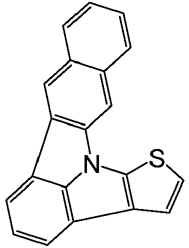 2306103-34-2 | 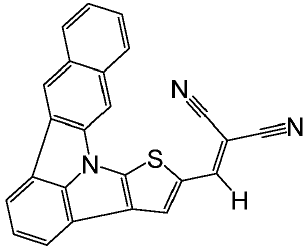 | 77 % |
| | nur Schritt 5 | | |
| B402 | 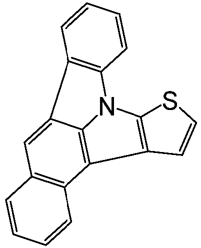 2306102-85-0 | 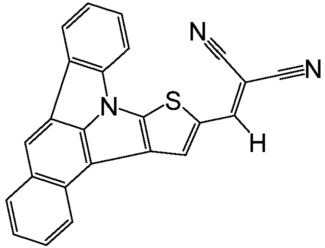 | 70 % |
| | nur Schritt 5 | | |
| B403 | 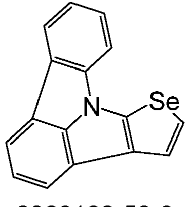 2306102-60-9 | 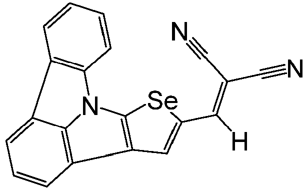 | 70 % |
| | nur Schritt 5 | | |

### Beispiel B500:

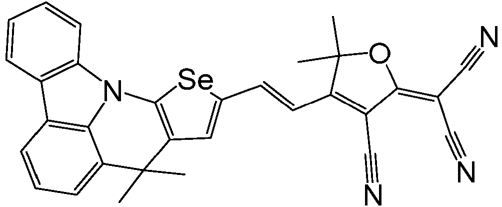

Anstelle von Maleodinitril (H₂C(CN)₂) kann Beispiel B1 mit 2-(4,5,5-Trimethyl-2(5H)-furanyliden)-propandinitril bzw. 2-(4,5,5-Trimethyl-2(5H)-thiophenyliden)-propandinitril und deren Derivaten durchgeführt werden. Ansatz: 36.4 g (100 mmol) LS1 und 25.9 g (130 mmol), 2-(3-Cyano-4,5,5-trimethyl-2(5H)-furanyliden)-propanedinitril [171082-32-9]. Ausbeute: 34.5 g (63 mmol) 63 %; Reinheit: ca. 99.9 %ig n. HPLC.

Analog können folgende Verbindungen, in den angegebenen Ausbeuten über die 5 Stufen, dargestellt werden:

| Bsp. | Edukte | Produkt | Ausbeute |
|---|---|---|---|
| B501 | 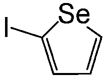 | 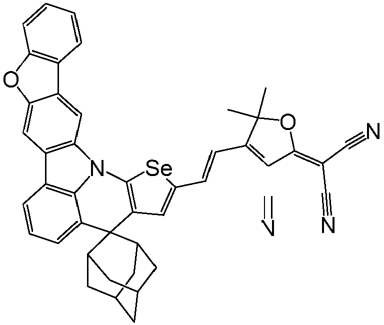 | 13 % |
| | 37686-36-5 | | |
| | 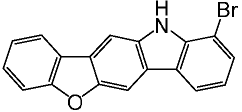 | | |
| | 2444315-80-2 | | |
| | 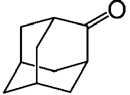 | | |
| | 700-58-3 | | |
| | 171082-32-9 | | |
| B502 | 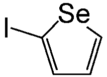 | 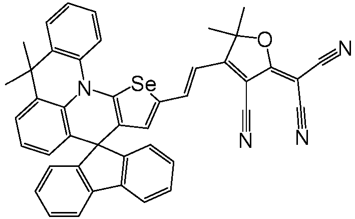 | 17 % |
| | 37686-36-5 | | |
| | 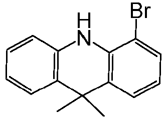 | | |
| | 2222130-32-5 | | |
| | 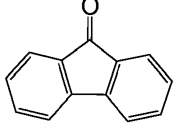 | | |
| | 486-25-9 | | |
| | 171082-32-9 | | |
| B503 | 37686-36-5 | 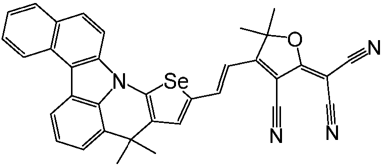 | 33 % |
| | 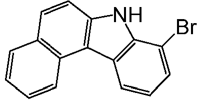 | | |
| | 1686099-80-8 | | |
| | 67-64-1 | | |
| | 171082-32-9 | | |
| B504 | 37686-36-5 | 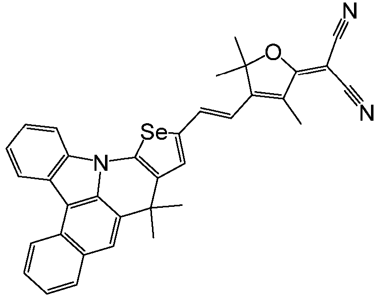 | 30 % |
| | 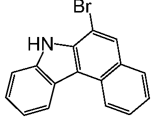 | | |
| | 1639452-47-3 | | |
| | 67-64-1 | | |
| | 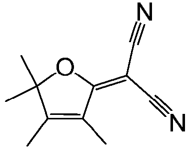 | | |
| | 1274821-70-3 | | |
| B505 | 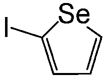 | 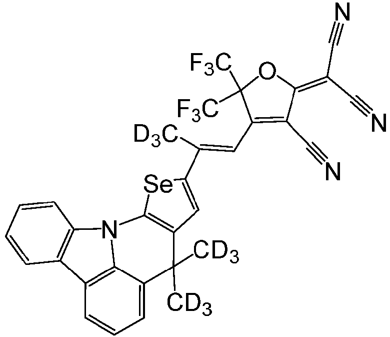 | 16 % |
| | 37686-36-5 | | |
| | 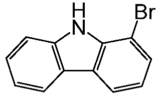 | | |
| | 16807-11-7 | | |
| | 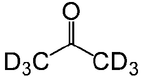 | | |
| | 666-52-4 | | |
| | 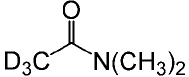 | | |
| | 20255-66-7 | | |
| | 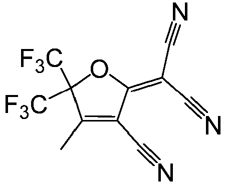 | | |
| | 2244199-35-5 | | |
| B506 | S5 | 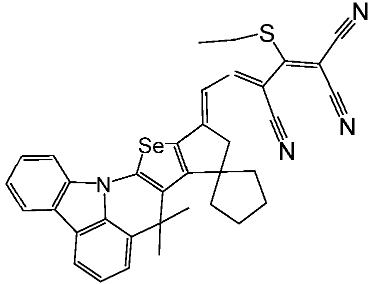 | 37 % |
| | nur Schritt 5 | | |
| | 100 °C / 5 h | | |
| | 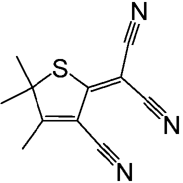 | | |
| | 400760-72-7 | | |
| B507 | 3437-95-4 | 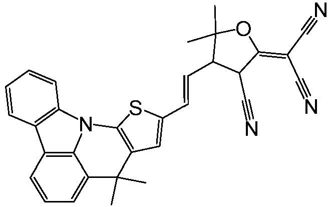 | 19 % |
| | 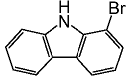 | | |
| | 16807-11-7 | | |
| | 67-64-1 | | |
| | 171082-32-9 | | |
| B508 | 3437-95-4 | 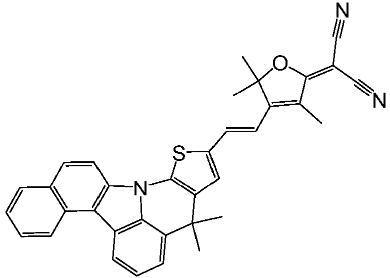 | 17 % |
| | 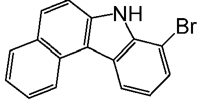 | | |
| | 1686099-80-8 | | |
| | 67-64-1 | | |
| | 1274821-70-3 | | |
| B509 | 3437-95-4 | 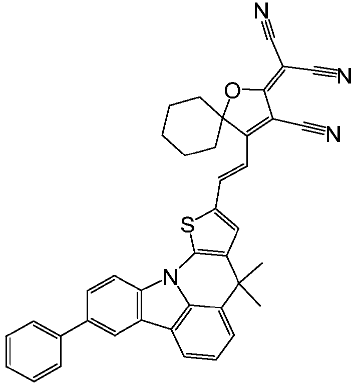 | |
| | 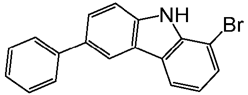 | | |
| | 1686099-79-5 | | |
| | 67-64-1 | | |
| | 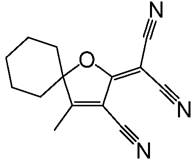 | | |
| | 383124-81-0 | | |
| B510 | 3437-95-4 | 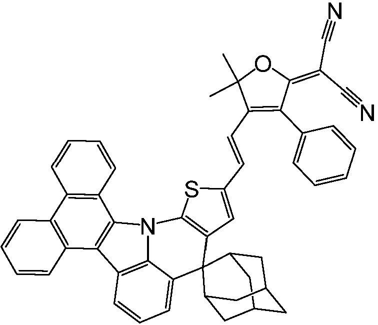 | 15 % |
| | 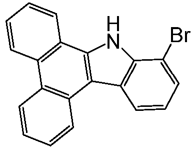 | | |
| | 1686100-44-6 | | |
| | 700-58-3 | | |
| | 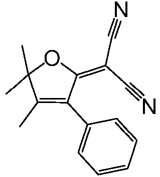 | | |
| | 1290106-98-7 | | |
| B511 | 3437-95-4 | 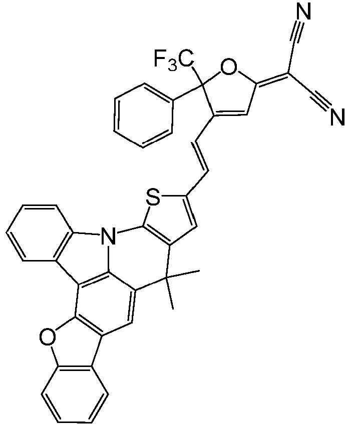 | 16 % |
| | 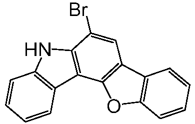 | | |
| | 1438427-35-0 | | |
| | 67-64-1 | | |
| | 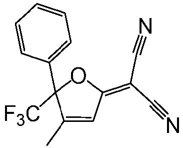 | | |
| | 2790414-09-2 | | |
| B512 | 3437-95-4 | 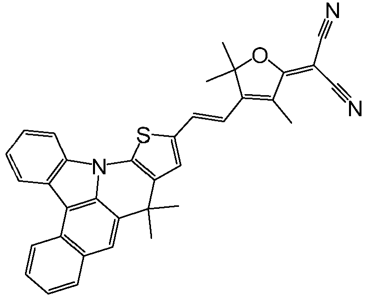 | 19 % |
| | 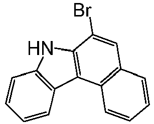 | | |
| | 1639452-47-3 | | |
| | 67-64-1 | | |
| | 1274821-70-3 | | |
| B513 | 3437-95-4 | 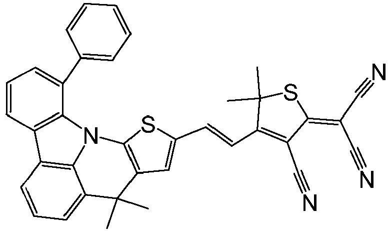 | 18 % |
| | 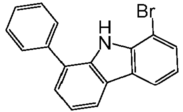 | | |
| | 2619631-74-0 | | |
| | 67-64-1 | | |
| | 400760-72-7 | | |
| B514 | 3437-95-4 | 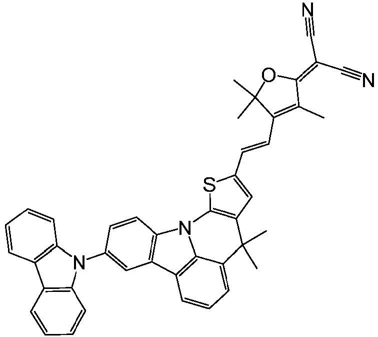 | 17 % |
| | 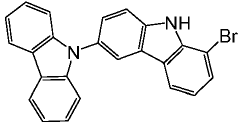 | | |
| | 2311845-06-2 | | |
| | 67-64-1 | | |
| | 1274821-70-3 | | |
| B515 | 3437-95-4 | 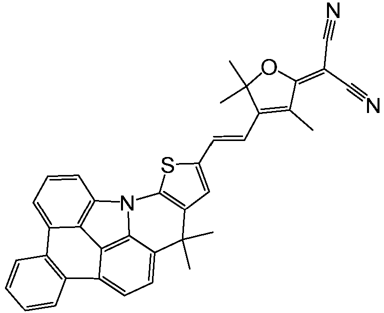 | 20 % |
| | 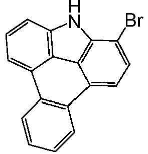 | | |
| | 2209068-40-4 | | |
| | 67-64-1 | | |
| | 1274821-70-3 | | |
| B516 | 3437-95-4 | 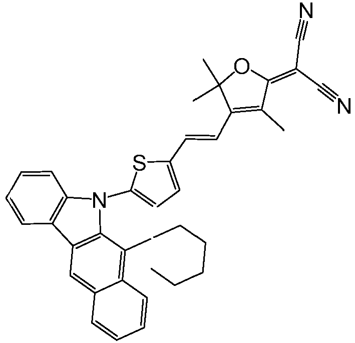 | 16 % |
| | 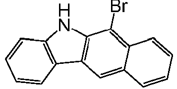 | | |
| | 2097681-89-3 | | |
| | 108-94-1 | | |
| | 1274821-70-3 | | |
| B517 | 3437-95-4 | 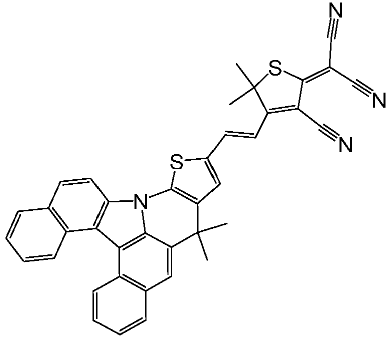 | 15 % |
| | 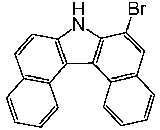 | | |
| | 2685783-06-4 | | |
| | 67-64-1 | | |
| | 400760-72-7 | | |
| B518 | 3437-95-4 | 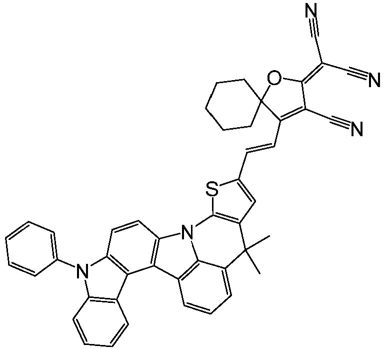 | 18 % |
| | 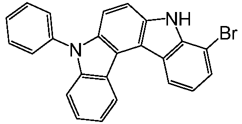 | | |
| | 1370555-67-1 | | |
| | 67-64-1 | | |
| | 383124-81-0 | | |
| B519 | 3437-95-4 | 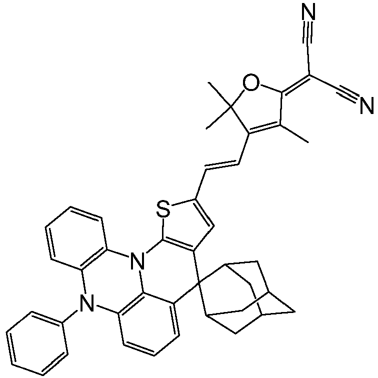 | 16 % |
| | 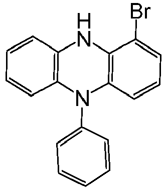 | | |
| | 2493276-29-0 | | |
| | 700-58-3 | | |
| | 1274821-70-3 | | |
| B520 | 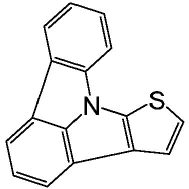 | 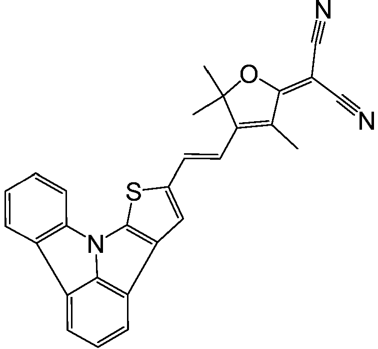 | 44 % |
| | 2201847-06-3 | | |
| | nur Schritt 5 | | |
| | 1274821-70-3 | | |

**Thermische Stabilität der erfindungsgemäßen Verbindungen:**
Tabelle 1 fasst die Ergebnisse der thermischen Auslagerungstest zusammen. Dazu werden 500 mg der Verbindungen B in einer 20 ml Duranglas-Ampulle unter Vakuum (p ~ 10⁻⁴ mbar) abgeschmolzen und dann 10 Tage bei der angegebenen Temperatur T (T = T-Vakuum-TGA (5% Gewichtsverlust + 40 °C) unter Lichtausschluss ausgelagert. Anschließend wird die Ampulle geöffnet, berochen und olfaktorisch bewertet, da schon geringste Mengen durch thermische Zersetzung gebildete, flüchtige Organo-Schwefel-, -Selen- und -Tellur-Verbindungen an ihrem charakteristischen Geruch nach fauligem Ei bzw. Rettich gut zu erkennen sind. Außerdem wird die Reinheit mittels HPLC bestimmt und mit der des Ausgangsmaterials, das eine Reinheit > 99.9 % aufweist, verglichen.

**Tabelle 1:**

| **Bsp.** | **Verbindung B** | **Temperatur T** | **Olfaktorische Bewertung** | **Reinheit HPLC** |
|---|---|---|---|---|
| TS1 | B1 | 236 °C | Kein Fehlgeruch | > 99.9 % |
| TS2 | B6 | 241 °C | Kein Fehlgeruch | > 99.9 % |
| TS3 | B22 | 251 °C | Kein Fehlgeruch | > 99.9 % |
| TS4 | B26 | 244 °C | Kein Fehlgeruch | > 99.9 % |
| TS5 | B25 | 255 °C | Kein Fehlgeruch | > 99.9 % |
| TS6 | B58 | 267 °C | Kein Fehlgeruch | > 99.9 % |
| TS7 | B500 | 266 °C | Kein Fehlgeruch | > 99.9 % |
| TS8 | B508 | 289 °C | Kein Fehlgeruch | > 99.9 % |

### Beispiele Photodioden:

### 1) Herstellung der Mono-Layer Photodioden (MLPD)

### Device-Typ 1:

Gereinigte Quarzsubstrate (15 min. Ultraschall im Aceton/iso-Propanol/ Wasser-Bad (1:1:1 v:v:v), dann UV-Ozon) werden via Sputtering mit einer 150 nm dicken Indium-Zinn-Oxid-Anode (ITO) versehen. Darauf wird im Hochvakuum durch co-Verdampfen eine 150 nm dicke Schichten aus den erfindungsgemäßen Verbindungen B und C₆₀ im Volumenverhältnis 1:1 aufgedampft. Abschließend wird durch Sputtering eine 10 nm dicke ITO Kathode aufgebracht. Anschließend wird die IPCE (Incident Photon to Charge Carrier Efficiency) der initialen Devices mit Hilfe eines PTS-2-QE, Fa. Photonic Solutions (UK) im Maximum der Absorption im Wellenlängenbereich 400-700 nm bei einer Spannung von 10 V bestimmt (Tabelle 2). Dann werden die Devices für 10 min. bei einer Temperatur von 180 °C thermisch ausgelagert, danach wird erneut IPCE ermittelt.

**Tabelle 2:**

| Bsp. | Verbindung | IPCE (initial, %) | IPCE (therm. ausgelagert 10 min. / 180 °C, %) |
|---|---|---|---|
| D1 | B1 | 70 | 68 |
| D2 | B100 | 75 | 72 |

### Device-Typ 2:

Gereinigte Quarzsubstrate (15 min. Ultraschall im Aceton/iso-Propanol/ Wasser-Bad (1:1:1 v:v:v), dann UV-Ozon) werden via Sputtering mit einer 150 nm dicken Indium-Zinn-Oxid-Anode (ITO) versehen. Darauf wird im Hochvakuum eine 30 nm dicke Schicht aus HTM2 (s. Tabelle 5) und dann durch co-Verdampfen eine 80 nm dicke Schicht aus den erfindungsgemäßen Verbindungen B und C₆₀ im Volumenverhältnis 1:1 aufgedampft. Anschließend wird eine 1.5 nm dicke Ytterbium-Schicht aufgedampft. Abschließend wird durch Sputtering eine 10 nm dicke ITO Kathode aufgebracht. Anschließend wird die IPCE (Incident Photon to Charge Carrier Efficiency) der initialen Devices mit Hilfe eines PTS-2-QE, Fa. Photonic Solutions (UK) im Maximum der Absorption im Wellenlängenbereich 400 - 700 nm bei einer Spannung von 3 V bestimmt (Tabelle 3).

**Tabelle 3:**

| Bsp. | Verbindung | IPCE |
|---|---|---|
| D10 | B3 | 21 |
| D11 | B4 | 17 |
| D12 | B8 | 61 |
| D13 | B11 | 51 |
| D14 | B14 | 48 |
| D15 | B26 | 37 |
| D16 | B35 | 40 |

### 2) Herstellung der Bi-Layer Photodioden (BLPD)

Gereinigte Quarzsubstrate (15 min. Ultraschall im Aceton/iso-Propanol/ Wasser-Bad (1:1:1 v:v:v), dann UV-Ozon) werden via Sputtering mit einer 150 nm dicken Indium-Zinn-Oxid-Anode (ITO) Versehen. Alle weiteren Materialien werden in einer Vakuumkammer thermisch aufgedampft. Die zur Herstellung der BLPDs verwendeten Materialien sind in Tabelle 5 gezeigt. Die Elektronentransportschicht 2 (ETL2) kann durch Co-Verdampfen zweier Materialen hergestellt werden. Eine Angabe wie ETM1:EIL (50:50) bedeutet, dass die co-verdampfte Schicht jeweils 50 Volumen-% der Einzelmaterialien enthält.

### Aufbau der BLPD:

ITO-Substrat-BLPD
Lochinjektionsschicht (HIL) aus HTM1 dotiert mit 5 % NDP-9 (kommerziell erhältlich von der Fa. Novaled), 10 nm
Lochtransportschicht 1 (HTL1), s. Tabelle 4
Lochtransportschicht 2 (HTL2), s. Tabelle 4
Elektronendonorschichtschicht (EDL), s. Tabelle 4
Elektronenakzeptorschicht (EAL), s. Tabelle 4
Elektronentransportschicht 1 (ETL1), s.Tabelle 4
Elektronentransportschicht 2 (ETL2), s.Tabelle 4
Elektroneninjektionsschicht 1 (EIL1), 3 nm EIM
Kathode aus Magnesium:Silber (10:90), 100 nm

Anschließend wird die IPCE (Incident Photon to Charge Carrier Efficiency) der initialen Devices mit Hilfe eines PTS-2-QE, Fa. Photonic Solutions (UK) im Maximum der Absorption im Wellenlängenbereich 400-700 nm bei einer Spannung von 3 V bestimmt (Tabelle 4).

**Tabelle 4: Aufbau Bi-Layer Photodioden (BLPD)**

| **Bsp.** | **HTL1** Dicke | **HTL2** Dicke | **EDL** Dicke | **EAL** Dicke | **ETL1** Dicke | **ETL2** Dicke | IPCE % |
|---|---|---|---|---|---|---|---|
| D30 | HTM1 70 nm | --- | B1 15 nm | EAM1 35 nm | --- | ETM2 30 nm | 25 |
| D31 | HTM1 70 nm | --- | B5 15 nm | EAM1 35 nm | ETM1 5 nm | ETM2 30 nm | 19 |
| D32 | HTM1 50 nm | HTM2 20 nm | B12 15 nm | EAM1 35 nm | ETM1 5 nm | ETM2 30 nm | 24 |
| D33 | HTM1 50 nm | HTM2 20 nm | B18 15 nm | EAM1 35 nm | ETM1 5 nm | ETM1:EIM (50:50) 30 nm | 29 |
| D34 | HTM1 50 nm | HTM2 20 nm | B19 10 nm | EAM1 35 nm | ETM1 5 nm | ETM1:EIM (50:50) 30 nm | 36 |
| D35 | HTM1 50 nm | HTM2 20 nm | B23 15 nm | EAM1 35 nm | ETM1 5 nm | ETM1:EIM (50:50) 30 nm | 39 |
| D36 | HTM1 50 nm | HTM2 20 nm | B27 15 nm | EAM1 35 nm | ETM1 5 nm | ETM1:EIM (50:50) 30 nm | 28 |
| D37 | HTM1 50 nm | HTM2 20 nm | B31 10 nm | EAM1 35 nm | ETM1 5 nm | ETM1:EIM (50:50) 30 nm | 27 |
| D38 | HTM1 50 nm | HTM2 20 nm | B37 10 nm | EAM1 35 nm | ETM1 5 nm | ETM1:EIM (50:50) 30 nm | 32 |
| D39 | HTM1 50 nm | HTM2 20 nm | B45 10 nm | EAM1 40 nm | ETM1 5 nm | ETM1:EIM (50:50) 30 nm | 30 |
| D40 | HTM1 50 nm | HTM2 20 nm | B52 15 nm | EAM1 35 nm | ETM1 5 nm | ETM1:EIM (50:50) 30 nm | 28 |
| D41 | HTM1 50 nm | HTM2 20 nm | B54 15 nm | EAM1 35 nm | ETM1 5 nm | ETM1:EIM (50:50) 30 nm | 24 |
| D42 | HTM1 50 nm | HTM2 20 nm | B58 15 nm | EAM1 35 nm | ETM1 5 nm | ETM1:EIM (50:50) 30 nm | 33 |
| D43 | HTM1 50 nm | HTM2 20 nm | B62 15 nm | EAM1 35 nm | ETM1 5 nm | ETM1:EIM (50:50) 30 nm | 35 |
| D44 | HTM1 50 nm | HTM2 20 nm | B64 15 nm | EAM1 35 nm | ETM1 5 nm | ETM1:EIM (50:50) 30 nm | 28 |
| D45 | HTM1 50 nm | HTM2 20 nm | B67 15 nm | EAM1 35 nm | ETM1 5 nm | ETM1:EIM (50:50) 30 nm | 22 |
| D46 | HTM1 50 nm | HTM2 20 nm | B74 15 nm | EAM1 35 nm | ETM1 5 nm | ETM1:EIM (50:50) 30 nm | 29 |
| D47 | HTM1 50 nm | HTM2 20 nm | B77 15 nm | EAM1 35 nm | ETM1 5 nm | ETM1:EIL (50:50) 30 nm | 35 |
| D48 | HTM1 50 nm | HTM2 20 nm | B82 15 nm | EAM1 35 nm | ETM1 5 nm | ETM1:EIM (50:50) 30 nm | 29 |
| D49 | HTM1 50 nm | HTM2 20 nm | B86 15 nm | EAM1 35 nm | ETM1 5 nm | ETM1:EIM (50:50) 30 nm | 27 |
| D50 | HTM1 50 nm | HTM2 20 nm | B89 15 nm | EAM1 35 nm | ETM1 5 nm | ETM1:EIM (50:50) 30 nm | 38 |
| D51 | HTM1 50 nm | HTM2 20 nm | B102 15 nm | EAM1 35 nm | ETM1 5 nm | ETM1:EIM (50:50) 30 nm | 33 |
| D52 | HTM1 50 nm | HTM2 20 nm | B201 15 nm | EAM1 35 nm | ETM1 5 nm | ETM1:EIM (50:50) 30 nm | 17 |
| D53 | HTM1 50 nm | HTM2 20 nm | B300 15 nm | EAM1 35 nm | ETM1 5 nm | ETM1:EIM (50:50) 30 nm | 28 |
| D54 | HTM1 50 nm | HTM2 20 nm | B401 15 nm | EAM1 35 nm | ETM1 5 nm | ETM1:EIM (50:50) 30 nm | 33 |
| D55 | HTM1 50 nm | HTM2 20 nm | B402 15 nm | EAM1 35 nm | ETM1 5 nm | ETM1:EIM (50:50) 30 nm | 35 |
| D56 | HTM1 50 nm | HTM2 20 nm | B500 15 nm | EAM1 35 nm | ETM1 5 nm | ETM1:EIM (50:50) 30 nm | 46 |
| D57 | HTM1 50 nm | HTM2 20 nm | B502 15 nm | EAM1 35 nm | ETM1 5 nm | ETM1:EIM (50:50) 30 nm | 45 |
| D58 | HTM1 50 nm | HTM2 20 nm | B504 15 nm | EAM1 35 nm | ETM1 5 nm | ETM1:EIM (50:50) 30 nm | 49 |
| D59 | HTM1 50 nm | HTM2 20 nm | B506 15 nm | EAM1 35 nm | ETM1 5 nm | ETM1:EIM (50:50) 30 nm | 47 |
| D60 | HTM1 50 nm | HTM2 20 nm | B507 15 nm | EAM1 35 nm | ETM1 5 nm | ETM1:EIM (50:50) 30 nm | 39 |
| D61 | HTM1 50 nm | HTM2 20 nm | B508 15 nm | EAM1 35 nm | ETM1 5 nm | ETM1:EIM (50:50) 30 nm | 42 |
| D62 | HTM1 50 nm | HTM2 20 nm | B510 15 nm | EAM1 35 nm | ETM1 5 nm | ETM1:EIM (50:50) 30 nm | 40 |
| D63 | HTM1 50 nm | HTM2 20 nm | B512 15 nm | EAM1 35 nm | ETM1 5 nm | ETM1:EIM (50:50) 30 nm | 41 |
| D64 | HTM1 50 nm | HTM2 20 nm | B516 15 nm | EAM1 35 nm | ETM1 5 nm | ETM1:EIM (50:50) 30 nm | 43 |
| D65 | HTM1 50 nm | HTM2 20 nm | B518 15 nm | EAM1 35 nm | ETM1 5 nm | ETM1:EIM (50:50) 30 nm | 40 |
| D66 | HTM1 50 nm | HTM2 20 nm | B519 15 nm | EAM1 35 nm | ETM1 5 nm | ETM1:EIM (50:50) 30 nm | 41 |
| D67 | HTM1 50 nm | HTM2 20 nm | B520 15 nm | EAM1 35 nm | ETM1 5 nm | ETM1:EIM (50:50) 30 nm | 44 |

**Tabelle 5: Verwendete Materialien**

| | |
|---|---|
| 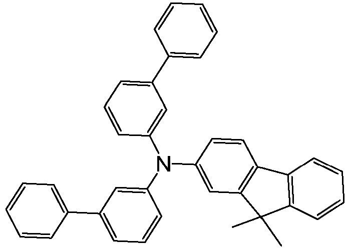 2306796-00-7 | 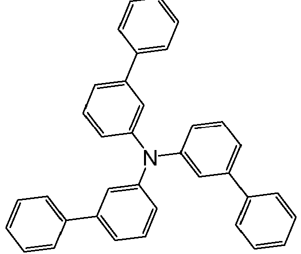 1092356-32-5 |
| HTM1 | HTM2 |
| 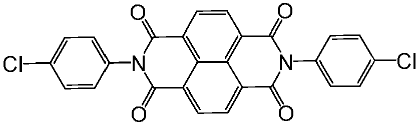 64005-91-0 | 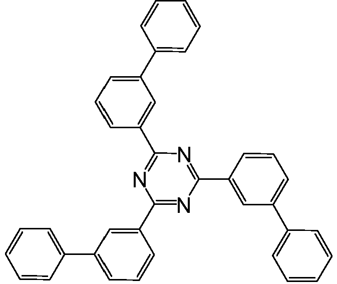 ETM1 |
| EAM1 | |
| 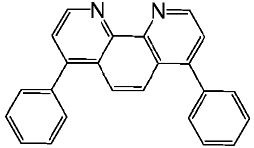 | 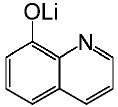 25387-93-3 |
| 1662-01-7 | EIM |
| ETM2 | |

## Patentansprüche

1. Verbindung umfassend mindestens eine Struktur der Formel (I), 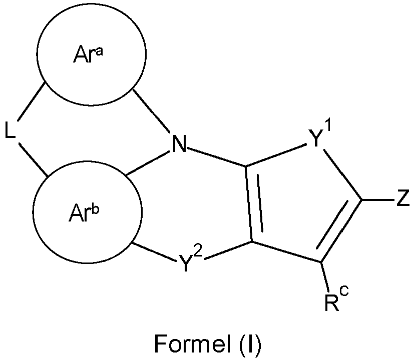 wobei für die verwendeten Symbole gilt:
Ar^{a} ist bei jedem Auftreten gleich oder verschieden ein aromatisches oder heteroaromatisches Ringsystem mit 5 bis 60 aromatischen Ringatomen, das mit einem oder mehreren Resten Ar oder R^{a} substituiert sein kann;
Ar^{b} ist bei jedem Auftreten gleich oder verschieden für aromatisches oder heteroaromatisches Ringsystem mit 5 bis 60 aromatischen Ringatomen, das mit einem oder mehreren Resten Ar oder R^{b} substituiert sein kann;
Z ist bei jedem Auftreten gleich oder verschieden eine Alkenylgruppe mit 2 bis 40 C-Atomen, wobei die Alkenylgruppe mit einem oder mehreren Resten R^{d} substituiert sein kann, wobei eine oder mehrere nicht benachbarte CH₂-Gruppen durch R^{d}C=CR^{d}, C=C, Si(R^{d})₂, C=O, C=S, C=Se, C=NR, C(=O)O, C(=O)NR^{d}, NR, P(=O)( R^{d}), O, S, SO oder SO₂ ersetzt sein können, oder ein aromatisches oder heteroaromatisches Ringsystem mit 5 bis 60 aromatischen Ringatomen, das jeweils durch einen oder mehrere Reste R^{d} substituiert sein kann; dabei kann die Gruppe mit einer weiteren Gruppe, beispielsweise einer Gruppe R oder R^{c}, ein Ringsystem bilden; mit der Maßgabe, dass die Gruppe Z mindestens zwei CN-Gruppen aufweist;
Y¹ ist bei jedem Auftreten gleich oder verschieden O, S, Se, Te, NAr, NR, C(R)₂,Ge(R)₂, Si(R)₂;
Y² ist bei jedem Auftreten gleich oder verschieden eine Bindung, O, S, Se, Te, NAr, NR^{e}, BAr, BR^{e}, C(R^{e})₂, Ge(R^{e})₂, Si(R^{e})₂, CR^{e}=CR^{e}, C=NR^{e}, S=O, SO₂;
L ist bei jedem Auftreten gleich oder verschieden eine Verbindungsgruppe, vorzugsweise eine Bindung, C(R)₂, O, S, NR, NAr, C(=O), BAr, B(R), Si(R)₂, C=NR, C=C(R)₂, S=O, SO₂, P(R) und P(=O)R, -CR=CR- oder ein aromatisches oder heteroaromatisches Ringsystem mit 5 bis 40 aromatischen Ringatomen darstellt, welches durch einen oder mehrere Reste R substituiert sein kann;
Ar ist bei jedem Auftreten gleich oder verschieden ein aromatisches oder heteroaromatisches Ringsystem mit 5 bis 60 aromatischen Ringatomen, das mit einem oder mehreren Resten R substituiert sein kann; hierbei kann die Gruppe Ar mit mindestens einer Gruppe Ar, R, R^{a}, R^{b}, R^{c}, R^{d}, R^{e} oder einer weiteren Gruppe ein Ringsystem bilden;
R, R^{a}, R^{b}, R^{c}, R^{d}, R^{e} ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, -C(=O)O-, -C(=O)NR¹-, NR¹, P(=O)(R¹), -Se-, -Te-, NAr',BR¹, Ge(R¹)₂, -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; eine Arylthio - oder Heteroarylthiogruppe mit 5 bis 60 aromatischen Ringatomen, die durch einen oder mehrere Reste R¹ substituiert sein kann, oder eine Diarylamino-, Arylheteroarylamino-, Diheteroarylaminogruppe mit 5 bis 60 aromatischen Ringatomen, die durch einen oder mehrere Reste R¹ substituiert sein kann, oder eine Aralkyl- oder Heteroaralkylgruppe mit 5 bis 60 aromatischen Ringatomen und 1 bis 10 C-Atomen im Alkylrest, die durch einen oder mehrere Reste R¹ substituiert sein kann; dabei können zwei Reste R, R^{a}, R^{b}, R^{c}, R^{d}, R^{e} auch miteinander oder mit einer weiteren Gruppe ein Ringsystem bilden;
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
R¹ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², C(=O)O, C(=O)NR², NR², P(=O)(R²), O, S, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere Reste R¹ miteinander ein Ringsystem bilden; dabei können einer oder mehrere Reste R¹ mit einem weiteren Teil der Verbindung ein Ringsystem bilden;
Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehrere Substituenten R² miteinander ein Ringsystem bilden.

2. Verbindung nach Anspruch 1, **dadurch gekennzeichnet, dass** die Gruppe Z eine Teilstruktur der Formel (Z-1) oder (Z-2) darstellt oder umfasst, 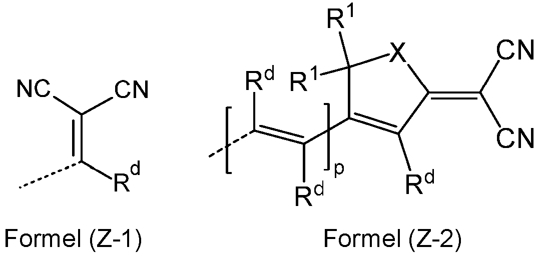 wobei R^{d} und R¹ die in Anspruch 1 genannte Bedeutung aufweist, die gestrichelte Bindung die Anbindungsstelle darstellt und weiterhin gilt:
X ist O, S oder Se;
p ist 0, 1 oder 2.

3. Verbindung nach Anspruch 1 oder 2, umfassend mindestens eine Struktur der Formel (II) oder (IIa), 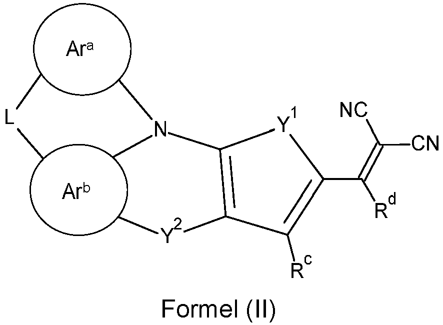 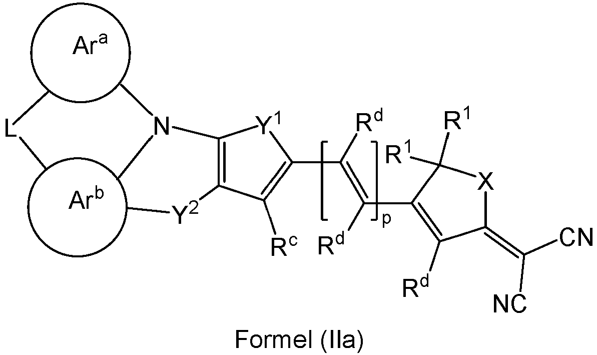 wobei die Symbole Ar^{a}, Ar^{b}, R^{c}, R^{d}, Y¹, Y², R¹ und L die in Anspruch 1 und X und p die in Anspruch 2 genannten Bedeutungen aufweisen.

4. Verbindung nach einem oder mehreren der Ansprüche 1 bis 3, umfassend mindestens eine Struktur der Formel (III) oder (IIIa), 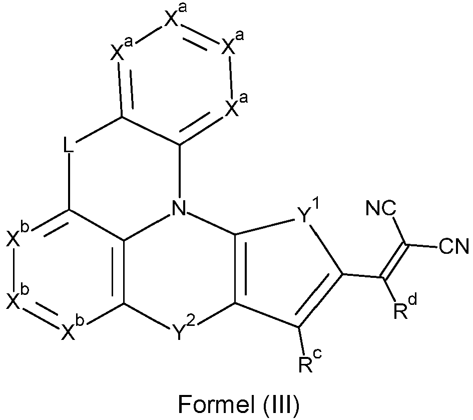 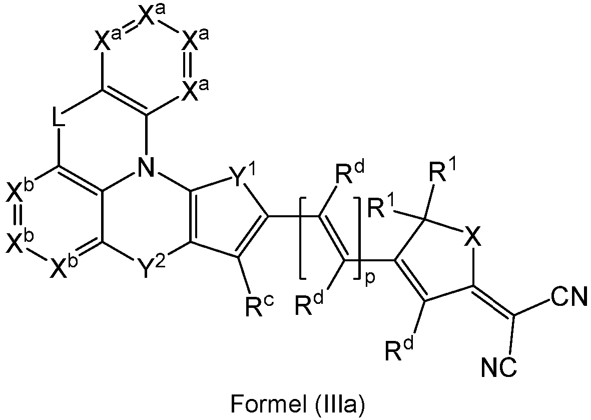 wobei die Symbole R^{c}, R^{d}, Y¹, Y², R¹ und L die in Anspruch 1 und X und p die in Anspruch 2 genannten Bedeutungen aufweisen und die weiteren Symbole die folgende Bedeutung aufweisen:
X^{a} steht bei jedem Auftreten gleich oder verschieden für N oder CR^{a}, mit der Maßgabe, dass nicht mehr als zwei der Gruppen X^{a} in einem Cyclus für N stehen, wobei R^{a} die in Anspruch 1 dargelegte Bedeutung aufweist; oder zwei benachbarte X^{a} stehen zusammen für eine Gruppe der folgenden Formel, 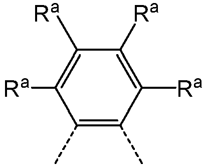 wobei die gestrichelten Bindungen die Verknüpfung der Gruppe innerhalb der Struktur darstellen;
X^{b} steht bei jedem Auftreten gleich oder verschieden für N oder CR^{b}, mit der Maßgabe, dass nicht mehr als zwei der Gruppen X^{b} in einem Cyclus für N stehen, wobei R^{b} die in Anspruch 1 dargelegte Bedeutung aufweist; oder zwei benachbarte X^{b} stehen zusammen für eine Gruppe der folgenden Formel, 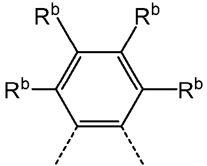 wobei die gestrichelten Bindungen die Verknüpfung der Gruppe innerhalb der Struktur darstellen.

5. Verbindung nach einem oder mehreren der Ansprüche 1 bis 4, umfassend mindestens eine Struktur der Formel (IV) oder (IVa) bis (IVf), 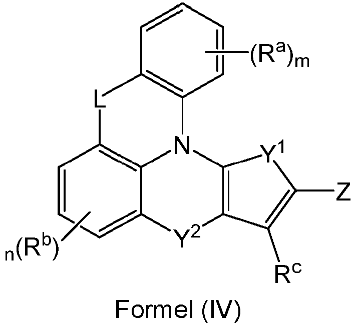 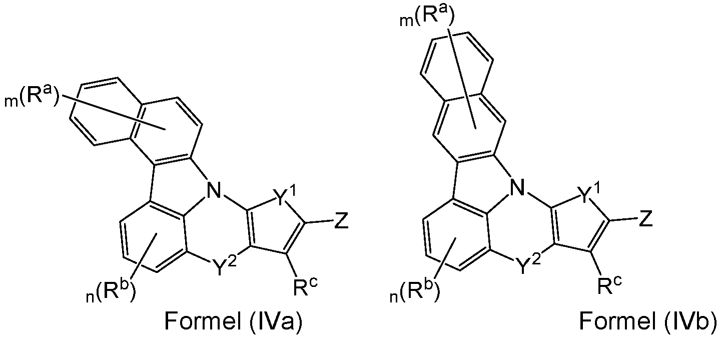 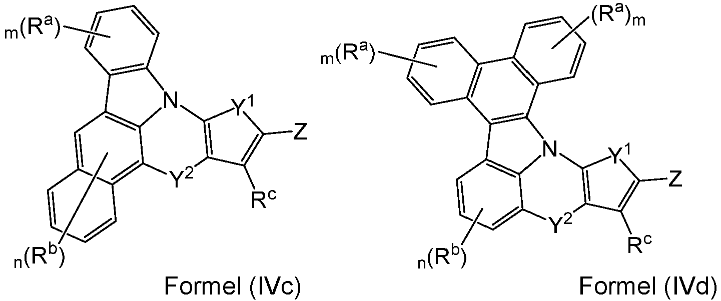 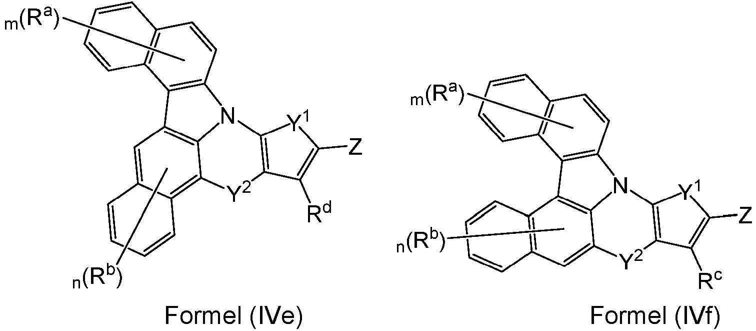 wobei die Verbindungen auch teilweise oder vollständig deuteriert sein können, die Symbole R^{a}, R^{b}, R^{c}, R^{d}, Y¹, y² und L die in Anspruch 1 genannten Bedeutungen aufweisen, Z die in Anspruch 2 genannten Bedeutungen aufweist und die weiteren Symbole die folgende Bedeutung aufweisen:
m ist 0, 1, 2, 3 oder 4;
n ist 0, 1, 2 oder 3.

6. Verbindung nach einem oder mehreren der Ansprüche 1 bis 5, **dadurch gekennzeichnet, dass** die Gruppe L für eine Bindung, C(R)₂, O, S, NAr oder NR steht, die Gruppe Y² für C(R^{e})₂ steht und die Gruppe Y¹ für O, S, Se, Te, NR, NAr, vorzugsweise S oder Se steht.

7. Verbindung nach einem oder mehreren der Ansprüche 1 bis 6, **dadurch gekennzeichnet, dass** der Rest R^{e} der Gruppe Y² für eine geradkettige Alkylgruppe mit 1 bis 10 C Atomen, für eine verzweigte oder cyclische Alkylgruppe mit 3 bis 12 C-Atomen, wobei die Alkylgruppe jeweils mit einem oder mehreren Resten R¹ substituiert sein kann, oder für eine Aryl- oder Heteroarylgruppe mit 5 bis 12 aromatischen Ringatomen steht, die durch einen oder mehrere Reste R¹ substituiert sein kann, wobei die zwei Reste R^{e} der Gruppe Y² zusammen einen Ring mit 5 bis 10 Ringatomen bilden können, der durch einen oder mehrere Reste R¹ substituiert sein kann.

8. Verbindung nach einem oder mehreren der Ansprüche 1 bis 7, **dadurch gekennzeichnet, dass** der Rest R^{c} für H, D, eine geradkettige Alkylgruppe mit 1 bis 10 C Atomen, eine verzweigte oder cyclische Alkylgruppe mit 3 bis 12 C-Atomen, wobei die Alkylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=Se, C=NR¹, C(=O)O, C(=O)NR¹, NR¹, P(=O)(R¹), O, S, SO oder SO₂ ersetzt sein können, oder eine Aryl- oder Heteroarylgruppe mit 5 bis 12 aromatischen Ringatomen steht, die durch einen oder mehrere Reste R¹ substituiert sein kann, wobei der Rest R^{c} mit dem Rest R^{d} zusammen einen Ring mit 5 bis 10 Ringatomen bilden kann, der durch einen oder mehrere Reste R¹ substituiert sein kann.

9. Verbindung nach einem oder mehreren der Ansprüche 1 bis 8, umfassend mindestens eine Struktur der Formeln (V-1) bis (V-80), 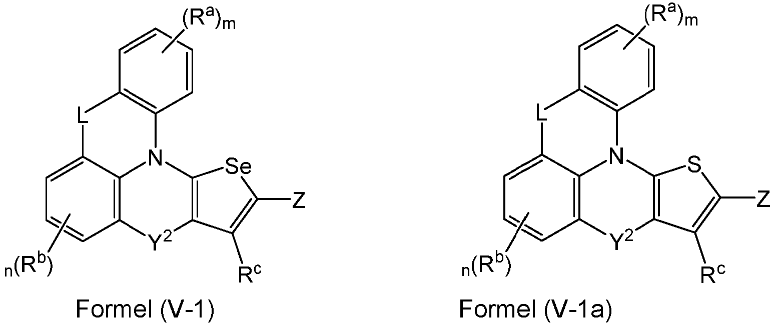 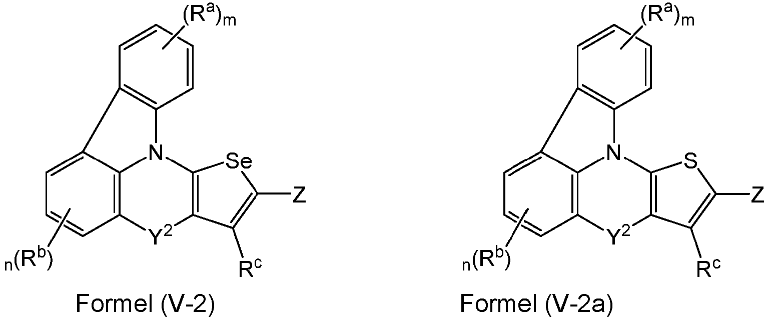 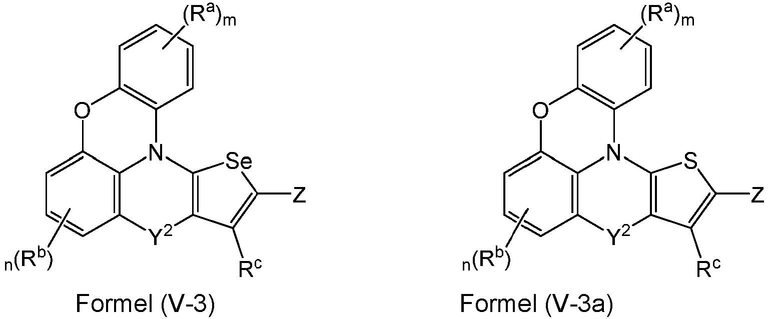 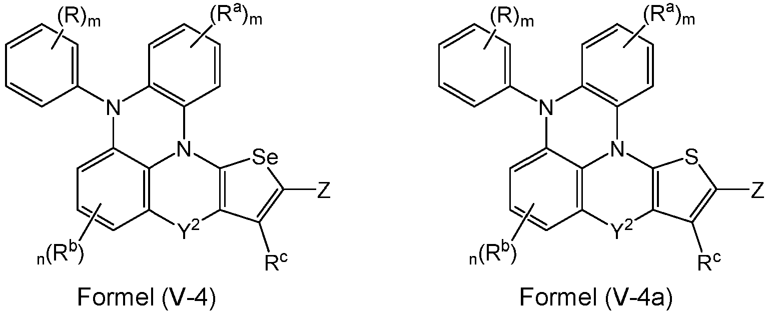 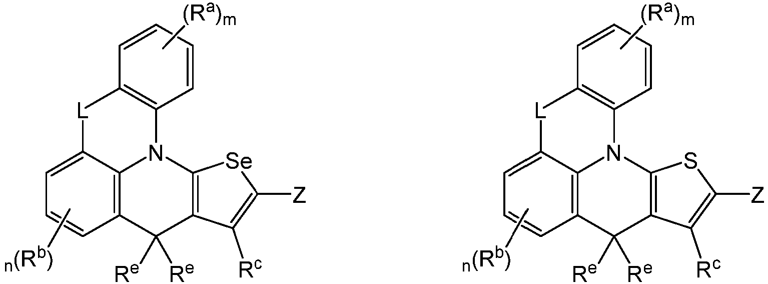 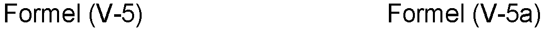 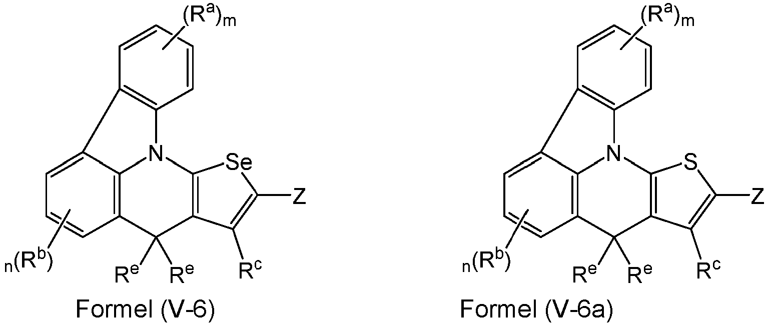 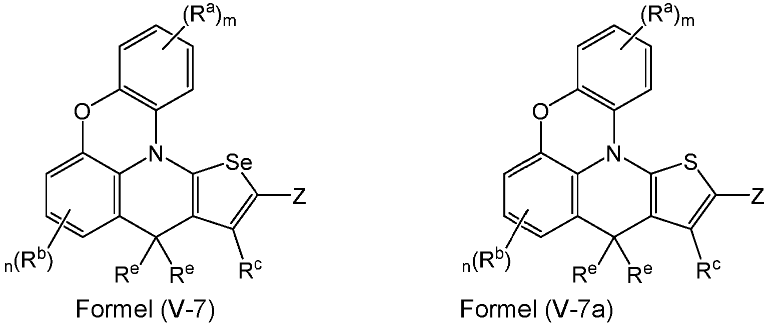 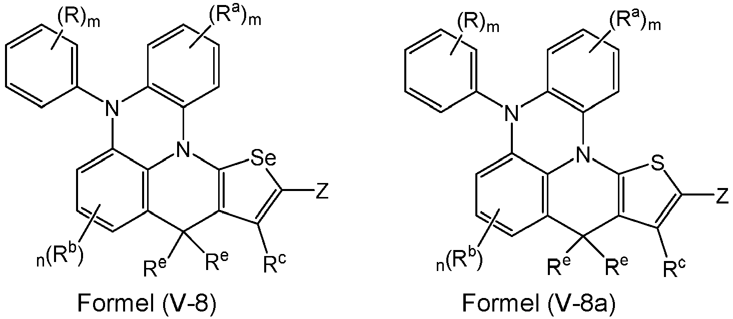 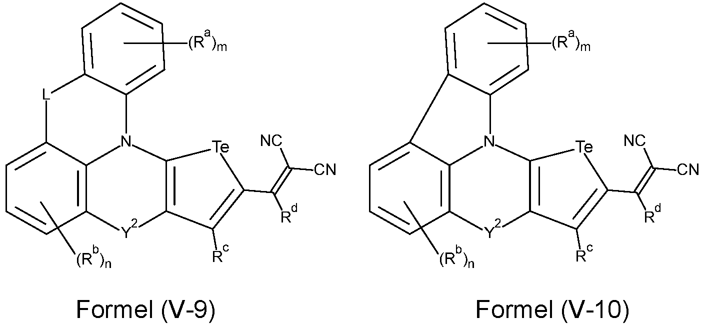 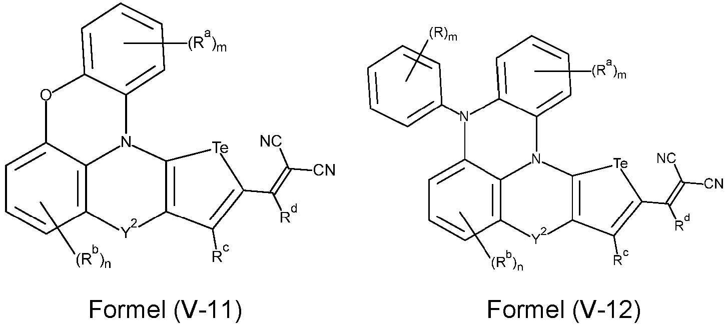 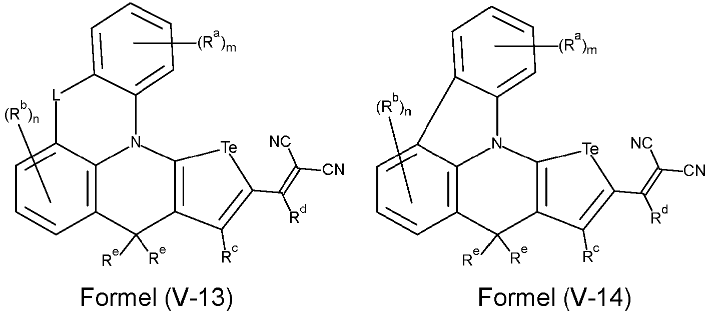 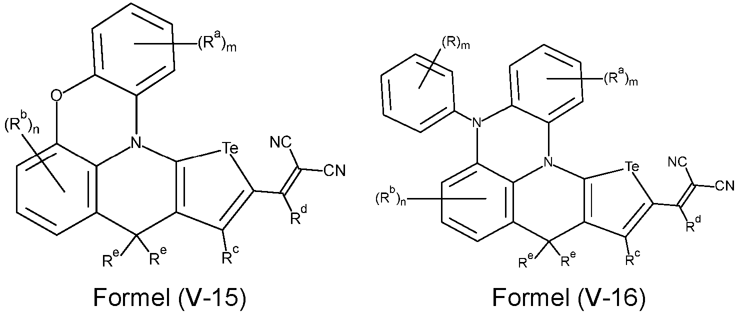 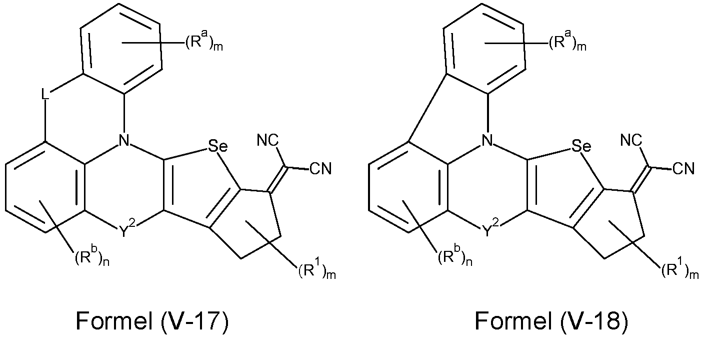 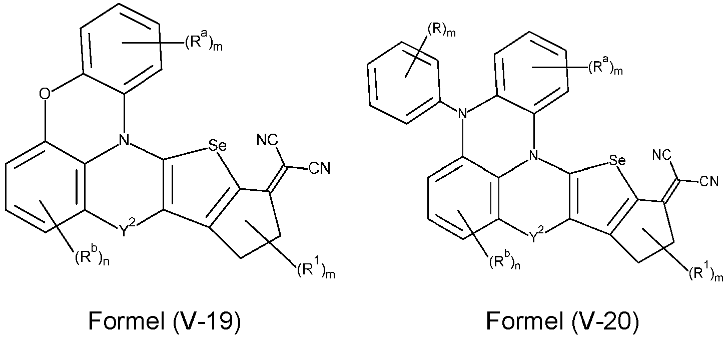 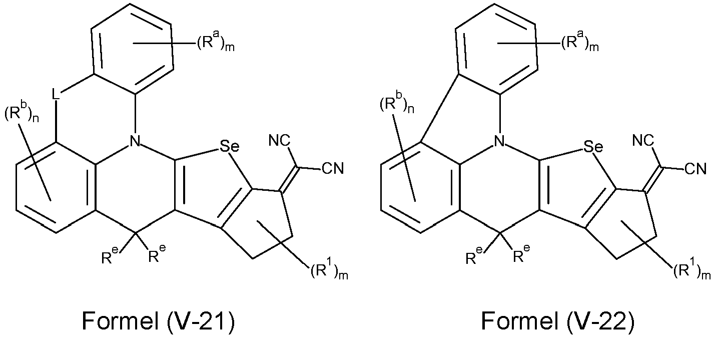 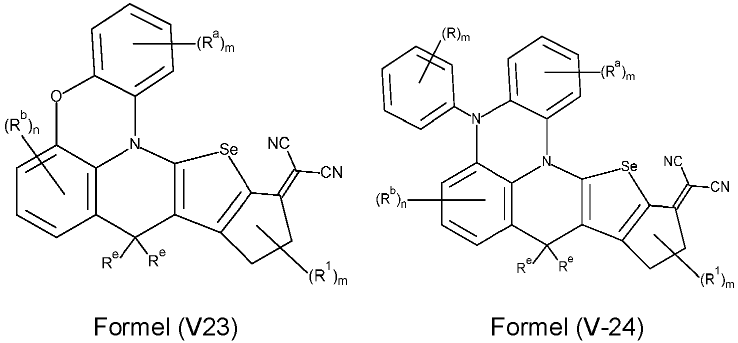 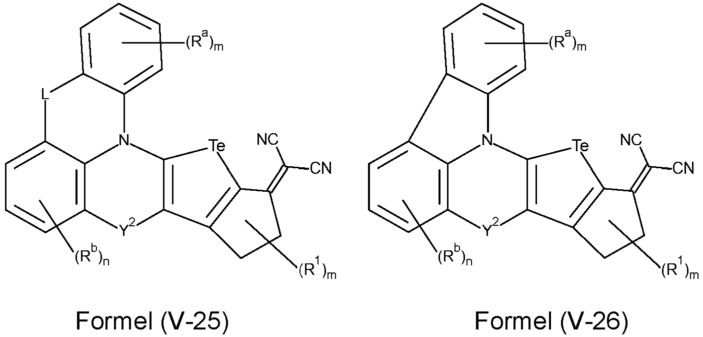 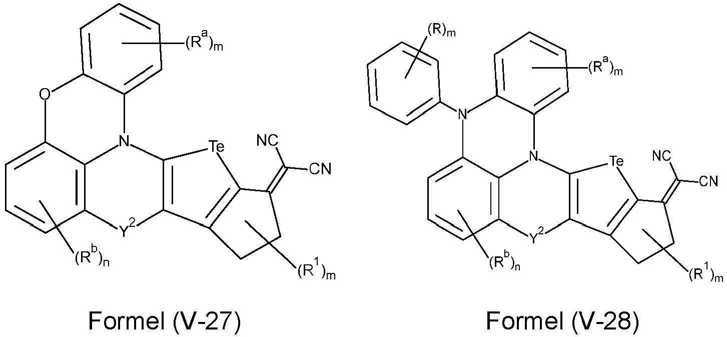 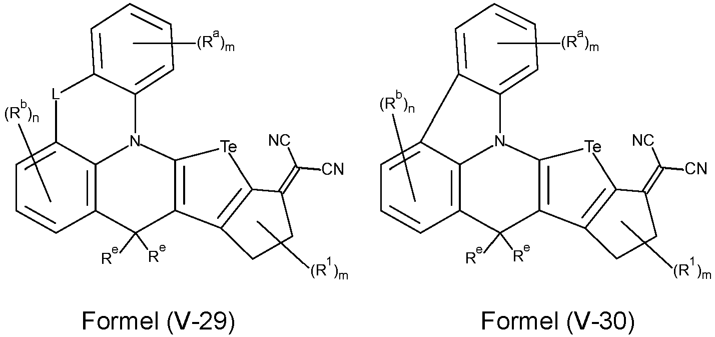 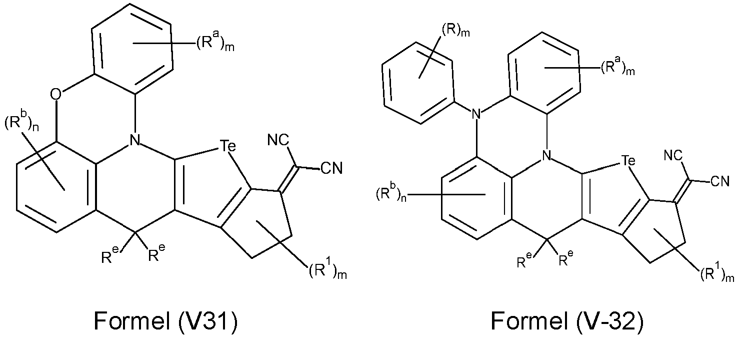 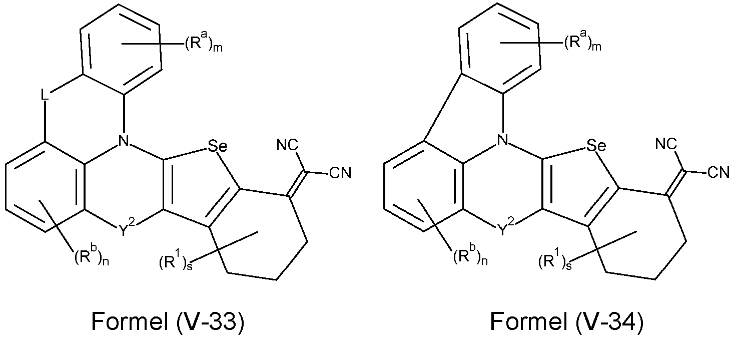 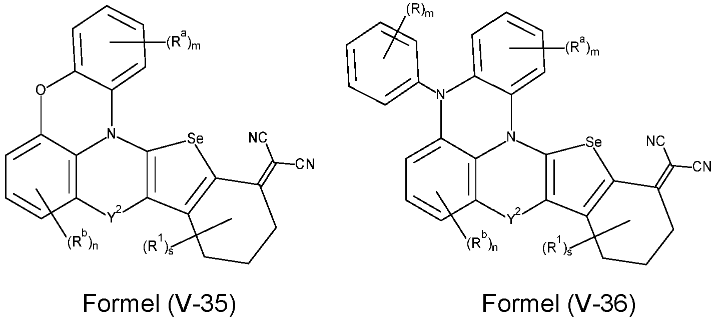 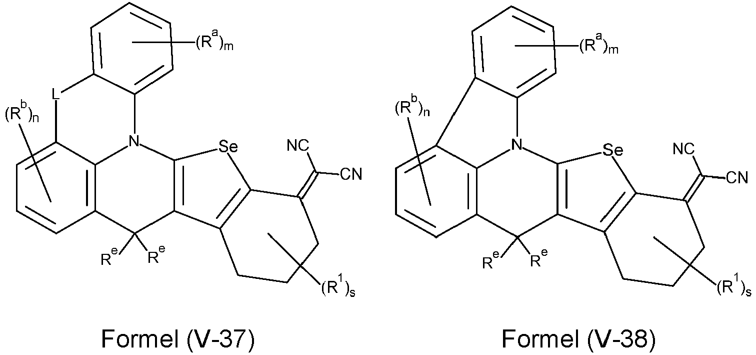 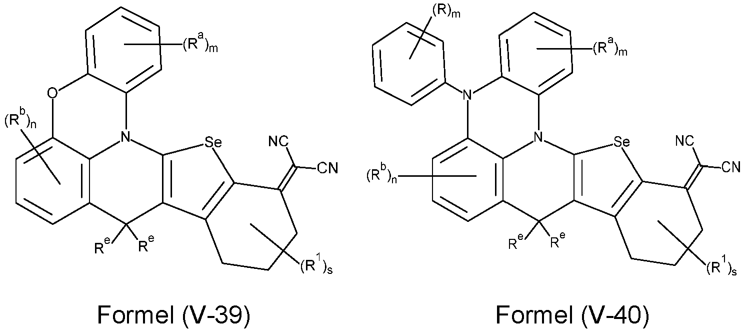 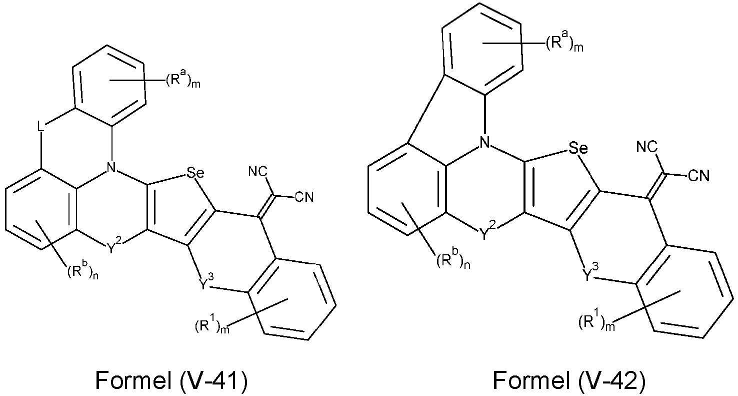 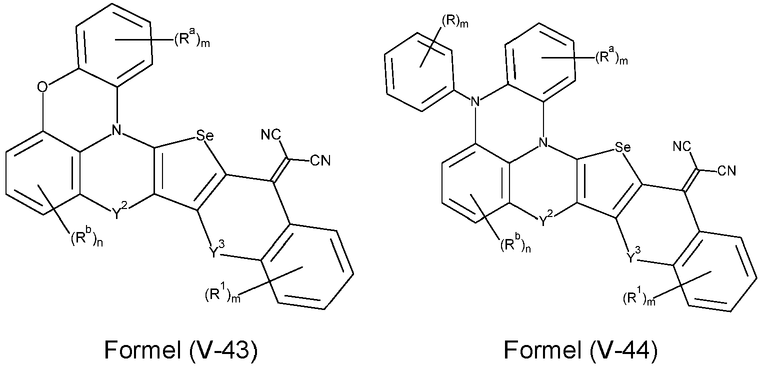 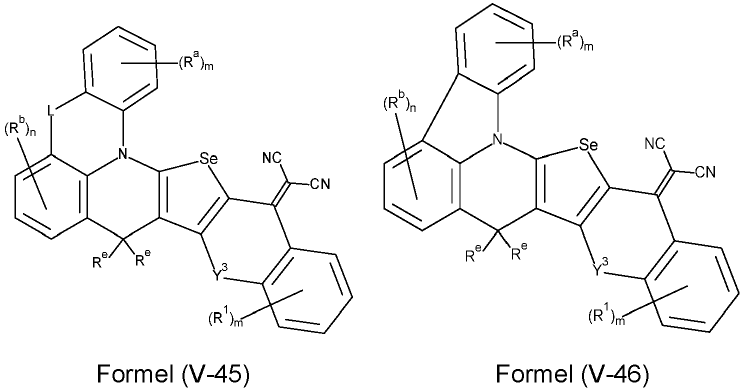 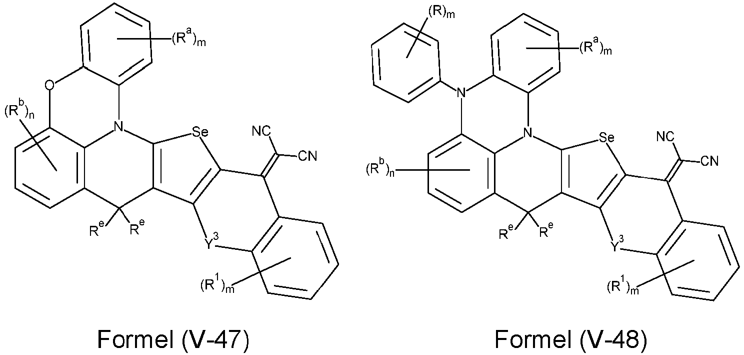 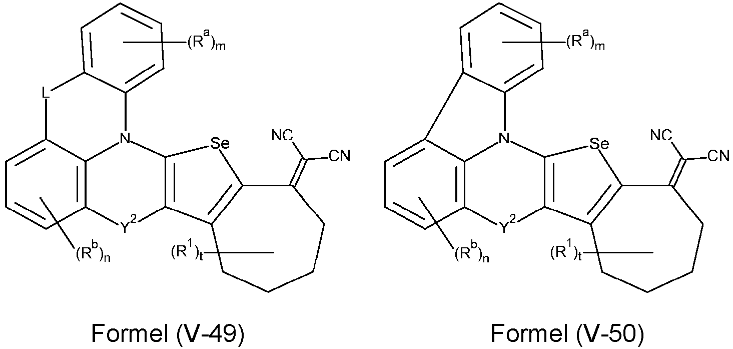 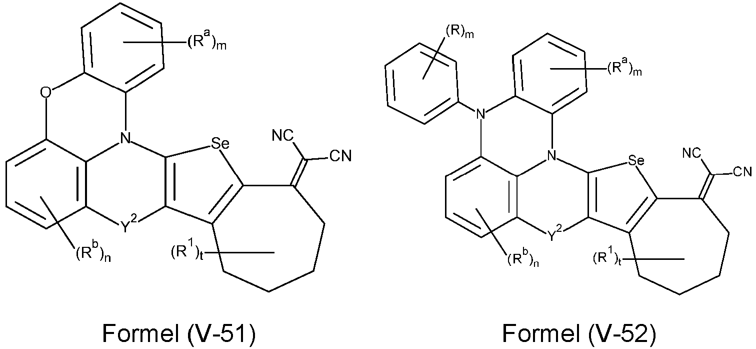 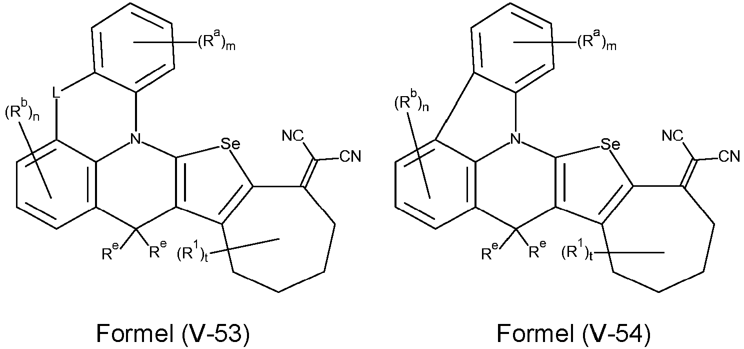 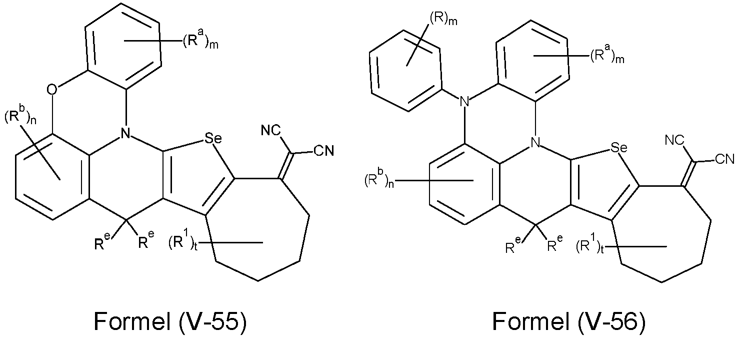 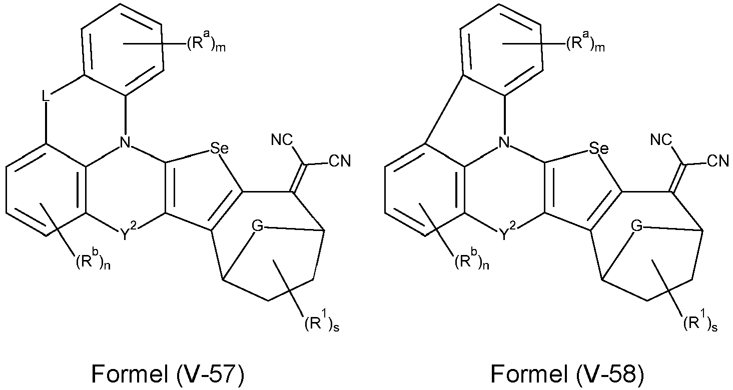 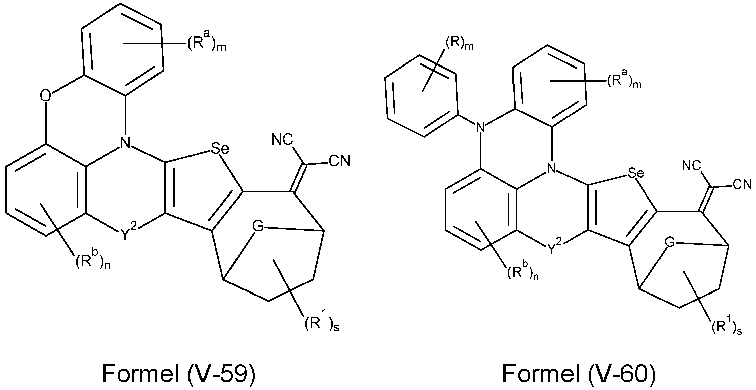 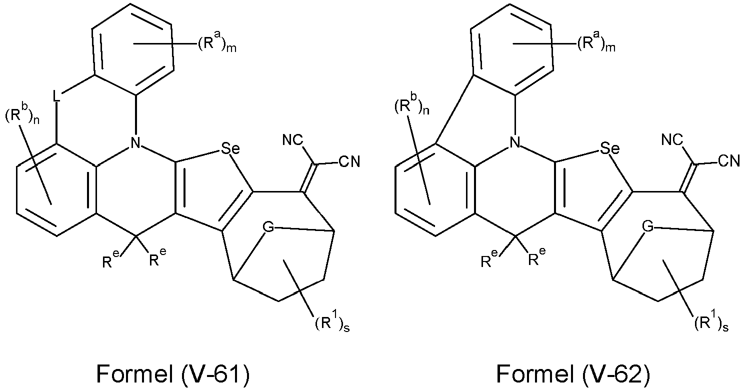 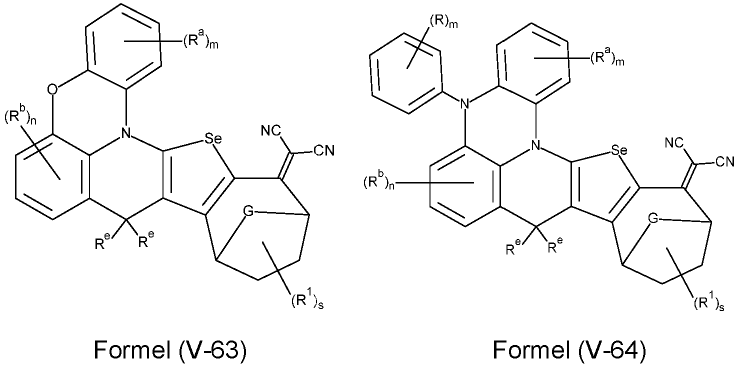 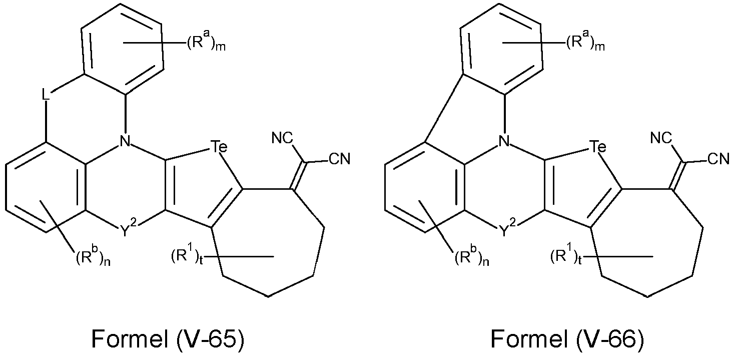 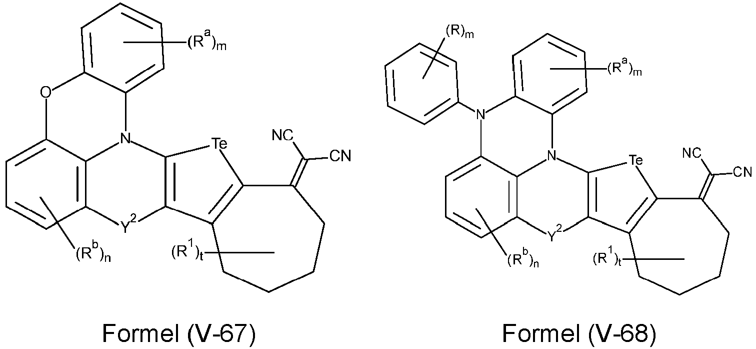 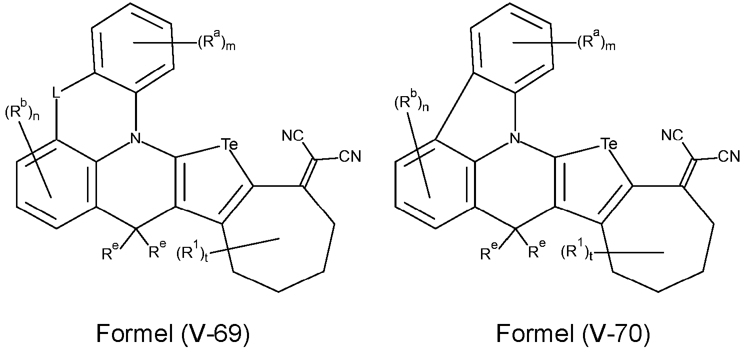 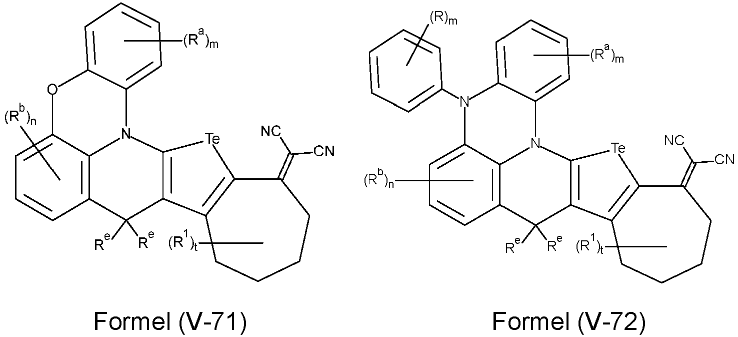 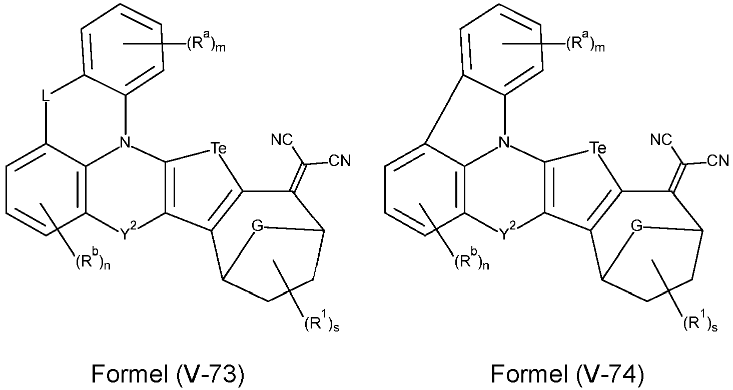 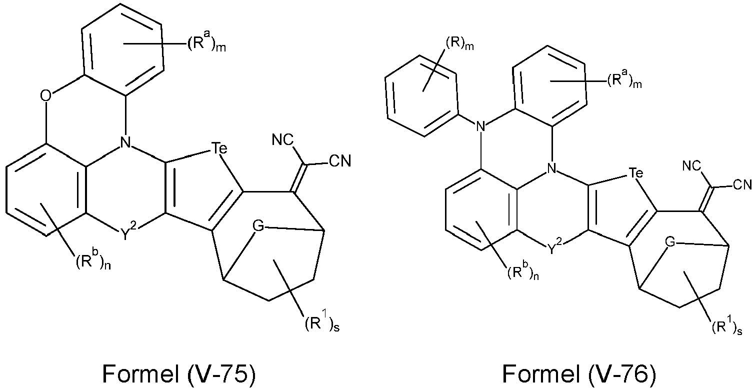 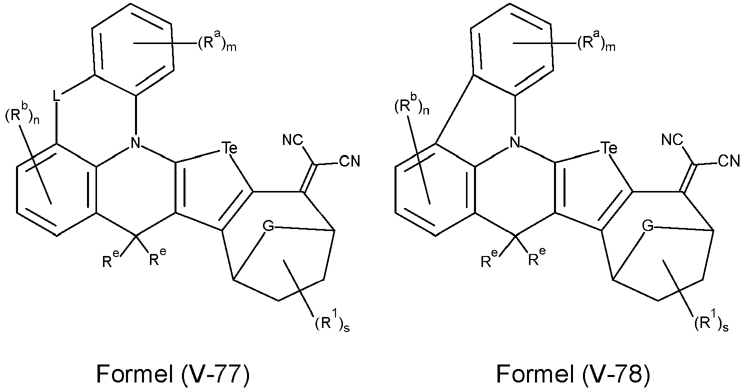 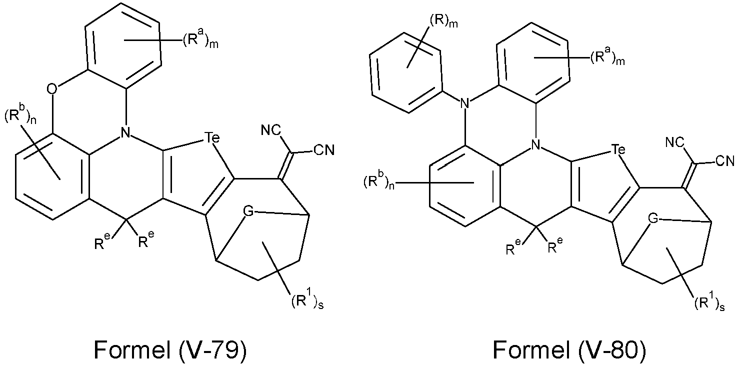 wobei die Symbole R^{a}, R^{b}, R^{c}, R^{d}, R^{e}, Y² und L die in Anspruch 1 genannten Bedeutungen aufweisen, Z die in Anspruch 2 genannten Bedeutungen aufweist und die weiteren Symbole die folgende Bedeutung aufweisen:
G ist bei jedem Auftreten gleich oder verschieden O, S, Se, Te, NAr', NR¹, BR¹, C(R¹)₂, Ge(R¹)₂, Si(R¹)₂, -C(R¹)₂-(CR¹)₂-, -CR¹=CR¹-, C=NR¹, S=O, SO₂ oder eine ortho-verknüpfte Arylen- oder Heteroarylengruppe mit 5 bis 14 aromatischen Ringatomen, welche durch einen oder mehrere Reste R¹ substituiert sein kann, wobei der Rest R¹ die in Anspruch 1 genannte Bedeutung aufweist;
Y³ ist bei jedem Auftreten gleich oder verschieden eine Bindung, O, S, Se, Te, NAr', NR¹, BR¹, C(R¹)₂, Ge(R¹)₂, Si(R¹)₂, CR¹=CR¹, C=NR¹, S=O, SO₂ oder eine ortho-verknüpfte Arylen- oder Heteroarylengruppe mit 5 bis 14 aromatischen Ringatomen, welche durch einen oder mehrere Reste R¹ substituiert sein kann, wobei der Rest R¹ die in Anspruch 1 genannte Bedeutung aufweist;
t ist 0, 1, 2, 3, 4, 5, 6, 7 oder 8;
s ist 0, 1, 2, 3, 4, 5 oder 6;
m ist 0, 1, 2, 3 oder 4;
n ist 0, 1, 2 oder 3.

10. Oligomer, Polymer oder Dendrimer enthaltend eine oder mehrere Verbindungen nach einem der Ansprüche 1 bis 9, wobei statt eines Wasserstoffatoms oder eines Substituenten eine oder mehrere Bindungen der Verbindungen zum Polymer, Oligomer oder Dendrimer vorhanden sind.

11. Formulierung, enthaltend mindestens eine Verbindung nach einem oder mehreren der Ansprüche 1 bis 9 oder ein Oligomer, Polymer oder Dendrimer nach Anspruch 10 und mindestens eine weitere Verbindung, wobei die weitere Verbindung bevorzugt ausgewählt ist aus einem oder mehreren Lösemitteln und/oder aus mindestens einer weiteren Verbindung ausgewählt aus der Gruppe bestehend aus Elektronentransportmaterialien, Elektroneninjektionsmaterialien, Lochleitermaterialien, Lochinjektionsmaterialien, Elektronenblockiermaterialien und Lochblockiermaterialien, vorzugsweise Elektronentransportmaterialien, Elektroneninjektionsmaterialien, Lochblockiermaterialien, besonders bevorzugt Subphthalocyanine, Subphthalocyanin-Derivate, Fullerene oder Fulleren-Derivate.

12. Verfahren zur Herstellung einer Verbindung nach einem oder mehreren der Ansprüche 1 bis 9, **dadurch gekennzeichnet, dass** ein Grundgerüst mit einer aromatischen Aminogruppe synthetisiert wird und mindestens ein heterocyclischer Rest eingeführt wird, vorzugsweise mittels einer nukleophilen aromatischen Substitutionsreaktion oder einer Kupplungsreaktion.

13. Verwendung einer Verbindung nach einem oder mehreren der Ansprüche 1 bis 9 oder eines Oligomers, Polymers oder Dendrimers nach Anspruch 10 in einer elektronischen Vorrichtung, vorzugsweise als Photosensitizer.

14. Elektronische Vorrichtung enthaltend mindestens eine Verbindung nach einem oder mehreren der Ansprüche 1 bis 9 oder ein Oligomer, Polymer oder Dendrimer nach Anspruch 10.

15. Elektronische Vorrichtung nach Anspruch 14, **dadurch gekennzeichnet, dass** die Verbindung nach einem oder mehreren der Ansprüche 1 bis 9 oder das Oligomer, Polymer oder Dendrimer nach Anspruch 10 als Photosensitizer in einer lichtabsorbierenden Schicht eingesetzt wird.

## Claims

1. Compound comprising at least one structure of the formula (I): 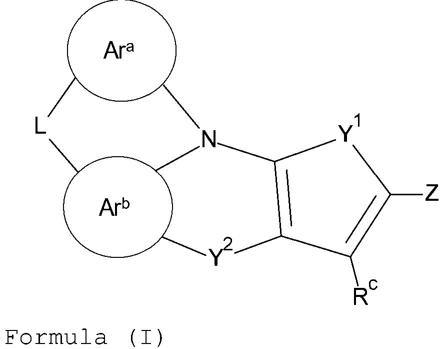 where the symbols used are as follows:
Ar^{a} is the same or different at each instance and is an aromatic or heteroaromatic ring system which has 5 to 60 aromatic ring atoms and may be substituted by one or more Ar or R^{a} radicals;
Ar^{b} is the same or different at each instance and is an aromatic or heteroaromatic ring system which has 5 to 60 aromatic ring atoms and may be substituted by one or more Ar or R^{d} radicals;
Z is the same or different at each instance and is an alkenyl group having 2 to 40 carbon atoms, where the alkenyl group may be substituted by one or more R^{d} radicals, where one or more nonadjacent CH₂ groups may be replaced by R^{d}C=CR^{d}, C=C, Si(R^{d})₂, C=O, C=S, C=Se, C=NR, C(=O)O, C(=O)NR^{d}, NR, P(=O)(R^{d}), O, S, SO or SO₂, or an aromatic or heteroaromatic ring system which has 5 to 60 aromatic ring atoms and may be substituted in each case by one or more R^{d} radicals; at the same time, the group may form a ring system with a further group, for example an R or R^{c} group; with the proviso that the Z group has at least two CN groups;
Y¹ is the same or different at each instance and is O, S, Se, Te, NAr, NR, C(R)₂, Ge(R)₂, Si(R)₂;
Y² is the same or different at each instance and is a bond, O, S, Se, Te, NAr, NR^{e}, BAr, BR^{e}, C(R^{e})₂, Ge(R^{e})₂, Si(R^{e})₂, CR^{e}=CR^{e}, C=NR^{e}, S=O, SO₂;
L is the same or different at each instance and is a connecting group, preferably a bond, C(R)₂, O, S, NR, NAr, C(=O), BAr, B(R), Si(R)₂, C=NR, C=C(R)₂, S=O, SO₂, P(R) and P(=O)R, -CR=CR- or an aromatic or heteroaromatic ring system which has 5 to 40 aromatic ring atoms and may be substituted by one or more R radicals;
Ar is the same or different at each instance and is an aromatic or heteroaromatic ring system which has 5 to 60 aromatic ring atoms and may be substituted by one or more R radicals; the Ar group here may form a ring system with at least one Ar, R, R^{a}, R^{b}, R^{c}, R^{d}, R^{e} group or a further group;
R, R^{a}, R^{b}, R^{c}, R^{d}, R^{e} is the same or different at each instance and is H, D, OH, F, Cl, Br, I, CN, NO₂, N(Ar')₂, N(R¹)₂, C(=O)N(Ar')₂, C(=O)N(R¹)₂, C(Ar')₃, C(R¹)₃ Si(Ar')₃, Si(R¹)₃, B(Ar')₂, B(R¹)₂, C(=O)Ar', C(=O)R¹, P(=O)(Ar')₂, P (=O)(R¹)₂, P(Ar')₂, P(R¹)₂, S(=O)Ar', S(=O)R¹, S(=O)₂Ar' , S(=O)₂R¹, OSO₂Ar' , OSO₂R¹, a straight-chain alkyl, alkoxy or thioalkoxy group having 1 to 40 carbon atoms or an alkenyl or alkynyl group having 2 to 40 carbon atoms or a branched or cyclic alkyl, alkoxy or thioalkoxy group having 3 to 20 carbon atoms, where the alkyl, alkoxy, thioalkoxy, alkenyl or alkynyl group may be substituted in each case by one or more R¹ radicals, where one or more nonadjacent CH₂ groups may be replaced by R¹C=CR¹, C=C, Si(R¹)₂, C=O, C=S, C=Se, C=NR¹, -C(=O)O-, -C(=O)NR¹-, NR¹, P(=O)(R¹), -Se-, -Te-, Nar', BR¹, Ge(R¹)₂, -O-, -S-, SO or SO₂, or an aromatic or heteroaromatic ring system which has 5 to 60 aromatic ring atoms and may be substituted in each case by one or more R¹ radicals, or an aryloxy or heteroaryloxy group which has 5 to 60 aromatic ring atoms and may be substituted by one or more R¹ radicals; an arylthio or heteroarylthio group which has 5 to 60 aromatic ring atoms and may be substituted by one or more R¹ radicals, or a diarylamino, arylheteroarylamino, diheteroarylamino group which has 5 to 60 aromatic ring atoms and may be substituted by one or more R¹ radicals, or an aralkyl or heteroaralkyl group which has 5 to 60 aromatic ring atoms and 1 to 10 carbon atoms in the alkyl radical and may be substituted by one or more R¹ radicals; at the same time, two R, R^{a}, R^{b}, R^{c}, R^{d}, R^{e} radicals together or with a further group may also form a ring system;
Ar' is the same or different at each instance and is an aromatic or heteroaromatic ring system which has 5 to 60 aromatic ring atoms and may be substituted by one or more R¹ radicals, at the same time, it is possible for two Ar' radicals bonded to the same carbon atom, silicon atom, nitrogen atom, phosphorus atom or boron atom also to be joined together via a bridge by a single bond or a bridge selected from B(R¹), C(R¹)₂, Si(R¹)₂, C=O, C=NR¹, C=C(R¹)₂, O, S, S=O, SO₂, N(R¹), P(R¹) and P(=O)R¹;
R¹ is the same or different at each instance and is H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, a straight-chain alkyl, alkoxy or thioalkoxy group having 1 to 40 carbon atoms or a branched or cyclic alkyl, alkoxy or thioalkoxy group having 3 to 40 carbon atoms or an alkenyl group having 2 to 40 carbon atoms, each of which may be substituted by one or more R² radicals, where one or more nonadjacent CH₂ groups may be replaced by R²C=CR², C=C, Si(R²)₂, C=O, C=S, C=Se, C=NR², C(=O)O, C(=O)NR², NR², P(=O)(R²), O, S, SO or SO₂ and where one or more hydrogen atoms may be replaced by D, F, Cl, Br, I, CN or NO₂, or an aromatic or heteroaromatic ring system which has 5 to 60 aromatic ring atoms and may be substituted in each case by one or more R² radicals, or an aryloxy or heteroaryloxy group which has 5 to 60 aromatic ring atoms and may be substituted by one or more R² radicals, or an aralkyl or heteroaralkyl group which has 5 to 60 aromatic ring atoms and may be substituted by one or more R² radicals, or a combination of these systems; at the same time, two or more R¹ radicals together may form a ring system; at the same time, one or more R¹ radicals may form a ring system with another part of the compound;
Ar" is the same or different at each instance and is an aromatic or heteroaromatic ring system which has 5 to 30 aromatic ring atoms and may be substituted by one or more R² radicals, at the same time, it is possible for two Ar" radicals bonded to the same carbon atom, silicon atom, nitrogen atom, phosphorus atom or boron atom also to be joined together via a bridge by a single bond or a bridge selected from B(R²), C(R²)₂, Si(R²)₂, C=O, C=NR², C=C(R²)₂, O, S, S=O, SO₂, N(R²), P(R²) and P(=O)R²;
R² is the same or different at each instance and is selected from the group consisting of H, D, F, CN, an aliphatic hydrocarbyl radical having 1 to 20 carbon atoms or an aromatic or heteroaromatic ring system which has 5 to 30 aromatic ring atoms and in which one or more hydrogen atoms may be replaced by D, F, Cl, Br, I or CN and which may be substituted by one or more alkyl groups each having 1 to 4 carbon atoms; at the same time, two or more substituents R² together may form a ring system.

2. Compound according to Claim 1, **characterized in that** the Z group is or comprises a substructure of the formula (Z-1) or (Z-2): 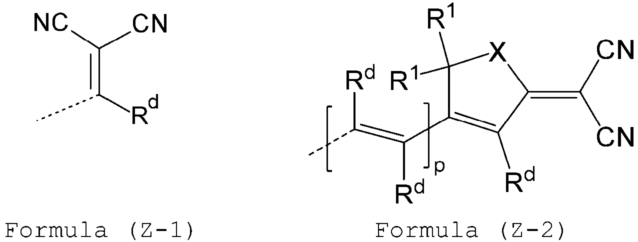 where R^{d} and R¹ have the definition given in Claim 1, the dashed bond represents the site of attachment, and in addition:
X is O, S or Se;
p is 0, 1 or 2.

3. Compound according to Claim 1 or 2, comprising at least one structure of the formula (II) or (IIa): 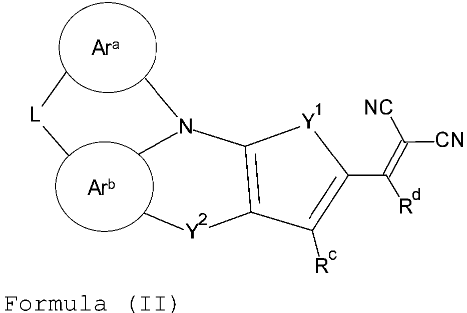 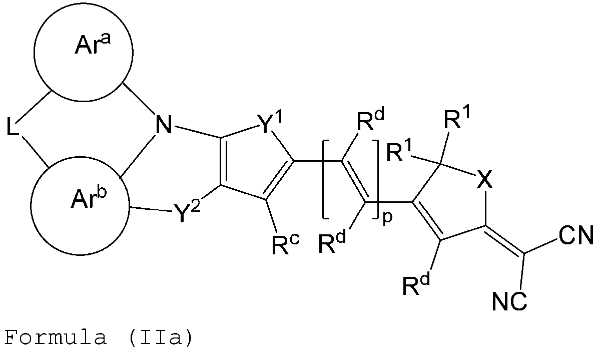 where the symbols Ar^{a}, Ar^{b}, R^{c}, R^{d}, Y¹, Y², R¹ and L have the definitions given in Claim 1, and X and p the definitions given in Claim 2.

4. Compound according to one or more of Claims 1 to 3, comprising at least one structure of the formula (III) or (IIIa) : 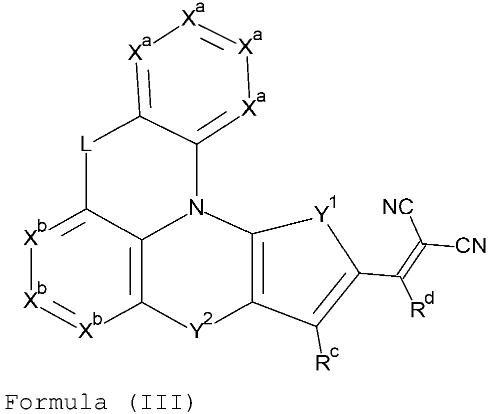 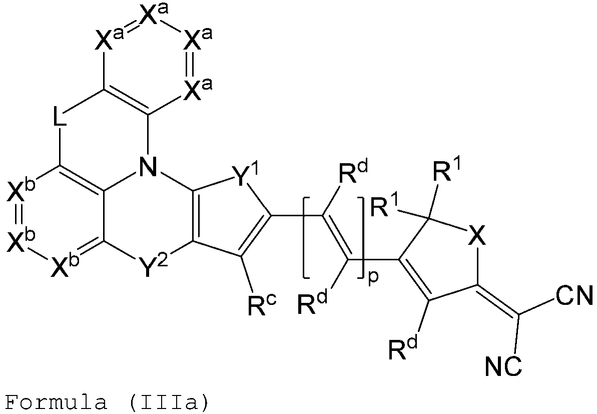 where the symbols R^{c}, R^{d}, Y¹, Y², R¹ and L have the definitions given in Claim 1, and X and p the definitions given in Claim 2, and the other symbols are defined as follows:
X^{a} is the same or different at each instance and is N or CR^{a}, with the proviso that not more than two of the X^{a} groups in one cycle are N, where R² has the definition detailed in Claim 1; or two adjacent X^{a} together are a group of the following formula: 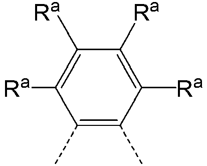
where the dashed bonds represent the linkage of the group within the structure;
X^{b} is the same or different at each instance and is N or CR^{b}, with the proviso that not more than two of the X^{b} groups in one cycle are N, where R^{d} has the definition detailed in Claim 1; or two adjacent X^{b} together are a group of the following formula: 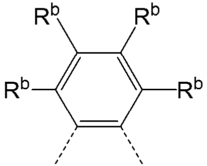
where the dashed bonds represent the linkage of the group within the structure.

5. Compound according to one or more of Claims 1 to 4, comprising at least one structure of the formula (IV) or (IVa) to (IVf): 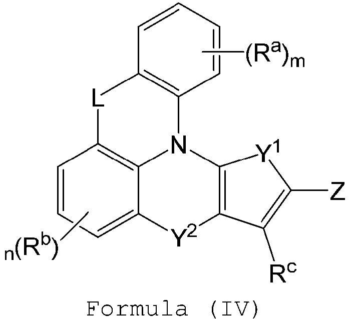 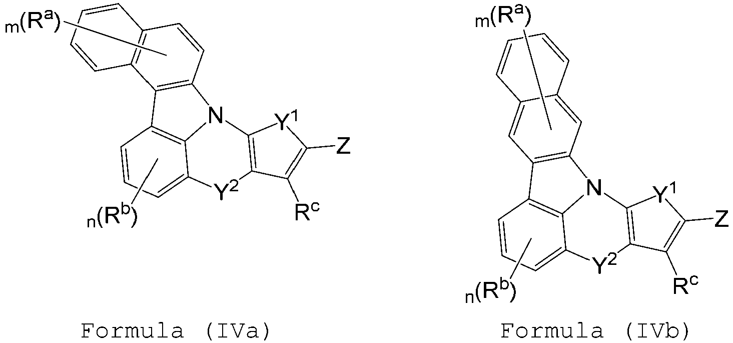 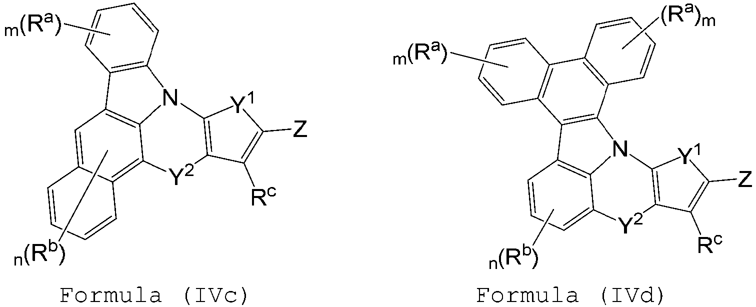 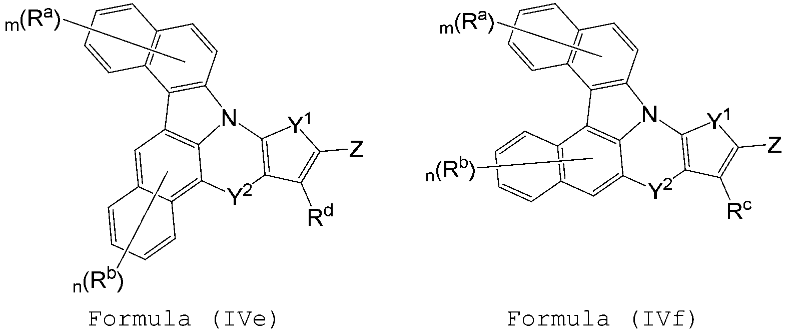 where the compounds may also be partly or fully deuterated, the symbols R^{a}, R^{b}, R^{c}, R^{d}, Y¹, Y² and L have the definitions given in Claim 1, Z has the definitions given in Claim 2, and the other symbols are defined as follows:
m is 0, 1, 2, 3 or 4;
n is 0, 1, 2 or 3.

6. Compound according to one or more of Claims 1 to 5, **characterized in that** the L group is a bond, C(R)₂, O, S, NAr or NR, the Y² group is C(R^{e})₂ and the Y¹ group is O, S, Se, Te, NR, NAr, preferably S or Se.

7. Compound according to one or more of Claims 1 to 6, **characterized in that** the R^{e} radical of the Y² group is a straight-chain alkyl group having 1 to 10 carbon atoms, a branched or cyclic alkyl group having 3 to 12 carbon atoms, where the alkyl group may be substituted in each case by one or more R¹ radicals, or an aryl or heteroaryl group which has 5 to 12 aromatic ring atoms and may be substituted in each case by one or more R¹ radicals, where the two R^{e} radicals of the Y² group may together form a ring which has 5 to 10 ring atoms and may be substituted by one or more R¹ radicals.

8. Compound according to one or more of Claims 1 to 7, **characterized in that** the R^{c} radical is H, D, a straight-chain alkyl group having 1 to 10 carbon atoms, a branched or cyclic alkyl group having 3 to 12 carbon atoms, where the alkyl group may be substituted in each case by one or more R¹ radicals, where one or more nonadjacent CH₂ groups may be replaced by R¹C=CR¹, C=C, Si(R¹)₂, C=O, C=S, C=Se, C=NR¹, C(=O)O, C(=O)NR¹, NR¹, P(=O)(R¹), O, S, SO or SO₂, or an aryl or heteroaryl group which has 5 to 12 aromatic ring atoms and may be substituted by one or more R¹ radicals, where the R^{c} radical together with the R^{d} radical may form a ring which has 5 to 10 ring atoms and may be substituted by one or more R¹ radicals.

9. Compound according to one or more of Claims 1 to 8, comprising at least one structure of the formulae (V-1) to (V-80) : 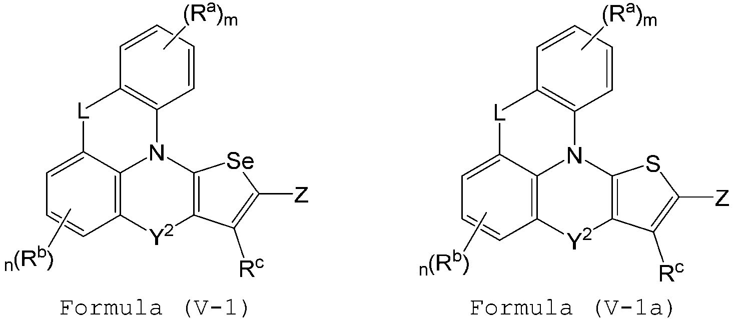 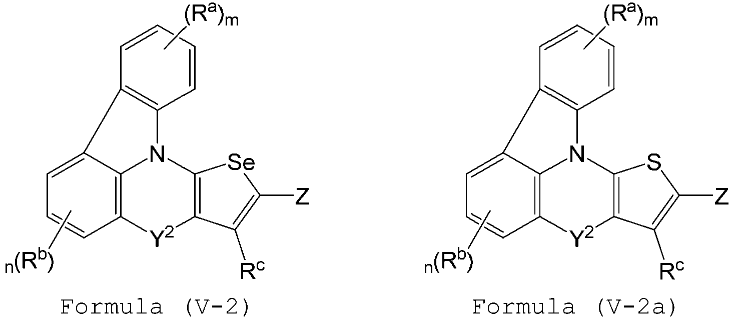 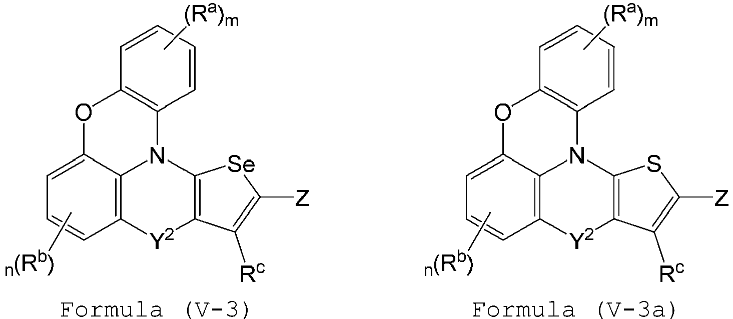 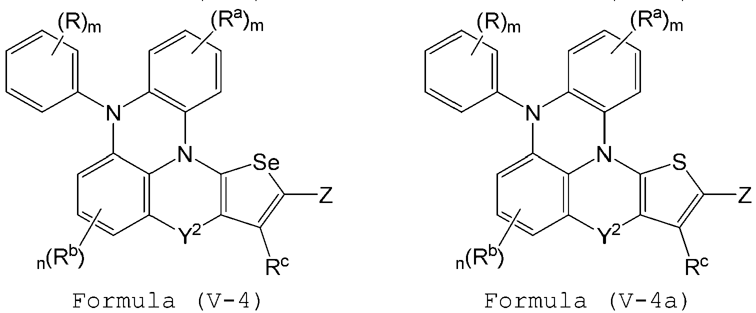 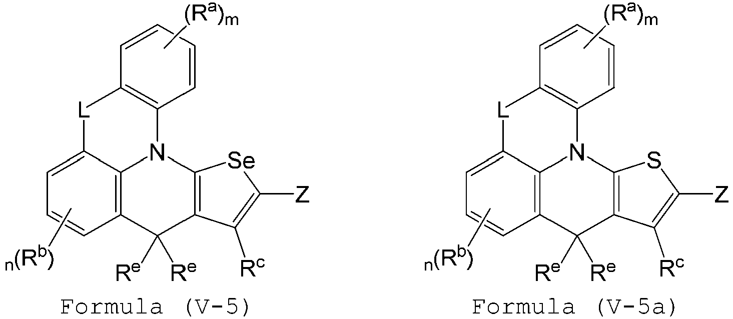 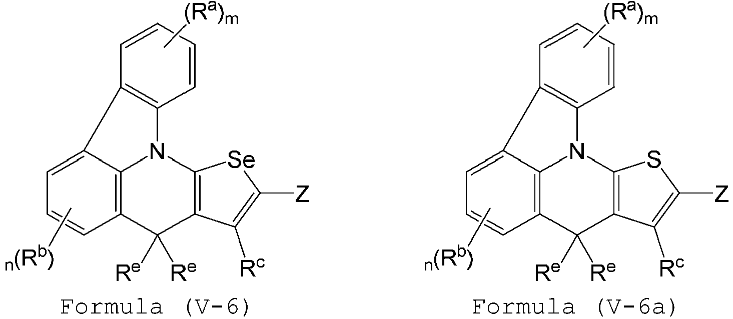 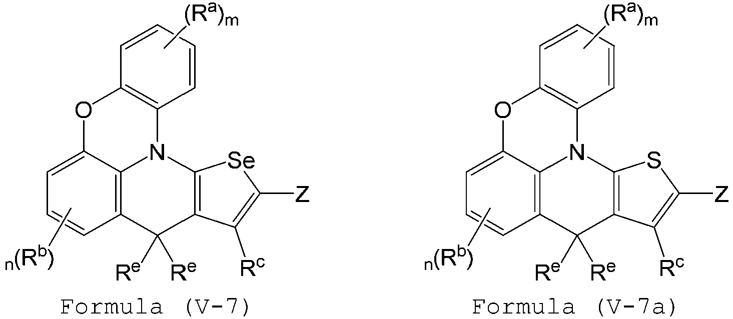 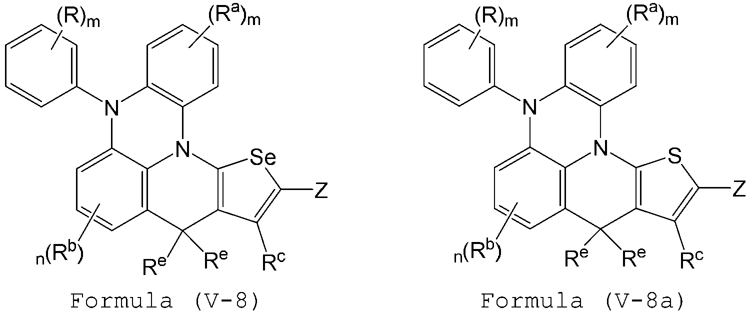 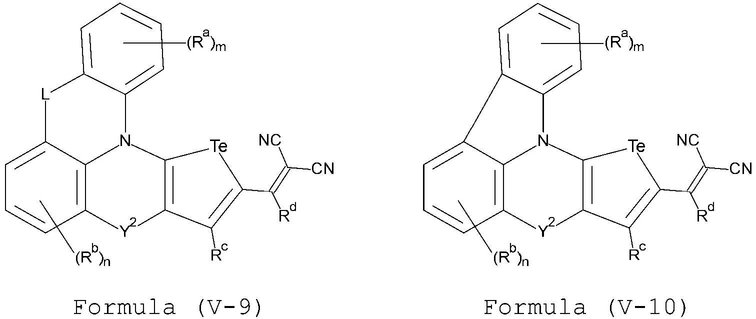 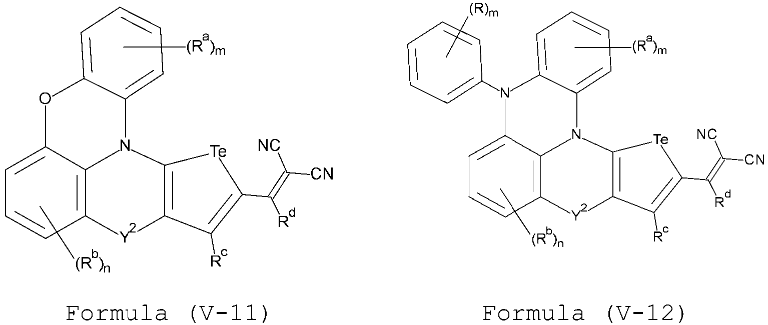 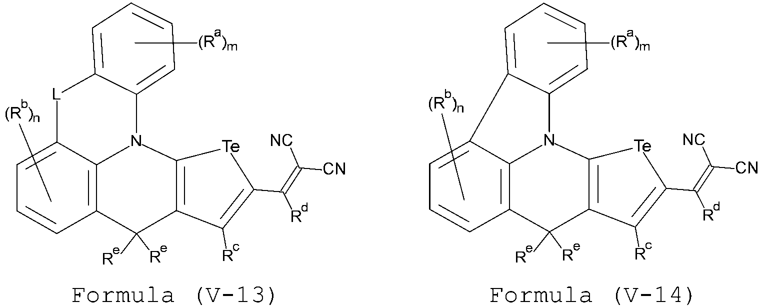 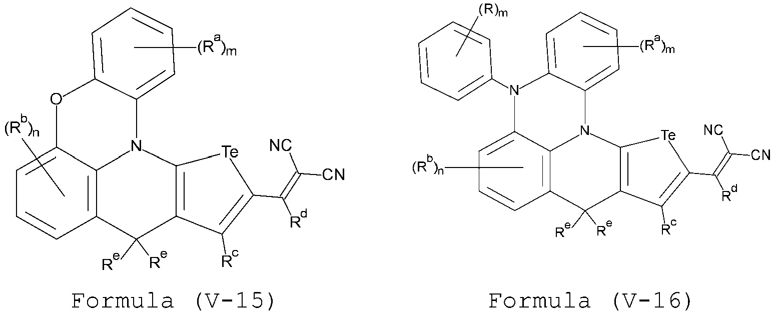 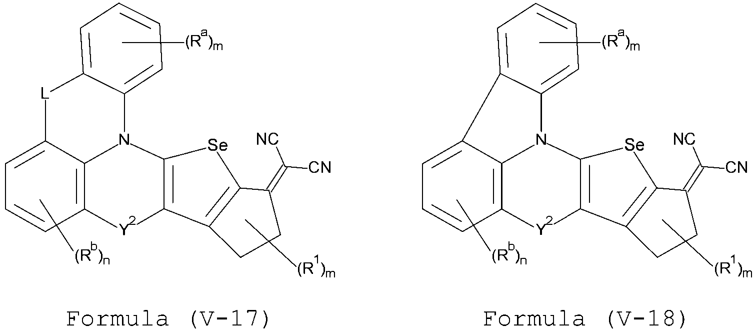 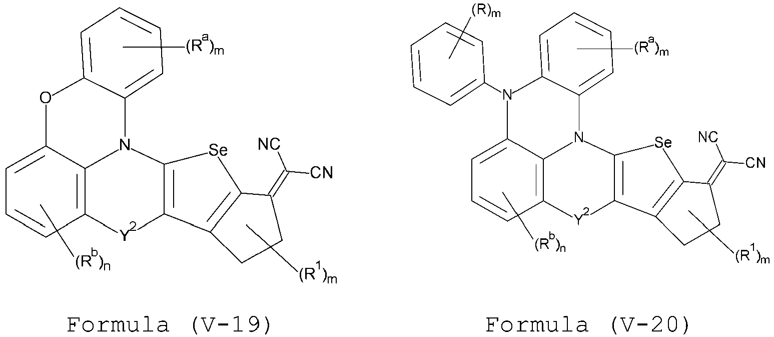 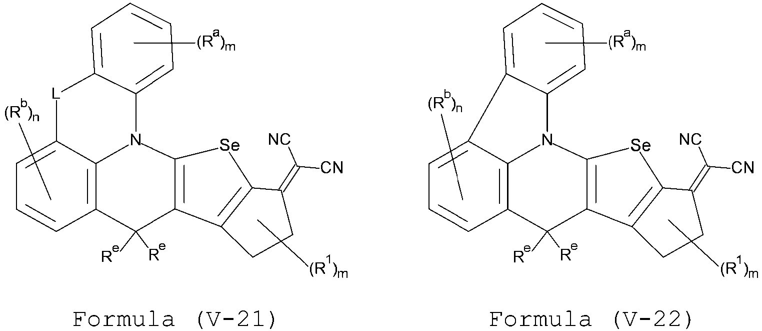 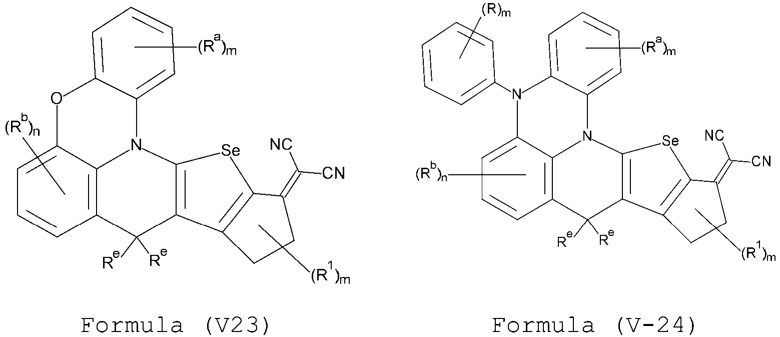 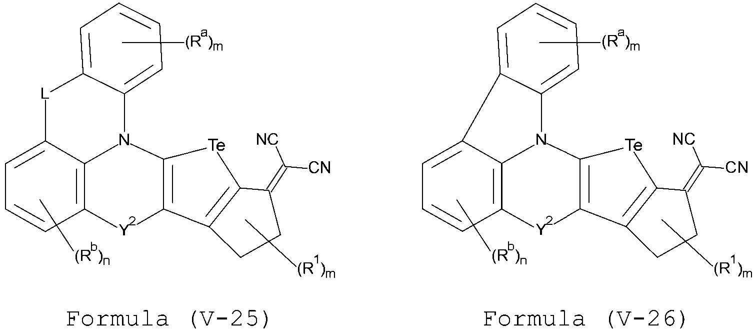 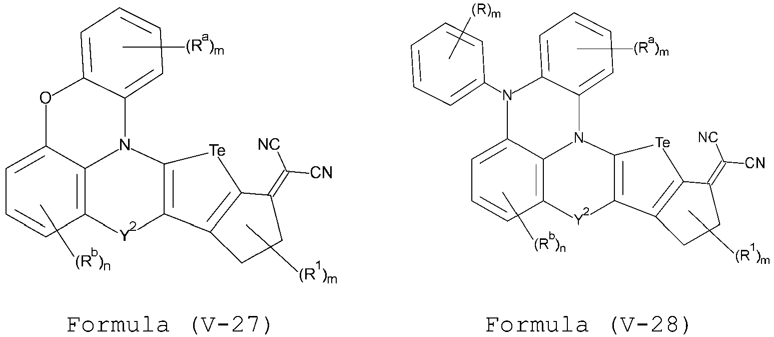 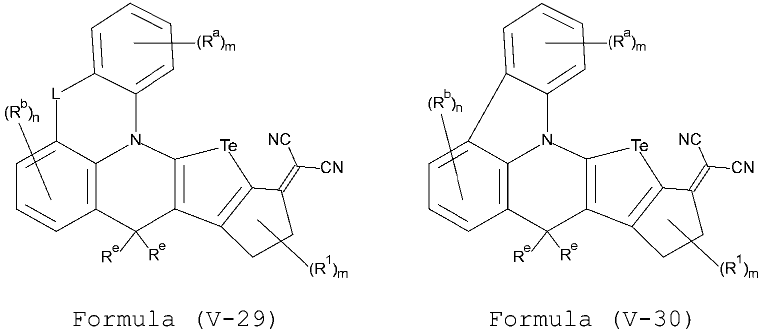 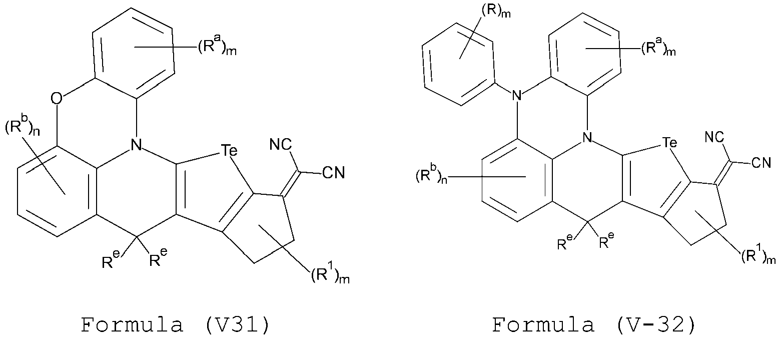 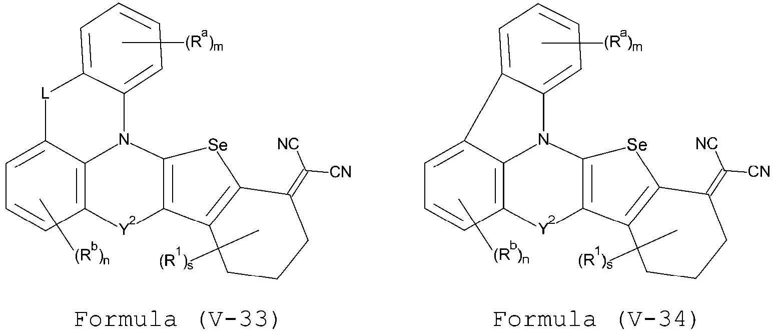 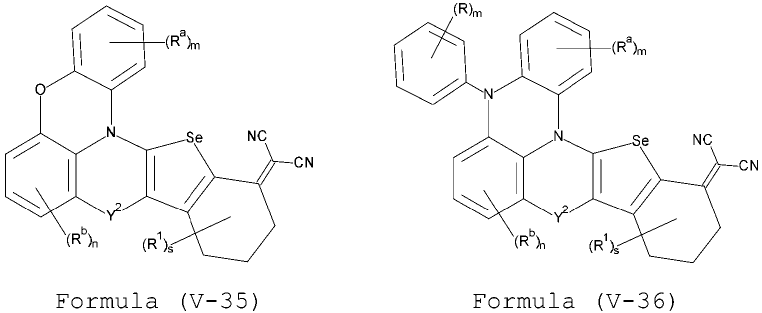 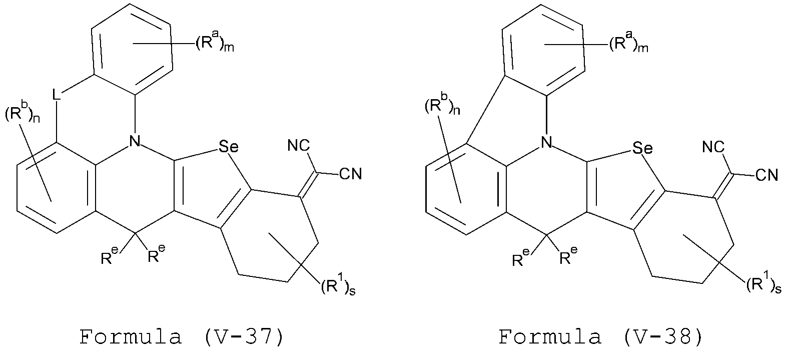 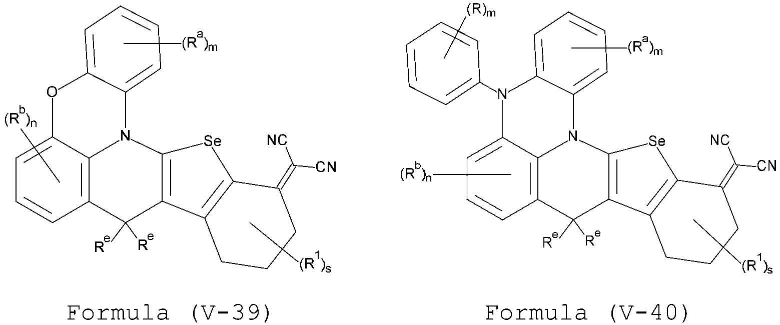 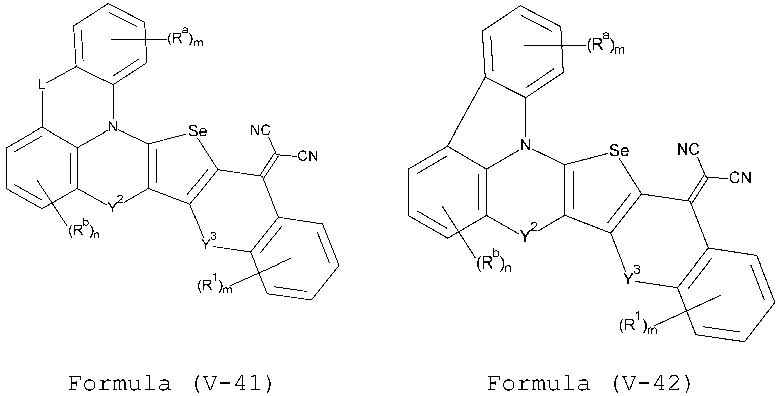 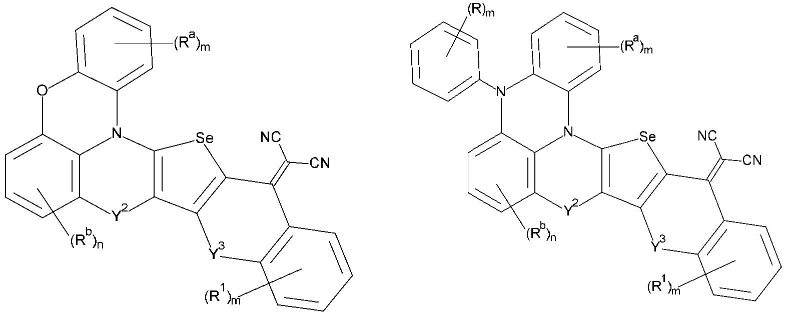 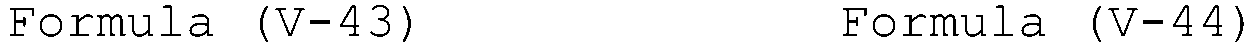 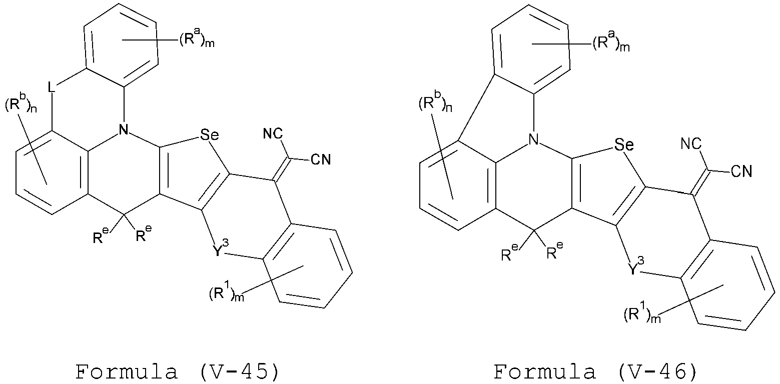 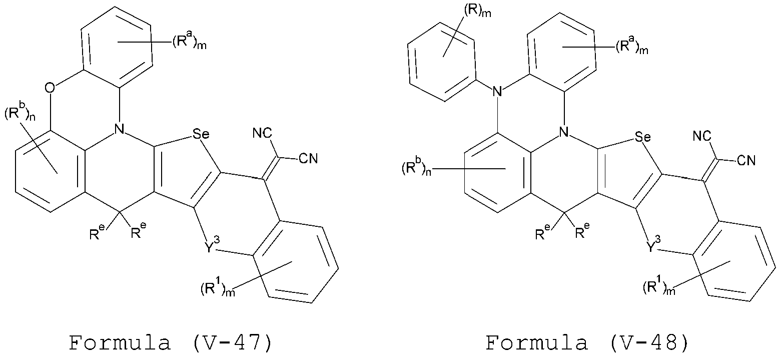 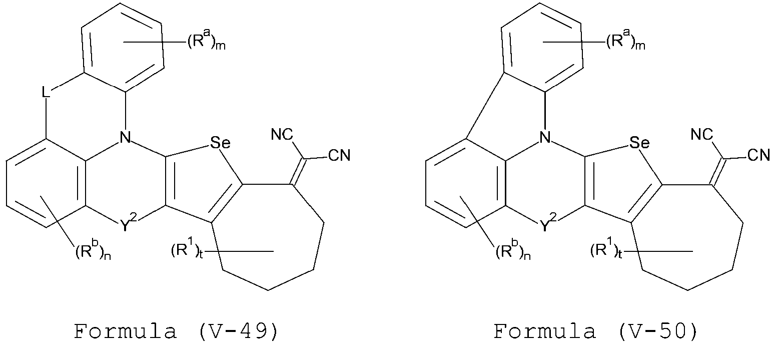 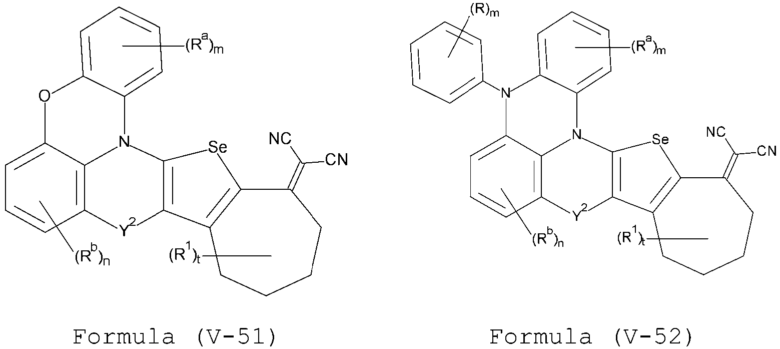 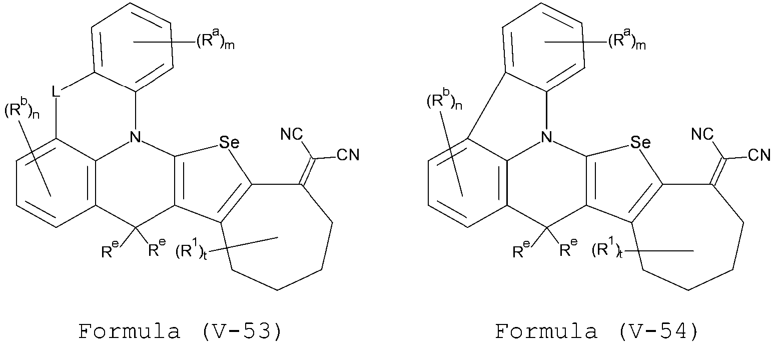 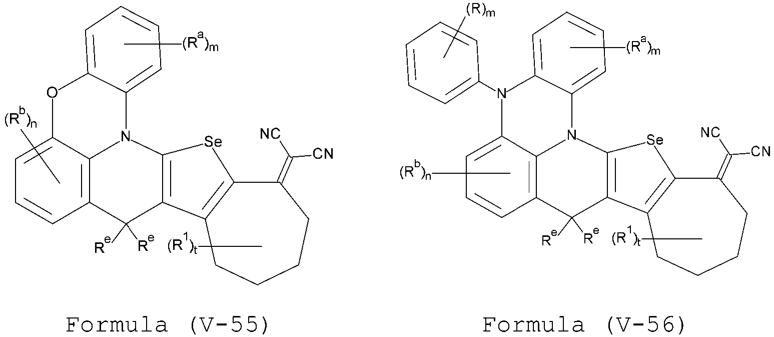 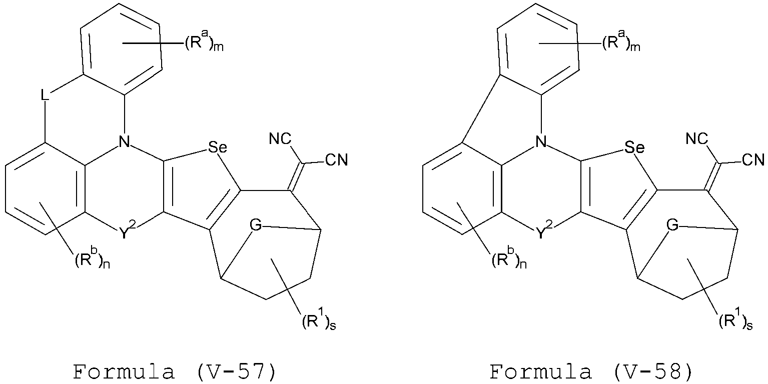 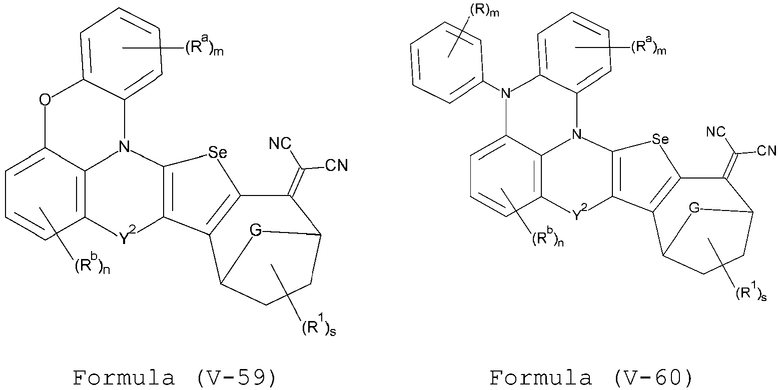 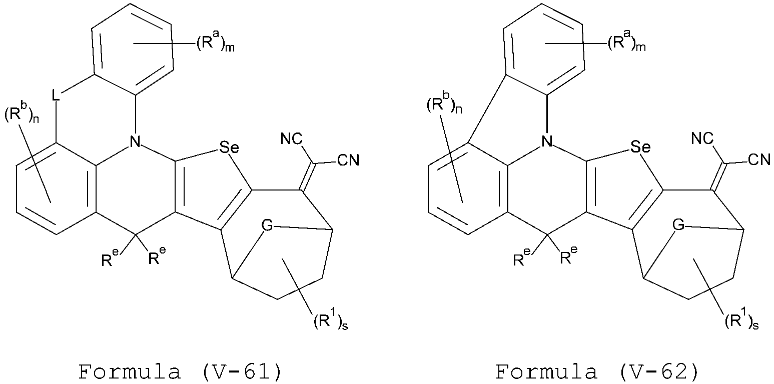 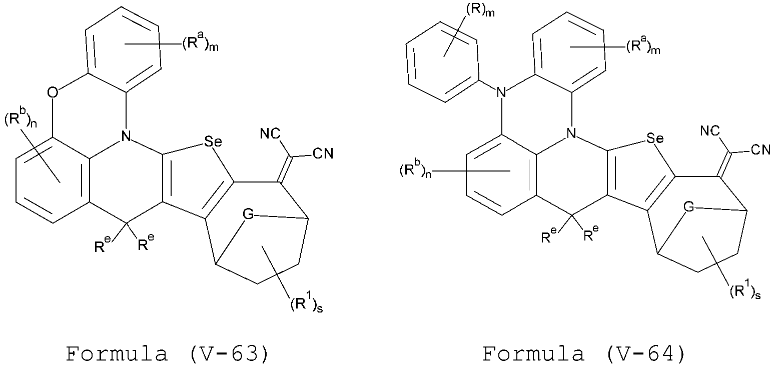 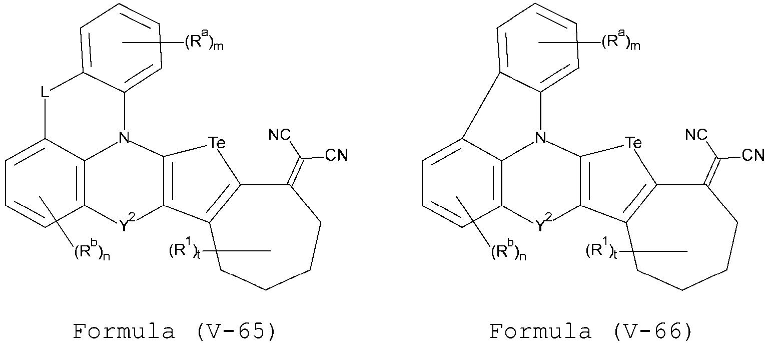 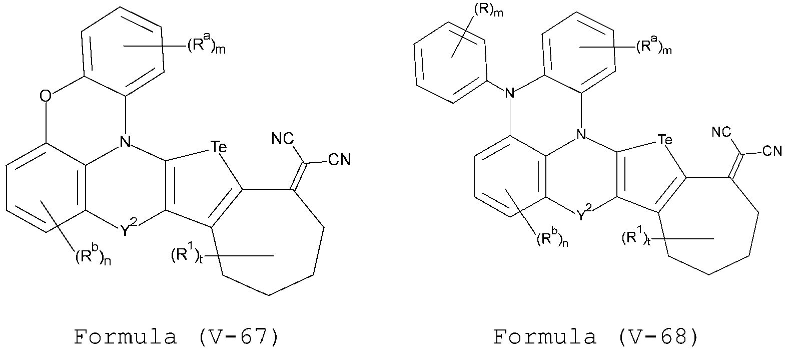 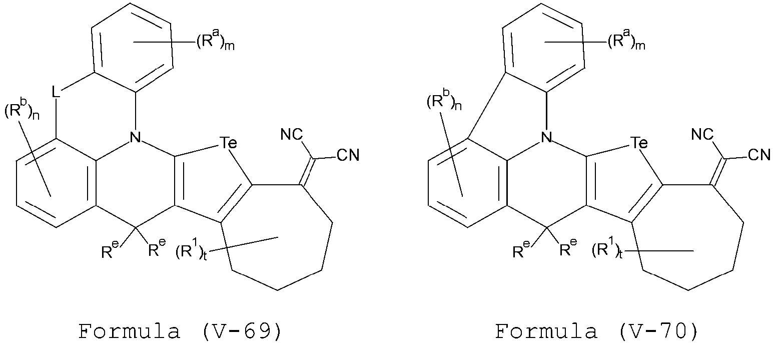 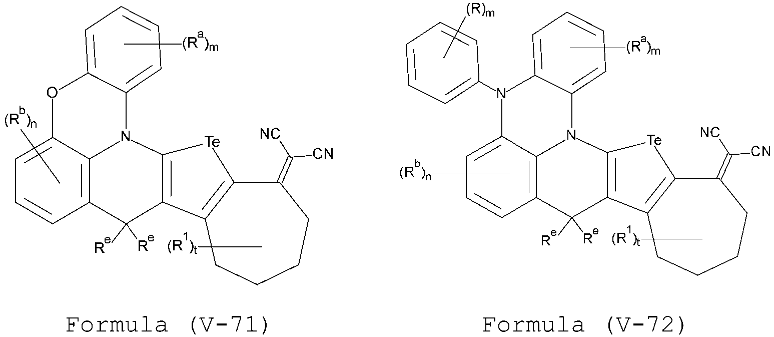 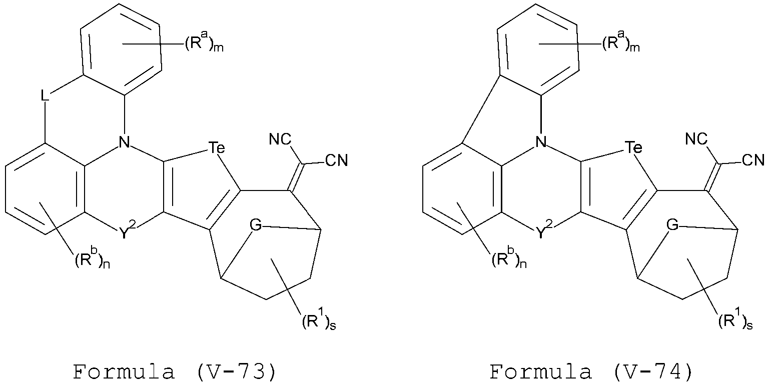 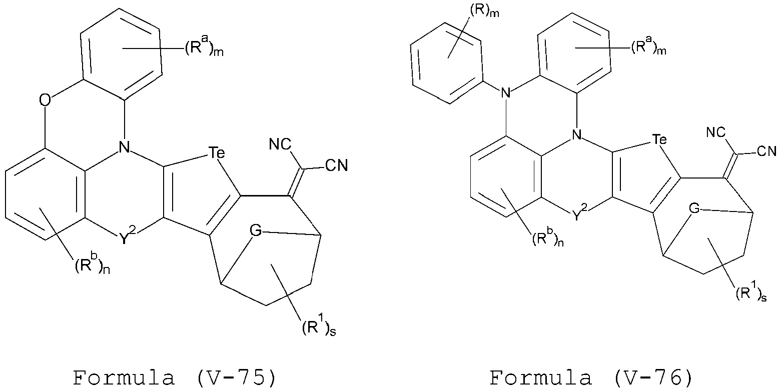 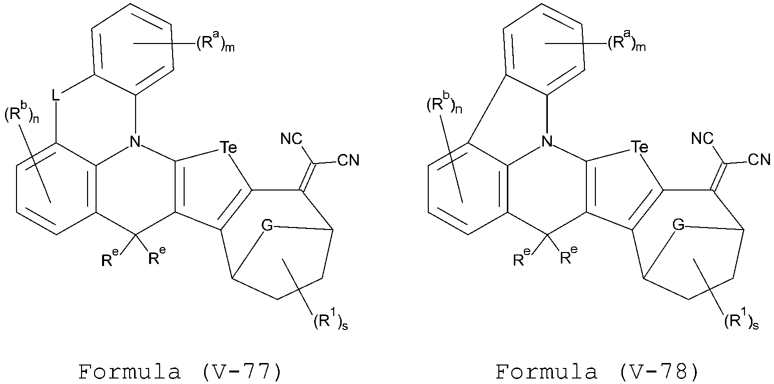 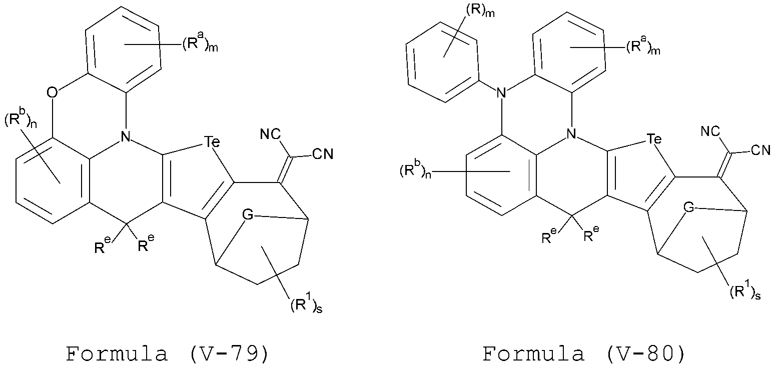 where the symbols R², R^{b}, R^{c}, R^{d}, R^{e}, Y² and L have the definitions given in Claim 1, Z has the definitions given in Claim 2, and the other symbols are defined as follows:
G is the same or different at each instance and is O, S, Se, Te, Nar', NR¹, BR¹, C(R¹)₂, Ge(R¹)₂, Si(R¹)₂, -C(R¹)₂-(CR¹)₂-, -CR¹=CR¹-, C=NR¹, S=O, SO₂ or an ortho-linked arylene or heteroarylene group which has 5 to 14 aromatic ring atoms and may be substituted by one or more R¹ radicals, where the R¹ radical has the definition given in Claim 1;
Y³ is the same or different at each instance and is a bond, O, S, Se, Te, Nar', NR¹, BR¹, C(R¹)₂, Ge(R¹)₂, Si(R¹)₂, CR¹=CR¹, C=NR¹, S=O, SO₂ or an ortho-linked arylene or heteroarylene group which has 5 to 14 aromatic ring atoms and may be substituted by one or more R¹ radicals, where the R¹ radical has the definition given in Claim 1;
t is 0, 1, 2, 3, 4, 5, 6, 7 or 8;
s is 0, 1, 2, 3, 4, 5 or 6;
m is 0, 1, 2, 3 or 4;
n is 0, 1, 2 or 3.

10. Oligomer, polymer or dendrimer containing one or more compounds according to any of Claims 1 to 9, wherein, in place of a hydrogen atom or a substituent, there are one or more bonds of the compounds to the polymer, oligomer or dendrimer.

11. Formulation containing at least one compound according to one or more of Claims 1 to 9 or an oligomer, polymer or dendrimer according to Claim 10 and at least one further compound, where the further compound is preferably selected from one or more solvents and/or from at least one further compound selected from the group consisting of electron transport materials, electron injection materials, hole conductor materials, hole injection materials, electron blocker materials and hole blocker materials, preferably electron transport materials, electron injection materials, hole blocker materials, more preferably subphthalocyanines, subphthalocyanine derivatives, fullerenes or fullerene derivatives.

12. Process for preparing a compound according to one or more of Claims 1 to 9, **characterized in that** a base skeleton having an aromatic amino group is synthesized and at least one heterocyclic radical is introduced, preferably by means of a nucleophilic aromatic substitution reaction or a coupling reaction.

13. Use of a compound according to one or more of Claims 1 to 9 or of an oligomer, polymer or dendrimer according to Claim 10 in an electronic device, preferably as photosensitizer.

14. Electronic device comprising at least one compound according to one or more of Claims 1 to 9 or an oligomer, polymer or dendrimer according to Claim 10.

15. Electronic device according to Claim 14, **characterized in that** the compound according to one or more of Claims 1 to 9 or the oligomer, polymer or dendrimer according to Claim 10 is used as photosensitizer in a light-absorbing layer.

## Revendications

1. Composé comprenant au moins une structure de formule (I), 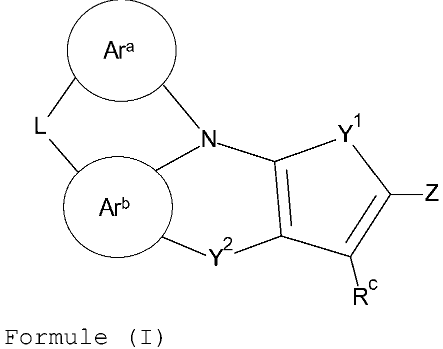 dans laquelle, pour les symboles utilisés, ce qui suit est d'application :
Ar^{a} représente, en chaque occurrence, de manière identique ou différente, un système cyclique aromatique ou hétéroaromatique comprenant 5 à 60 atomes de cycle aromatique, qui peut être substitué par un ou plusieurs radicaux Ar ou R^{a} ;
Ar^{b} représente, en chaque occurrence, de manière identique ou différente, un système cyclique aromatique ou hétéroaromatique comprenant 5 à 60 atomes de cycle aromatique, qui peut être substitué par un ou plusieurs radicaux Ar ou R^{b} ;
Z représente, en chaque occurrence, de manière identique ou différente, un groupe alcényle comprenant 2 à 40 atomes de carbone, le groupe alcényle pouvant être substitué par un ou plusieurs radicaux R^{d}, un ou plusieurs groupes CH₂ non adjacents pouvant être remplacés par R^{d}C=CR^{d}, C≡C, Si(R^{d})₂, C=O, C=S, C=Se, C=NR, C(=O)O, C(=O)NR^{d}, NR, P(=O)(R^{d}), O, S, SO ou SO₂, ou un système cyclique aromatique ou hétéroaromatique comprenant 5 à 60 atomes de cycle aromatique, qui peut à chaque fois être substitué par un ou plusieurs radicaux R^{d} ; le groupe pouvant former un système cyclique avec un autre groupe, par exemple un groupe R ou R^{c} ; étant entendu que le groupe Z présente au moins deux groupes CN ;
Y¹ représente, en chaque occurrence, de manière identique ou différente, O, S, Se, Te, NAr, NR, C(R)₂, Ge(R)₂, Si(R)₂ ;
Y² représente, en chaque occurrence, de manière identique ou différente, une liaison, O, S, Se, Te, NAr, NR^{e}, BAr, BR^{e}, C(R^{e})₂, Ge(R^{e})₂, Si(R^{e})₂, CR^{e}=CR^{e}, C=NR^{e}, S=O, SO₂ ;
L représente, en chaque occurrence, de manière identique ou différente, un groupe de liaison, de préférence une liaison, C(R)₂, O, S, NR, NAr, C(=O), BAr, B(R), Si(R)₂, C=NR, C=C(R)₂, S=O, SO₂, P(R) et P(=O)R, -CR=CR- ou un système cyclique aromatique ou hétéroaromatique comprenant 5 à 40 atomes de cycle aromatique, qui peut être substitué par un ou plusieurs radicaux R ;
Ar représente, en chaque occurrence, de manière identique ou différente, un système cyclique aromatique ou hétéroaromatique comprenant 5 à 60 atomes de cycle aromatique, qui peut être substitué par un ou plusieurs radicaux R ; auquel cas le groupe Ar peut former un système cyclique avec au moins un groupe Ar, R, R^{a}, R^{b}, R^{c}, R^{d}, R^{e} ou avec un autre groupe ;
R, R^{a}, R^{b}, R^{c}, R^{d}, R^{e} représentent, en chaque occurrence, de manière identique ou différente, H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, un groupe alkyle, alcoxy ou thioalcoxy linéaire comprenant 1 à 40 atomes de carbone ou un groupe alcényle ou alcynyle comprenant 2 à 40 atomes de carbone, ou un groupe alkyle, alcoxy ou thioalcoxy ramifié ou cyclique comprenant 3 à 20 atomes de carbone, le groupe alkyle, alcoxy, thioalcoxy, alcényle ou alcynyle pouvant à chaque fois être substitué par un ou plusieurs radicaux R¹, un ou plusieurs groupes CH₂ non adjacents pouvant être remplacés par R¹C=CR¹, C=C, Si(R¹)₂, C=O, C=S, C=Se, C=NR¹, -C(=O)O-, -C(=O)NR¹-, NR¹, P(=O)(R¹), -Se-, -Te-, NAr', BR¹, Ge(R¹)₂, -O-, -S-, SO ou SO₂ ; ou un système cyclique aromatique ou hétéroaromatique comprenant 5 à 60 atomes de cycle aromatique, qui peut à chaque fois être substitué par un ou plusieurs radicaux R¹, ou un groupe aryloxy ou hétéroaryloxy comprenant 5 à 60 atomes de cycle aromatique, qui peut être substitué par un ou plusieurs radicaux R¹ ; ou un groupe arylthio ou hétéroarylthio comprenant 5 à 60 atomes de cycle aromatique, qui peut être substitué par un ou plusieurs radicaux R¹, ou un groupe diarylamino, arylhétéroarylamino, dihétéroarylamino comprenant 5 à 60 atomes de cycle aromatique, qui peut être substitué par un ou plusieurs radicaux R¹, ou un groupe aralkyle ou hétéroaralkyle comprenant 5 à 60 atomes de cycle aromatique et 1 à 10 atomes de carbone dans le radical alkyle, qui peut être substitué par un ou plusieurs radicaux R¹ ; deux radicaux R, R^{a}, R^{b}, R^{c}, R^{d}, R^{e} pouvant également former un système cyclique l'un avec l'autre ou avec un autre groupe ;
Ar' représente en chaque occurrence, de manière identique ou différente, un système cyclique aromatique ou hétéroaromatique comprenant 5 à 60 atomes de cycle aromatique, qui peut être substitué par un ou plusieurs radicaux R¹, deux radicaux Ar', qui sont liés au même atome de C, de Si, de N, de P ou de B, également par une simple liaison ou par un pont, choisi parmi B(R¹), C(R¹)₂, Si(R¹)₂, C=O, C=NR¹, C=C(R¹)₂, O, S, S=O, SO₂, N(R¹), P(R¹) et P(=O)R¹, pouvant être pontés l'un avec l'autre ;
R¹ représente, en chaque occurrence, de manière identique ou différente, H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, un groupe alkyle, alcoxy ou thioalcoxy linéaire comprenant 1 à 40 atomes de carbone ou un groupe alkyle, alcoxy ou thioalcoxy ramifié ou cyclique comprenant 3 à 40 atomes de carbone ou un groupe alcényle comprenant 2 à 40 atomes de carbone, qui peut à chaque fois être substitué par un ou plusieurs radicaux R², un ou plusieurs groupes CH₂ non adjacents pouvant être remplacés par R²C=CR², C=C, Si(R²)₂, C=O, C=S, C=Se, C=NR², C(=O)O, C(=O)NR², NR², P(=O)(R²) , O, S, SO ou SO₂ et un ou plusieurs atomes H pouvant être remplacés par D, F, Cl, Br, I, CN ou NO₂, ou un système cyclique aromatique ou hétéroaromatique comprenant 5 à 60 atomes de cycle aromatique, qui peut à chaque fois être substitué par un ou plusieurs radicaux R², ou un groupe aryloxy ou hétéroaryloxy comprenant 5 à 60 atomes de cycle aromatique, qui peut être substitué par un ou plusieurs radicaux R², ou un groupe aralkyle ou hétéroaralkyle comprenant 5 à 60 atomes de cycle aromatique, qui peut être substitué par un ou plusieurs radicaux R², ou une combinaison de ces systèmes ; deux ou plusieurs radicaux R¹ pouvant former un système cycle l'un avec l'autre ; un ou plusieurs radicaux R¹ pouvant former un système cyclique avec une autre partie du composé ;
Ar" représente en chaque occurrence, de manière identique ou différente, un système cyclique aromatique ou hétéroaromatique comprenant 5 à 30 atomes de cycle aromatique, qui peut être substitué par un ou plusieurs radicaux R², deux radicaux Ar", qui sont liés au même atome de C, de Si, de N, de P ou de B, également par une simple liaison ou par un pont, choisi parmi B(R²), C(R²)₂, Si(R²)₂, C=O, C=NR², C=C(R²)₂, O, S, S=O, SO₂, N (R²), P(R²) et P(=O)R², pouvant être pontés l'un avec l'autre ;
R² est choisi en chaque occurrence, de manière identique ou différente, dans le groupe constitué par H, D, F, CN, un radical hydrocarboné aliphatique comprenant 1 à 20 atomes de carbone ou un système cyclique aromatique ou hétéroaromatique comprenant 5 à 30 atomes de cycle aromatique, dans lequel un ou plusieurs atomes H peuvent être remplacés par D, F, Cl, Br, I ou CN et qui peut être substitué par un ou plusieurs groupes alkyle comprenant à chaque fois 1 à 4 atomes de carbone ; deux substituants R² ou plus pouvant former un système cyclique les uns avec les autres.

2. Composé selon la revendication 1, **caractérisé en ce que** le groupe Z représente ou comprend une structure partielle de formule (Z-1) ou (Z-2), 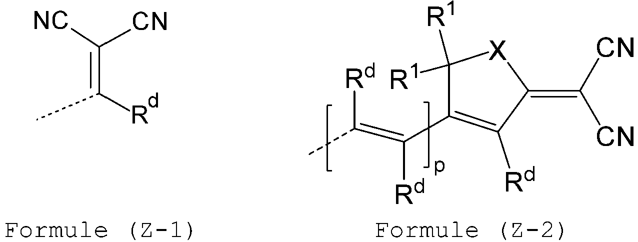 dans lesquelles R^{d} et R¹ présentent la signification mentionnée dans le revendication 1, la liaison en pointillés représente le site de liaison en ce qui suit est d'application :
X représente O, S ou Se ;
p représente 0, 1 ou 2.

3. Composé selon la revendication 1 ou 2, comprenant au moins une structure de formule (II) ou (IIa), 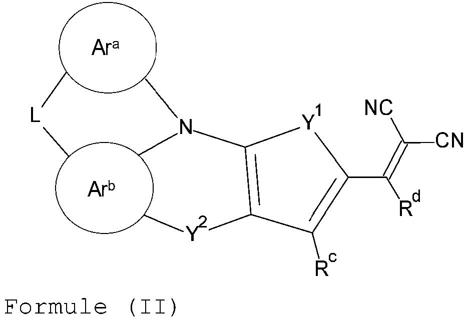 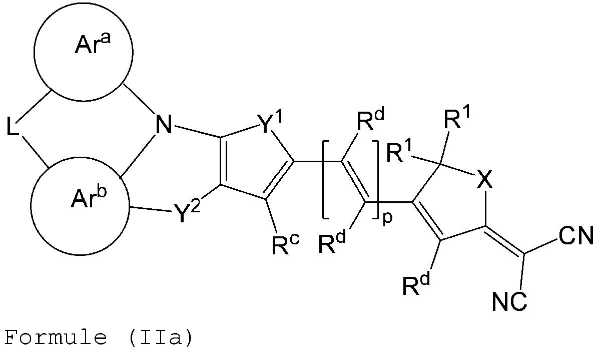 dans lesquelles les symboles Ar^{a}, Ar^{b}, R^{c}, R^{d}, Y¹, Y², R¹ et L présentent les significations mentionnées dans la revendication 1 et X et p présentent les significations mentionnées dans la revendication 2.

4. Composé selon l'une ou plusieurs des revendications 1 à 3, comprenant au moins une structure de formule (III) ou (IIIa), 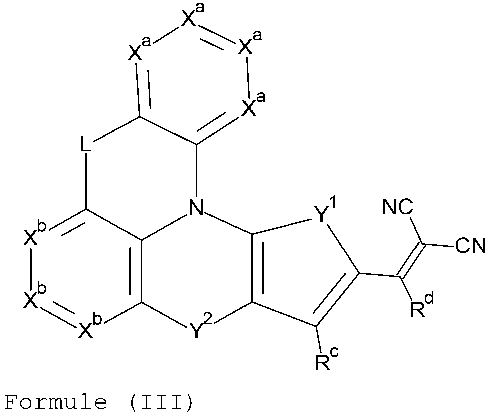 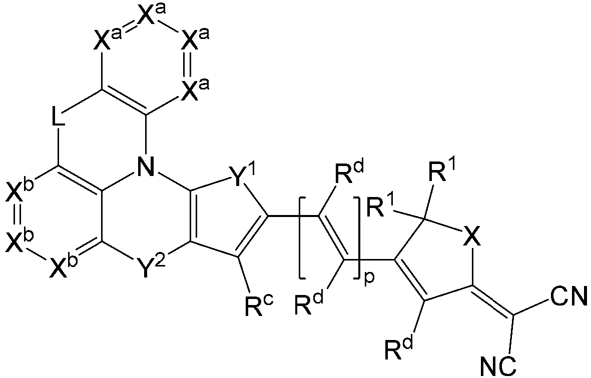 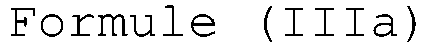 dans lesquelles les symboles R^{c}, R^{d}, Y¹, Y², R¹ et L présentent les significations mentionnées dans la revendication 1 et X et p présentent les significations mentionnées dans la revendication 2 et les autres symboles présentent la signification suivante :
X^{a} représente, en chaque occurrence, de manière identique ou différente, N ou CR^{a}, étant entendu que pas plus de deux des groupes X^{a} dans un cycle ne représentent N, R^{a} présentant la signification indiquée dans la revendication 1 ; ou deux radicaux x^{a} adjacents représentent ensemble un groupe de la formule suivante, 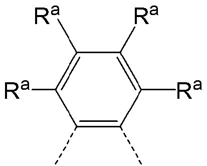
dans laquelle les liaisons en pointillés représentent la liaison du groupe dans la structure ;
X^{b} représente, en chaque occurrence, de manière identique ou différente, N ou CR^{b}, étant entendu que pas plus de deux des groupes X^{b} dans un cycle ne représentent N, R^{b} présentant la signification indiquée dans la revendication 1 ; ou deux radicaux X^{b} adjacents représentent ensemble un groupe de la formule suivante, 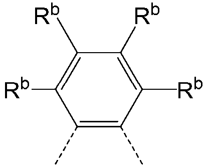
dans laquelle les liaisons en pointillés représentent la liaison du groupe dans la structure.

5. Composé selon l'une ou plusieurs des revendications 1 à 4, comprenant au moins une structure de formule (IV) ou (IVa) à (IVf), 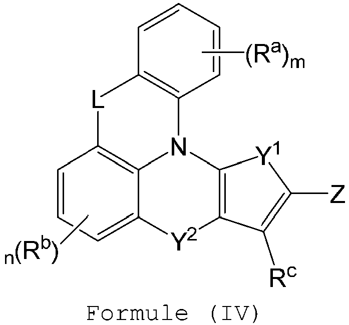 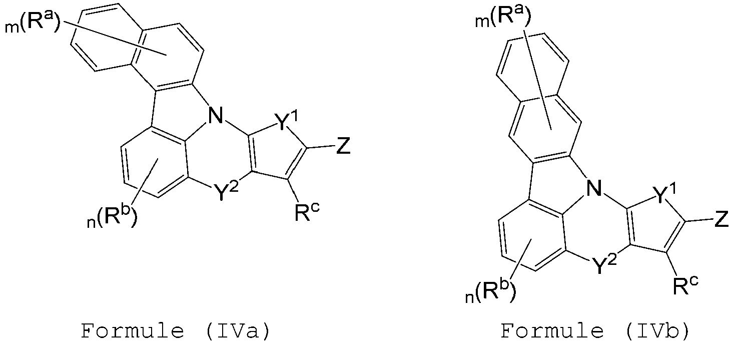 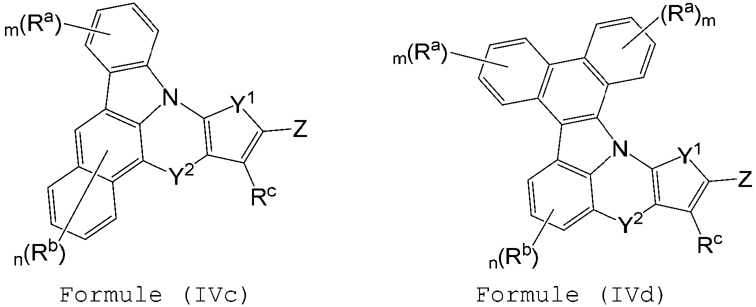 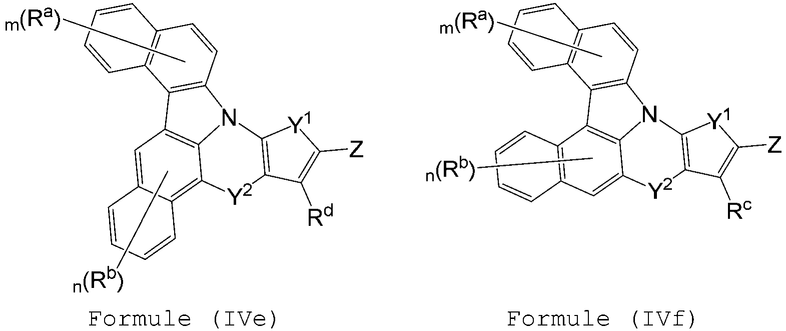 dans lesquelles les composés peuvent également être partiellement ou complètement deutérisés, les symboles R^{a}, R^{b}, R^{c}, R^{d}, Y¹, Y² et L présentent les significations mentionnées dans la revendication 1, Z présente les significations mentionnées dans la revendication 2 et les autres symboles présentent la signification suivante :
m représente 0, 1, 2, 3 ou 4 ;
n représente 0, 1, 2 ou 3.

6. Composé selon l'une ou plusieurs des revendications 1 à 5, **caractérisé en ce que** le groupe L représente une liaison, C(R)₂, O, S, NAr ou NR, le groupe Y² représente C(R^{e})₂ et le groupe Y¹ représente O, S, Se, Te, NR, NAr, de préférence S ou Se.

7. Composé selon l'une ou plusieurs des revendications 1 à 6, **caractérisé en ce que** le radical R^{e} du groupe Y² représente un groupe alkyle linéaire comprenant 1 à 10 atomes de carbone, un groupe alkyle ramifié ou cyclique comprenant 3 à 12 atomes de carbone, le groupe alkyle pouvant à chaque fois être substitué par un ou plusieurs radicaux R¹, ou représente un groupe aryle ou hétéroaryle comprenant 5 à 12 atomes de cycle aromatique, qui peut être substitué par un ou plusieurs radicaux R¹, les deux radicaux R^{e} du groupe Y² pouvant former ensemble un cycle comprenant 5 à 10 atomes de cycle, qui peut être substitué par un ou plusieurs radicaux R¹.

8. Composé selon l'une ou plusieurs des revendications 1 à 7, **caractérisé en ce que** le radical R^{c} représente H, D, un groupe alkyle linéaire comprenant 1 à 10 atomes de carbone, un groupe alkyle ramifié ou cyclique comprenant 3 à 12 atomes de carbone, le groupe alkyle pouvant à chaque fois être substitué par un ou plusieurs radicaux R¹, un ou plusieurs groupes CH₂ non adjacents pouvant être remplacés par R¹C=CR¹, C=C, Si(R¹)₂, C=O, C=S, C=Se, C=NR¹, C(=O)O, C(=O)NR¹, NR¹, P(=O)(R¹), O, S, SO ou SO₂, ou un groupe aryle ou hétéroaryle comprenant 5 à 12 atomes de carbone, qui peut être substitué par un ou plusieurs radicaux R¹, le radical R^{c} pouvant former, ensemble avec le radical R^{d}, un cycle comprenant 5 à 10 atomes de carbone, qui peut être substitué par un ou plusieurs radicaux R¹.

9. Composé selon l'une ou plusieurs des revendications 1 à 8, comprenant au moins une structure des formules (V-1) à (V-80), 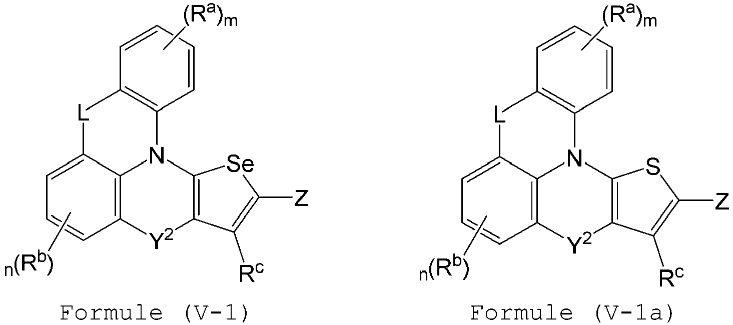 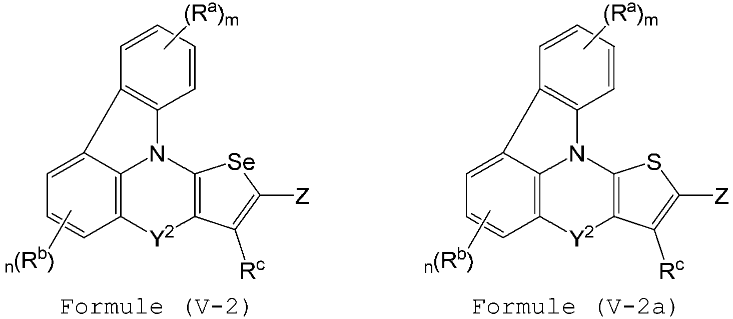 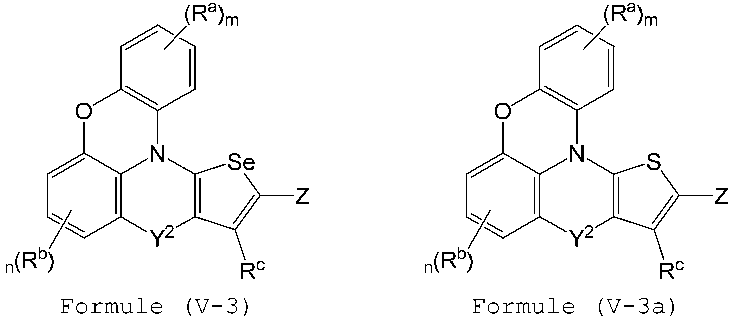 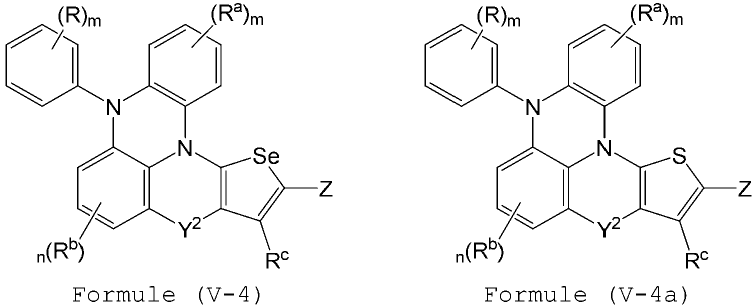 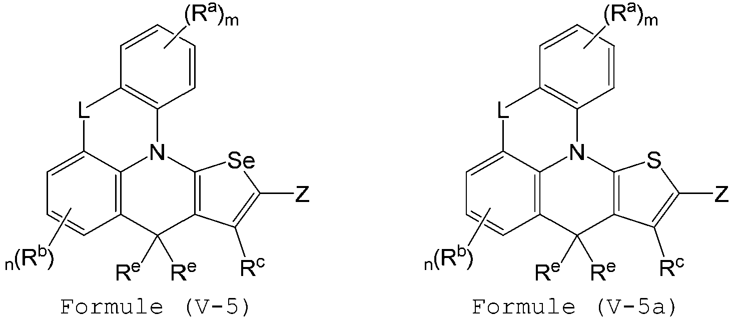 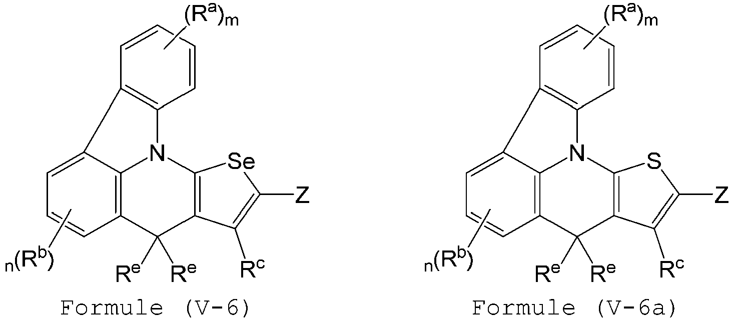 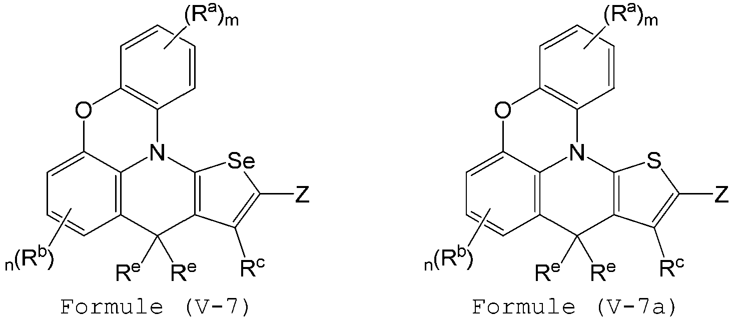 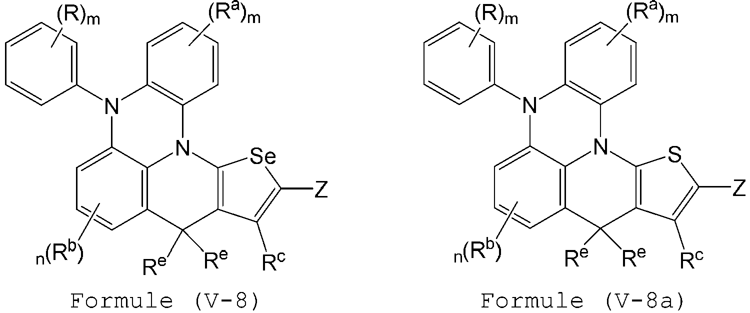 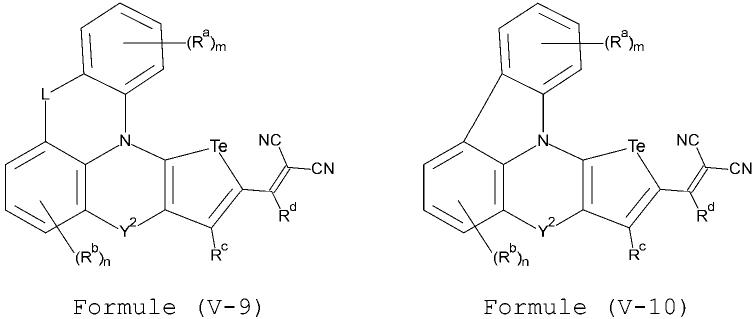 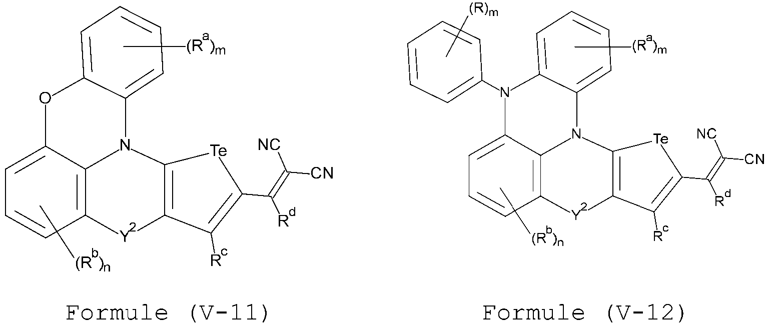 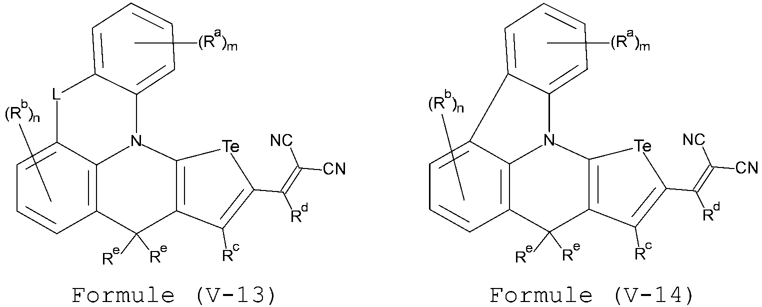 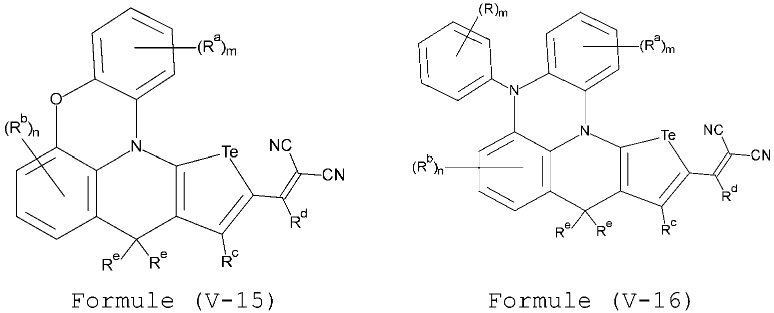 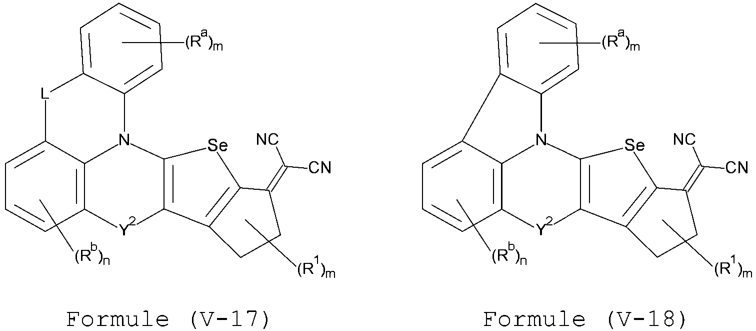 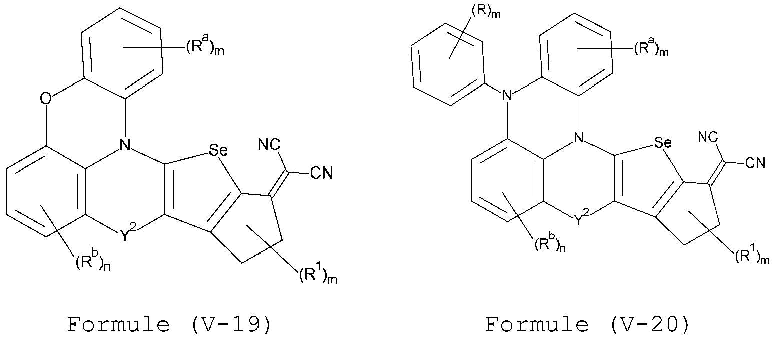 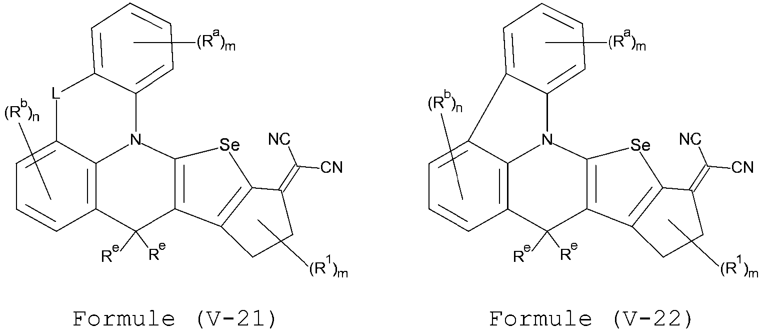 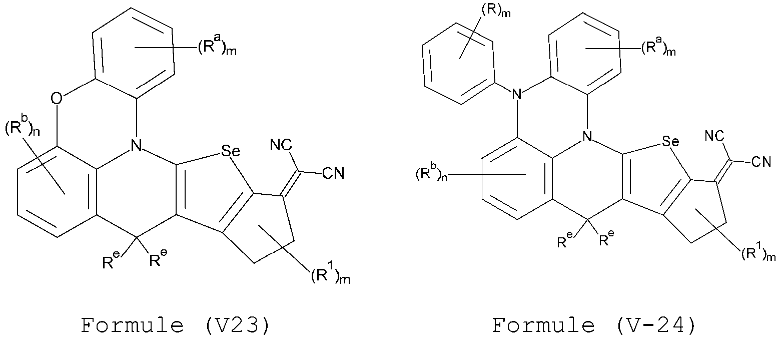 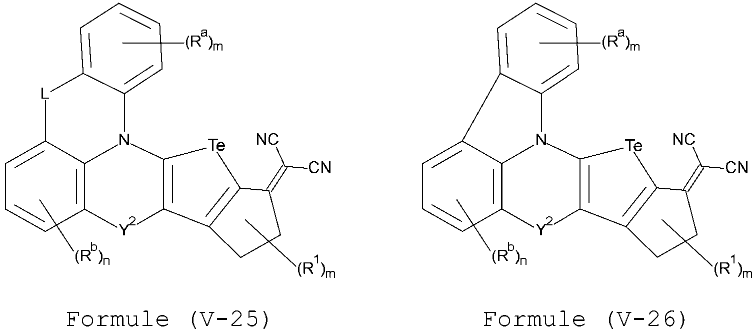 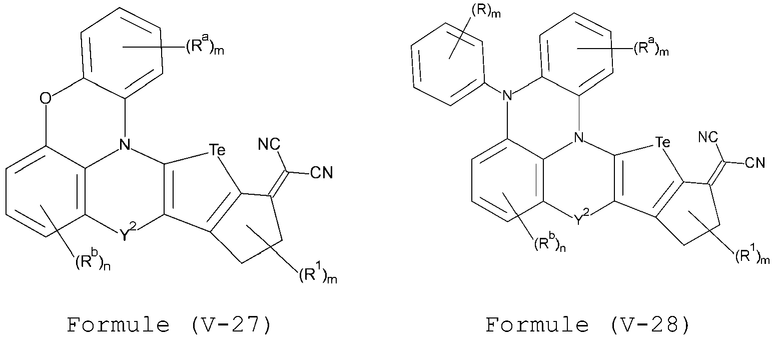 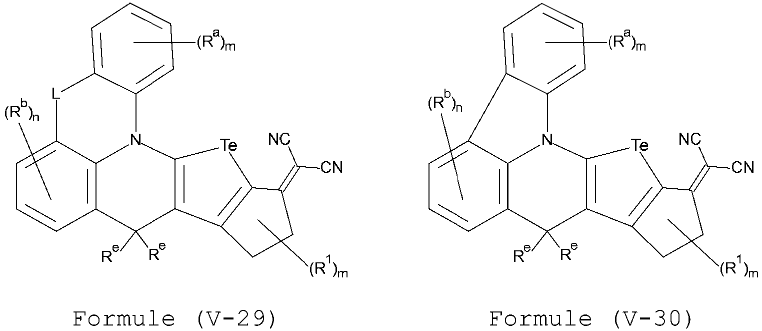 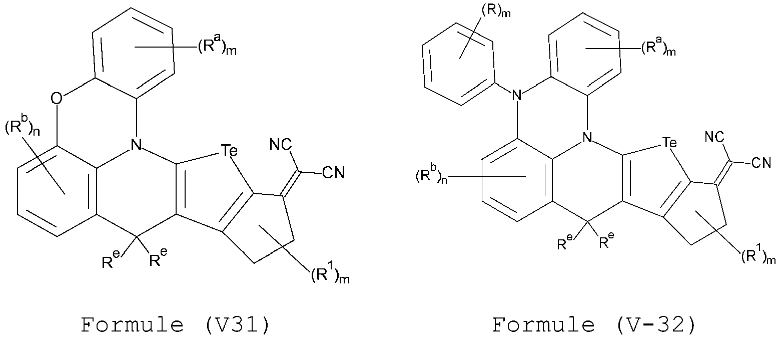 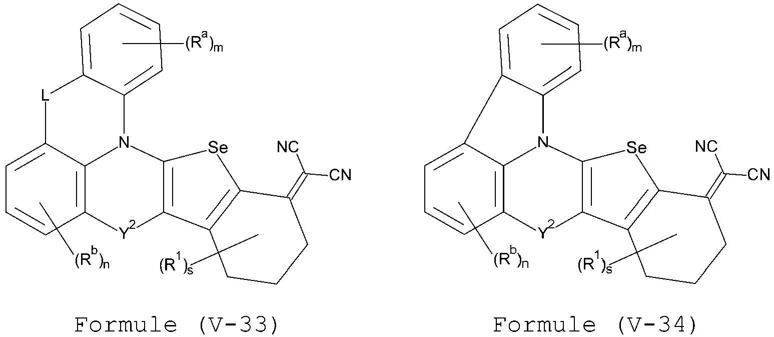 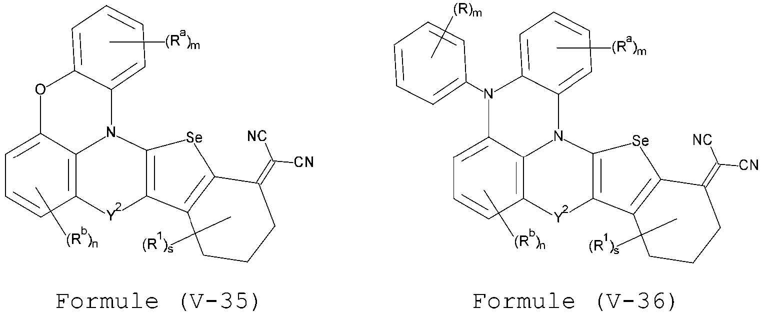 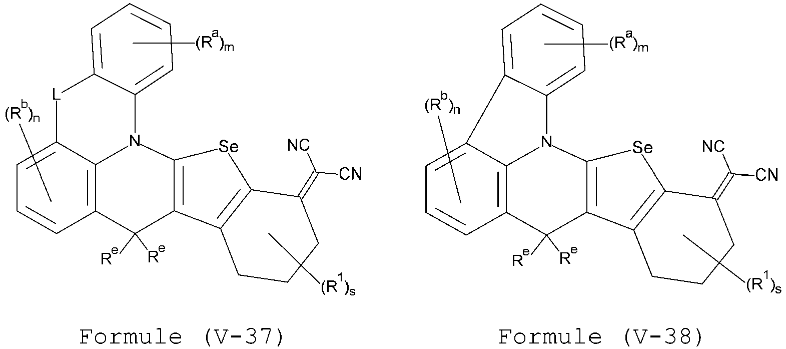 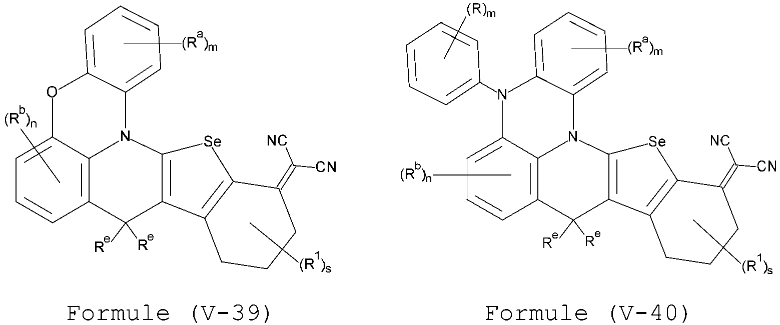 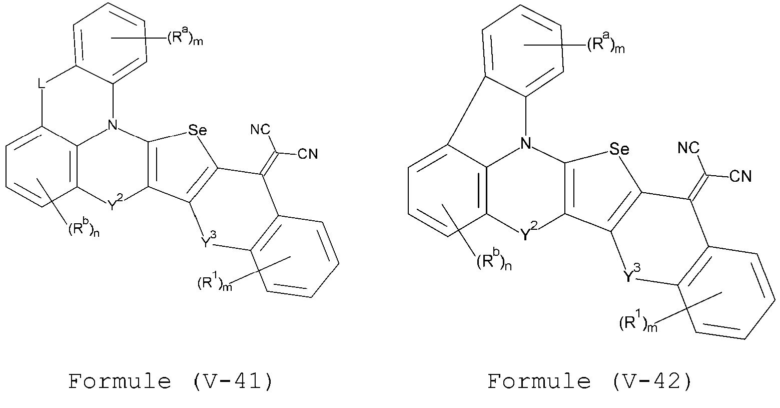 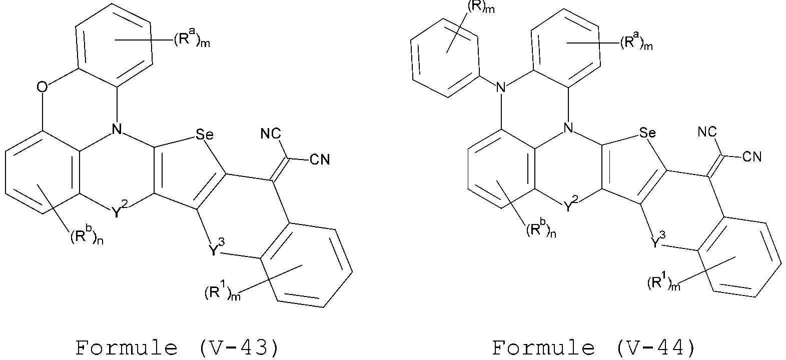 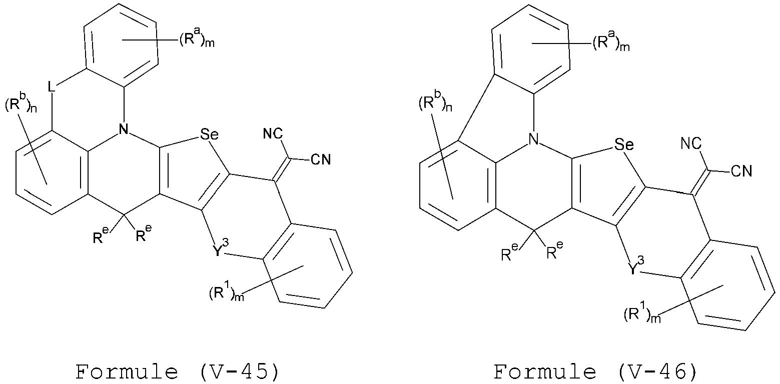 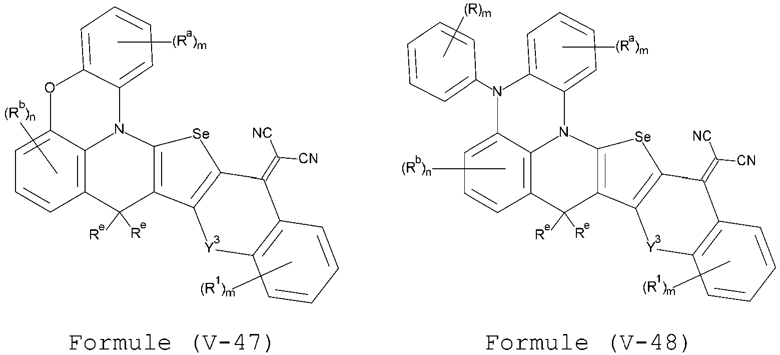 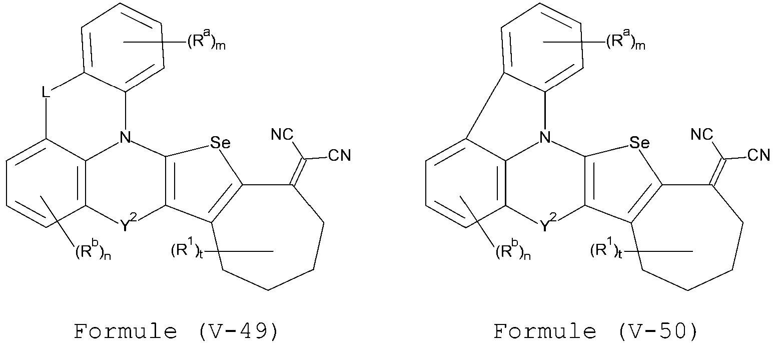 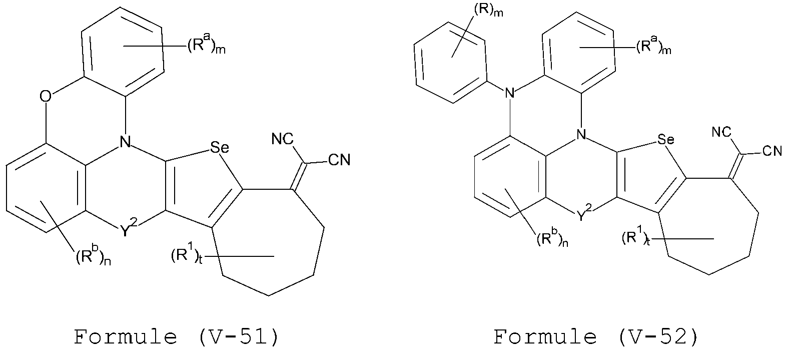 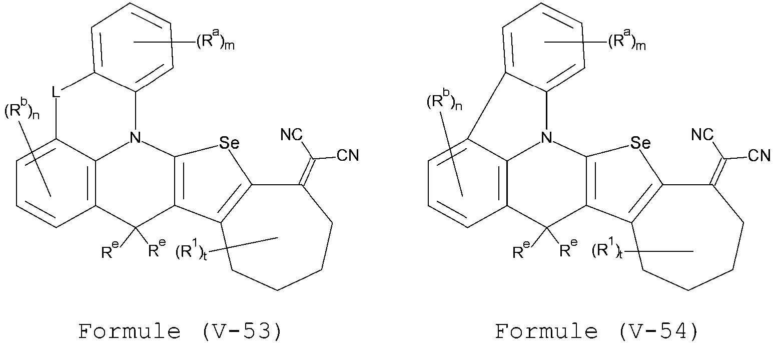 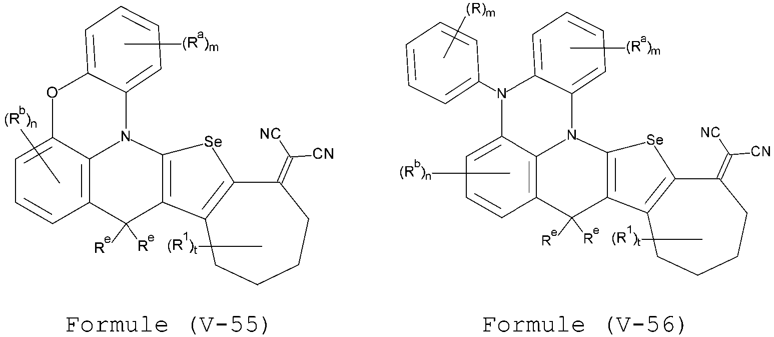 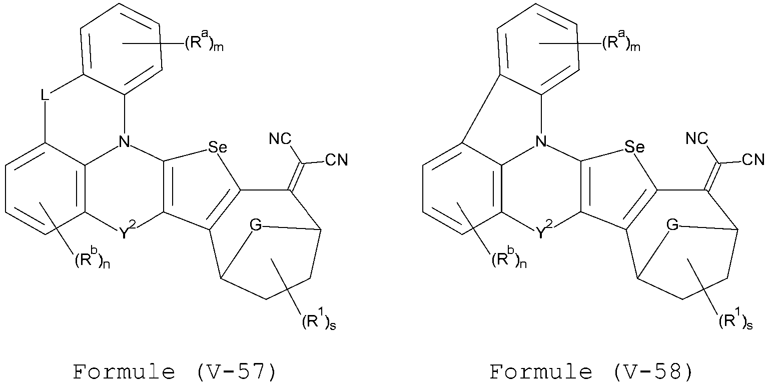 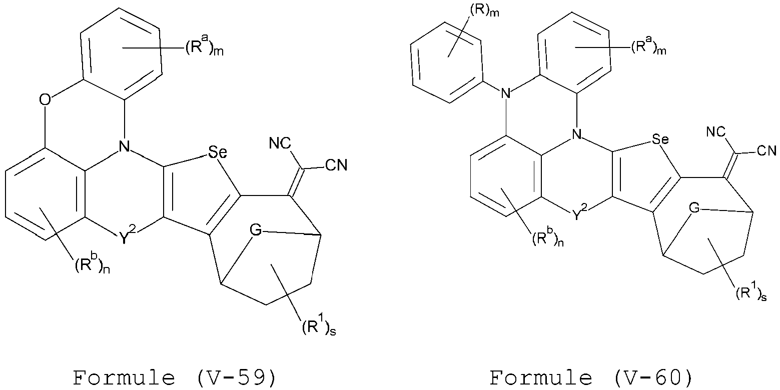 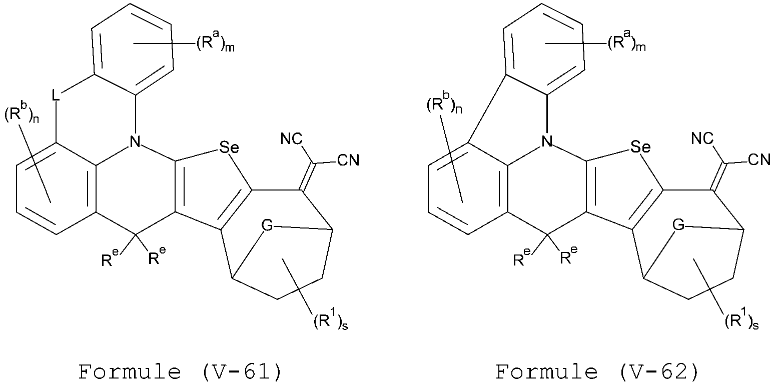 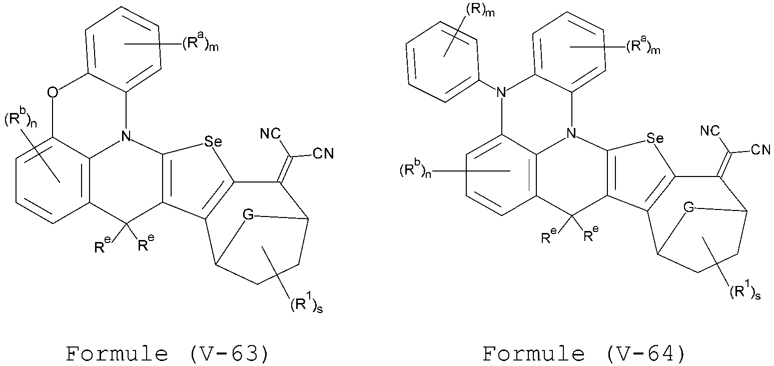 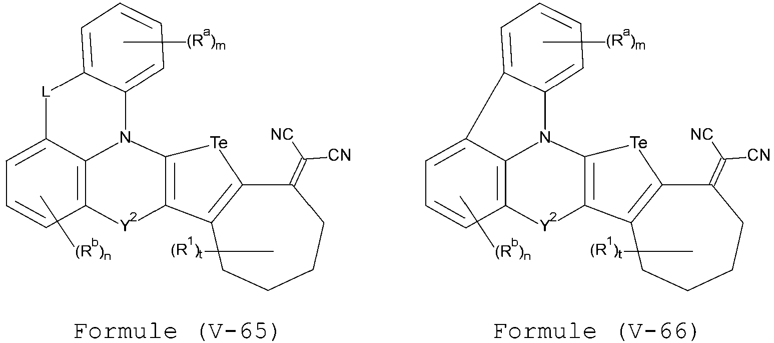 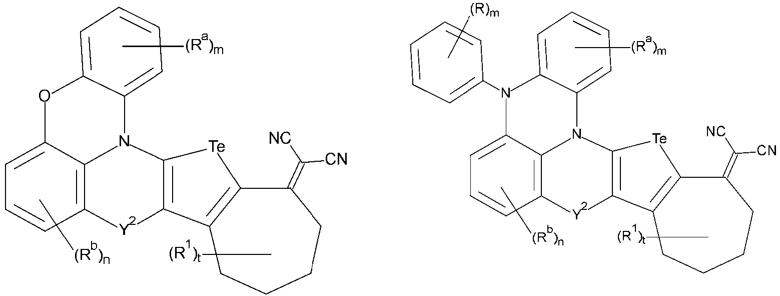 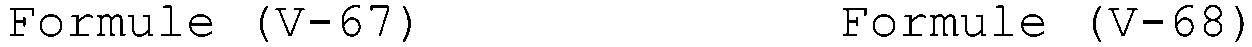 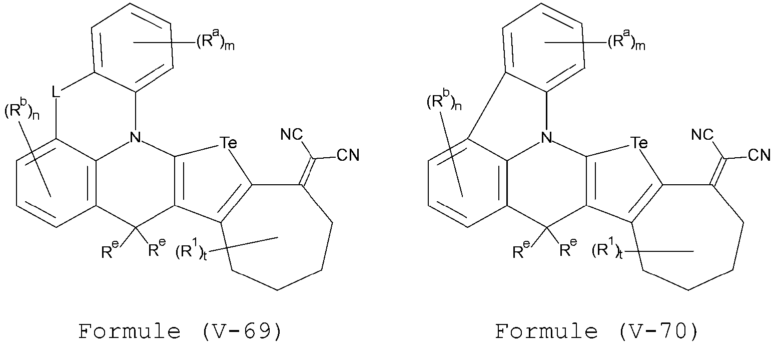 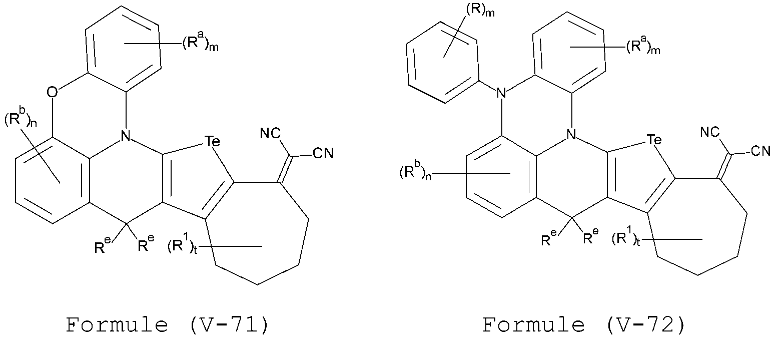 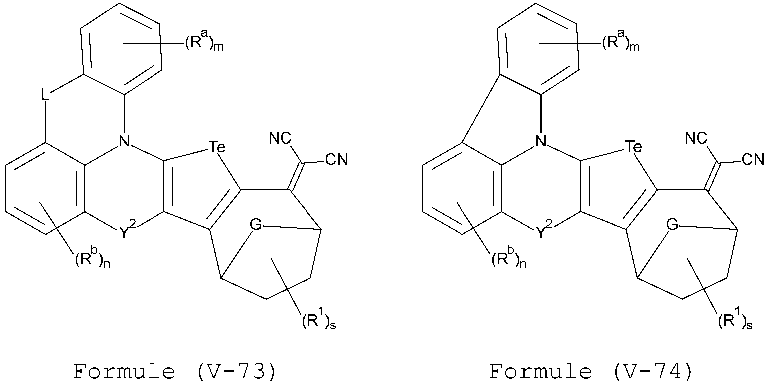 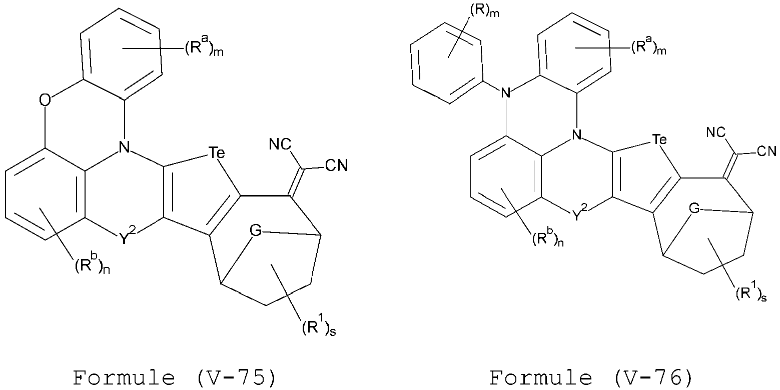 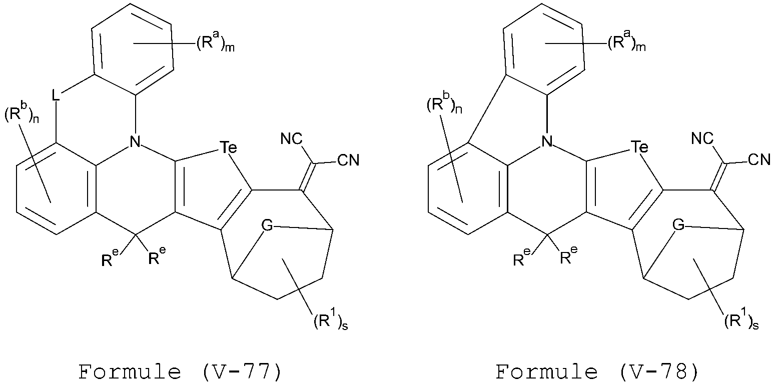 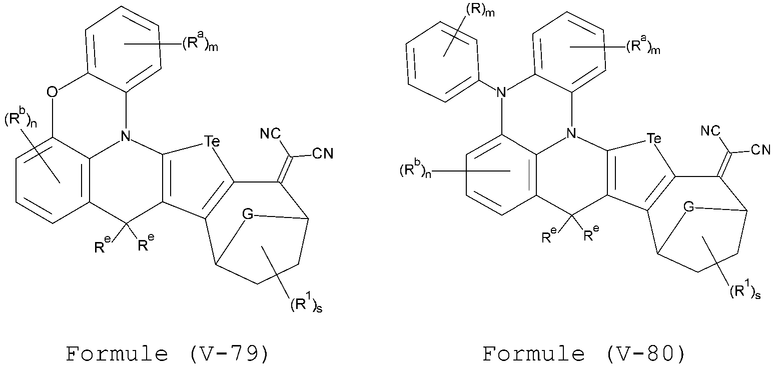 dans lesquelles les symboles R^{a}, R^{b}, R^{c}, R^{d}, R^{e}, Y² et L présentent les significations mentionnées dans la revendication 1, Z présente les significations mentionnées dans la revendication 2 et les autres symboles présentent la signification suivante :
G représente, en chaque occurrence, de manière identique ou différente, O, S, Se, Te, NAr', NR¹, BR¹, C(R¹)₂, Ge(R¹)₂, Si(R¹)₂, -C(R¹)₂-(CR¹)₂-, -CR¹=CR¹-, C=NR¹, S=O, SO₂ ou un groupe arylène ou hétéroarylène lié en position ortho, comprenant 5 à 14 atomes de cycle aromatique, qui peut être substitué par un ou plusieurs radicaux R¹, le radical R¹ présentant la signification mentionnée dans la revendication 1 ;
Y³ représente, en chaque occurrence, de manière identique ou différente, une liaison, O, S, Se, Te, NAr', NR¹, BR¹, C(R¹)₂, Ge(R¹)₂, Si(R¹)₂, CR¹=CR¹, C=NR¹, S=O, SO₂ ou un groupe arylène ou hétéroarylène, lié en position ortho, comprenant 5 à 14 atomes de cycle aromatique, qui peut être substitué par un ou plusieurs radicaux R¹, le radical R¹ présentant la signification mentionnée dans la revendication 1 ;
t représente 0, 1, 2, 3, 4, 5, 6, 7 ou 8 ;
s représente 0, 1, 2, 3, 4, 5 ou 6 ;
m représente 0, 1, 2, 3 ou 4 ;
n représente 0, 1, 2 ou 3.

10. Oligomère, polymère ou dendrimère contenant un ou plusieurs composés selon l'une des revendications 1 à 9, dans lequel il existe, au lieu d'un atome d'hydrogène ou d'un substituant, une ou plusieurs liaisons des composés au polymère, à l'oligomère ou au dendrimère.

11. Formulation contenant au moins un composé selon l'une ou plusieurs des revendications 1 à 9 ou un oligomère, un polymère ou un dendrimère selon la revendication 10 et au moins un autre composé, l'autre composé étant de préférence choisi parmi un ou plusieurs solvants et/ou parmi au moins un autre composé, choisi dans le groupe constitué par les matériaux de transport d'électrons, les matériaux d'injection d'électrons, les matériaux conducteurs de trous, les matériaux d'injection de trous, les matériaux de blocage d'électrons et les matériaux de blocage de trous, de préférence les matériaux de transport d'électrons, les matériaux d'injection d'électrons, les matériaux de blocage de trous, de manière particulièrement préférée les sous-phtalocyanines, les dérivés de sous-phtalocyanine, les fullerènes ou les dérivés de fullerène.

12. Procédé de préparation d'un composé selon l'une ou plusieurs des revendications 1 à 9, **caractérisé en ce qu'**on synthétise une structure de base présentant un groupe amino aromatique et on introduit au moins un radical hétérocyclique, de préférence au moyen d'une réaction de substitution aromatique nucléophile ou d'une réaction de couplage.

13. Utilisation d'un composé selon l'une ou plusieurs des revendications 1 à 9 ou d'un oligomère, d'un polymère ou d'un dendrimère selon la revendication 10 dans un dispositif électronique, de préférence en tant qu'agent de photosensibilisation.

14. Dispositif électronique contenant au moins un composé selon l'une ou plusieurs des revendications 1 à 9 ou un oligomère, un polymère ou un dendrimère selon la revendication 10.

15. Dispositif électronique selon la revendication 14, **caractérisé en ce que** le composé selon l'une ou plusieurs des revendications 1 à 9 ou l'oligomère, le polymère ou le dendrimère selon la revendication 10 est utilisé en tant qu'agent de photosensibilisation dans une couche d'absorption de lumière.
